# EUROPEAN PATENT SPECIFICATION

(11) **EP 3 119 913 B1**
(45) Date of publication and mention of the grant of the patent: **06.01.2021**
(21) Application number: 15765469.0
(22) Date of filing: 20.03.2015
(51) Int. Cl.: C12Q 1/68, A61K 39/395, A61K 45/06, C07K 16/42, A61K 39/00, G01N 33/574

(54) **METHODS AND COMPOSITIONS FOR TREATMENT OF IMMUNE-RELATED DISEASES OR DISORDERS AND/OR THERAPY MONITORING**
VERFAHREN UND ZUSAMMENSETZUNGEN ZUR BEHANDLUNG VON IMMUNBEDINGTEN ERKRANKUNGEN ODER STÖRUNGEN UND/ODER THERAPIEÜBERWACHUNG
PROCÉDÉS ET COMPOSITIONS POUR LE TRAITEMENT DE MALADIES IMMUNITAIRES OU DE TROUBLES ET/OU LA SURVEILLANCE THÉRAPEUTIQUE

(30) Priority: 21.03.2014 US 201461968835 P; 17.04.2014 US 201461981019 P
(43) Date of publication of application: 25.01.2017
(73) Proprietor: The Brigham and Women's Hospital, Inc., Boston, MA 02115 (US)
(72) Inventor: KUCHROO, Vijay, Newton, Massachusetts 02459 (US); JOLLER, Nicole, CH-8006 Zurich (CH); ANDERSON, Ana C., Newton, MA 02458 (US); BURKETT, Patrick, Cambridge, Massachusetts 02139 (US)
(74) Representative: Barker Brettell LLP
(86) International application number: PCT/US2015/021784
(87) International publication number: WO 2015/143343

(56) References cited:
- WO-A2-2009/126688
- WO-A2-2012/031008
- US-A1- 2005 169 913
- US-A1- 2008 003 199
- US-A1- 2010 298 418
- US-A1- 2013 251 720
- N. JOLLER ET AL: "Cutting Edge: TIGIT Has T Cell-Intrinsic Inhibitory Functions", THE JOURNAL OF IMMUNOLOGY, vol. 186, no. 3, 3 January 2011 (2011-01-03), pages 1338-1342, XP55319470, US ISSN: 0022-1767, DOI: 10.4049/jimmunol.1003081
- NICOLE JOLLER ET AL: "Good guys gone bad: exTreg cells promote autoimmune arthritis", NATURE MEDICINE, vol. 20, no. 1, 1 January 2014 (2014-01-01), pages 15-17, XP055389212, ISSN: 1078-8956, DOI: 10.1038/nm.3439
- AMANDA C. FOKS ET AL: "Agonistic Anti-TIGIT Treatment Inhibits T Cell Responses in LDLr Deficient Mice without Affecting Atherosclerotic Lesion Development", PLOS ONE, vol. 8, no. 12, 20 December 2013 (2013-12-20), page e83134, XP55215806, DOI: 10.1371/journal.pone.0083134
- J. FOURCADE ET AL: "PD-1 and Tim-3 Regulate the Expansion of Tumor Antigen-Specific CD8+ T Cells Induced by Melanoma Vaccines", CANCER RESEARCH, vol. 74, no. 4, 15 February 2014 (2014-02-15), pages 1045-1055, XP055275102, us ISSN: 0008-5472, DOI: 10.1158/0008-5472.CAN-13-2908
- KHATTAR ET AL.: 'Targeted deletion of FGL2 leads to increased early viral replication and enhanced adaptive immunity in a murine model of acute viral hepatitis caused by LCMV WE' PLOS ONE vol. 8, 11 October 2013, pages 1 - 11, XP055358672
- JOLLER ET AL.: 'Immune checkpoints in central nervous system autoimmunity' IMMUNOL REV. vol. 248, 01 July 2012, pages 122 - 139, XP055145273
- JOLLER ET AL.: 'Cutting edge: TIGIT has T cell -intrinsic inhibitory functions' J IMMUNOL. vol. 186, 01 February 2011, pages 1338 - 1342, XP055319470
- FOERSTER ET AL.: 'The novel immunoregulatory molecule FGL2: a potential biomarker for severity of chronic hepatitis C virus infection' J HEPATOL. vol. 53, 17 June 2010, pages 608 - 615, XP027241346
- YU ET AL.: 'THE SURFACE PROTEIN TIGIT SUPPRESSES T CELL ACTIVATION BY PROMOTING THE GENERATION OF MATURE IMMUNOREGULATORY DENDRITIC CELLS' NAT IMMUNOL. vol. 10, 16 November 2008, pages 48 - 57, XP055273979

## Description

### CROSS-REFERENCE TO RELATED APPLICATIONS

This Application claims benefit under 35 U.S. C. § 119(e) of the U.S. Provisional Application No. 61/968,835 filed March 21, 2014, and the U.S. Provisional Application No. 61/981,019 filed April 17, 2014.

### GOVERNMENT SUPPORT

This invention was made with government support under Grant No. P01AI039671 awarded by the National Institutes of Health.

### FIELD OF THE INVENTION

The present invention relates to molecular immunology and cell biology. More specifically, various aspects of the present embodiments provide for methods and compositions for treatment of immune-related diseases or disorders and/or therapy monitoring. In some embodiments, the methods and compositions described herein are directed to treatment and/or therapy monitoring of cancer. In some embodiments, the methods and compositions described herein are directed to treatment and/or therapy monitoring of inflammatory diseases such as infections, allergy, asthma, autoimmune diseases and/or inflammation. Methods for identifying patients who are more likely to be responsive to and benefit from an immunotherapy are also described herein.

### BACKGROUND

The immune system protects the body from foreign invaders and diseased cells; but immune disorders, particularly those associated with T-cell tolerance, such as cancers, can wreak havoc. According to the most recent data from the World Health Organization, ten million people around the world were diagnosed with cancer in 2000, and six million died from it. Moreover, statistics indicate that the cancer incidence rate is on the rise around the globe. In America, for example, projections suggest that fifty percent of those alive today will be diagnosed with some form of cancer at some point in their lives.

T-cell tolerance is also implicated in immune suppression that can be desirable, for example, in autoimmune diseases and in organ transplant situations, wherein an overactive immune response can cause great permanent damage to the afflicted individual and or donor organ. More specifically, autoimmune disorders are caused by dysfunctional immune responses directed against the body's own tissues, resulting in chronic, multisystem impairments that differ in clinical manifestations, course, and outcome. Autoimmune diseases are on the rise in the U.S. and around the world. In the U.S. alone, some fifty million are affected, and autoimmune disease is one of the top ten causes of death in women under the age of 65, is the second highest cause of chronic illness, and the top cause of morbidity in women.

Hence, there remains an urgent need for compositions and approaches to treating immune-related disorders or T-cell tolerance mediated immune disorders.

### SUMMARY

In a first aspect of the invention, there is provided an agent that inhibits TIGIT (T cell Ig and ITIM domain) activity for use in treating a patient diagnosed with cancer and/or infection that has an elevated level of Fgl2, wherein the agent that inhibits TIGIT activity comprises an antibody or an antigen-binding fragment thereof that specifically binds TIGIT.

In another aspect of the invention, there is provided an *in vitro* method of determining the efficacy of an agent that inhibits TIGIT activity in the treatment of a patient diagnosed with cancer and/or infection that has an elevated level of Fgl2, the method comprising: (a) determining a first level of Fgl2 expression or activity in a sample provided by the patient diagnosed with cancer and/or infection that has an elevated level of Fgl2 prior to the administration of the agent that inhibits TIGIT activity; (b) determining a second level of Fgl2 expression or activity in a sample provided by the patient after administration of the agent that inhibits TIGIT activity ; and (c) comparing said first and second levels of Fgl2 expression or activity, wherein the agent is considered effective if said second level of Fgl2 expression or activity is lower than said first level, and wherein the agent administered in (b) is ineffective if said second level of Fgl2 expression is the same as or higher than said first level; and (d) if said anti-TIGIT therapy is considered ineffective, administration of said agent that inhibits TIGIT activity at a higher dose or administration of a therapy comprising an activator of a proinflammatory T cell response pathway and/or a suppressor of an anti-inflammatory T cell response pathway is advised, wherein the agent that inhibits TIGIT activity comprises an antibody or an antigen-binding fragment thereof that specifically binds TIGIT.

In another aspect of the invention, there is provided a composition for use in treating a patient diagnosed with cancer that has an elevated level of Fgl2, wherein the composition comprises a TIGIT inhibitor comprising an anti-TIGIT antibody or an antigen-binding fragment thereof and a therapeutic agent selected from the group consisting of a TIM-3 inhibitor, an anti-galectin-9 molecule, a PD-1 antagonist, a PD-L1 antagonist, a CTLA-4 antagonist, a Lag-3 antagonist, a CD155 inhibitor or any combination thereof.

In another aspect of the invention, there is provided a composition for use in treating a patient diagnosed with cancer that has an elevated level of Fgl2 and who has received immunotherapy for the cancer, the composition comprising a TIGIT antagonist, wherein the TIGIT antagonist comprises an antibody or an antigen-binding fragment thereof that specifically binds TIGIT.

### BRIEF DESCRIPTION OF THE DRAWINGS

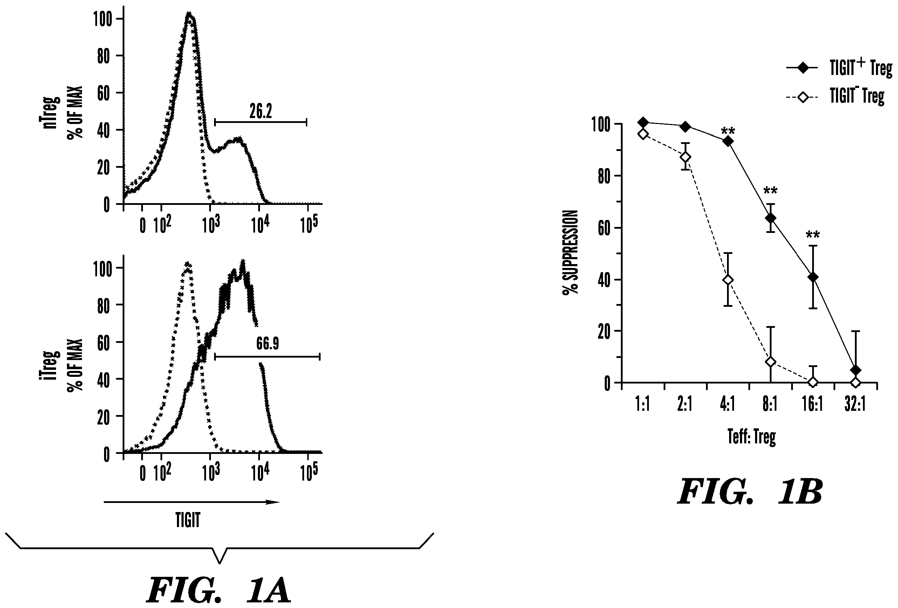

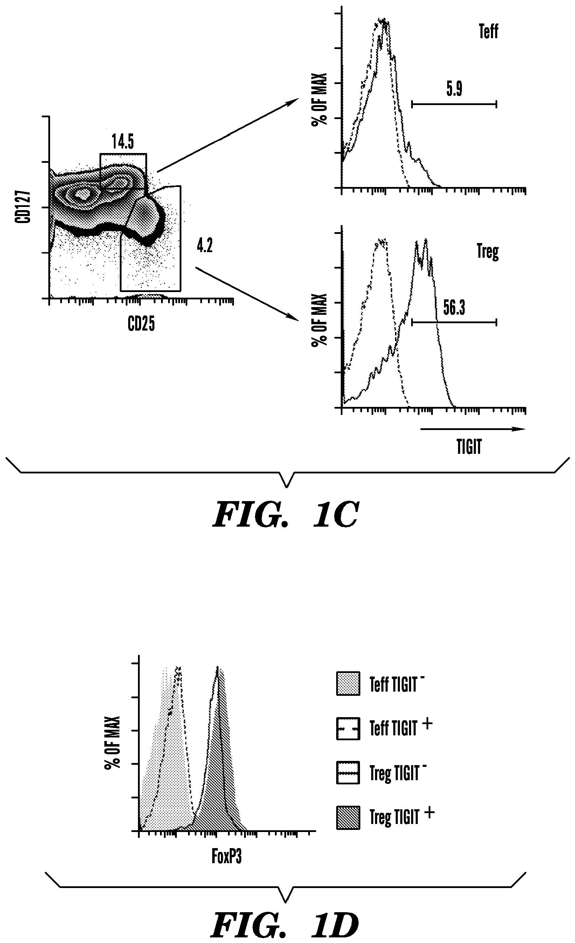

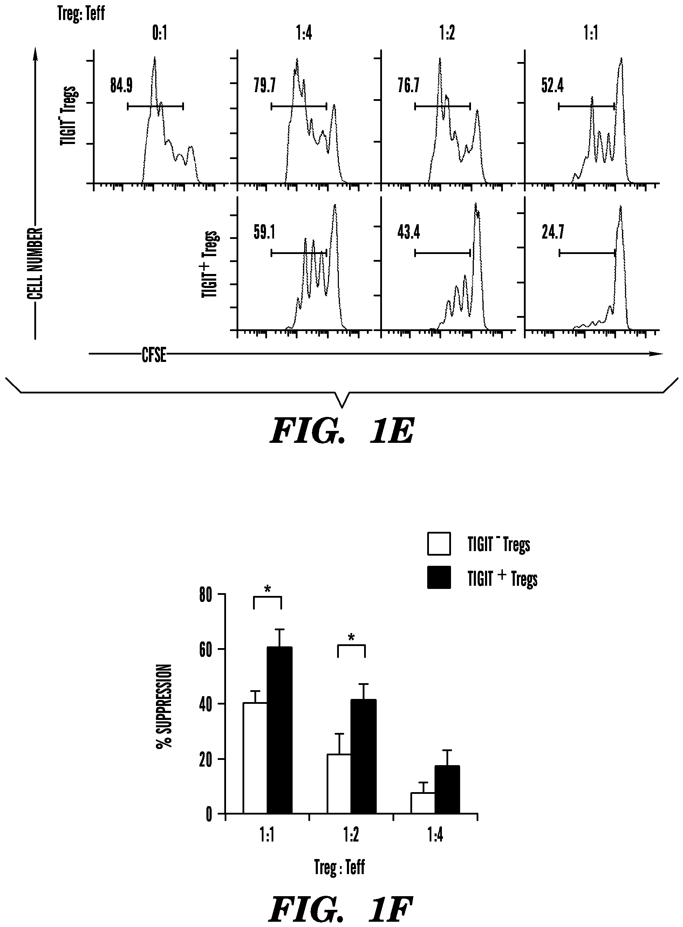

**Figures 1A-1F** are experimental data showing that TIGIT is expressed on highly suppressive Tregs and promotes Treg conversion. (**Figure 1A**) CD4+ T cells were purified from Foxp3-GFP.KI mice and the Foxp3+ and Foxp3- cells were sorted. Foxp3+ nTregs were stained directly for TIGIT (solid line) or with an isotype control (dotted line) and analyzed by flow cytometry. Foxp3+ induced Tregs (iTregs) were analyzed after 4 days of stimulation with TGF-β. (**Figure 1B**) CD4+Foxp3+TIGIT+ (υ) or CD4+Foxp3+TIGIT- (◊) Tregs were sorted from Foxp3-GFP.KI mice and titrated onto Foxp3-GFP- effector T cells stimulated with anti-CD3 and APCs. Proliferation was measured after 72 h by ³H-thymidine incorporation. (Mean ± s.d.; * P<0.01; representative experiment of >10 independent experiments). (**Figure 1C**) Sorting strategy of ex vivo FACS sorted human effector T cells (CD4+CD25+CD127+) and Tregs (CD4+CD25^{high}CD127-) sorted into TIGIT+ and TIGIT-. (**Figure 1D**) Tregs sorted as outlined in Figure 1C showed >96% purity in both subsets measured by Foxp3 staining after isolation. (**Figure 1E**) Representative suppression assay with human CD4+CD25^{high}CD127-TIGIT+ and TIGIT- Tregs co-cultured with CFSE-labeled CD25-depleted CD4+ effector T cells for 4 days. (**Figure 1F**) Statistical summary of Figure 1E of six healthy donors (mean ± SEM; * P<0.05).

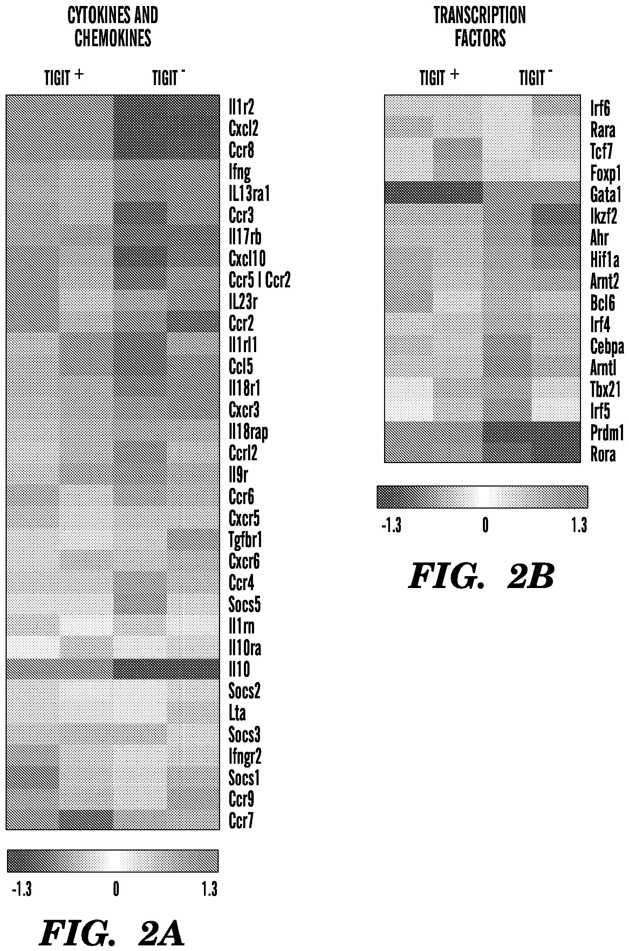

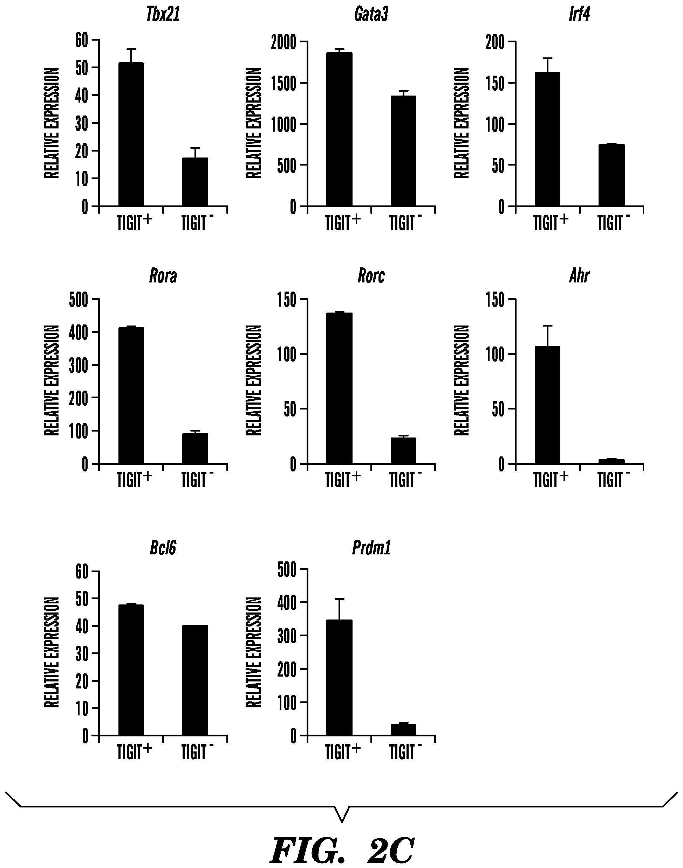

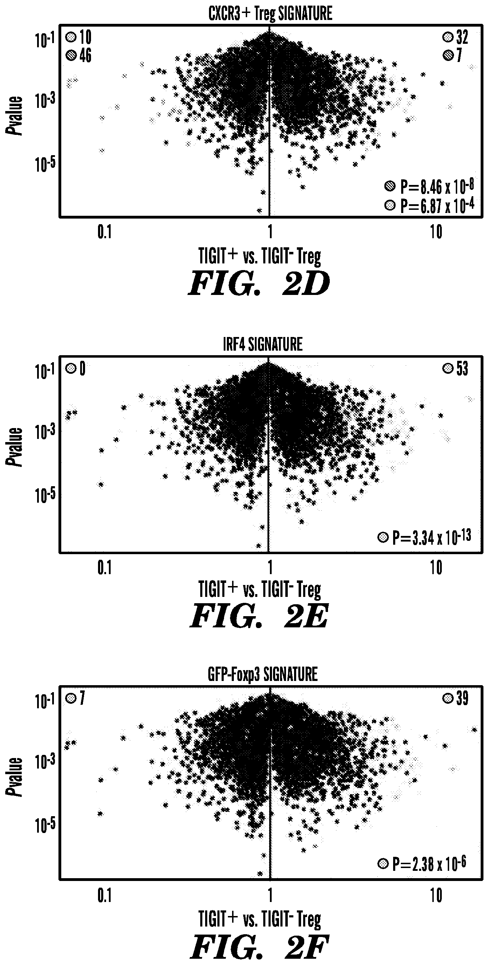

**Figures 2A-2F** are experimental data showing expression profiling of TIGIT+ regulatory T cells. Heat map of chemokine (receptor) and cytokine (receptor) (**Figure 2A**) or transcription factor (**Figure 2B**) genes that are differentially expressed (>1.5-fold) in CD4+Foxp3+TIGIT+ and CD4+Foxp3+TIGIT- Tregs (duplicate samples are shown). (**Figure 2C**) Differential expression of a selection of genes from Figure 2B was determined by quantification of mRNA levels in CD4+Foxp3+TIGIT+ and CD4+Foxp3+TIGIT- Tregs by RT-PCR. Mean ± s.d. of at least 3 independent experiments is shown. (**Figures 2D-2F**) Volcano plots comparing the P value versus fold-change for probes from TIGIT+ versus TIGIT- Treg cells. Treg signatures generated from (**Figure 2D**) CXCR3+ versus CXCR3- Tregs, (**Figure 2E**) WT versus IRF4 KO Tregs and (**Figure 2F**) Tregs from GFP-Foxp3 fusion protein reporter mice versus Foxp3-IRES-GFP mice are highlighted as overexpressed or underrepresented. P values form a chi-squared test. P values from a chi-squared test. Genes and Probe IDs included in the signatures are listed in Table 2 in the Examples.

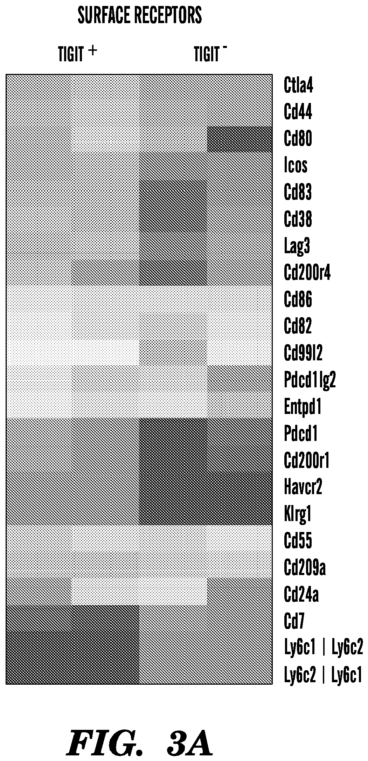

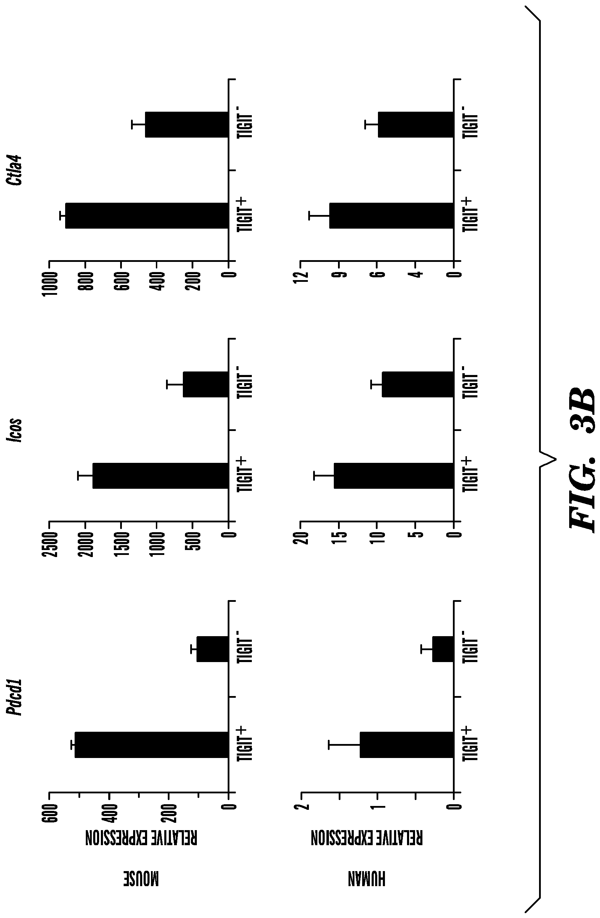

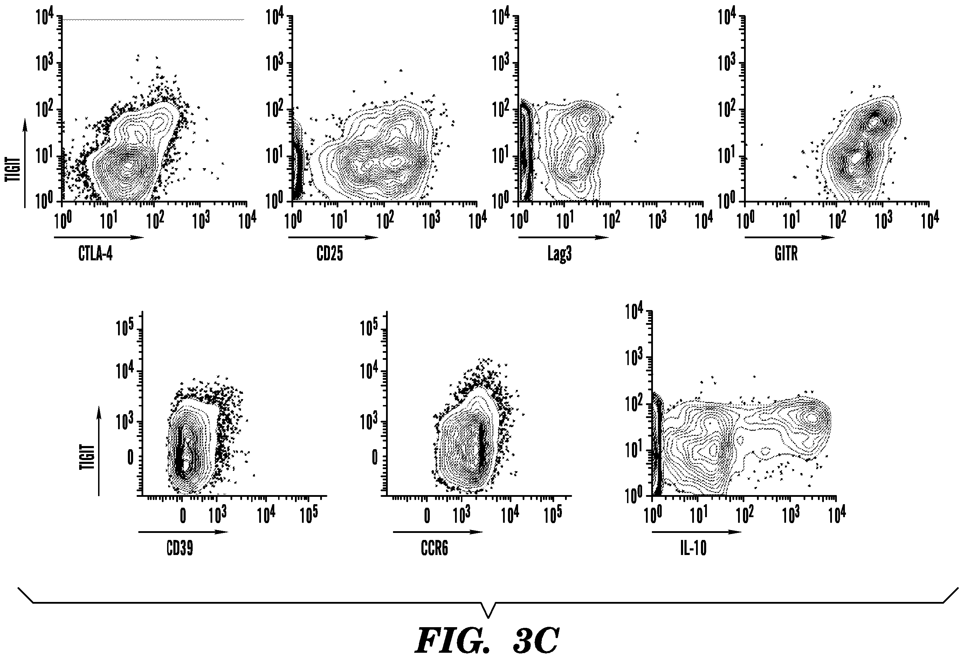

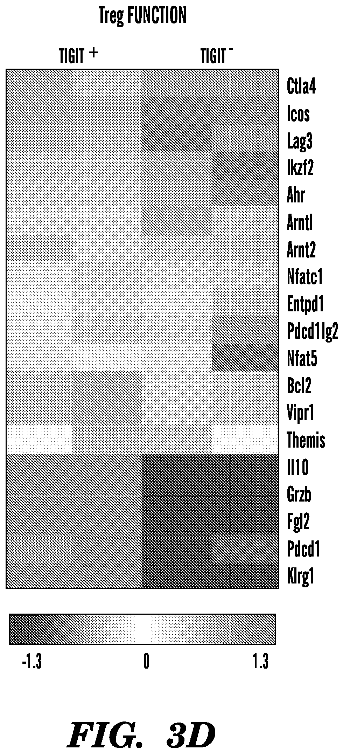

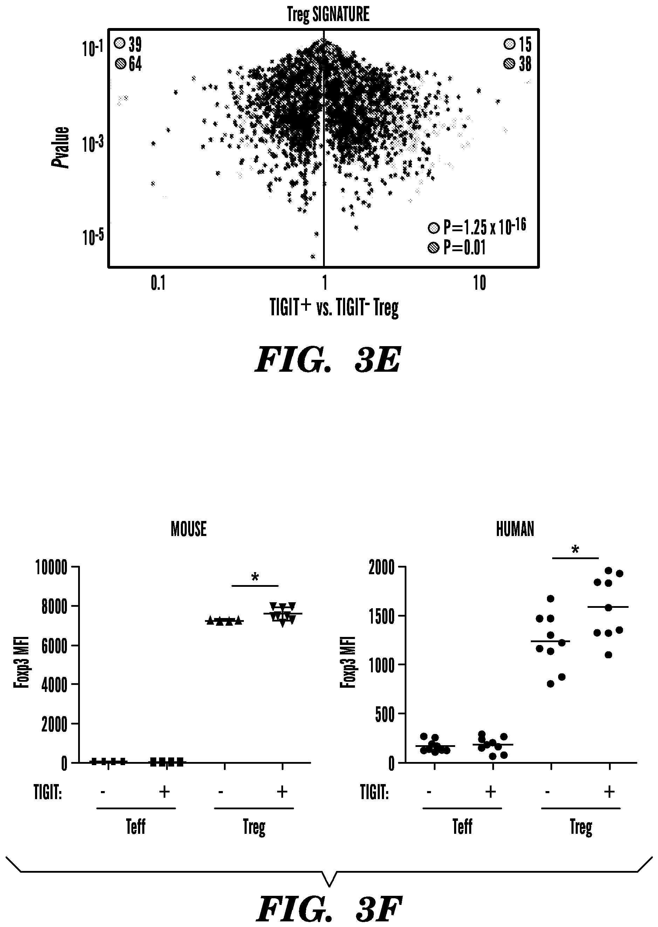

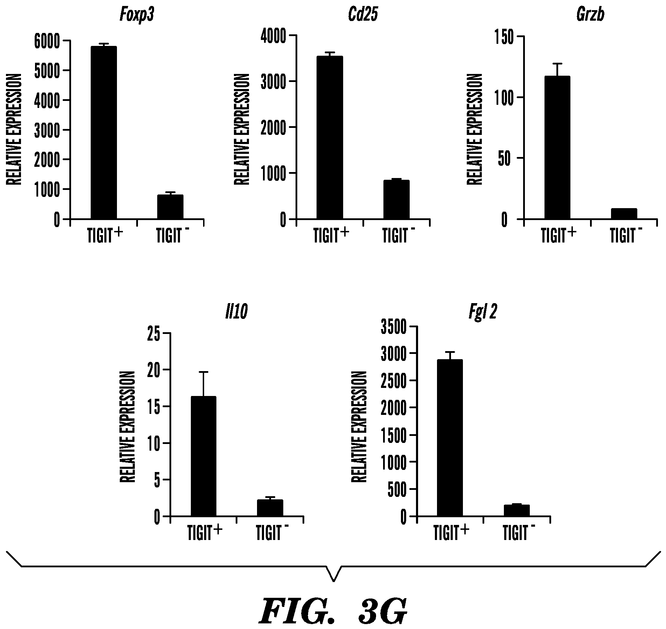

**Figures 3A-3G** are experimental data showing that TIGIT+ Tregs are better equipped for suppression. (**Figure 3A**) Heat map of surface receptor genes that are differentially expressed (>1.5-fold, duplicate samples) in CD4+Foxp3+TIGIT+ and CD4+Foxp3+TIGIT- Tregs. Quantitative RT-PCR (**Figure 3B**) and flow cytometric (**Figure 3C**) confirmation for a selection of genes from Figure 3A and Figure 3D. (**Figure 3D**) Heat map of differentially expressed genes involved in Treg differentiation and function. (**Figure 3E**) Volcano plot comparing the P value versus fold-change for probes from TIGIT+ versus TIGIT- Treg cells. The canonical Treg signature is highlighted in red (transcripts upregulated in Treg cells) and green (transcripts downregulated in Treg cells). (**Figure 3F**) Foxp3 protein expression was quantified by flow cytometry in mouse Teff (Foxp3-) or Tregs (Foxp3+) and human memory T cells (CD4⁺CD127⁺CD25^{med}) and Tregs (CD4⁺CD127^{low}CD25^{high}) (n=9; *p<0.05). (**Figure 3G**) Relative expression of the indicated genes in CD4+Foxp3+TIGIT+ and CD4+Foxp3+TIGIT- Tregs was determined by quantitative PCR.

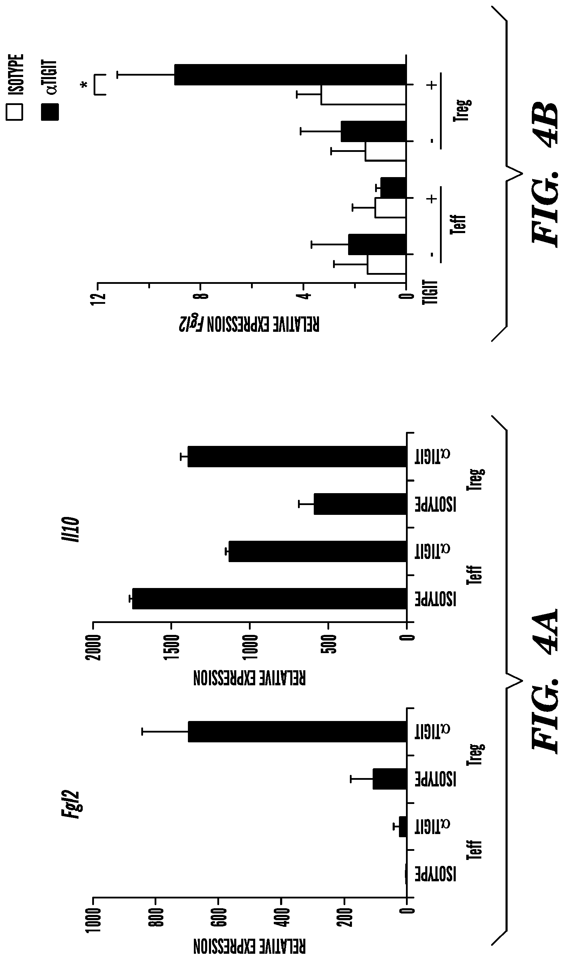

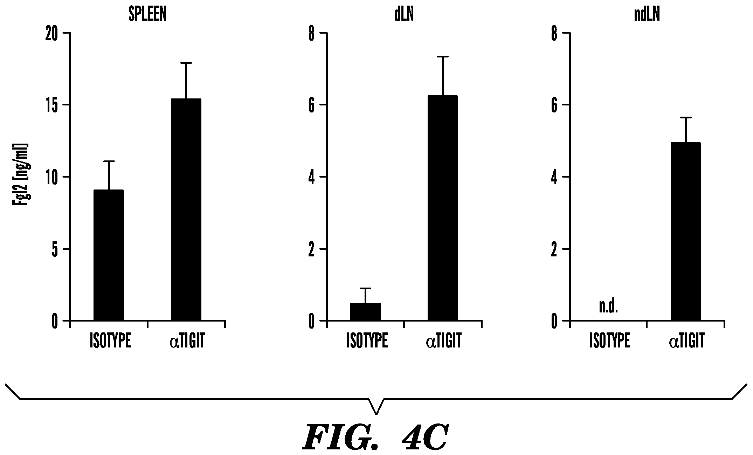

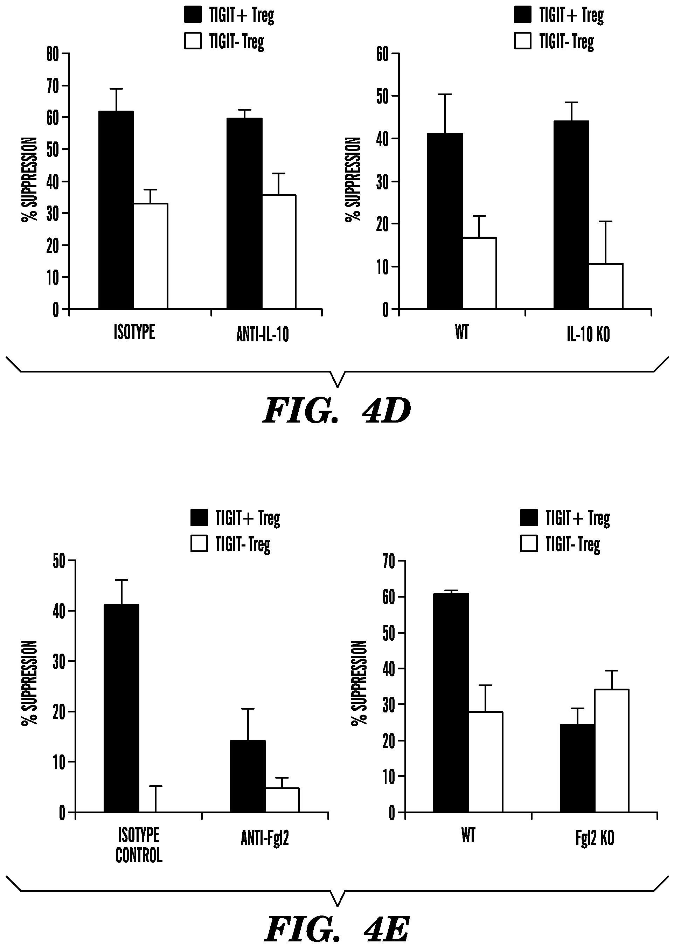

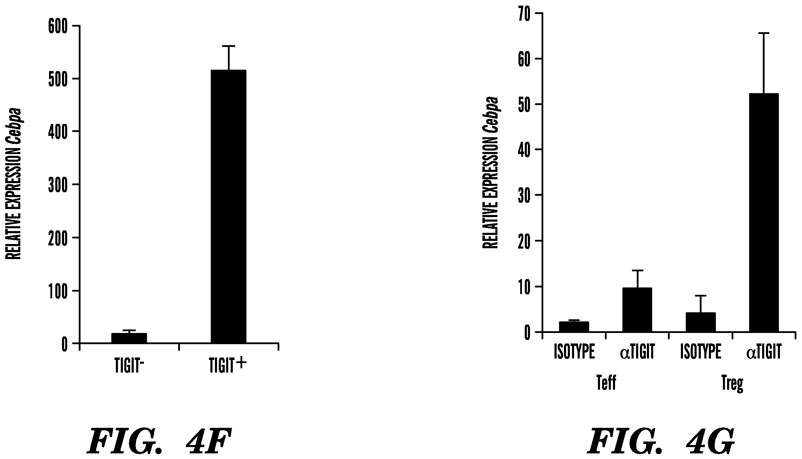

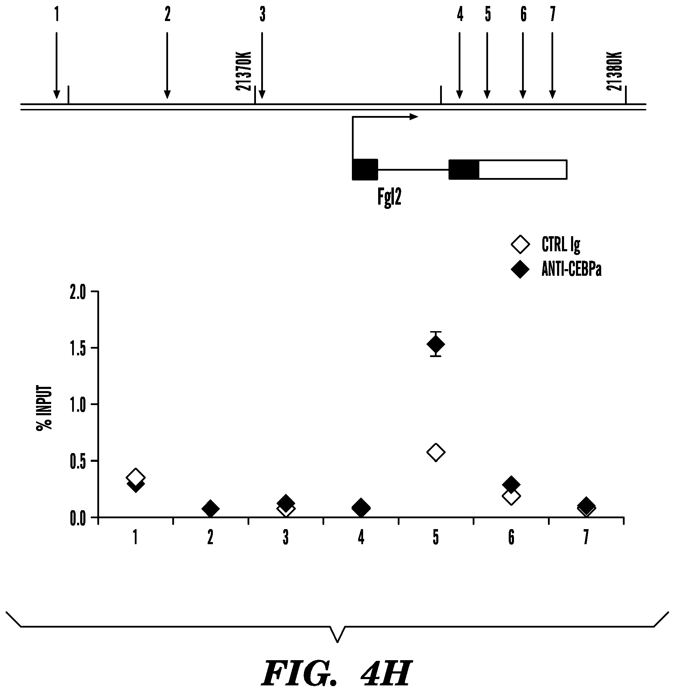

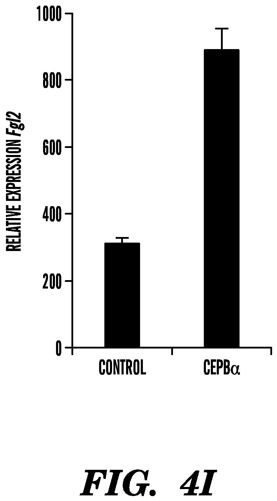

**Figures 4A-4I** are experimental data showing that TIGIT ligation induces Fgl2 expression. (**Figure 4A**) Foxp3- (Teff) and Foxp3+ (Treg) cells were sorted from Foxp3-GFP.KI mice, stimulated with anti-CD3/anti-CD28 in the presence of agonistic anti-TIGIT Ab. After 3 days RNA was extracted and Fgl2 and 1110 mRNA levels were determined by quantitative RT-PCR. (**Figure 4B**) Ex vivo human memory T cells (CD4+CD127+CD25^{med}) and Tregs (CD4+CD127^{low}CD25^{high}) were sorted gating into TIGIT+ and TIGIT-. After isolation, cells were cultured in the presence of agonistic anti-TIGIT or isotype control for 4 days. Fgl2 expression was quantified by RT-PCR (n=6; * P<0.05). (**Figure 4C**) Mice were immunized s.c. with MOG₃₅₋₅₅ peptide in CFA and treated with anti-TIGIT or isotype control antibody. On day 10 cells were re-stimulated with MOG₃₅₋₅₅ peptide for 48h. Fgl2 concentrations in the supernatants were determined by ELISA. (**Figures 4D, 4E**) CD4+CD25+TIGIT+ (closed bars) or CD4+CD25+TIGIT- (open bars) Tregs were sorted from WT, IL-10 KO (**Figure 4D**) or Fgl2 KO (**Figure 4E**) mice and co-cultured with CD25- effector T cells stimulated with anti-CD3 and APCs at a ratio of 1:8. Where indicated neutralizing anti-IL10 (**Figure 4D**) or anti-Fgl2 (**Figure 4E**) Ab or the respective isotype control Ab was added to the culture. Proliferation was measured after 72 h by 3H-thymidine incorporation. **(****Figure 4F****)** CD4+Foxp3+TIGIT+ and CD4+Foxp3+TIGIT- Tregs were sorted from Foxp3-GFP.KI mice and mRNA levels for Cebpa were determined by RT-PCR. (**Figure 4G**) Cells were isolated and stimulated as in Figure 4A and on day 3 Cebpa mRNA levels were determined by quantitative RT-PCR. (**Figure 4H**) ChIP assays were performed on P815 cells expressing TIGIT using anti-CEBPα antibody or rabbit IgG isotype control. The precipitated chromatin was analyzed by quantitative PCR with primers specific for 3 promoter and 4 intragenic regions of the Fgl2 gene with predicted CEBPα binding sites. Signals are displayed as % of the total input chromatin. (**Figure 4I**) CD4+Foxp3+ Treg cells were sorted from Foxp3-GFP.KI mice and transfected with a CEBPα over-expression construct (CEBPα) or the empty vector as control (control) and stimulated with anti-CD3/CD28 Dynabeads. Relative expression of Fgl2 mRNA was determined by RT-PCR 4 days later. (all panels represent mean ± s.d)

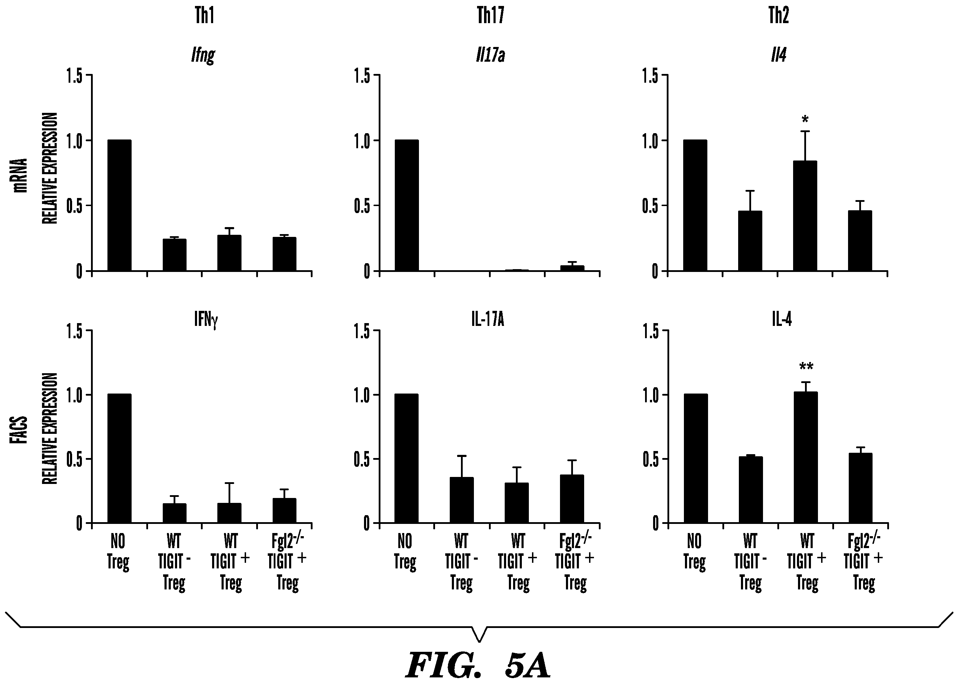

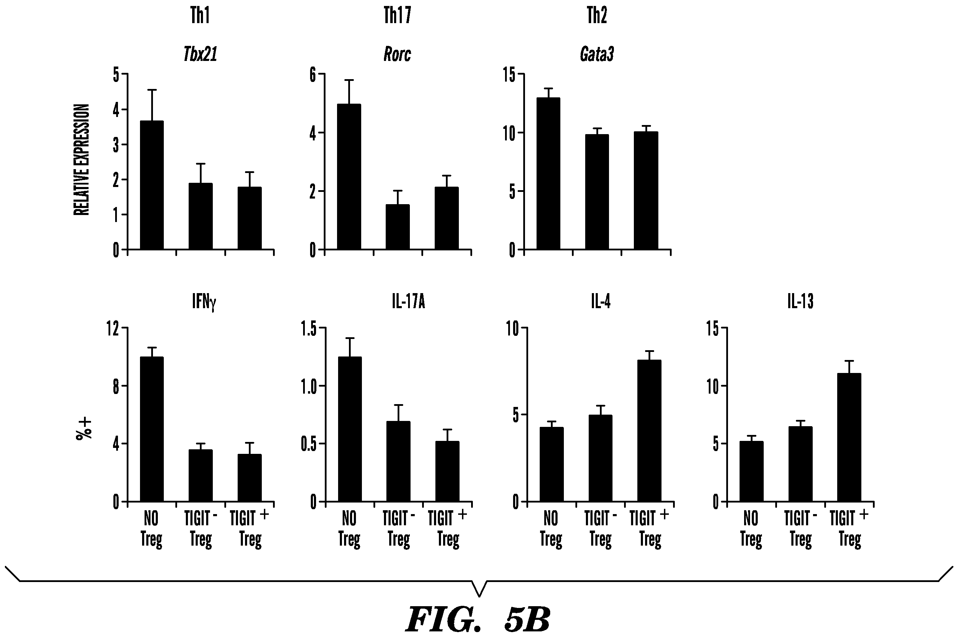

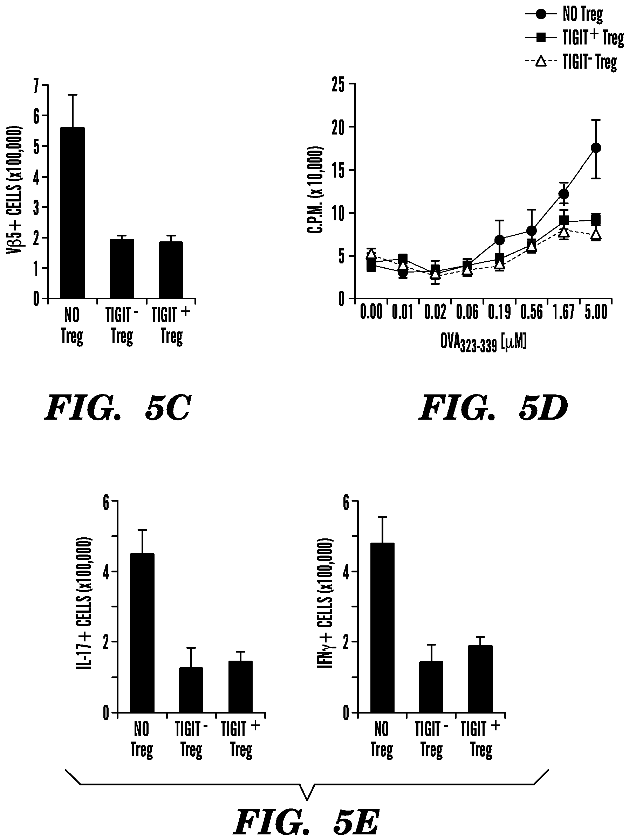

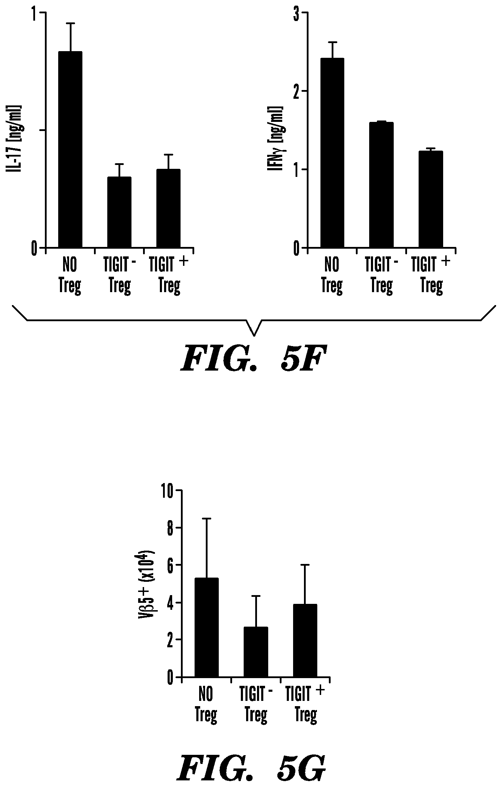

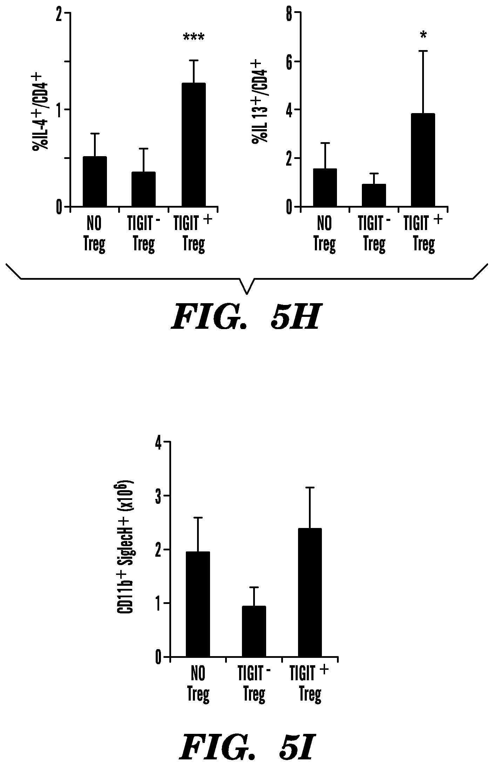

**Figures 5A-5I** are experimental data showing that TIGIT+ regulatory T cells suppress Th1 and/or Th17 but not a Th2 response. (**Figure 5A**) Naive effector T cells, WT Foxp3+TIGIT-, WT Foxp3+TIGIT+ Tregs, and Fgl2-/- Foxp3+TIGIT+ Tregs were sorted and co-cultured at a ratio of 1:10 under Th1, Th2, or Th17 polarizing conditions. After 3 days mRNA levels were determined by quantitative RT-PCR. On day 5 intracellular cytokines in CD45.1+ T effector cells were determined by flow cytometry (values normalized to unsuppressed controls, mean ± SEM; * P<0.05, ** P<0.001, paired student's t-test). (**Figure 5B**) Human TIGIT+ and TIGIT- Tregs (CD4+CD25^{high}CD127^{neg}) were sorted and co-cultured with CFSE-labeled CD25-depleted CD4+ T effector cells. Gene expression (qRT-PCR) and intracellular cytokine levels (flow cytometry) were determined on day 4 (mean ± SEM; n=6). (**Figures 5C-5F**) CD25- effector OT-II cells and CD25high OT-II Tregs (TIGIT-, TIGIT+ or no Treg control) were transferred i.v. into WT recipients and mice were immunized with OVA in CFA. (**Figure 5C**) Expansion of Vβ5⁺ OT-II T cells and (**Figure 5D**) proliferation in response to OVA₃₂₃₋₃₃₉ were determined 10 days later. (**Figure 5E**) Intracellular cytokine levels were determined by flow cytometry and (**Figure 5F**) cytokine concentration in the culture supernatants was determined by cytometric bead array. (**Figures 5G-5I**) CD25- effector OT-II cells and CD25high OT-II Tregs (TIGIT-, TIGIT+ or no Treg control) were transferred i.v. into WT recipients. Mice were then sensitized with OVA (i.p.) on days 0 and 7 and challenged with aerosolized OVA on days 14-17 to induce allergic airway inflammation. (**Figure 5G**) Total numbers of Vβ5⁺ OT-II cells in lungs, (**Figure 5H**) intracellular cytokine levels from lung-infiltrating CD4+ T cells, and (**Figure 5I**) total eosinophil numbers in bronchio-alveolar lavage fluid were determined by flow cytometry. Pooled data from two experiments are shown (mean ± SEM; n=8).

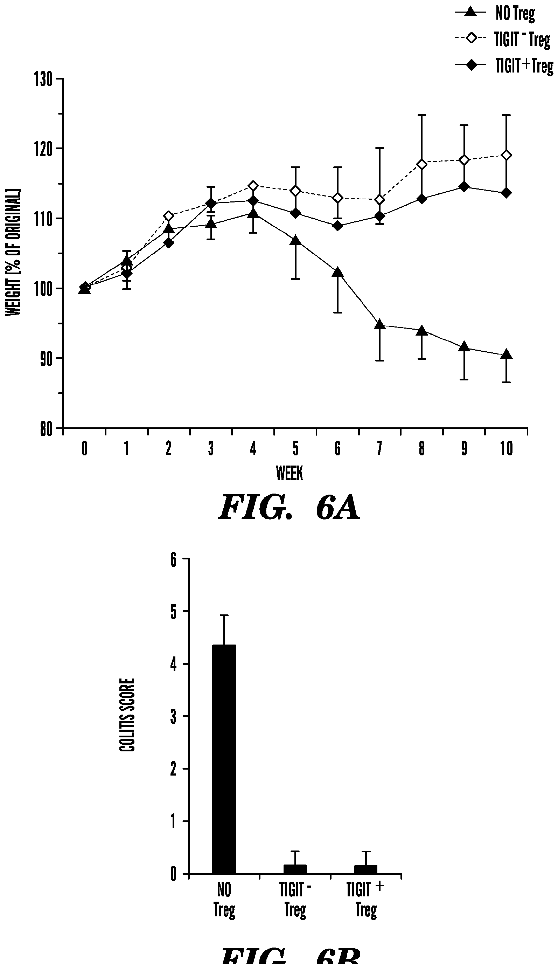

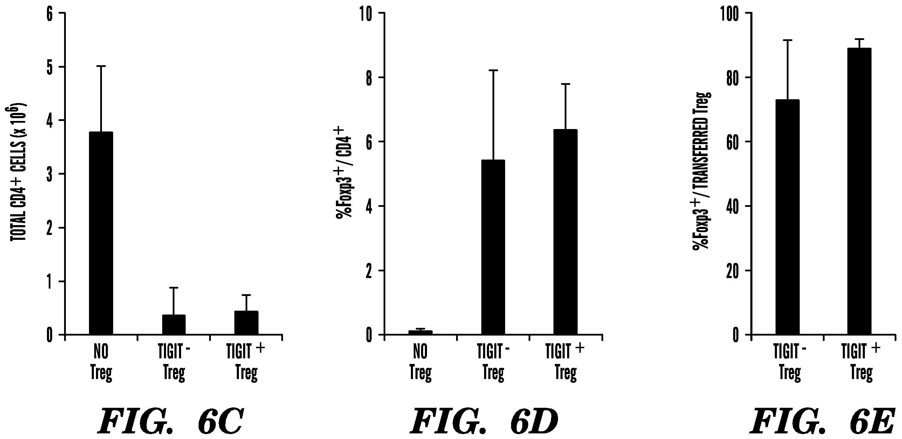

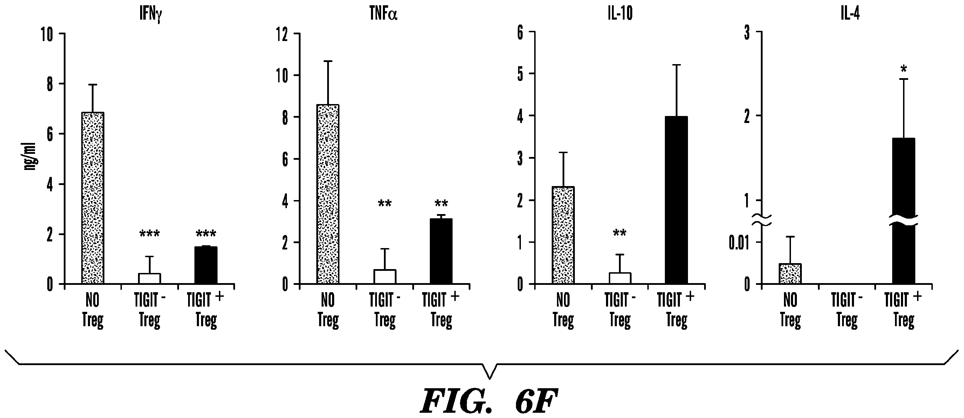

**Figures 6A-6F** are experimental data showing that TIGIT+ regulatory T cells suppress pro-inflammatory responses in vivo. To induce colitis CD45RB^{hi} effector T cells (CD45.1) were transferred into Rag1-/- mice together with TIGIT+ or TIGIT- Tregs (CD45.2) or no Tregs as controls (Teff:Treg ratio was 4.4:1 for TIGIT+ Tregs and 3.6:1 for TIGIT- Tregs). (**Figure 6A**) Mice were monitored for weight loss over 10 weeks and (**Figure 6B**) total colitis scores were determined by histopathology. (**Figures 6C-6E**) At 10 weeks after transfer mesenteric LNs were harvested and (**Figure 6C**) total number of infiltrating CD4+ T cells, (**Figure 6D**) proportion of Foxp3+ Tregs among CD4+ T cells, and (**Figure 6E**) Foxp3 expression among the transferred Treg population (CD45.2+) were determined by flow cytometry. (**Figure 6F**) Mesenteric LN cells were re-stimulated in vitro with 0.5 µg/ml anti-CD3 for 3 days and cytokine secretion was determined by cytometric bead array in supernatants (P<0.05 (*), P<0.01 (**), P<0.005 (***)).

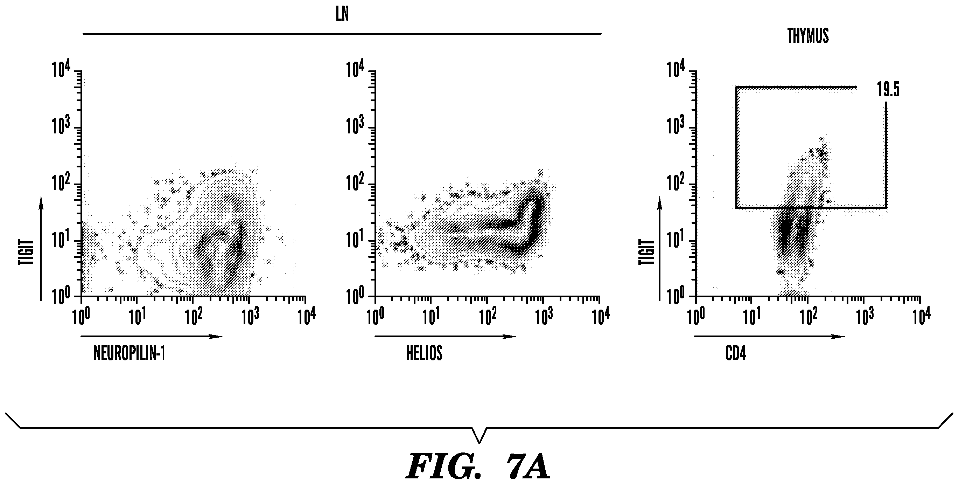

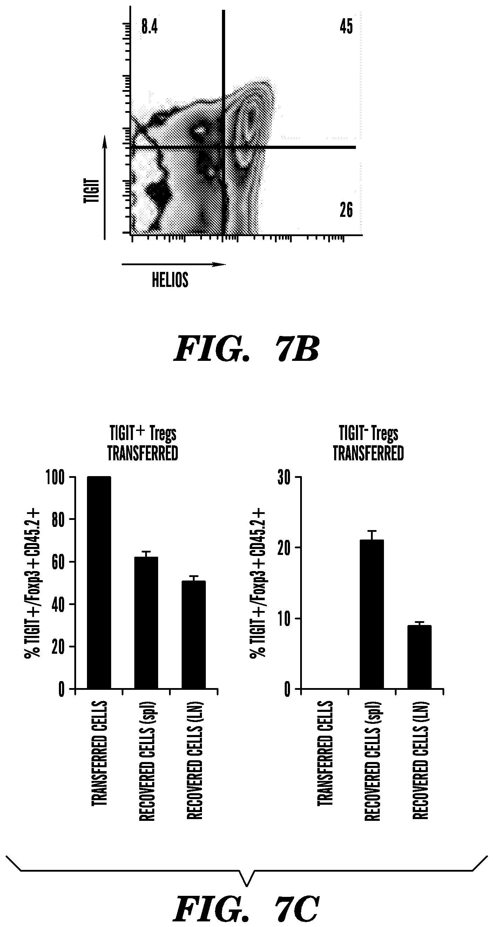

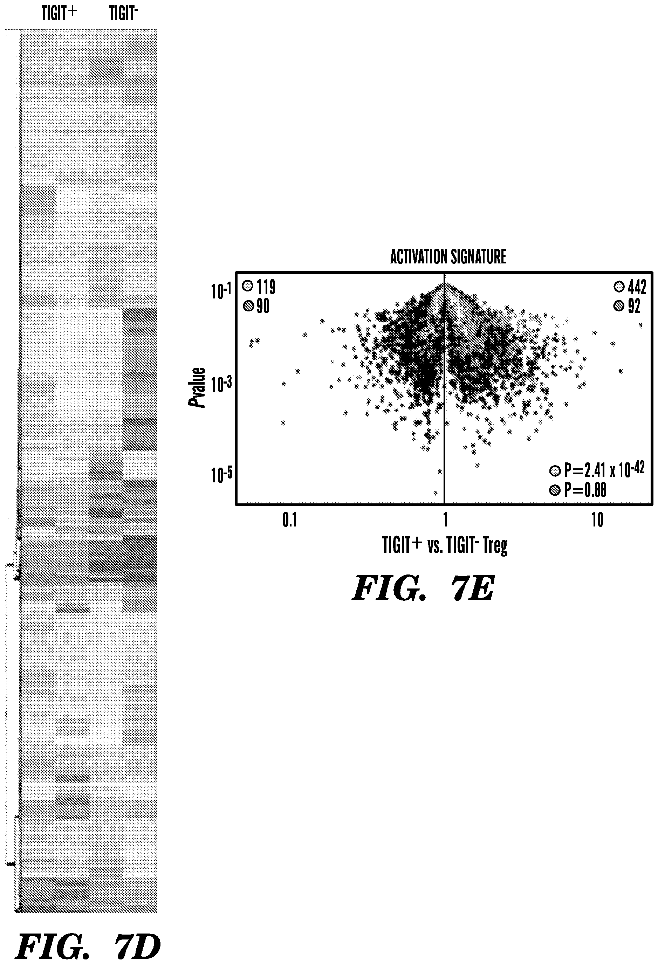

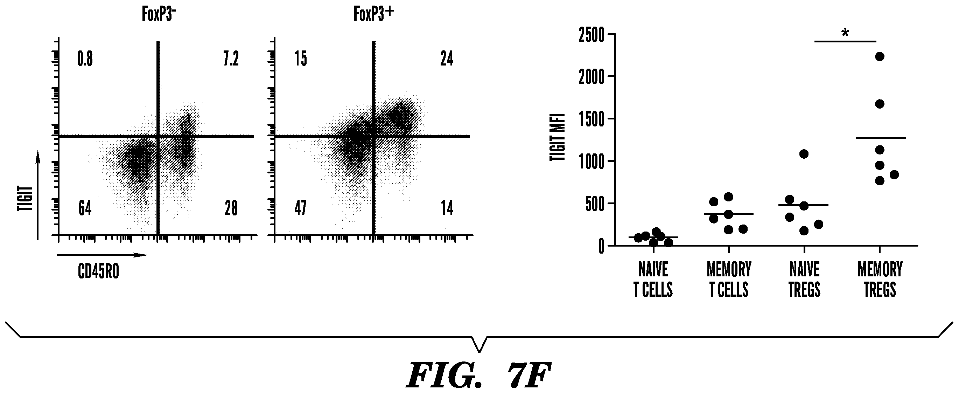

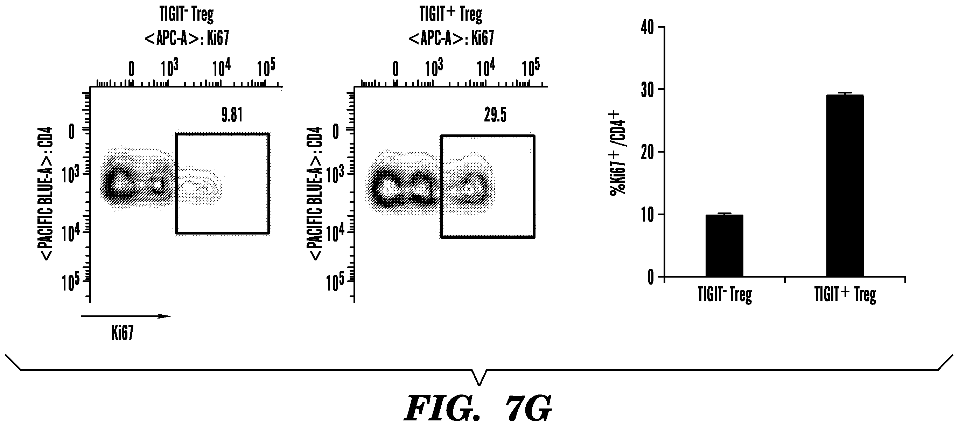

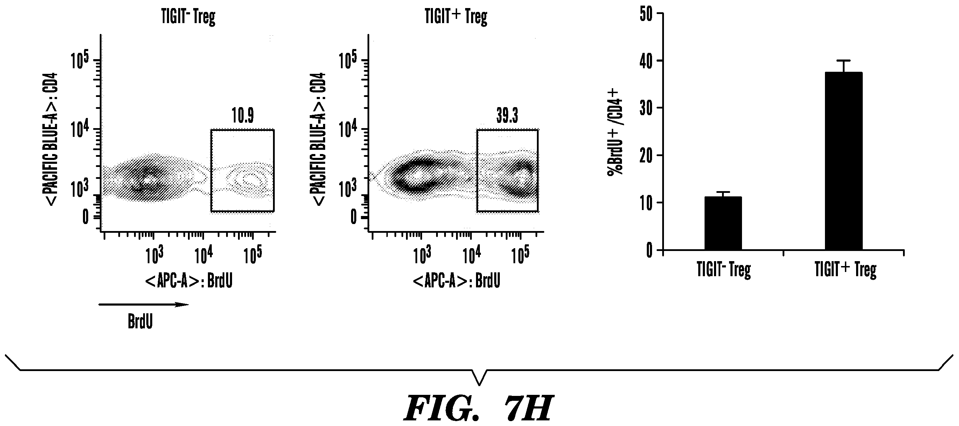

**Figures 7A-7H** are experimental data showing that TIGIT+ regulatory T cells display an activated phenotype. (**Figure 7A-7B**) Expression of TIGIT in conjunction with the natural Treg markers Neuropilin-1 and Helios was analyzed in (**Figure 7A**) murine CD4+CD8-Foxp3+ and (**Figure 7B**) human CD4+ CD127^{low} CD25^{high} Tregs using flow cytometry. (**Figure 7C**) CD4+Foxp3+TIGIT+ and CD4+Foxp3+TIGIT- Tregs (CD45.2) were sorted and transferred i.v. into WT recipients (CD45.1). After 20 days TIGIT expression on donor cells (CD45.2) was assessed by flow cytometry in lymph nodes (LN) and spleen. (**Figure 7D**) Heat map of the microarray analysis of CD4+Foxp3+TIGIT+ and CD4+Foxp3+TIGIT- Tregs of genes that display differential expression (>1.5-fold); duplicate samples are shown. (**Figure 7E**) Volcano plot comparing P value versus fold-change for probes from TIGIT+ versus TIGIT- Treg cells. Genes from the T cell activation/proliferation-responsive genes are highlighted in red (overexpressed) and green (underrepresented). (**Figure 7F**) Ex vivo human CD4+ T cells were stained for CD45RO, FoxP3, and TIGIT and analyzed by flow cytometry. Flow analysis from one representative healthy donor and pooled data of TIGIT MFI in each T cell subset are depicted (n=6; ANOVA *P<0.05). (**Figure 7G**) Ki67 expression in Foxp3+TIGIT+ and Foxp3+TIGIT- cells was determined by intracellular staining and flow cytometry. (**Figure 7H**) Naive Foxp3-GFP.KI mice were administered 1mg of BrdU/day by i.p. injection over 4 days. On day5 BrdU incorporation was assessed by flow cytometry. (**Figures 7G** **and** **7H****:** representative plots and mean ± s.d.).

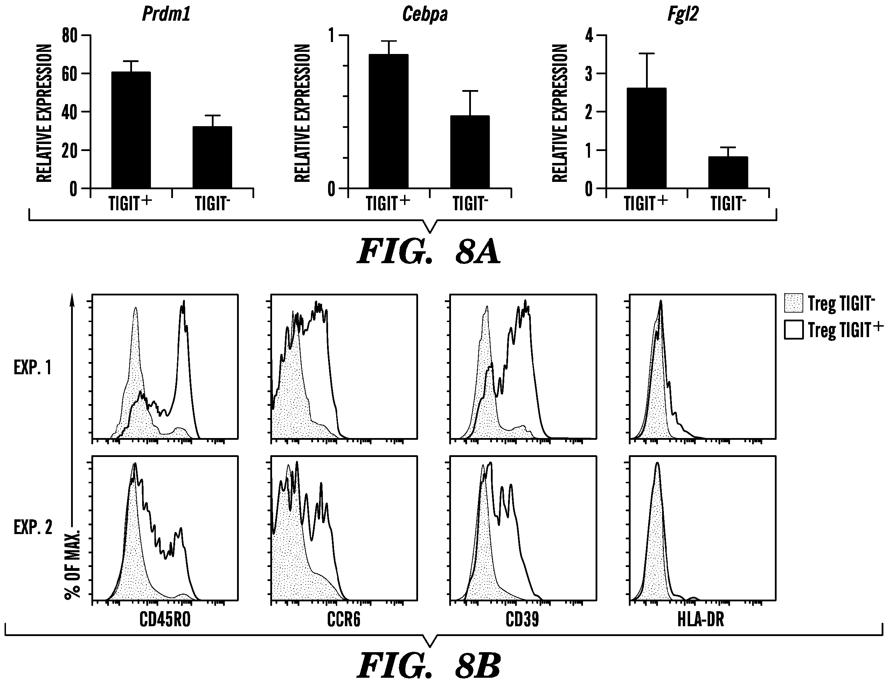

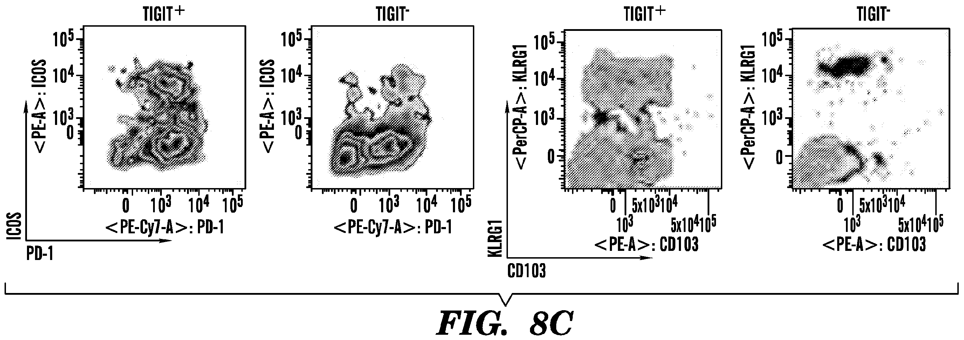

**Figures 8A-8C** are experimental data showing phenotypic characterization of TIGIT+ Tregs. (**Figures 8A, 8B**) Ex vivo human Tregs (CD4+CD127^{low}CD25^{high}) were FACS-sorted gating into TIGIT+ and TIGIT-. (**Figure 8A**) RNA was isolated for gene expression analysis by RT-PCR and (**Figure 8B**) cell surface expression of T cell markers was determined in both subsets by flow cytometry (n=6). (**Figure 8C**) Differential expression of cell surface molecules ICOS, PD-1, KLRG1, and CD103 in murine CD4+Foxp3+TIGIT+ and CD4+Foxp3+TIGIT- Tregs was determined by flow cytometry.

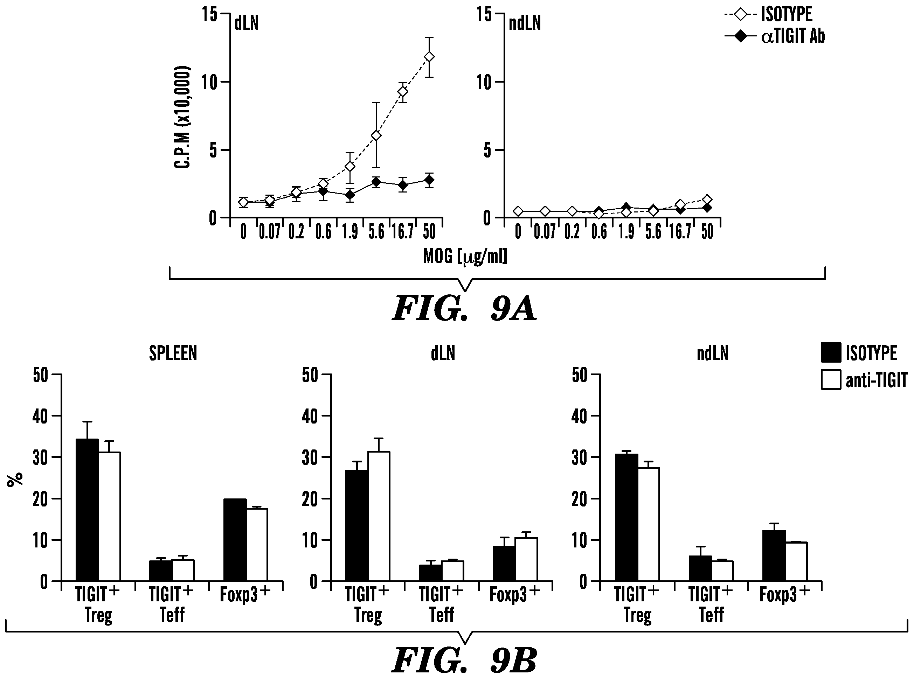

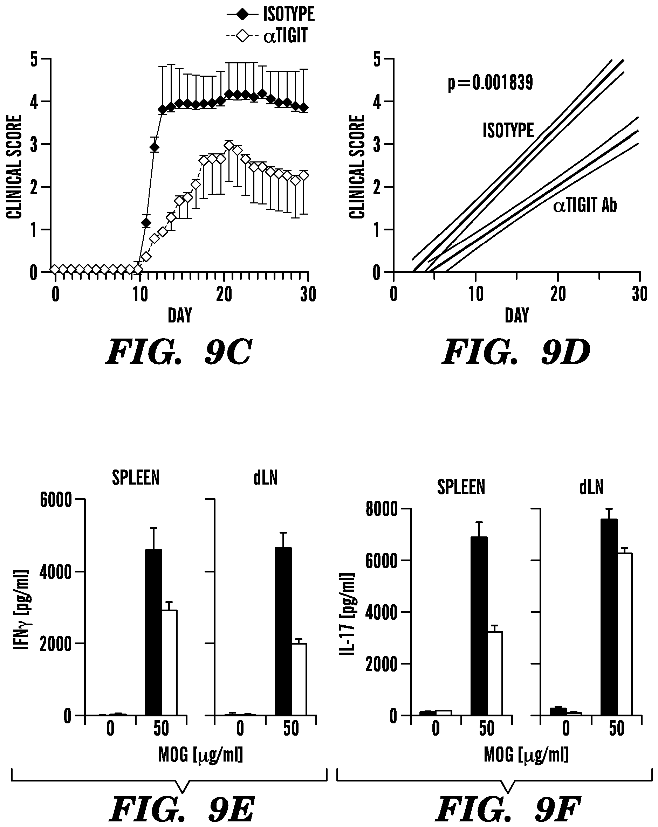

**Figures 9A-9F** are experimental data showing that an anti-TIGIT antibody acts agonistic in vivo. (**Figures 9A, 9B**) Mice were immunized s.c. with MOG₃₅₋₅₅ peptide in CFA and treated i.p. with 100 µg of anti-TIGIT or isotype control antibody on days 0, 2, and 4. Spleens and lymph nodes were collected on day 10. (**Figure 9A**) Cells were re-stimulated for 48 h in the presence of MOG₃₅₋₅₅ peptide, pulsed with 3H-thymidine and proliferation was determined 18 h later (mean ± s.d.). (**Figure 9B**) Frequencies of TIGIT+ Treg, TIGIT+ effector T cells (Teff) and Tregs (Foxp3+) were determined by flow cytometry at the time of sacrifice. (**Figures 9C-9F**) TIGIT suppresses Th1 and Th17 responses *in vivo.* Mice were immunized s.c. with 50µg MOG₃₅₋₅₅ peptide in CFA, followed by injection of 100ng pertussis toxin i.v. on day 0 and day 2. In addition, animals were treated with 100µg anti-TIGIT Ab (open symbols and bars) or an isotype control (filled symbols and bars) i.p. on days 0, 2, 4, 10, and 17. (**Figures 9C-9D**) Mice were monitored daily for EAE. Mean clinical score ± SEM (**Figure 9C**) and linear regressions (**Figure 9D**) are shown (n=14). (**Figures 9E and 9F**) Spleens and draining LN were harvested at disease onset (day 10) and re-stimulated with 30µg/ml MOG₃₅₋₅₅ *in vitro.* After 48h supernatants were harvested and analyzed for (**Figure 9E**) IFNγ and (**Figure 9F**) IL-17 by ELISA.

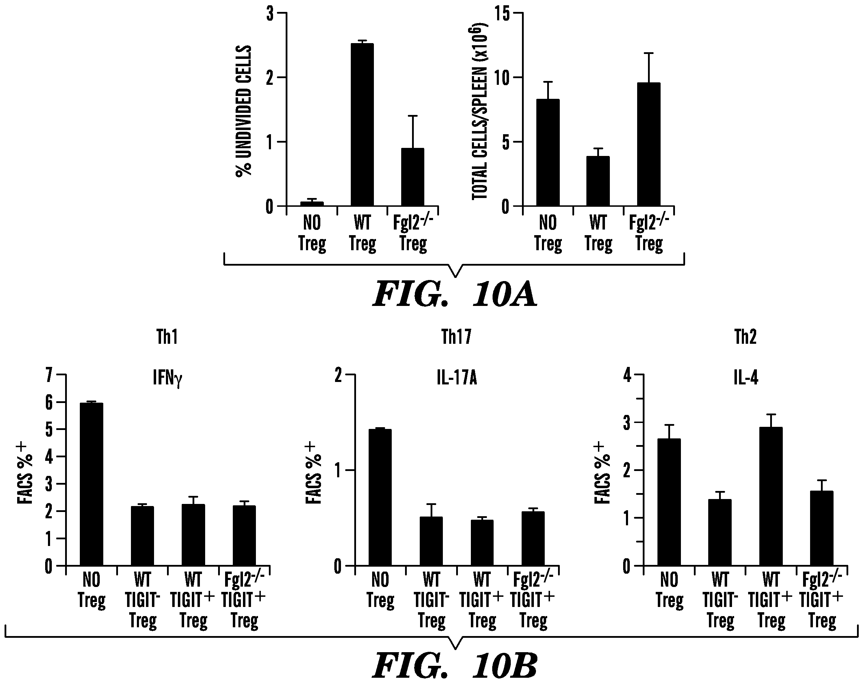

**Figures 10A-10B** are experimental data showing that TIGIT+ regulatory T cells suppress Th1 and/or Th17 but not Th2 differentiation. (**Figure 10A**) Naive CFSE-labeled effector T cells (CD45.1) were transferred into Rag1-/- mice together with WT or Fg12-/- Tregs (CD45.2) or no Tregs as controls (Teff:Treg ratio 5:1). After 8 days, splenocytes were analyzed for CFSE dilution and the number of undivided (CFSE^{high}) CD45.1+ cells as well as the total number of lymphocytes/spleen was quantified (mean ± SEM). (**Figure 10B**) Naive WT CD4+CD62L+CD25-effector T cells (CD45.1) and WT CD4+Foxp3+TIGIT+, WT CD4+Foxp3+TIGIT-Tregs, and Fgl2-/-CD4+Foxp3+TIGIT+ (CD45.2) were sorted and co-cultured at a ratio of 1:10 under Th1, Th2, or Th17 polarizing conditions. On day 5 cells were re-stimulated with PMA/Ionomycin and cytokine levels in CD45.1+ T effector cells were determined by flow cytometry. A representative experiment is shown.

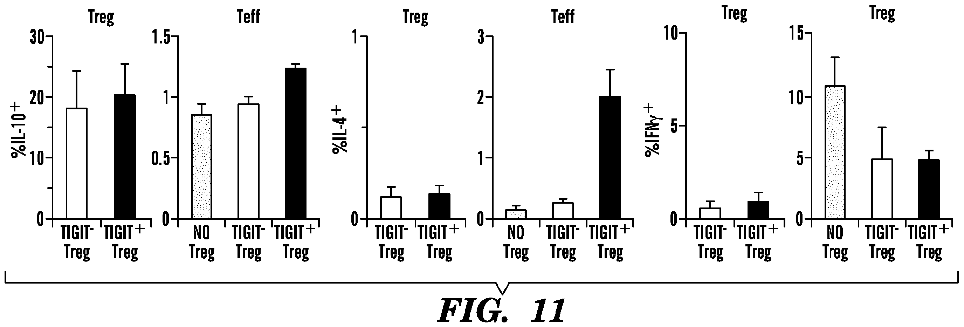

**Figure 11** shows that TIGIT+ regulatory T cells promote Th2-like responses in vivo. To induce colitis CD45RB^{hi} effector T cells (CD45.1) were transferred into Rag1-/- mice together with TIGIT+ or TIGIT- Tregs (CD45.2) or no Tregs as controls (Teff:Treg ratio was 4.4:1 for TIGIT+ Tregs and 3.6:1 for TIGIT- Tregs). At 10 weeks after transfer mesenteric LNs were harvested and cultured in vitro with 0.5µg/ml anti-CD3 for 3 days. Cells were then re-stimulated with PMA/Ionomycin for 4 hours and IL-10, IL-4, and IFNγ secretion was determined by intracellular cytokine staining. Plots show quantification of IL-10+, IL-4+ or IFNγ+ cells among CD4+CD45.1+ effector T cells or CD4+CD45.2+ regulatory T cells (mean ± s.d.).

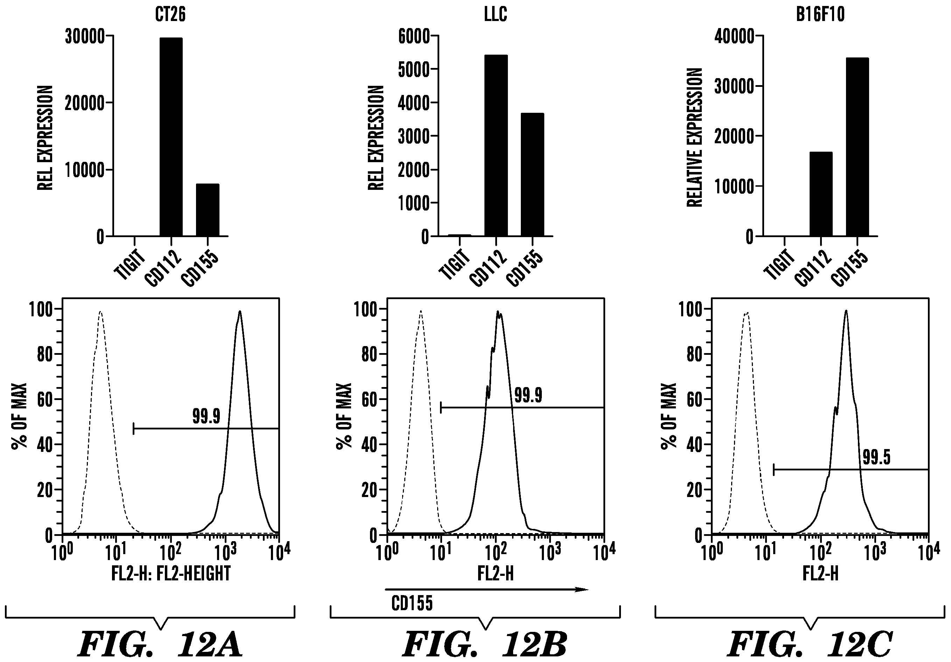

**Figures 12A-12C** are data graphs showing expression of TIGIT ligand (CD112 and CD155) on various murine tumors such as colon carcinoma (CT26: **Figure 12A**), Lewis lung carcinoma (LLC: **Figure 12B**) and melanoma (B16F10: **Figure 12C**). RNA was extracted from mouse colorectal carcinoma (CT26), lewis lung carcinoma (LLC) and melanoma (B16F10) for examination of TIGIT and the TIGIT ligands CD155 and CD112 by real-time quantitative PCR. CD155 expression was further confirmed by flow cytometry. No detectable expression of TIGIT receptors was found in murine tumors, but they have displayed TIGIT ligand expression. These data show that TIGIT ligands are expressed on the mouse tumor lines, consistent with a role of the TIGIT pathway in cancer.

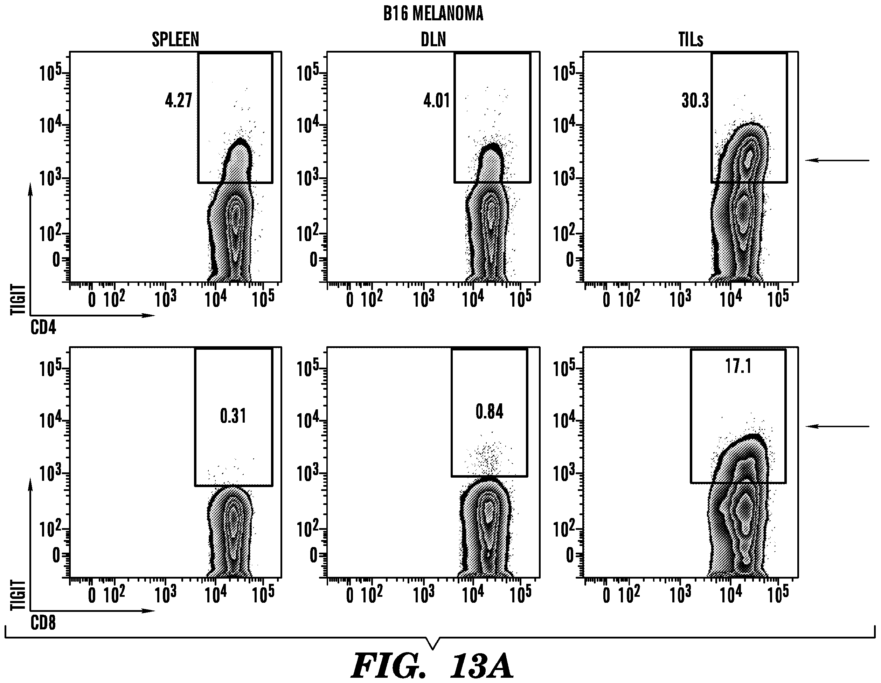

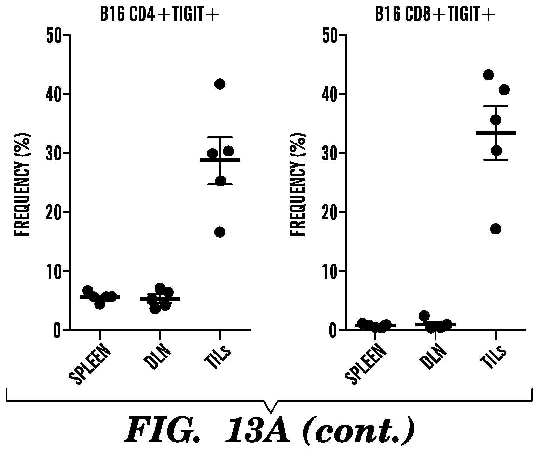

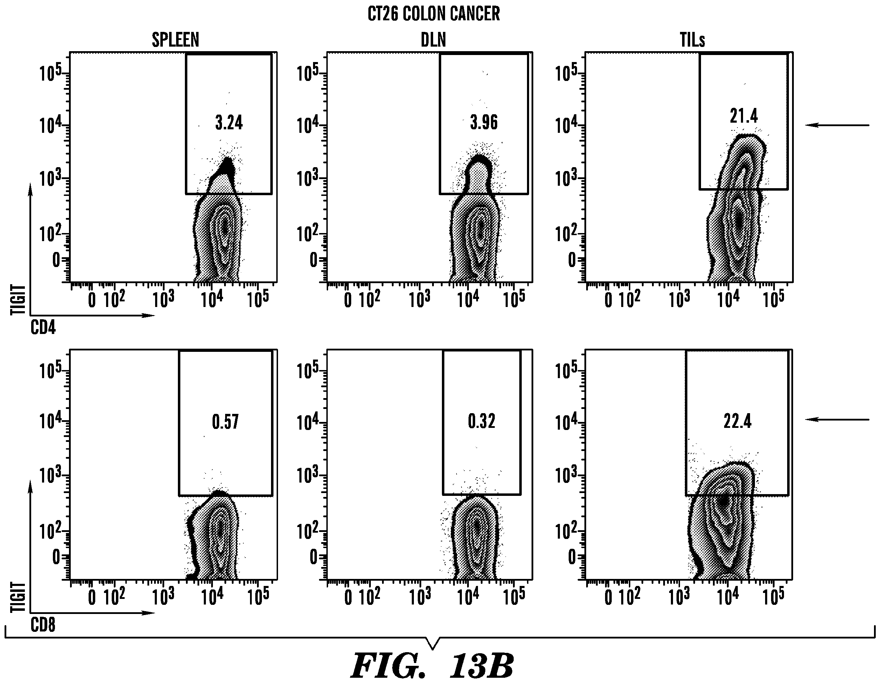

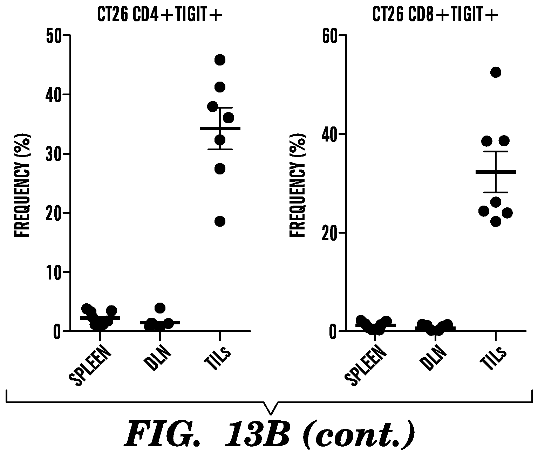

**Figures 13A-13B** show that TIGIT is enriched on both CD4 and CD8 T cells that infiltrate tumor (tumor-infiltrating lymphocytes; TILs; right panels), as compared to lymphocytes from the spleen (left panels) or tumor-draining lymph nodes (DLN; middle panels). B16F10 (5x10⁵) cells were inoculated into wild type C57BL/6 mice (n=5) and CT26 (1x10⁶) were inoculated in wild type Balb/c mice (n=7). Representative flow cytometry data showing TIGIT expression is enriched in both CD4 and CD8 tumor infiltrating lymphocytes (TILs) in melanoma. Spleen, tumor draining lymph node (DLN), and TILs were harvested and examined for TIGIT expression. **Figure 13A** shows the raw data and quantification of CD4+TIGIT+ T cells or CD8+TIGIT+ T cells from spleen, DLN, or TILs of a murine melanoma tumor (B16F10). **Figure 13B** shows the raw data and quantification of CD4+TIGIT+ T cells or CD8+TIGIT+ T cells from spleen, DLN, or TILs of a murine colon tumor (CT26). In each figure, upper panels show representative flow data, while bottom panels show summary data. These data show that TIGIT expression is highly enriched on T cells that infiltrate tumor tissue, indicating that targeting TIGIT can have significant effects in tumor tissue but not elsewhere. Thus, there can be fewer systemic effects, and decreased possibility for autoimmune-like toxicities as have been observed with targeting CTLA-4.

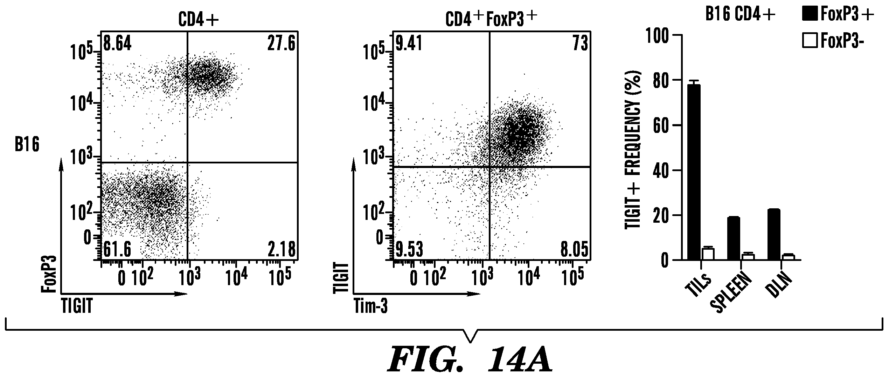

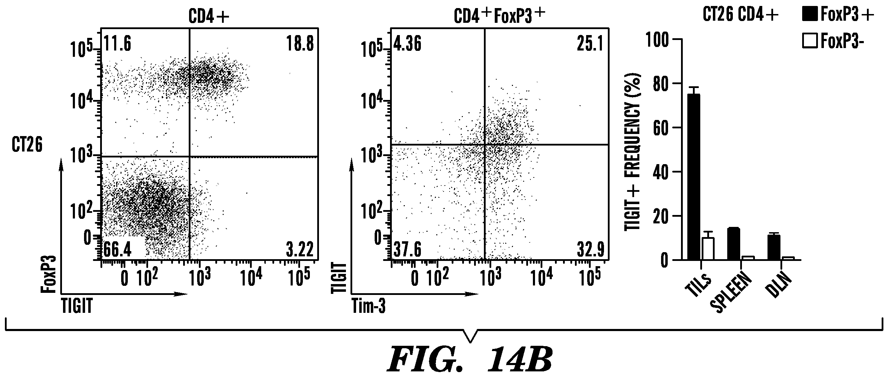

**Figures 14A-14B** show that TIGIT+ CD4+ T cells are predominantly FoxP3+ Treg in tumor-bearing mice. B16F10 (5x10⁵) and CT26 (1x10⁶) were inoculated into FoxP3-GFP Knock-in mice on the C57BL/6 (n=5) and Balb/c (n=7) backgrounds, respectively. **Figure 14A** corresponds to a murine melanoma tumor model (B16F10). **Figure 14B** corresponds to a murine colon tumor model (CT26). In each figure, left panel shows TIGIT and FoxP3 expression on CD4+ TILs; middle panel show TIGIT and Tim-3 expression on CD4+FoxP3+ TILs; and the right panel (a bar graph) shows summary data for TIGIT expression on FoxP3+ and FoxP3- cells in TILs, spleen, and tumor draining lymph node (DLN). The data in **Figures 14A-14B** show that TIGIT+ Treg are highly enriched in tumor tissue and TIGIT+ Tregs coexpress Tim-3. These data also show TIGIT expression is selective to FoxP3+ Treg in CD4 TILs.

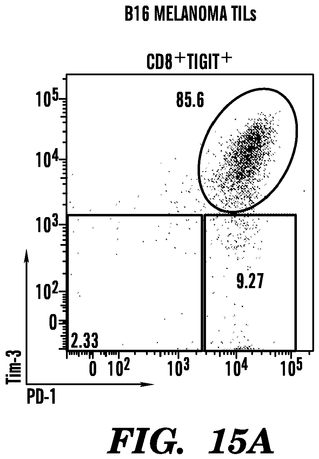

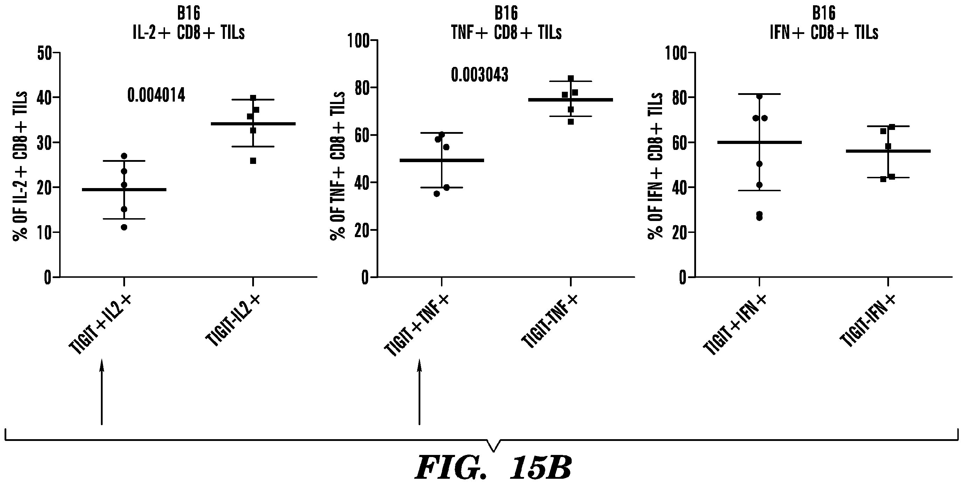

**Figures 15A-15B** show that TIGIT+ CD8+ tumor infiltrating lymphocytes (TILs) co-express the T cell inhibitory receptors such as Tim-3 and PD1 (**Figure 15A**), and also exhibit exhausted/dysfunctional phenotype, for example, defective IL-2 (**Figure 15B**, left panel), TNFa production (**Figure 15B**, middle panel), and increased IL-10 production (not shown). B16F10 (5x10⁵) cells were inoculated into wild type C57BL/6 mice. (**Figure 15A**) Representative Tim-3 and PD-1 staining on CD8 TILs from B16F10 melanoma. (**Figure 15B**) TILs were harvested from B16F10 melanoma tumors and restimulated ex vivo with PMA/ionomycin for 4 hrs prior to intracytoplasmic staining. Expression of IL-2, TNF, and IFNγ on TIGIT CD8+ TILs is shown, n=5. These data indicate that TIGIT is found on T cells that co-express other markers of T cell dysfunction/exhaustion such as Tim-3 and PD-1 and show that TIGIT+ TILs are defective in IL-2 and TNF production. The TIGIT+ CD8+ TILs exhibit no significant defects in IFNγ (**Figure 15B****,** right panel).

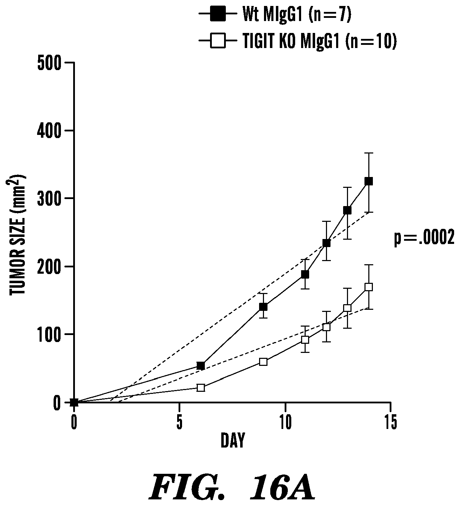

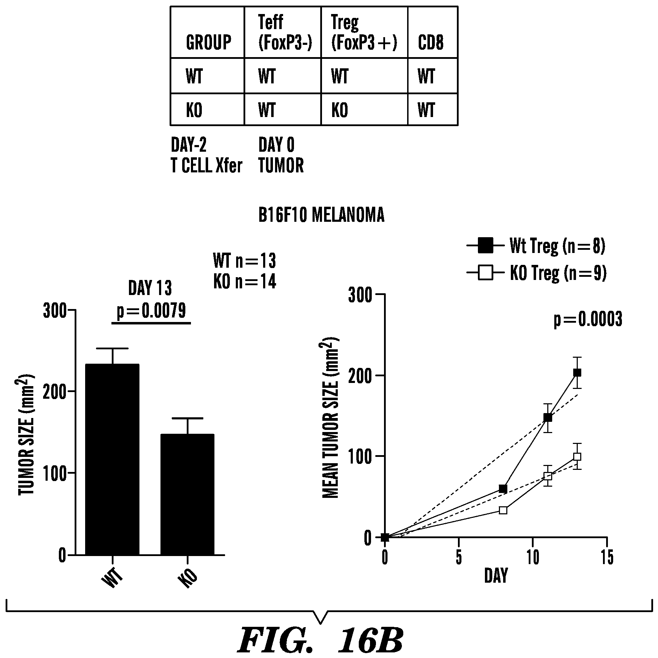

**Figures 16A-16B** are experimental data showing roles of TIGIT+ Treg in tumor growth and tumor immunity. **Figure 16A** is a line graph showing better control of tumor growth in mice bearing melanoma (B16F10) with TIGIT knock-out (KO). 7 week old female C57BL/6 mice were inoculated with B16F10 (5x10⁵). Tumor growth was measured in two dimensions using a caliper. Mean tumor growth is shown. Error bars indicate SEM. Dashed lines indicate linear regression. Difference in slope is statistically significant, p=0.0002. **Figure 16B** shows role of TIGIT Treg in tumor immunity. At Day 0, 7 week old female Rag-deficient mice were reconstituted with wildtype CD4 FoxP3-GFP- effectors, wildtype FoxP3-GFP+ Treg and wildtype CD8 T cells (WT group) or with wildtype CD4 FoxP3-GFP- effectors, TIGIT- deficient FoxP3-GFP+ Treg and wildtype CD8 T cells (KO group). At Day 2, mice were inoculated with B16F10 (5x10⁵). Tumor growth was measured in two dimensions using a caliper. Mean tumor growth is shown. Error bars indicate sem. Dashed lines indicate linear regression. Difference in slope is statistically significant, p=0.0003. Bar group shows mean tumor size at Day 13 from 3 experiments. P=0.0079.

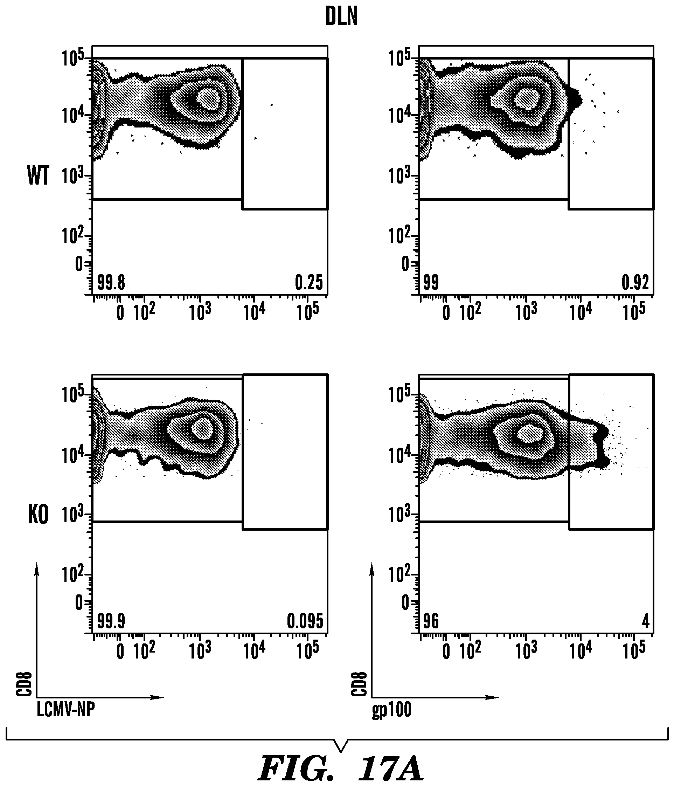

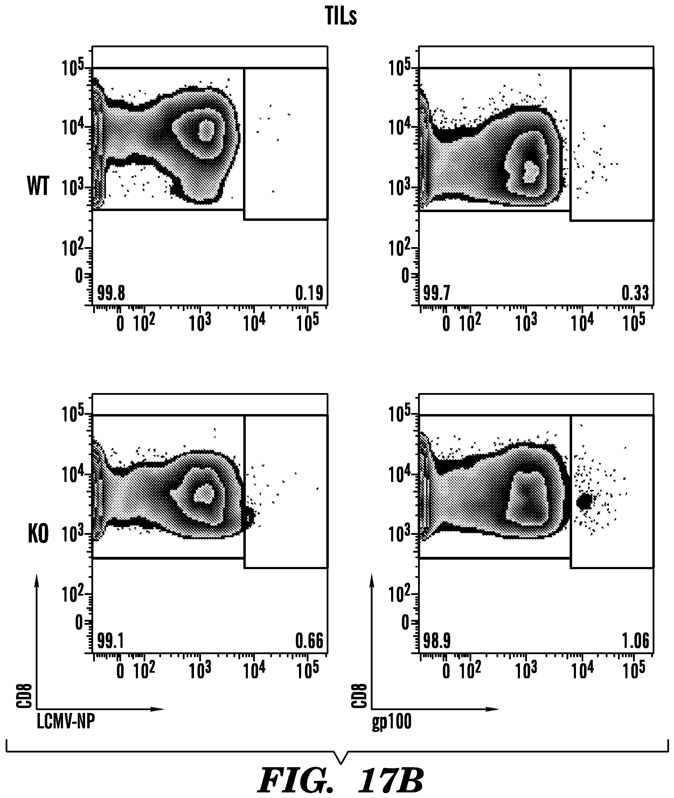

**Figures 17A-17B** are experimental data showing that increase in tumor-specific CD8+ T cells from DLN (**Figure 17A**) and TILs (**Figure 17B**) in mice lacking TIGIT+ Tregs.

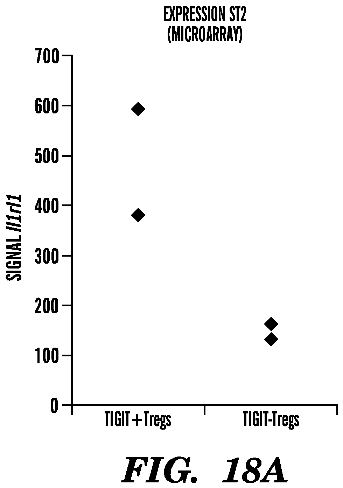

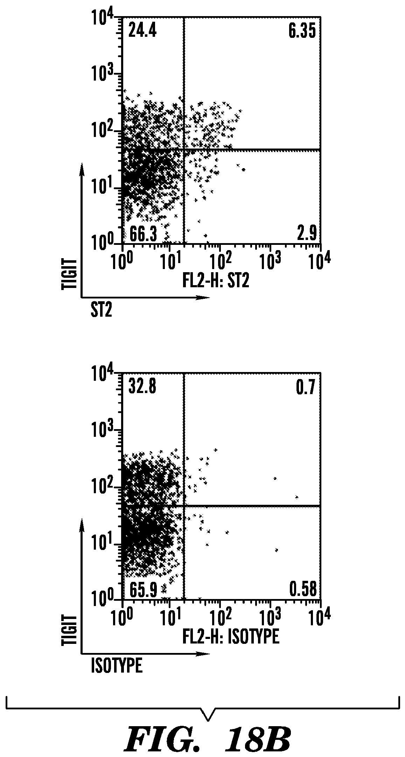

**Figures 18A-18B** are experimental data showing that TIGIT+ Tregs express ST2. (**Figure 18A**) Microarray analysis of naive CD4+Foxp3+TIGIT+ and CD4+Foxp3+TIGIT- Tregs was performed. Signal intensity for probes for the ST2 gene Il1rl1 is depicted (duplicate samples). (**Figure 18B**) Expression of ST2 and TIGIT was analyzed in naive CD4+Foxp3+ Tregs isolated from spleen. Representative plots from one of >10 independent experiments are shown. Cells are gated on live CD4+Foxp3+ Tregs.

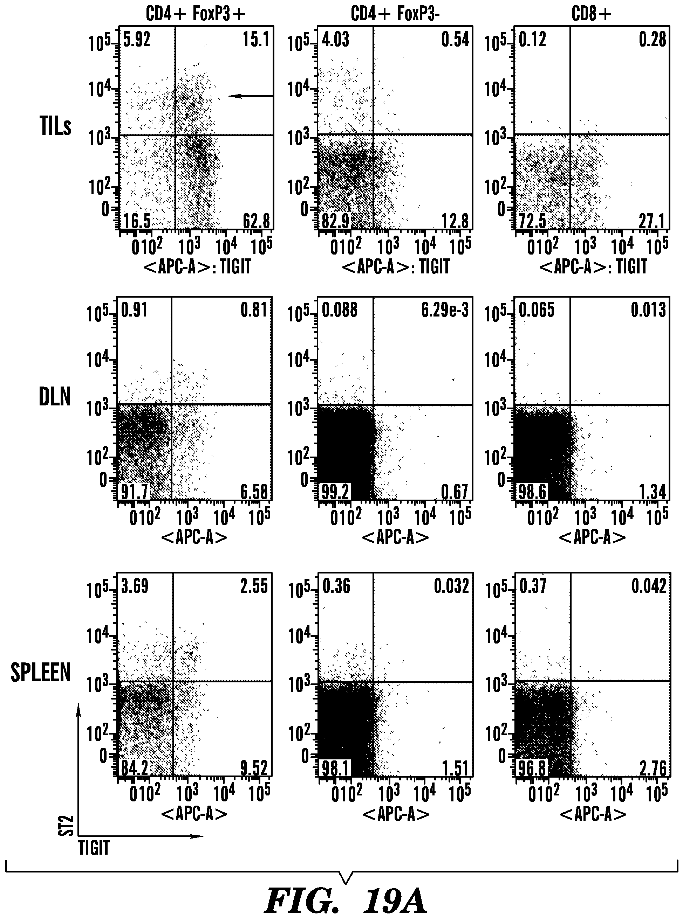

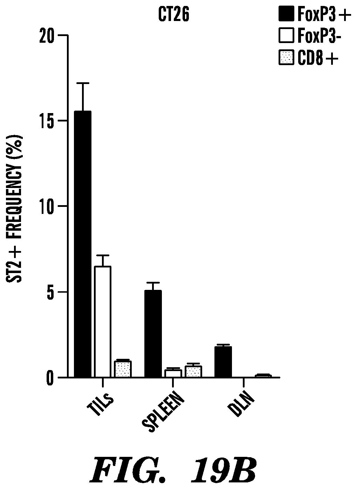

**Figures 19A-19B** are experimental data showing expression of an interleukin-1 receptor family member ST2 and TIGIT on T cells in CT26 colon cancer. CT26 (1x10⁶) were inoculated in wild type Balb/c mice. **Figure 19A** are representative flow cytometry data showing expression of ST2 and TIGIT on T cells (CD4+FoxP3+; CD4+FoxP3-; CD8+) from TILs (top panels), DLN (middle panels) and spleen (bottom panels), and indicating that ST2 is most highly expressed on TIGIT+ Treg in tumor tissue in CT26 colon carcinoma. **Figure 19B** is a bar graph showing the number of ST2+ T cells from TILs, spleen and DLN, and indicating that ST2+ Treg are highly enriched in tumor tissue. Error bars indicate SEM. These data show the role of IL-33/ST2 in promoting TIGIT Treg in tissue.

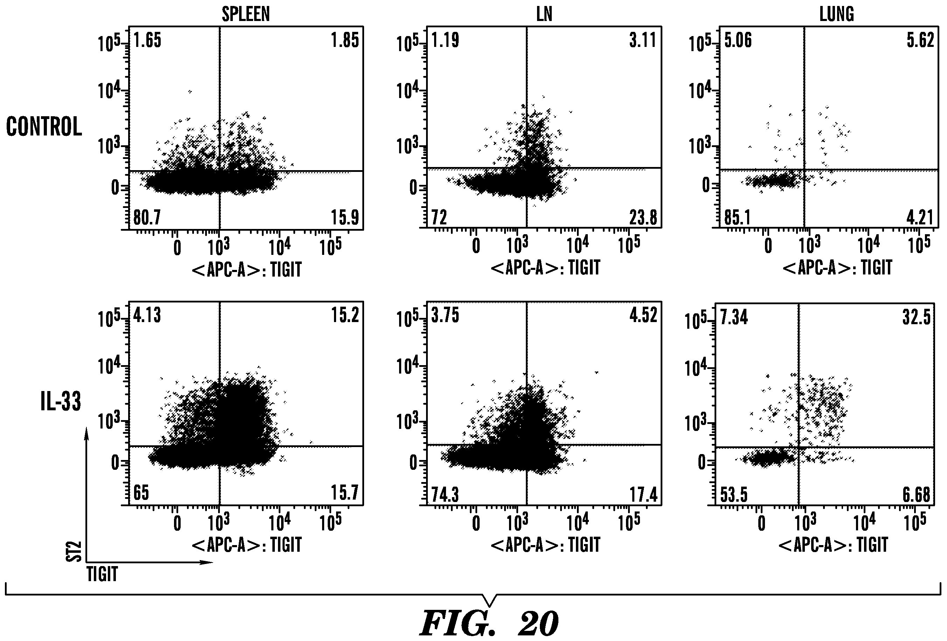

**Figure 20** is a set of plots showing that IL-33 expands TIGIT⁺ Tregs *in vivo.* Mice were treated with either 200ng IL-33 or PBS (control) i.p. for 4 days. On day 5, cells were isolated from spleen, lymph nodes (LN), and lung and expression of ST2 and TIGIT was analyzed in Tregs. Representative plots from one of three independent experiments are shown. Cells are gated on live CD4⁺Foxp3⁺ Tregs.

### DETAILED DESCRIPTION

Embodiments of the present disclosure are, in part, based on the discovery that TIGIT expression defines a functionally distinct subset of regulatory T cells (Tregs) that selectively suppress pro-inflammatory Th1 and Th17 responses but spare or promote an anti-inflammatory Th2 response by inducing the secretion of the soluble effector molecule Fgl2. Further, the inventors discovered that TIGIT+ Treg cells can be induced and/or expanded by IL-33. The inventors have also discovered that tumors express TIGIT ligands such as CD112 and CD155, which can induce tumor immune evasion where TIGIT+ Tregs infiltrate the tumors and induce suppression of Th1 and/or Th17 responses. Thus, not only can agents that modulate the activity and/or expression of TIGIT, Fgl2, and/or IL-33 be used for treatment of immune related diseases or disorders such as autoimmune disease, infection, chronic inflammation, cancer, asthma, allergy, and atopy, but TIGIT, Fgl2 and/or IL-33 can also be used as predictive markers to identify subjects who are more likely to benefit from an immunotherapy that targets TIGIT, Fgl2 and/or IL-33. Accordingly, various aspects of the present disclosure provide for methods of identifying subjects with an immune-related disease or disorder who are more likely to be responsive to an immunotherapy that targets TIGIT, Fgl2 and/or IL-33, as well as monitoring the treatment efficacy. Methods and compositions for treating subjects with an immune-related disease or disorder are also provided herein.

### Methods and compositions for treating immune-related diseases where an inhibition of Th2 response and/or a shift of balance toward a Th1and/or Th17 response is desirable

In some immune-related diseases or disorders, e.g., but not limited to cancer and/or infections, it can be desirable to induce proinflammatory responses, e.g., Th1 and/or Th17 responses, at a target site (e.g., a tumor) for a therapeutic effect. Accordingly, these immune-related diseases or disorders, e.g., but not limited to cancer and/or infections (including, e.g., but not limited to chronic viral infection, intracellular bacterial infection, extracellular bacterial infection, and/or fungal infection), where upregulation of immune response (e.g., Th1 and/or Th17 responses) is desirable, can be treated by inhibiting or reducing the expression or activity of TIGIT, Fgl2 and/or IL-33. However, not every patient, e.g., not every patient with cancer and/or infection, would necessarily benefit from a treatment that inhibits the level of TIGIT, Fgl2 and/or IL-33 expression and/or activity. The inventors discovered that the regulatory T cells in a subject can be separated into distinct populations, TIGIT+ cells with more strongly suppressive phenotypes and TIGIT negative (-) cells, and that Fgl2 and IL-33 are involved in regulation of TIGIT. The TIGIT+ cell population and Fgl2 and/or IL-33 expression and/or activity can vary in each individual. Tumors which include high levels of TIGIT+ T cell infiltration would be expected to respond poorly to immunotherapies designed to stimulate Th1 and/or Th17-type responses, because the TIGIT axis as defined below works to suppress activity and/or activation of Th1/Th7 responses. Only after the TIGIT axis is inhibited would one expect a strong anti-tumor immune response. The TIGIT axis suppresses proinflammatory responses, e.g., via suppression of Th1 and/or Th17 mediated responses. As defined herein, the "TIGIT axis" refers to an immunosuppressive pathway including TIGIT and Fgl2. The inventors have also discovered that TIGIT induces expression and/or activity of a transcription factor CEBPα, which in turn induces Fgl2 expression. Accordingly, in some embodiments, the TIGIT axis can further include CEBPα, and thus the "TIGIT axis" refers to an immunosuppressive pathway including TIGIT, CEBPα, and Fgl2. In some embodiments, the TIGIT axis further includes IL-33, where IL-33 induces or expands the TIGIT+ T cells such as TIGIT+ Tregs, and thus the "TIGIT axis" refers to an immunosuppressive pathway including IL-33, TIGIT, and Fgl2. IL-33 induces TIGIT expression and/or increases the TIGIT+ regulatory T cells (Tregs), where TIGIT induces transcription and secretion of the effector molecule Fgl2 in Tregs, thus resulting in suppression of pro-inflammatory Th1 and/or Th17 cells but not Th2 response

Accordingly, some aspects of the present disclosure relate to methods of identifying a patient who is diagnosed with cancer and/or infection, and is more likely to be responsive to a Th1 and/or Th17 pro-inflammatory agent or to an anti-TIGIT, anti-Fgl2 and/or anti-IL-33 therapy. In some embodiments, patients diagnosed with cancer and/or infection can be identified as more likely to be responsive to a Th1 and/or Th17 pro-inflammatory agent or to an anti-TIGIT and/or anti-IL-33 therapy based on the patients' level of Fgl2 activity or expression in a sample. In some embodiments, patients with cancer and/or infection can be identified as more likely to be responsive to a Th1 and/or Th17 pro-inflammatory agent or to an anti-IL-33 therapy and/or anti-Fgl2 therapy based on the patients' level of TIGIT activity or expression in a sample. In some embodiments, patients diagnosed with cancer and/or infection can be identified as more likely to be responsive to a Th1 and/or Th17 pro-inflammatory agent or to an anti-TIGIT and/or anti-Fgl2 therapy based on the patients' level of IL-33 activity or expression in a sample.

As used herein, the phrase "more likely to be responsive" generally refers to likelihood of a subject to respond to a treatment. In accordance with one aspect of the discovery that selective suppression by TIGIT+ Tregs is Fgl2-dependent and IL-33 can induce or expand the Treg population, by determining the level of TIGIT, Fgl2 and/or IL-33 expression or activity, one can predict the immune response of a subject subjected to an agent that modulates TIGIT, Fgl2 and/or IL-33 expression or activity (e.g., an agent that activates or suppresses a Th1 or Th17 response), which can in turn produce an effect on a disease or condition. Patients whose tumors and/or cells (including, e.g., normal cells and/or diseased cells such as infected cells) express TIGIT ligands (e.g., CD112 and/or CD155), Fgl2 and/or IL-33 would likely have a greater population of TIGIT+, Fgl2+, Fgl2 receptor+ and/or IL-33 receptor+ T cells infiltrated therein and thus suppress activity or activation of Th1 and/or Th17 cells. Accordingly, these patients would not be expected to respond effectively to an anti-tumor and/or anti-infection therapy designed to stimulate Th1 and/or Th17 cells on its own, as relative to patients whose tumors and/or tissues (including, e.g., normal tissues or diseased tissues such as infected tissues) (i) lack or express low levels of TIGIT ligands (e.g., CD112 and/or CD155), Fgl2 and/or IL-33; and/or (ii) lack or are low in T cells with these markers. The same patients with (i) tumors and/or cells (including, e.g., normal cells and/or diseased cells such as infected cells) expressing TIGIT ligands (e.g., CD112 and/or CD155), Fgl2 and/or IL-33; and/or (ii) TIGIT+, Fgl2+, Fgl2 receptor+ and/or IL-33 receptor+ tumor or tissue (including, e.g., normal tissue or diseased tissue such as infected tissue) T cells would, however, more likely benefit from anti-TIGIT, anti-Fgl2, and/or anti-IL-33 therapy, which would likely permit either spontaneous Th1 or Th17 responses, or Th1 and/or Th17 responses induced via coadministered agents that stimulate Th1 and/or Th17 cells.

As used herein, an "immune response" being modulated refers to a response by a cell of the immune system, such as a B cell, T cell (CD4 or CD8), regulatory T cell, antigen-presenting cell, dendritic cell, monocyte, macrophage, NKT cell, NK cell, basophil, eosinophil, or neutrophil, to a stimulus. In some embodiments, the response is specific for a particular antigen (an "antigen-specific response"), and refers to a response by a CD4 T cell, CD8 T cell, or B cell via their antigen-specific receptor. In some embodiments, an immune response is a T cell response, such as a CD4+ response or a CD8+ response. Such responses by these cells can include, for example, cytotoxicity, proliferation, cytokine or chemokine production, trafficking, or phagocytosis, and can be dependent on the nature of the immune cell undergoing the response.

By way of example only, one aspect of the present disclosure relates to methods of identifying a patient who is diagnosed with cancer and/or infection, and is more likely to be responsive to a proinflammatory immunotherapy, or an anti-TIGIT and/or anti-IL-33 therapy, based on the level of Fgl2 activity or expression in the patient's sample. The method comprises (a) measuring the level of Fgl2 activity or expression in a sample from a patient who is diagnosed with cancer and/or infection; and (b) comparing the level of Fgl2 or expression in the sample with an Fgl2 reference; and (c) (i) identifying the patient to be more likely to be responsive to a proinflammatory immunotherapy, or an anti-TIGIT and/or anti-IL-33 therapy, when the level of Fgl2 activity or expression is greater than the Fgl2 reference; or (ii) identifying the patient to be likely to respond to an alternative, proinflammatory immunotherapy comprising an activator of a proinflammatory T cell response pathway and/or a suppressor of an anti-inflammatory T cell response pathway, when the level of Fgl2 activity or expression is the same as or less than the Fgl2 reference. A pro-inflammatory immunotherapy comprises either an agent that directly activates a proinflammatory response, or an agent that suppresses an anti-inflammatory response.

Fgl2, also known as fibroleukin or fibrinogen-like protein 2, is a member of the fibrinogen-related protein superfamily of proteins. Fgl2 was first cloned from human CTLs and is secreted by CD4+ and CD8+ T cells or Tregs. Methods for measuring the secreted form of Fgl2 from a sample are known in the art, including, but not limited to mRNA expression using PCR or real-time PCR, protein analysis using western blot, immunoassay, and/or ELISA, and/or sequencing analysis. Thus, in some embodiments, nucleic acid molecules can be isolated from a patient's sample to measure Flg2 mRNA expression, or proteins can be isolated to measure Fgl2 protein expression.

As used herein, the term "expression" refers to the protein or mRNA amount of a target molecule (e.g., TIGIT, Fgl2 or IL-33) in a sample.

As used herein, the term "activity" refers to the ability of a target molecule (e.g., TIGIT, Fgl2, or IL-33) to directly or indirectly produce an immune response in a subject.

As used herein, the term "reference" refers to a pre-determined value for the level of expression or activity of a target molecule to be measured, which can be used in comparison with the expression or activity of the target molecule measured from a patient's sample. In the methods of various aspects of the present disclosure, a reference used for comparison to measured levels of TIGIT, Fgl2 and/or IL-33 activity or expression in a patient's sample can be determined from a normal healthy subject, or from a patient who has shown responsiveness to a treatment. In some embodiments, a reference can correspond to the level of expression or activity of the target molecule (e.g., TIGIT, Fgl2 or IL-33) in a normal healthy subject. The term "normal healthy subject" generally refers to a subject who has no symptoms of any diseases or disorders, or who is not identified with any diseases or disorders, or who is not on any medication treatment, or a subject who is identified as healthy by a physician based on medical examinations. In some embodiments, a reference can correspond to the level of expression or activity of the target molecule (e.g., TIGIT, Fgl2 or IL-33) in a normal tissue of the same type or lineage as a tissue biopsy obtained from a target site (e.g., a tumor or an inflammatory tissue) in a patient subjected to at least one aspect of the methods disclosed herein. In some embodiments, a reference can correspond to the level of expression or activity of the target molecule (e.g., TIGIT, Fgl2, or IL-33) at a prior time point in a patient from which a sample is derived or obtained. In some embodiments, a reference can correspond to a threshold level of expression or activity of the target molecule (e.g., TIGIT, Fgl2 or IL-33), above or below which the level of expression or activity of the target molecule (e.g., TIGIT, Fgl2 or IL-33) measured in a patient's sample would indicate the likelihood of a subject to respond to a treatment. In some embodiments, a reference can be a standard numeric level or threshold.

Accordingly, in some embodiments of the present disclosure, the Fgl2 reference can correspond to the level of expression or activity of Fgl2 in a normal healthy subject. In some embodiments, the Fgl2 reference can correspond to the level of expression or activity of Fgl2 in a normal tissue of the same type or lineage as a tissue biopsy obtained from a patient. The normal tissue of the same type or lineage can be obtained from the same or a different patient. In some embodiments, the Fgl2 reference can correspond to a threshold level of expression or activity of Fgl2, above which the level of Fgl2 expression activity measured in a patient's sample would indicate the likelihood of the patient diagnosed with cancer and/or infection to respond to a treatment. When the level of Fgl2 activity or expression is greater than the Fgl2 reference, e.g., by at least about 10% or more, including, e.g., at least about 20%, at least about 30%, at least about 40%, at least about 50%, at least about 60%, at least about 70%, at least about 80%, at least about 90%, at least about 100% or more, the patient diagnosed with cancer and/or infection is identified to be more likely to be responsive to an anti-TIGIT and/or anti-IL-33 therapy. In some embodiments, when the level of Fgl2 activity or expression is greater than the Fgl2 reference, e.g., by at least about 1.1-fold or more, including, e.g., at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold, or more, the patient diagnosed with cancer and/or infection can be identified to be more likely to be responsive to an anti-TIGIT and/or anti-IL-33 therapy. On the other hand, when the level of Fgl2 activity or expression is substantially the same as or less than the Fgl2 reference, e.g., by at least about 10% or more, including, e.g., at least about 20%, at least about 30%, at least about 40%, at least about 50%, at least about 60%, at least about 70%, at least about 80%, at least about 90%, or more, the patient diagnosed with cancer and/or infection is identified as likely to respond to an alternative, proinflammatory immunotherapy comprising an activator of a proinflammatory T cell response pathway and/or a suppressor of an anti-inflammatory T cell response pathway, e.g., without the need to suppress TIGIT, Fgl2, or IL-33 activity.

As used herein, the term "proinflammatory T cell response" refers to response of T cells to produce proinflammatory factors such as Th1 and/or Th17 cytokines, e.g., but not limited to IFNγ, TNFα, GM-CSF, IL-2, IL-9, IL-17, IL-21, and IL-22. In some embodiments, the term "proinflammatory T cell response" can refer to activation of "stimulatory immune checkpoints," including, but not limited to CD28, ICOS, 4-1BB, OX40, and/or CD27.

As used herein, the term "anti-inflammatory T cell response" refers to response of T cells to produce anti-inflammatory factors such as Th2 cytokines or immunosuppressive cytokines, e.g., but not limited to IL-4, IL-5, IL-6, IL-10, IL-13, TGFβ, IL-35, and/or IL-27. In some embodiments, the term "anti-inflammatory T cell response" can refer to activation of "inhibitory immune checkpoints," including, but not limited to PD-1, CTLA-4, BTLA, LAG-3, and/or TIM-3.

In this aspect and other aspects of the present disclosure, any appropriate modulators of a T cell response pathway that are known in the art can be used in the alternative, pro-inflammatory immunotherapy for patients with cancer and/or infection. For example, activators of a proinflammatory T cell response or suppressors of an anti-inflammatory T-cell response pathway can comprise a TIM-3 inhibitor, an anti-galectin-9 molecule, a PD-1 antagonist, a PD-L1 antagonist, a CTLA-4 antagonist, a Lag-3 antagonist, a DD1α antagonist, an agonist of an stimulatory immune checkpoint molecule, an antagonist of an inhibitory immune checkpoint molecule, or any combination thereof.

Some aspects of the present disclosure relate to methods of treating a patient diagnosed with cancer and/or infection. In some embodiments, the patient diagnosed with an infection can have chronic viral infection, intracellular bacterial infection, extracellular bacterial infection, and/or fungal infection. An anti-TIGIT, anti-Fgl2 and/or anti-IL33 therapy can be selected for administration to a patient diagnosed with cancer and/or infection, based on the level of Fgl2, IL-33 and/or TIGIT in the patient's sample. In some embodiments, an anti-TIGIT and/or anti-IL-33 therapy can be selected for administration to a patient diagnosed with cancer and/or infection, based on the patient's level of Fgl2 activity or expression in a sample. In some embodiments, an anti-IL-33 therapy and/or anti-Fgl2 therapy can be selected for administration to a patient diagnosed with cancer and/or infection, based on the patient's level of TIGIT activity or expression in a sample. In some embodiments, an anti-TIGIT and/or anti-Fgl2 therapy can be selected for administration to a patient diagnosed with cancer and/or infection, based on the patient's level of IL-33 activity or expression in a sample.

For example, in one aspect of the present disclosure is a method for treating a patient diagnosed with cancer and/or infection, wherein the method comprises (a) measuring the level of IL-33 activity or expression in a sample from a patient diagnosed with cancer and/or infection; (b) comparing the level of IL-33 activity or expression in the sample with an IL-33 reference, and (c) performing one of the following actions:
(i) administering to the patient a composition comprising a TIGIT inhibitor and/or an Fgl2 inhibitor, when the level of IL-33 activity or expression is greater than the IL-33 reference;
(ii) administering an alternative, e.g., proinflammatory immunotherapy, treatment without the TIGIT inhibitor or Fgl2 inhibitor, when the level of IL-33 activity or expression is the same as or less than the IL-33 reference; or
(iii) determining if the level of at least one other inhibitory immune regulator in the sample is greater than the level of the corresponding reference, or if the level of at least one activating immune regulator in the sample is less than the level of the corresponding reference, when the level of IL-33 activity or expression is the same as or less than the IL-33 reference.

Examples of inhibitory immune regulators include, but are not limited to Fgl2, TIGIT, ST2, CD155, CD112, PD-1, PD-L1, DD1α, TIM-3, galectin-9, CTLA-4, Lag-3, and any combination thereof. Examples of activating immune regulators include, but are not limited to CD28, ICOS, 4-1BB, OX40, CD27, and any combination thereof.

In some embodiments, the IL-33 reference can correspond to the level of expression or activity of IL-33 in a normal healthy subject. In some embodiments, the IL-33 reference can correspond to the level of expression or activity of IL-33 in a normal tissue of the same type or lineage as a tissue biopsy obtained from a patient. The normal tissue of the same type or lineage can be obtained from the same or a different patient. In some embodiments, the IL-33 reference can correspond to the level of expression or activity of IL-33 in a patient's sample obtained at a different or prior time point. In some embodiments, the IL-33 reference can correspond to a threshold level of expression or activity of IL-33, above which the level of IL-33 expression or activity measured in a patient's sample would indicate the likelihood of the patient diagnosed with cancer and/or infection to respond to a treatment. When the level of IL-33 activity or expression is greater than the IL-33 reference, e.g., by at least about 10% or more, including, e.g., at least about 20%, at least about 30%, at least about 40%, at least about 50%, at least about 60%, at least about 70%, at least about 80%, at least about 90%, at least about 100% or more, the patient diagnosed with cancer and/or infection is identified to be more likely to be responsive to a pro-inflammatory immunotherapy and/or a TIGIT inhibitor and/or Fgl2 inhibitor. In some embodiments, when the level of IL-33 activity or expression is greater than the IL-33 reference, e.g., by at least about 1.1-fold or more, including, e.g., at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold or more, the patient diagnosed with cancer and/or infection is identified to be more likely to be responsive to a pro-inflammatory immunotherapy and/or a TIGIT inhibitor and/or Fgl2 inhibitor. On the other hand, when the level of IL-33 activity or expression is substantially the same as or less than the IL-33 reference, e.g., by at least about 10% or more, including, e.g., at least about 20%, at least about 30%, at least about 40%, at least about 50%, at least about 60%, at least about 70%, at least about 80%, at least about 90%, or more, the patient diagnosed with cancer and/or infection is identified as likely to respond to a pro-inflammatory immunotherapy, e.g., an activator of a proinflammatory T cell response pathway and/or a suppressor of an anti-inflammatory T cell response pathway, without the need to suppress the TIGIT axis.

In some embodiments where the level of IL-33 activity or expression is the same as or less than the IL-33 reference, the method can further comprise (a) measuring the level of Fgl2 activity or expression in a sample from the patient, (b) comparing the level of Fgl2 activity or expression in the sample with an Fgl2 reference, and (c) administering to the patient a composition comprising a TIGIT inhibitor and/or an Fgl2 inhibitor, when the level of Fgl2 activity and/or expression is greater than the Fgl2 reference (e.g., by at least about 30% or more, including, e.g., at least about 40%, at least about 50%, at least about 60%, at least about 70%, at least about 80%, at least about 90%, at least about 95%, at least about 100% or more); or administering a proinflammatory immunotherapy without a TIGIT inhibitor or Fgl2 inhibitor, when the level of Fgl2 activity or expression is the same as or less than the reference (e.g., by at least about 30% or more, including, e.g., at least about 40%, at least about 50%, at least about 60%, at least about 70%, at least about 80%, at least about 90%, at least about 95% or more).

In some embodiments, when the level of Fgl2 activity and/or expression is greater than the Fgl2 reference, e.g., by at least about 1.1-fold or higher, including, e.g., at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold or higher, the patient can be administered a composition comprising a TIGIT inhibitor and/or an Fgl2 inhibitor.

In some embodiments where the level of IL-33 and/or Fgl2 activity or expression is the same as or less than the reference (e.g., by at least about 30% or more, including, e.g., at least about 40%, at least about 50%, at least about 60%, at least about 70%, at least about 80%, at least about 90%, at least about 95% or more), a proinflammatory immunotherapy without a TIGIT inhibitor or Fgl2 inhibitor to be administered can be an alternative, proinflammatory immunotherapy treatment comprising an activator of a proinflammatory T cell response pathway and/or a suppressor of an anti-inflammatory T cell response pathway as described earlier.

As used herein, the term "administering," or "administration" refer to the placement of an agent (e.g., a pro-or anti-inflammatory immunotherapy agent or an agent that modulates the expression and/or activity of TIGIT, Fgl2 and/or IL-33 into a subject by a method or route which results in at least partial localization of such agents at a desired site, such as a site of inflammation or tumor, such that a desired effect(s) is produced.

As used herein, "modulating" or "modulate" generally means either reducing or inhibiting the expression and/or activity of, or alternatively increasing the expression and/activity of, a target molecule, e.g., TIGIT, Fgl2 and/or IL-33, e.g., as measured using a suitable in vitro, cellular, or in vivo assay. In particular, "modulating" or "modulate" can mean either reducing or inhibiting the expression and/or activity of, or alternatively increasing a (relevant or intended) biological activity and/or expression of, a target molecule, e.g., TIGIT, Fgl2 and/or IL-33, as measured using a suitable in vitro, cellular or in vivo assay (which will usually depend on the target involved), by at least 5%, at least 10%, at least 25%, at least 50%, at least 60%, at least 70%, at least 80%, at least 90%, or more, inclusive, compared to activity of the target in the same assay under the same conditions but without the presence of an agent. Thus, as used herein, the term "modulating" can refer to an increase or decrease in the expression and/or activity of TIGIT, Fgl2 and/or IL-33 relative to a subject not treated with an agent that modulates the expression and/or activity of TIGIT, Fgl2 and/or IL-33. An "increase" or "decrease" refers to a statistically significant increase or decrease respectively. For the avoidance of doubt, an increase or decrease will be at least 10% relative to a reference, such as at least 10%, at least 20%, at least 30%, at least 40%, at least 50%,a t least 60%, at least 70%, at least 80%, at least 90%, at least 95%, at least 97%, at least 98%, or more, up to and including at least 100% or more, inclusive, in the case of an increase, for example, at least 2-fold, at least 3-fold, at least 4-fold, at least 5-fold, at least 6-fold, at least 7-fold, at least 8-fold, at least 9-fold,at least 10-fold, at least 50-fold, at least 100-fold, or more.

As will be clear to the skilled person, "modulating" can also involve effecting a change (which can either be an increase or a decrease) in affinity, avidity, specificity and/or selectivity of a target molecule, e.g., TIGIT, Fgl2 and/or IL-33, for one or more of its ligands, receptors, binding partners, partners for association into a homomultimeric or heteromultimeric form, or substrates; and/or effecting a change (which can either be an increase or a decrease) in the sensitivity of the target molecule, e.g., TIGIT, Fgl2 and/or IL-33, for one or more conditions in the medium or surroundings in which the target molecule is present (such as pH, ion strength, the presence of co-factors, etc.), compared to the same conditions but without the presence of the target molecule, e.g., TIGIT, Fgl2 and/or IL-33. Again, this can be determined in any suitable manner and/or using any suitable assay known per se or described herein, depending on the target involved. "Modulating" can also mean effecting a change (i.e., an activity as an agonist, as an antagonist or as a reverse agonist, respectively, depending on the target molecule, e.g., TIGIT, Fgl2 and/or IL-33, and the desired biological or physiological effect) with respect to one or more biological or physiological mechanisms, effects, responses, functions, pathways or activities in which the target or antigen (or in which its substrate(s), ligand(s) or pathway(s) are involved, such as its signaling pathway or metabolic pathway and their associated biological or physiological effects) is involved. Again, as will be clear to the skilled person, such an action as an agonist or an antagonist can be determined in any suitable manner and/or using any suitable (in vitro and usually cellular or in assay) assay known per se or described herein, depending on the target or antigen involved.

Modulating can, for example, also involve allosteric modulation of the target molecule, such as TIGIT, Fgl2 and/or IL-33; and/or reducing or inhibiting the binding of the target to one of its substrates, receptors, or ligands and/or competing with a natural ligand, receptor or substrate for binding to the target. Modulating can also involve activating the target or the mechanism or pathway in which it is involved. Modulating can for example also involve effecting a change in respect of the folding or confirmation of the target, or in respect of the ability of the target to fold, to change its conformation (for example, upon binding of a ligand), to associate with other (sub)units, or to disassociate. Modulating can for example also involve effecting a change in the ability of the target to signal, phosphorylate, dephosphorylate, and the like.

Thus, TIGIT, Fgl2 and/or IL-33 expression and/or activity is "decreased" or "reduced" if one or more signaling activities or downstream read-outs of TIGIT, Fgl2 and/or IL-33 activity is reduced by a statistically significant amount, such as by at least 10%, at least 20%, at least 30%, at least 40%, at least 50%,a t least 60%, at least 70%, at least 80%, at least 90%, at least 95%, at least 97%, at least 98%, or more, up to and including at least 100%, in the presence of an agent or stimulus relative to the absence of such modulation. As will be understood by one of ordinary skill in the art, in some embodiments, if TIGIT, Fgl2 and/or IL-33 expression and/or activity is decreased or reduced, some downstream read-outs will decrease but others can increase (i.e. things that are normally suppressed by TIGIT, Fgl2 and/or IL-33 expression and/or activity), and the converse would be in those embodiments where TIGIT, Fgl2 and/or IL-33 expression and/or activity is increased.

Conversely, TIGIT, Fgl2 and/or IL-33 expression and/or activity is "increased" if one or more signaling activities or downstream read-outs of TIGIT, Fgl2 and/or IL-33 expression and/or activity is increased by a statistically significant amount, for example by at least 10%, at least 20%, at least 30%, at least 40%, at least 50%,a t least 60%, at least 70%, at least 80%, at least 90%, at least 95%, at least 97%, at least 98%, or more, up to and including at least 100% or more, at least 2-fold, at least 3-fold, at least 4-fold, at least 5-fold, at least 6-fold, at least 7-fold, at least 8-fold, at least 9-fold,at least 10-fold, at least 50-fold, at least 100-fold, or more, in the presence of an agent or stimulus, relative to the absence of such agent or stimulus.

In some embodiments of this aspect and other aspects of the present disclosure, the agents described herein for modulating the expression and/or activity of TIGIT, Fgl2 and/or IL-33 can be administered to a subject by any mode of administration that delivers the agent systemically or to a desired surface, organ, or target, and can include, but is not limited to injection, infusion, instillation, and inhalation administration. To the extent that such agents can be protected from inactivation in the gut, oral administration forms are also contemplated. "Injection" includes, without limitation, intravenous, intramuscular, intraarterial, intrathecal, intraventricular, intracapsular, intraorbital, intracardiac, intradermal, intraperitoneal, transtracheal, subcutaneous, subcuticular, intraarticular, sub capsular, subarachnoid, intraspinal, intracerebro spinal, and intrasternal injection and infusion. In some embodiments, the agents for modulating the expression and/or activity of TIGIT, Fgl2 and/or IL-33 for use in the methods described herein are administered by intravenous infusion or injection.

The phrases "parenteral administration" and "administered parenterally" as used herein, refer to modes of administration other than enteral and topical administration, usually by injection. The phrases "systemic administration," "administered systemically", "peripheral administration" and "administered peripherally" as used herein refer to the administration of an agent for modulating expression and/or activity of TIGIT, Fgl2 and/or IL-33 other than directly into a target site, tissue, or organ, such as a tumor site, such that it enters the subject's circulatory system and, thus, is subject to metabolism and other like processes.

In another aspect of the present disclosure are methods of treating a patient diagnosed with cancer and/or infection comprising (a) measuring the level of Fgl2 activity or expression in a sample from a patient diagnosed with cancer and/or infection; (b) comparing the level of Fgl2 activity or expression in the sample with an Fgl2 reference; and (c) administering to the patient a composition comprising a TIGIT inhibitor and/or an IL-33 inhibitor when the level of Fgl2 activity or expression is greater than the Fgl2 reference (e.g., by at least about 30% or more, including, e.g., at least about 40%, at least about 50%, at least about 60%, at least about 70%, at least about 80%, at least about 90%, at least about 95%, at least about 100% or more), or administering an alternative, pro-inflammatory immunotherapy treatment without a TIGIT inhibitor or IL-33 inhibitor when the level of Fgl2 activity or expression is the same as or less than the Fgl2 reference (e.g., by at least about 30% or more, including, e.g., at least about 40%, at least about 50%, at least about 60%, at least about 70%, at least about 80%, at least about 90%, at least about 95% or more). In some embodiments, the alternative, proinflammatory immunotherapy treatment without a TIGIT inhibitor or IL-33 inhibitor can be a therapy comprising an activator of a proinflammatory T cell response pathway and/or a suppressor of an anti-inflammatory T cell response pathway.

In some embodiments, when the level of Fgl2 activity and/or expression is greater than the Fgl2 reference, e.g., by at least about 1.1-fold or more, including, e.g., at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold or more, the patient can be administered a composition comprising a TIGIT inhibitor and/or an IL-33 inhibitor.

In some embodiments, the patient with an Fgl2 level greater than the Fgl2 reference can be further administered with a therapy comprising an activator of a proinflammatory T cell response pathway and/or a suppressor of an anti-inflammatory T cell response pathway as described earlier.

In some aspects of the present disclosure, TIGIT, Fgl2 and/or IL-33 can be used as a predicative marker to determine or monitor the efficacy of an anti-TIGIT, anti-Fgl2 and/or anti-IL-33 therapy administered to a patient diagnosed with cancer and/or infection. In some embodiments, the patient diagnosed with an infection can have chronic viral infection, intracellular bacterial infection, extracellular bacterial infection, and/or fungal infection. In some embodiments, Fgl2 can be used as a predictive marker to determine or monitor the efficacy of an anti-TIGIT and/or anti-IL-33 therapy administered to a patient diagnosed with cancer and/or infection. In some embodiments, TIGIT can be used as a predictive marker to determine or monitor the efficacy of an anti-IL-33 therapy and/or anti-Fgl2 therapy administered to a patient diagnosed with cancer and/or infection. In some embodiments, IL-33 can be used as a predictive marker to determine or monitor the efficacy of an anti-TIGIT and/or anti-Fgl2 therapy administered to a patient diagnosed with cancer and/or infection.

As an example, methods of treating a patient who is diagnosed with cancer and/or infection and has an elevated level of Fgl2 are disclosed herein. The method comprises: (a) determining a first level of Fgl2 expression or activity in a sample from a patient diagnosed with cancer and/or infection that has an elevated level of Fgl2; (b) administering an agent that inhibits IL-33 activity and/or TIGIT activity; (c) determining a second level of Fgl2 expression or activity after the administering; and (d) comparing the first and second levels of Fgl2 expression or activity, wherein the agent administered in (b) is effective if the second level of Fgl2 expression or activity is lower than the first level, and wherein the agent administered in (b) is ineffective if the second level of Fgl2 expression is the same as or higher than the first level.

By monitoring the effects of the anti-IL-33 and/or anti-TIGIT therapy on the level of Fgl2 expression or activity, one can determine the efficacy of the treatment regimen and adjust the treatment regimen if necessary. Accordingly, in some embodiments, the method can further comprise, when the anti-IL-33 or anti-TIGIT therapy is effective, continuing to administer the agent that inhibits IL-33 activity and/or TIGIT activity. In some embodiments, the method can further comprise, when the anti-IL-33 therapy or the anti-TIGIT therapy is ineffective, administering the agent that inhibits IL-33 activity and/or TIGIT activity at a higher dose. In some embodiments, the method can further comprise, when the anti-IL-33 therapy or the anti-TIGIT therapy is ineffective, discontinuing the anti-IL-33 therapy or the anti-TIGIT therapy. In these embodiments, the method can further comprise administering a therapy comprising an activator of a proinflammatory T cell response pathway and/or a suppressor of an anti-inflammatory T cell response pathway.

Similarly, a further aspect of the present disclosure relates to methods of treating a patient diagnosed with cancer and/or infection that exhibits an elevated level of IL-33. The method comprises: (a) determining a first level of TIGIT and/or Fgl2 expression or activity in a sample from a patient diagnosed with cancer and/or infection and having an elevated level of IL-33; (b) administering an agent that inhibits IL-33 activity; (c) determining a second level of TIGIT or Fgl2 expression or activity after the administering; and (d) comparing the first and second levels of TIGIT and/or Fgl2 expression or activity, wherein anti-IL-33 therapy is effective if the second level of TIGIT and/or Fgl2 expression or activity is lower that the first level, and wherein anti-IL-33 therapy is ineffective if the second level of TIGIT and/or Fgl2 expression is the same as or higher than the first level.

In some embodiments, the method can further comprise, when the anti-IL-33 therapy is effective, continuing to administer the agent that inhibits IL-33 activity. In some embodiments, the method can further comprise, when the anti-IL-33 therapy is ineffective, administering the agent that inhibits IL-33 activity at a higher dose. In other embodiments, the method can further comprise, when the anti-IL-33 therapy is ineffective, discontinuing the anti-IL-33 therapy. In these embodiments, the method can further comprise administering a therapy comprising an activator of a proinflammatory T cell response pathway and/or a suppressor of an anti-inflammatory T cell response pathway.

In yet another aspect of the present disclosure, methods of treating a patient diagnosed with cancer and/or infection comprising administering to the patient one or more embodiments of the pharmaceutical compositions described herein are also provided. The pharmaceutical composition can be taken alone or in combination with another anti-cancer agent and/or an anti-infection agent. In some embodiments, the patient diagnosed with an infection can have chronic viral infection, intracellular bacterial infection, extracellular bacterial infection, and/or fungal infection.

As used herein, the term "in combination with" or "co-administer" in the context of therapy administration generally refers to administrating a first agent and at least a second agent. The first agent and the second agent can be administered concurrently or simultaneously (e.g., in the same or separate unit dosage forms), or separately at different times. The first agent and the second agent can be administered by the same or different route.

As used herein, an "anti-cancer agent" or "anti-cancer therapy" is generally an agent or a therapy for treatment of cancer, e.g., an agent that kills cancer cells, and/or reduces or prohibits tumor growth and/or progression. Examples of anti-cancer agents include, but are not limited to cancer vaccines, chemotherapy, targeted therapy (e.g., kinase inhibitors), radiation therapy, surgery, immunotherapy, and any combinations thereof. One of skill in the art can readily identify a chemotherapeutic agent for use in treatment of cancer (e.g. see Physicians' Cancer Chemotherapy Drug Manual 2014, Edward Chu, Vincent T. DeVita Jr., Jones & Bartlett Learning; Principles of Cancer Therapy, Chapter 85 in Harrison's Principles of Internal Medicine, 18th edition; Therapeutic Targeting of Cancer Cells: Era of Molecularly Targeted Agents and Cancer Pharmacology, Chs. 28-29 in Abeloff's Clinical Oncology, 2013 Elsevier; and Fischer D S (ed): The Cancer Chemotherapy Handbook, 4th ed. St. Louis, Mosby-Year Book, 2003).

As used herein, an "anti-infection agent" or "anti-infection therapy" is generally an agent or a therapy that kills or inhibits a cellular process, development and/or replication of a target infectious agent. Examples of an anti-infection agent or therapy include, but are not limited to anti-viral agent agent or therapy, anti-bacterial agent or therapy, anti-fungal agent or therapy, and a combination of two or more thereof.

As used herein, an "anti-viral agent" or "anti-viral therapy" is generally an agent or a therapy that kills or inhibits cellular process, development and/or replication of a target virus. For example, an anti-viral agent can be an agent that interferes with one or more viral components and/or interferes with replication or propagation of a virus. Examples of anti-viral agents include, but are not limited to, virus protein specific antibodies, reverse transcriptase inhibitors, protease inhibitors, immunomodulatory agents (e.g., cytokines, various nucleoside analogs, and/or Zn²⁺), plant extracts demonstrated to have an antiviral effect, and any combinations thereof.

As used herein, the term "anti-bacterial agent" or "anti-bacterial therapy" refers to an agent that has bactericidal and/or bacteriostatic activity. The anti-bacterial agent can be naturally occurring or synthetic. In some embodiments, an anti-bacterial agent or therapy can comprise an antibiotic, e.g., to suppress the growth of other microorganisms. Non-limiting examples of anti-bacterial agents include β-lactam antibacterial agents including, e.g., ampicillin, cloxacillin, oxacillin, and piperacillin, cephalosporins and other cephems including, e.g., cefaclor, cefamandole, cefazolin, cefoperazone, cefotaxime, cefoxitin, ceftazidime, ceftriaxone, and cephalothin; carbapenems including, e.g., imipenem and meropenem; and glycopeptides, macrolides, quinolones, tetracyclines, and aminoglycosides . In general, if an antibacterial agent is bacteriostatic, it means that the agent essentially stops bacterial cell growth (but does not necessarily kill the bacteria); if the agent is bacteriocidal, it means that the agent kills the bacterial cells (and may stop growth before killing the bacteria).

As used herein, the term "anti-fungal agent" or "anti-fungal therapy" refers to an agent that is able to exert an inhibitory effect on the growth and/or development of a fungus. Such an effect can be classified as fungicidal, fungistatic, sporocidal, sporostatic, or a combination thereof. Examples of anti-fungal agent or therapy include, but are not limited to polyene-based, imidazole-based, triazole-based, thiazole-based, allyalmine-based, echinocandin-based, and a combination of two or more thereof.

In some embodiments, the method can further comprise administering the patient an immunotherapy. As used herein, the term "immunotherapy" refers to a treatment that modifies or affects (e.g., stimulates or suppresses) response and/or number of at least a subset of immune cells. For example, the immunotherapy for treatment of cancer and/or infection can comprise an agent that increases a proinflammatory T cell response and/or an agent that suppresses an anti-inflammatory T cell response.

In some embodiments of this aspect and other related aspects of the present disclosure, the patient can be previously treated with or is being treated an anti-cancer therapy and/or anti-infection therapy. Thus, the anti-TIGIT, anti-Fgl2 and/or anti-IL-33 therapy can be used, alone or in combination with another anti-cancer agent and/or anti-infection agent. In some embodiments of this aspect and other related aspects of the present disclosure, the methods described herein can further comprise administering to the patient a selected therapy (e.g., anti-TIGIT or anti-IL-33 therapy) after they have been identified to be more likely to benefit from one immunotherapy over another.

As used herein, the term "selected therapy" or "selected treatment" refers to a therapy or treatment selected based on the level and/or activity of a target molecule (e.g., TIGIT, Fgl2 and/or IL-33) as measured in a sample of a subject to be treated according to the methods of various aspects disclosed herein. In accordance with one aspect of the discovery that selective suppression by TIGIT+ Tregs is Fgl2-dependent and that IL-33 can induce or expand the Treg population, one can predict the responsiveness of a patient to an agent that modulates TIGIT, Fgl2 and/or IL-33 expression and/or activity, by determining the level of TIGIT, Fgl2 and/or IL-33 expression or activity in the patient's sample, and thus select for the patient an appropriate therapy to which the patient is more likely to respond.

In some embodiments of this aspect and other related aspects of the present disclosure, the TIGIT inhibitor, Fgl2 inhibitor and/or IL-33 inhibitor administered to a patient diagnosed with cancer and/or infection can be constructed to specifically target TIGIT+ regulatory T cells (Tregs) that infiltrate the tumor or infected tissue. For example, for treatment of a patient diagnosed with cancer, the TIGIT inhibitor, Fgl2 inhibitor and/or IL-33 inhibitor can comprise a tumor-targeting moiety. For treatment of a patient diagnosed with infection, the TIGIT inhibitor, Fgl2 inhibitor and/or IL-33 inhibitor can comprise a targeting moiety against infected tissue.

As used herein, the terms "treat," "treatment," "treating," refer to therapeutic treatments, wherein the object is to reverse, alleviate, ameliorate, inhibit, slow down or stop the progression or severity of a condition associated with a disease or disorder. The term "treating" includes reducing or alleviating at least one adverse effect or symptom of a condition, disease or disorder, such as an autoimmune disease, infection or a cancer. Treatment is generally "effective" if one or more symptoms or clinical markers are reduced. Alternatively, treatment is "effective" if the progression of a disease is reduced or halted. That is, "treatment" includes not just the improvement of symptoms or markers, but also a cessation of at least slowing of progress or worsening of symptoms that would be expected in absence of treatment. Beneficial or desired clinical results include, but are not limited to alleviation of one or more symptom(s), diminishment of extent of disease, stabilized (i.e., not worsening) state of disease, delay or slowing of disease progression, amelioration or palliation of the disease state, and remission (whether partial or total), whether detectable or undetectable. The term "treatment" of a disease also includes providing relief from the symptoms or side-effects of the disease (including palliative treatment).

The term "effective amount" as used herein refers to the amount of an agent for modulating expression and/or activity of TIGIT, Fgl2 and/or IL-33 needed to alleviate at least one or more symptom of the disease or disorder, and relates to a sufficient amount of pharmacological composition to provide the desired effect, i.e., promote or inhibit T cell tolerance, for example. The term "therapeutically effective amount" therefore refers to an amount of an agent for modulating expression and/or activity of TIGIT, Fgl2 and/or IL-33 using the methods as disclosed herein, that is sufficient to effect a particular effect when administered to a subject. An effective amount as used herein would also include an amount sufficient to delay the development of a symptom of the disease, alter the course of a symptom of disease (for example but not limited to slow the progression of a symptom of the disease), or reverse a symptom of disease. Thus, it is not possible to specify the exact "effective amount". However, for any given case, an appropriate "effective amount" can be determined by one of ordinary skill in the art using only routine experimentation.

Effective amounts, toxicity, and therapeutic efficacy can be determined by standard pharmaceutical procedures in cell cultures or experimental animals, e.g., for determining the LD50 (the dose lethal to 50% of the population) and the ED50 (the dose therapeutically effective in 50% of the population). The dosage can vary depending upon the dosage form employed and the route of administration utilized. The dose ratio between toxic and therapeutic effects is the therapeutic index and can be expressed as the ratio LD50/ED50. Compositions and methods that exhibit large therapeutic indices are preferred. A therapeutically effective dose can be estimated initially from cell culture assays. Also, a dose can be formulated in animal models to achieve a circulating plasma concentration range that includes the IC50 (i.e., the concentration of the agent for modulating expression and/or activity of TIGIT, Fgl2 and/or IL-33), which achieves a half-maximal inhibition of symptoms) as determined in cell culture, or in an appropriate animal model. Levels in plasma can be measured, for example, by high performance liquid chromatography. The effects of any particular dosage can be monitored by a suitable bioassay. The dosage can be determined by a physician and adjusted, as necessary, to suit observed effects of the treatment.

A "cancer" or "tumor" as used herein refers to an uncontrolled growth of cells which interferes with the normal functioning of the bodily organs and systems. A subject that has a cancer or a tumor is a subject having objectively measurable cancer cells present in the subject's body. Included in this definition are benign and malignant cancers, as well as dormant tumors, metastases, or micrometastases. Cancers which migrate from their original location and seed vital organs can eventually lead to the death of the subject through the functional deterioration of the affected organs. Hemopoietic cancers, such as leukemia, are able to out-compete the normal hemopoietic compartments in a subject, thereby leading to hemopoietic failure (in the form of anemia, thrombocytopenia and neutropenia) ultimately causing death.

By "metastasis" is meant the spread of cancer from its primary site to other places in the body. Cancer cells can break away from a primary tumor, penetrate into lymphatic and blood vessels, circulate through the bloodstream, and grow in a distant focus (metastasize) in normal tissues elsewhere in the body. Metastasis can be local or distant. Metastasis is a sequential process, contingent on tumor cells breaking off from the primary tumor, traveling through the bloodstream, and stopping at a distant site. At the new site, the cells establish a blood supply and can grow to form a life-threatening mass. Both stimulatory and inhibitory molecular pathways within the tumor cell regulate this behavior, and interactions between the tumor cell and host cells in the distant site are also significant.

Metastases are most often detected through the sole or combined use of magnetic resonance imaging (MRI) scans, computed tomography (CT) scans, blood and platelet counts, liver function studies, chest X-rays and bone scans in addition to the monitoring of specific symptoms.

Examples of cancer include, but are not limited to carcinoma, lymphoma, blastoma, sarcoma, and leukemia. More particular examples of such cancers include, but are not limited to, basal cell carcinoma, biliary tract cancer; bladder cancer; bone cancer; brain and CNS cancer; breast cancer; cancer of the peritoneum; cervical cancer; choriocarcinoma; colon and rectum cancer; connective tissue cancer; cancer of the digestive system; endometrial cancer; esophageal cancer; eye cancer; cancer of the head and neck; gastric cancer (including gastrointestinal cancer); glioblastoma; hepatic carcinoma; hepatoma; intra-epithelial neoplasm; kidney or renal cancer; larynx cancer; leukemia; liver cancer; lung cancer (e.g., small-cell lung cancer, non-small cell lung cancer, adenocarcinoma of the lung, and squamous carcinoma of the lung); lymphoma including Hodgkin's and non-Hodgkin's lymphoma; melanoma; myeloma; neuroblastoma; oral cavity cancer (e.g., lip, tongue, mouth, and pharynx); ovarian cancer; pancreatic cancer; prostate cancer; retinoblastoma; rhabdomyosarcoma; rectal cancer; cancer of the respiratory system; salivary gland carcinoma; sarcoma; skin cancer; squamous cell cancer; stomach cancer; testicular cancer; thyroid cancer; uterine or endometrial cancer; cancer of the urinary system; vulval cancer; as well as other carcinomas and sarcomas; as well as B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia); chronic lymphocytic leukemia (CLL); acute lymphoblastic leukemia (ALL); Hairy cell leukemia; chronic myeloblastic leukemia; and post-transplant lymphoproliferative disorder (PTLD), as well as abnormal vascular proliferation associated with phakomatoses, edema (such as that associated with brain tumors), and Meigs' syndrome.

As used herein, the term "chronic viral infection" refers to a viral infection having the provirus or virus material in the nucleus or cytoplasm of a host cell and which, until induced, has little or no detectable viral RNA or protein. Such infections can persist for many years, or even for the lifetime of the infected individual. Examples of chronic viral infection include, but are not limited to, Hepatitis B virus (HBV), hepatitis C virus (HCV), human immunodeficiency virus (HIV) infections, and Herpes viruses.

In some embodiments of various aspects of the present disclosure, at least one or more TIGIT antagonist(s), Fgl2 antagonist(s) and/or IL-33 antagonist(s) can be administered to a patient with a bacterial infection. The bacterial infection can be casused by intracellular bacteria and/or extracellular bacteria. Examples of infectious bacteria include: *Helicobacterpyloris, Borelia burgdorferi, Chlamydia trachomatis, Legionella pneumophilia, Mycobacteria sps (*such as *M. tuberculosis, M. avium, M. intracellulare, M. kansaii, M. gordonae), Staphylococcus aureus, Neisseria gonorrhoeae, Neisseria meningitidis, Listeria monocytogenes, Streptococcus pyogenes* (Group A *Streptococcus), Streptococcus agalactiae* (Group B *Streptococcus), Streptococcus (viridans group), Streptococcus faecalis, Streptococcus bovis, Streptococcus (anaerobic sps.), Streptococcus pneumoniae, pathogenic Campylobacter sp., Enterococcus sp., Haemophilus influenzae, Bacillus anthracis, corynebacterium diphtheriae, corynebacterium sp., Erysipelothrix rhusiopathiae, Clostridium perfringens, Clostridium tetani, Enterobacter aerogenes, Klebsiella pneumoniae, Pasturella multocida, Bacteroides sp., Fusobacterium nucleatum, Streptobacillus moniliformis, Treponema pallidium, Treponema pertenue, Leptospira, and Actinomyces israelli.* The compositions and methods described herein are contemplated for use in treating infections caused by these bacterial agents.

In some embodiments of various aspects of the present disclosure, at least one or more TIGIT antagonist(s), Fgl2 antagonist(s) and/or IL-33 antagonist(s) can be administered to a patient with a viral infection. In some embodiments, the viral infection is a chronic viral infection. Examples of infectious viruses include: *Retroviridae* (for example, HIV); *Picornaviridae* (for example, polio viruses, hepatitis A virus; enteroviruses, human coxsackie viruses, rhinoviruses, echoviruses); *Calciviridae* (such as strains that cause gastroenteritis); *Togaviridae* (for example, equine encephalitis viruses, rubella viruses); *Flaviridae* (for example, dengue viruses, encephalitis viruses, yellow fever viruses); *Coronaviridae* (for example, coronaviruses); *Rhabdoviridae* (for example, vesicular stomatitis viruses, rabies viruses); *Filoviridae* (for example, ebola viruses); *Paramyxoviridae* (for example, parainfluenza viruses, mumps virus, measles virus, respiratory syncytial virus); *Orthomyxoviridae* (for example, influenza viruses); *Bungaviridae* (for example, Hantaan viruses, bunga viruses, phleboviruses and Nairo viruses); *Arena viridae* (hemorrhagic fever viruses); Reoviridae (e.g., reoviruses, orbiviurses and rotaviruses); *Birnaviridae; Hepadnaviridae* (Hepatitis B virus); *Parvoviridae* (parvoviruses); *Papovaviridae* (papilloma viruses, polyoma viruses); *Adenoviridae* (most adenoviruses); *Herpesviridae* (herpes simplex virus (HSV) 1 and HSV-2, varicella zoster virus, cytomegalovirus (CMV), herpes viruses); *Poxviridae* (variola viruses, vaccinia viruses, pox viruses); and *Iridoviridae* (such as African swine fever virus); and unclassified viruses (for example, the etiological agents of Spongiform encephalopathies, the agent of delta hepatitis (thought to be a defective satellite of hepatitis B virus), the agents of non-A, non-B hepatitis (class 1=internally transmitted; class 2=parenterally transmitted (i.e., Hepatitis C); Norwalk and related viruses, and astroviruses). The compositions and methods described herein are contemplated for use in treating infections caused by these viral agents.

In some embodiments of various aspects of the present disclosure, at least one or more TIGIT antagonist(s), Fgl2 antagonist(s) and/or IL-33 antagonist(s) can be administered to a patient with a fungal infection. The compositions and methods described herein that dampen Th2 responses are contemplated for use in treating infections caused by fungi agents. Examples of fungal infections include but are not limited to: *aspergillosis; thrush (caused by Candida albicans); cryptococcosis (caused by Cryptococcus); and histoplasmosis. Thus, examples of infectious fungi include, but are not limited to, Cryptococcus neoformans, Histoplasma capsulatum, Coccidioides immitis, Blastomyces dermatitidis, Candida albicans.* The compositions and methods described herein that promote Th17 cell activity or responses are contemplated for use in treating infections with these fungal agents.

**CD8+ T cell exhaustion**: It is contemplated that TIGIT signaling can play a role in establishing or maintain T cell exhaustion. A further aspect of the present disclosure relates to a method for increasing the differentiation and/or proliferation of functionally exhausted CD8+ T cells, or decreasing CD8+ T cell exhaustion, in a subject in need thereof. The method comprises administering to the subject in need thereof a pharmaceutical composition comprising a TIGIT antagonist or inhibitor described herein.

As used herein, the term "T cell exhaustion" refers to a state of T cell dysfunction. The T cell exhaustion generally arises during many chronic infections and cancer. T cell exhaustion can be defined by poor effector function, sustained expression of inhibitory receptors, and/or a transcriptional state distinct from that of functional effector or memory T cells. T cell exhaustion generally prevents optimal control of infection and tumors. See, e.g., Wherry EJ, Nat Immunol. (2011) 12: 492-499, for additional information about T cell exhaustion.

In some embodiments, the subject in need thereof of can be diagnosed with cancer. In some embodiments, the subject diagnosed with cancer has been receiving a cancer therapy, including, e.g., vaccine, chemotherapy, targeted therapy (e.g., kinase inhibitors), radiation therapy, surgery, immunotherapy, or any combination thereof.

In some embodiments, the subject in need thereof can be diagnosed with infection, e.g., but not limited to chronic viral infection, intracellular bacterial infection, extracellular bacterial infection, and/or fungal infection.

In some embodiments, the subject in need thereof can be diagnosed with chronic infection, e.g., chronic viral infection.

When the TIGIT antagonist administered to the subject is determined to be ineffective (e.g., no significant decrease in the level of TIGIT and/or Fgl2 expression and/or activity relative to a reference), the subject can be administered with an alternative therapy that suppresses anti-inflammatory T cell response pathway. Non-limiting examples of such alternative therapy include a TIM-3 antagonist, a PD-1 antagonist, a PD-L1 antagonist, a CTLA-4 antagonist, a Lag-3 antagonist, a BTLA antagonist, and any combinations thereof.

### Methods and compositions for treating autoimmune diseases and other immune-related diseases where an inhibition of Th1 and/or Th17 responses and/or a shift of balance toward a Th2 response is desirable

In some other immune-related diseases or disorders, e.g., but not limited to, inflammatory diseases or disorders such as parasitic infections and autoimmune diseases, it can be desirable to suppress proinflammatory Th1 and/or Th17 responses for a therapeutic effect, while sparing or promoting a Th2 response. Accordingly, these inflammatory diseases or disorders where an inhibition of Th1 and/or Th17 responses and/or a shift of balance toward a Th2 response is desirable can be treated by enhancing or stimulating the expression or activity of TIGIT, Fgl2 and/or IL-33 (or promoting the TIGIT axis signaling).

Accordingly, in some aspects of the present disclosure are methods of identifying a patient diagnosed to have an inflammatory disease or disorder who is more likely to be responsive to an anti-inflammatory immunotherapy, or a TIGIT agonist, Fgl2 agonist and/or IL-33 agonist therapy. Nonlimiting examples of an inflammatory disease or disorder where an inhibition of Th1 and/or Th17 responses and/or a shift of balance toward a Th2 response is desirable include autoimmune disease, parasitic infection, acute inflammation, chronic inflammation, and any combinations thereof. In some embodiments, patients having an inflammatory disease or disorder where an inhibition of Th1 and/or Th17 responses and/or a shift of balance toward a Th2 response is desirable can be identified as more likely to be responsive to a TIGIT agonist and/or an IL-33 agonist therapy based on the patients' level of Fgl2 activity or expression in a sample. In some embodiments, patients having an inflammatory disease or disorder where an inhibition of Th1 and/or Th17 responses and/or a shift of balance toward a Th2 response is desirable can be identified as more likely to be responsive to an IL-33 agonist therapy and/or an Fgl2 agonist therapy based on the patients' level of TIGIT activity or expression in a sample. In some embodiments, patients having an inflammatory disease or disorder where an inhibition of Th1 and/or Th17 responses and/or a shift of balance toward a Th2 response is desirable can be identified as more likely to be responsive to a TIGIT agonist and/or an Fgl2 agonist therapy based on the patients' level of IL-33 activity or expression in a sample.

By way of example only, some aspects disclosed herein relate to methods of identifying a patient with an inflammatory disease or disorder, who is more likely to be responsive to a TIGIT agonist and/or IL-33 agonist therapy, based on the level of Fgl2 activity or expression in the patient's sample. In some embodiments, the methods are directed to patients with inflammatory diseases or disorders where an inhibition of Th1 and/or Th17 responses and/or a shift of balance toward a Th2 response is desirable. The method comprises (a) measuring the level of Fgl2 activity or expression in a sample from a patient diagnosed to have an inflammatory disease or disorder where an inhibition of Th1 and/or Th17 responses and/or a shift of balance toward a Th2 response is desirable, e.g., autoimmune disease and/or parasitic infection; and (b) comparing the level of Fgl2 or expression in the sample with an Fgl2 reference; and: (i) when the level of Fgl2 activity or expression is lower than the Fgl2 reference, the patient is identified to be more likely to be responsive to a TIGIT agonist and/or IL-33 agonist therapy; or (ii) when the level of Fgl2 activity or expression is the same as or greater than the Fgl2 reference, the patient is identified as likely to respond to an alternative, anti-inflammatory immunotherapy comprising an activator of an anti-inflammatory T cell response pathway and/or a suppressor of a proinflammatory T cell response pathway.

In this aspect and other aspects of the present disclosure, any appropriate modulators of a T cell response pathway that are known in the art can be used in the alternative immunotherapy for patients with an inflammatory disease or disorder where an inhibition of Th1 and/or Th17 responses and/or a shift of balance toward a Th2 response is desirable, e.g., autoimmune diseases and/or parasitic infection. For example, activators of an anti-inflammatory T cell response or suppressors of a proinflammatory T-cell response pathway can comprise a TIM-3 agonist, a galectin-9 molecule, a PD-1 agonist, a PD-L1 agonist, a CTLA-4 agonist, a Lag-3 agonist, a DD1α agonist, an antagonist of an immune checkpoint activating molecule, an agonist of an immune checkpoint inhibitory molecule, or any combination thereof.

Some aspects of the present disclosure relate to methods of treating a patient who is determined to have an inflammatory disease or disorder. A TIGIT agonist, Fgl2 agonist and/or IL-33 agonist therapy can be selected for administration to a patient with an inflammatory disease or disorder where an inhibition of Th1 and/or Th17 responses and/or a shift of balance toward Th2 responses is desirable, based on the level of Fgl2, IL-33 and/or TIGIT in the patient's sample. In some embodiments, a TIGIT agonist and/or IL-33 agonist therapy can be selected for administration to a patient with an inflammatory disease or disorder where an inhibition of Th1 and/or Th17 responses and/or a shift of balance toward Th2 responses is desirable, based on the patient's level of Fgl2 activity or expression in a sample. In some embodiments, an IL-33 agonist therapy and/or Fgl2 agonist therapy can be selected for administration to a patient with an inflammatory disease or disorder where an inhibition of Th1 and/or Th17 responses and/or a shift of balance toward Th2 responses is desirable, based on the patient's level of TIGIT activity or expression in a sample. In some embodiments, a TIGIT agonist and/or Fgl2 agonist therapy can be selected for administration to a patient with an inflammatory disease or disorder where an inhibition of Th1 and/or Th17 responses and/or a shift of balance toward Th2 responses is desirable, based on the patient's level of IL-33 activity or expression in a sample.

For example, some aspects of the present disclosure relate to methods for treating a patient who is determined to have an inflammatory disease or disorder based on the level of IL-33 activity or expression in a sample from the patient. In some embodiments where an inhibition of Th1 and/or Th17 responses and/or a shift of balance toward Th2 responses is desirable, the method comprises (a) measuring the level of IL-33 activity or expression in a sample from a patient who is determined to have this type of an inflammatory disease or disorder ; (b) comparing the level of IL-33 activity or expression in the sample with an IL-33 reference, and (c) performing one of the following actions:
(i) administering to the patient a composition comprising a TIGIT agonist and/or an Fgl2 agonist, when the level of IL-33 activity or expression is lower than the IL-33 reference;
(ii) administering an alternative, anti-inflammatory immunotherapy treatment without the TIGIT agonist or Fgl2 agonist, when the level of IL-33 activity or expression is the same as or greater than the IL-33 reference; or
(iii) determining if the level of at least one other activating immune regulator in the sample is greater than the level of the corresponding reference, or if the level of at least one inhibitory immune regulator in the sample is less than the level of the corresponding reference, when the level of IL-33 activity or expression is the same as or greater than the IL-33 reference. Examples of inhibitory immune regulator include, but are not limited to, Fgl2, TIGIT, ST2, CD155, CD112, PD-1, PD-L1, DD1α, TIM-3, galectin-9, CTLA-4, Lag-3, and any combination thereof.

In some embodiments where the level of IL-33 activity or expression is the same as or greater than the IL-33 reference, the method can further comprise (a) measuring the level of TIGIT activity or expression, or frequency of TIGIT+ T cells in a sample from the patient, and (b) comparing the level of TIGIT activity or expression, or frequency of TIGIT+ T cells in the sample with a TIGIT reference. If the level of TIGIT activity or expression, or frequency of TIGIT+ T cells is low relative to a TIGIT reference, an increase in TIGIT via the TIGIT axis signaling can help inflammatory conditions where an inhibition of Th1 and/or Th17 responses and/or a shift of balance toward Th2 responses is desirable. For example, a composition comprising a TIGIT agonist and/or an Fgl2 agonist can be administered to such a patient when the level of TIGIT activity or expression, or frequency of TIGIT+ cells is lower than the TIGIT reference. However, if the level of TIGIT activity or expression, or frequency of TIGIT+ T cells is the same or high relative to a TIGIT reference, an alternative, anti-inflammatory immunotherapy treatment without a TIGIT agonist or Fgl2 agonist can be administered.

In some embodiments where the level of IL-33 activity or expression is the same as or greater than the IL-33 reference, the method can further comprise (a) measuring the level of Fgl2 activity or expression in a sample from the patient, and (b) comparing the level of Fgl2 activity or expression in the sample with an Fgl2 reference. The patient can be administered with a composition comprising a TIGIT agonist and/or an Fgl2 agonist, when the level of Fgl2 activity or expression is lower than the Fgl2 reference; or with an alternative, anti-inflammatory immunotherapy treatment without a TIGIT agonist or Fgl2 agonist, when the level of Fgl2 activity or expression is the same as or greater than the reference.

In some embodiments where the level of IL-33 and/or Fgl2 activity or expression is the same as or greater than the reference, an alternative, anti-inflammatory immunotherapy treatment without the TIGIT agonist or Fgl2 agonist to be administered can be a therapy comprising an activator of an anti-inflammatory T cell response pathway and/or a suppressor of a proinflammatory T cell response pathway.

In some other aspects of the present disclosure are methods of treating a patient determined to have an inflammatory disease or disorder based on the level of Fgl2 activity or expression in the patient's sample. In some embodiments where an inhibition of Th1 and/or Th17 responses and/or a shift of balance toward Th2 responses is desirable, the method comprises (a) measuring the level of Fgl2 activity or expression in a sample from a patient determined to have an inflammatory disease or disorder; (b) comparing the level of Fgl2 activity or expression in the sample with an Fgl2 reference; and (c) administering to the patient a composition comprising a TIGIT agonist and/or an IL-33 agonist when the level of Fgl2 activity or expression is lower than the Fgl2 reference, or administering an alternative, anti-inflammatory immunotherapy treatment without a TIGIT agonist or IL-33 agonist when the level of Fgl2 activity or expression is the same as or greater than the Fgl2 reference. In some embodiments, the alternative, anti-inflammatory immunotherapy treatment without a TIGIT agonist or IL-33 agonist can be a therapy comprising an activator of an anti-inflammatory T cell response pathway and/or a suppressor of a proinflammatory T cell response pathway.

In some embodiments, the patient with an Fgl2 level lower than the Fgl2 reference can be further administered a therapy comprising an activator of an anti-inflammatory T cell response pathway and/or a suppressor of a proinflammatory T cell response pathway.

In some aspects of the present disclosure, TIGIT, Fgl2 and/or IL-33 can be used as a marker to determine or monitor the efficacy of a TIGIT agonist, Fgl2 agonist and/or IL-33 agonist therapy administered to a patient diagnosed to have an inflammatory disease or disorder where an inhibition of Th1 and/or Th17 responses and/or a shift of balance toward Th2 responses is desirable. In some embodiments, Fgl2 can be used as a predictive marker to determine or monitor the efficacy of a TIGIT agonist and/or IL-33 agonist therapy administered to a patient diagnosed to have an inflammatory disease or disorder where an inhibition of Th1 and/or Th17 responses and/or a shift of balance toward Th2 responses is desirable. In some embodiments, TIGIT can be used as a predictive marker to determine or monitor the efficacy of an IL-33 agonist therapy and/or Fgl2 agonist therapy administered to a patient diagnosed to have an inflammatory disease or disorder where an inhibition of Th1 and/or Th17 responses and/or a shift of balance toward Th2 responses is desirable. In some embodiments, IL-33 can be used as a predictive marker to determine or monitor the efficacy of a TIGIT agonist and/or Fgl2 agonist therapy administered to a patient diagnosed to have an inflammatory disease or disorder where an inhibition of Th1 and/or Th17 responses and/or a shift of balance toward Th2 responses is desirable.

As an example, methods of treating a patient having an inflammatory disease or disorder and a low level of Fgl2 are provided herein. In some embodiments where an inhibition of Th1 and/or Th17 responses and/or a shift of balance toward Th2 responses is desirable, the method comprises: (a) determining a first level of Fgl2 expression or activity in a sample from a patient having an inflammatory disease or disorder and a low level of Fgl2; (b) administering an agent that activates IL-33 activity and/or TIGIT activity; (c) determining a second level of Fgl2 expression or activity after the administering; and (d) comparing the first and second levels of Fgl2 expression or activity, wherein the agent administered in (b) is effective if the second level of Fgl2 expression or activity is greater than the first level, and wherein the agent administered in (b) is ineffective if the second level of Fgl2 expression is the same as or lower than the first level.

By monitoring the effects of the IL-33 agonist and/or TIGIT agonist therapy on the level of Fgl2 expression or activity, one can determine the efficacy of the treatment regimen and adjust the treatment regimen if necessary. Accordingly, in some embodiments, the method can further comprise, when the IL-33 agonist or TIGIT agonist therapy is effective, continuing to administer the agent that activates IL-33 activity and/or TIGIT activity. In some embodiments, the method can further comprise, when the IL-33 agonist therapy or the TIGIT agonist therapy is ineffective, administering the agent that activates IL-33 activity and/or TIGIT activity at a higher dose. In some embodiments, the method can further comprise, when the IL-33 agonist therapy or the TIGIT agonist therapy is ineffective, discontinuing the IL-33 agonist therapy or the TIGIT agonist therapy. In these embodiments, the method can further comprise administering a therapy comprising an activator of an anti-inflammatory T cell response pathway and/or a suppressor of a proinflammatory T cell response pathway.

Similarly, a further aspect of the present disclosure relates to methods of treating a patient having an inflammatory disease or disorder that exhibits a reduced level of IL-33. In some embodiments where an inhibition of Th1 and/or Th17 responses and/or a shift of balance toward Th2 responses is desirable, the method comprises: (a) determining a first level of TIGIT and/or Fgl2 expression or activity in a sample from a patient having an inflammatory disease or disorder with a reduced level of IL-33; (b) administering an agent that activates IL-33 activity; (c) determining a second level of TIGIT or Fgl2 expression or activity after the administering; and (d) comparing the first and second levels of TIGIT and/or Fgl2 expression or activity, wherein IL-33 agonist therapy is effective if the second level of TIGIT and/or Fgl2 expression or activity is greater that the first level, and wherein IL-33 agonist therapy is ineffective if the second level of TIGIT and/or Fgl2 expression is the same as or lower than the first level.

In some embodiments, the method can further comprise, when the IL-33 agonist therapy is effective, continuing to administer the agent that activates IL-33 activity. In some embodiments, the method can further comprise, when the IL-33 agonist therapy is ineffective, administering the agent that activates IL-33 activity at a higher dose. In other embodiments, the method can further comprise, when the IL-33 agonist therapy is ineffective, discontinuing the IL-33 agonist therapy. In these embodiments, the method can further comprise administering a therapy comprising an activator of an anti-inflammatory T cell response pathway and/or a suppressor of a proinflammatory T cell response pathway.

In yet another aspect of the present disclousre, methods of treating a patient determined to have an inflammatory disease or disorder comprising administering to a patient determined to have an inflammatory disease or disorder one or more embodiments of the pharmaceutical compositions described herein are also provided. The pharmaceutical composition can be taken alone or in combination with another agent for treatment of an inflammatory disease or disorder. An exemplary anti-inflammatory agent includes, but is not limited to, an immunotherapy. In some embodiments, the method can further comprise administering the patient an immunotherapy for treatment of an inflammatory disease or disorder. For example, the immunotherapy can comprise an agent that activates an anti-inflammatory T cell response and/or an agent that suppresses a proinflammatory T cell response.

In some embodiments of this aspect and other related aspects of the present disclosure, the patient having an inflammatory disease or disorder can be previously treated with or is being treated an anti-inflammatory therapy. Thus, the TIGIT agonist, Fgl2 agonist and/or IL-33 agonist therapy can be used, alone or in combination with another anti-inflammatory agent. In some embodiments of this aspect and other related aspects of the present disclosure, the methods described herein can further comprise administering to the patient a selected therapy (e.g., TIGIT agonist or IL-33 agonist therapy) after they have been identified to be more likely to benefit from one immunotherapy over another.

In some embodiments of this aspect and other related aspects of the present disclosure, the TIGIT agonist, Fgl2 agonist and/or IL-33 agonist administered to a patient can be constructed or adapted to specifically target TIGIT+ regulatory T cells (Tregs). For example, the TIGIT agonist, Fgl2 agonist and/or IL-33 agonist can comprise a cell-targeting moiety.

As used herein, the term "cell-targeting moiety" refers to a molecule or entity that facilitates delivery of an agent to a target cell. For example, a cell-targeting moiety can be a molecule or entity that interacts with a binding site on the surface of a target cell. Thus, the targeting moiety provides specificity or binding affinity for one or more cell types. The molecule on a target cell which is targeted by the targeting moiety can be any selected target, for instance a cell surface receptor. Cell-targeting moieties include, but are not limited to, antibodies, antigen-binding antibody fragments, ligands for a cell-surface receptor, viral surface components, proteins that bind viral surface components, growth factors, lectins, carbohydrates, fatty acids or other hydrophobic substituents, peptides and peptidomimetic molecules. In one embodiment, the cell-targeting moiety is a molecule or entity that interacts with a binding site on the surface of a TIGIT+ regulatory T cell.

In some embodiments of this aspect and other related aspects of the present disclosure, the TIGIT agonist, Fgl2 agonist and/or IL-33 agonist can be co-administered with an agent, such as a chemokine, that promotes recruitment of TIGIT+ regulatory T cells (Tregs) to an inflammatory site or tissue.

In some embodiments of various aspects of the present disclosure, the TIGIT agonist, Fgl2 agonist and/or IL-33 agonist can be administered to a patient with an autoimmune disease. "Autoimmune disease" refers to a class of diseases in which a subject's own antibodies react with host tissue or in which immune effector T cells are autoreactive to endogenous self-peptides and cause destruction of tissue. Thus an immune response is mounted against a subject's own antigens, referred to as self-antigens. A "self-antigen" as used herein refers to an antigen of a normal host tissue. Normal host tissue does not include cancer cells.

Accordingly, in some embodiments, the autoimmune diseases to be treated or prevented using the methods described herein, include, but are not limited to: rheumatoid arthritis, Crohn's disease, multiple sclerosis, systemic lupus erythematosus (SLE), autoimmune encephalomyelitis, myasthenia gravis (MG), Hashimoto's thyroiditis, Goodpasture's syndrome, pemphigus (e.g., pemphigus vulgaris), Grave's disease, autoimmune hemolytic anemia, autoimmune thrombocytopenic purpura, scleroderma with anti-collagen antibodies, mixed connective tissue disease, polymyositis, pernicious anemia, idiopathic Addison's disease, autoimmune- associated infertility, glomerulonephritis (e.g., crescentic glomerulonephritis, proliferative glomerulonephritis), bullous pemphigoid, Sjogren's syndrome, insulin resistance, and autoimmune diabetes mellitus (type 1 diabetes mellitus; insulin- dependent diabetes mellitus). Autoimmune disease has been recognized also to encompass atherosclerosis and Alzheimer's disease. In one embodiment of the aspects disclosed herein, the autoimmune disease is selected from the group consisting of multiple sclerosis, type-I diabetes, Hashinoto's thyroiditis, Crohn's disease, rheumatoid arthritis, systemic lupus erythematosus, gastritis, autoimmune hepatitis, hemolytic anemia, autoimmune hemophilia, autoimmune lymphoproliferative syndrome (ALPS), autoimmune uveoretinitis, glomerulonephritis, Guillain-Barre syndrome, psoriasis and myasthenia gravis.

In some embodiments of various aspects disclosed herein, the TIGIT agonist, Fgl2 agonist and/or IL-33 agonist can be administered to a patient having an infection with a pathogen, such as a parasite. In some embodiments of these aspects and all such aspects of the present disclosure, the subject has a chronic infection.

The compositions and methods described herein that promote suppression of Th1 and/or Th17 responses and thus shift the balance toward Th2 responses are contemplated for the treatment of infection caused by parasites (e.g., helmiths among others) and intracellular pathogens. Other infectious organisms (such as protists) include: *Plasmodium falciparum* and *Toxoplasma gondii.*

### Methods for treating asthma, allergy, and/or atopy

Without wishing to be bound by theory, TIGIT can promote allergy/asthma/atopy, e.g., by inducing the level of expression and/or activity of Fgl2, thereby suppressing the Th1 and/or Th17 versus Th2 balance in favor of Th2 cytokine responses. Accordingly, it is also contemplated that other inflammatory diseases or disorders, including, e.g., allergy/asthma/atopy, where a dampening of the Th2 response is desirable, could be treated by downregulating the expression or activity of TIGIT, Fgl2 and/or IL-33 (or suppressing the TIGIT axis signaling).

In one aspect of the present disclosure is a method for treating asthma, allergy, and/or atopy. The method comprises administering to a patient diagnosed with asthma, allergy, and/or atopy a composition comprising an anti-Fgl2 therapy. In some embodiments, the method can further comprise identifying a patient diagnosed with asthma, allergy, and/or atopy who is more likely to respond to an anti-Fgl2 therapy, e.g., based on the level of expression and/or activity of TIGIT and/or IL-33. When the level of TIGIT and/or IL-33 activity or expression is greater than the TIGIT and/or IL-33 reference, the patient is identified to be more likely to be responsive to an anti-Fgl2 therapy; or (ii) when the level of TIGIT and/or IL-33 activity or expression is the same as or less than the TIGIT and/or IL-33 reference, the patient is identified as likely to respond to an alternative, Th2-dampening therapy or immunotherapy. In some embodiments, the alternative, Th2-dampening therapy or immunotherapy can comprise, e.g., an activator of proinflammatory T cell response pathway and/or a suppressor of an anti-inflammatory T cell response pathway.

Generally, methods and compositions for treatment of cancer and/or infections described herein that stimulate Th1 and/or Th17 responses and thus shift the balance away from Th2 responses can be adapted accordingly for treatment of allergy, asthma and/or atopy, where a dampening Th2 response is desirable.

Accordingly, in some aspects of the present disclosure are methods for guiding selection of a treatment for a subject diagnosed with asthma, allergy, and/or atopy. In some embodiments, patients diagnosed with asthma, allergy, and/or atopy can be identified as more likely to be responsive to an anti-TIGIT and/or anti-IL-33 therapy based on the patients' level of Fgl2 activity or expression in a sample. In some embodiments, patients diagnosed with asthma, allergy, and/or atopy can be identified as more likely to be responsive to an anti-IL-33 therapy and/or anti-Fgl2 therapy based on the patients' level of TIGIT activity or expression in a sample. In some embodiments, patients diagnosed with asthma, allergy, and/or atopy can be identified as more likely to be responsive to an anti-TIGIT and/or anti-Fgl2 therapy based on the patients' level of IL-33 activity or expression in a sample.

By way of example only, one aspect disclosed herein relates to methods for guiding selection of a treatment for a subject diagnosed with asthma, allergy, and/or atopy, based on the level of Fgl2 activity or expression in the patient's sample. The method comprises (a) measuring the level of Fgl2 activity or expression in a sample from a patient diagnosed with asthma, allergy, and/or atopy; and (b) comparing the level of Fgl2 or expression in the sample with an Fgl2 reference; and: (i) when the level of Fgl2 activity or expression is greater than the Fgl2 reference, the patient is identified to be more likely to be responsive to an anti-TIGIT and/or anti-IL-33 therapy; or (ii) when the level of Fgl2 activity or expression is the same as or less than the Fgl2 reference, the patient is identified as likely to respond to an alternative, Th2-dampening therapy or immunotherapy. In some embodiments, the alternative, Th2-dampening therapy or immunotherapy can comprise, e.g., an activator of proinflammatory T cell response pathway and/or a suppressor of an anti-inflammatory T cell response pathway.

Accordingly, in some embodiments, the Fgl2 reference can correspond to the level of expression or activity of Fgl2 in a normal healthy subject. In some embodiments, the Fgl2 reference can correspond to the level of expression or activity of Fgl2 in a normal tissue of the same type or lineage as a blood or tissue biopsy obtained from a patient. The normal tissue of the same type or lineage can be obtained from the same or a different patient. In some embodiments, the Fgl2 reference can correspond to a threshold level of expression or activity of Fgl2, above which the level of Fgl2 expression activity measured in a sample from a patient diagnosed with asthma, allergy, and/or atopy would indicate the likelihood of the patient to respond to a treatment. When the level of Fgl2 activity or expression is greater than the Fgl2 reference, e.g., by at least about 10% or more, including, e.g., at least about 20%, at least about 30%, at least about 40%, at least about 50%, at least about 60%, at least about 70%, at least about 80%, at least about 90%, at least about 100% or more, the patient diagnosed with asthma, allergy, and/or atopy is identified to be more likely to be responsive to an anti-TIGIT and/or anti-IL-33 therapy. In some embodiments, when the level of Fgl2 activity or expression is greater than the Fgl2 reference, e.g., by at least about 1.1-fold or more, including, e.g., at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold or more, the patient diagnosed with asthma, allergy, and/or atopy is identified to be more likely to be responsive to an anti-TIGIT and/or anti-IL-33 therapy. On the other hand, when the level of Fgl2 activity or expression is substantially the same as or less than the Fgl2 reference, e.g., by at least about 10% or more, including, e.g., at least about 20%, at least about 30%, at least about 40%, at least about 50%, at least about 60%, at least about 70%, at least about 80%, at least about 90%, or more, the patient diagnosed with asthma, allergy, and/or atopy is identified as likely to respond to an alternative, Th2-dampening therapy or immunotherapy without the anti-TIGIT and/or anti-IL-33 therapy. In some embodiments, the alternative, Th2-dampening therapy or immunotherapy can comprise, e.g., an activator of a proinflammatory T cell response pathway and/or a suppressor of an anti-inflammatory T cell response pathway, e.g., without the need to suppress TIGIT, Fgl2, or IL-33 activity.

As used herein, the term "Th2-dampening T cell response" refers to a response resulting in reduced production of Th2 cytokines by T cells. In some embodiments, the Th2-dampening T cell response can encompass promoting Th1 and/or Th17 responses.

In some embodiments, the methods can further comprise administering to the patient the selected treatment. Accordingly, methods for treating a patient diagnosed with asthma, allergy and/or atopy are also provided herein.

For example, in addition to using Fgl2 as a diagnostic marker, another aspect of the present disclousre relates to a method for treating a patient diagnosed with asthma, allergy, and/or atopy, wherein the method comprises (a) measuring the level of IL-33 activity or expression in a sample from a patient diagnosed with asthma, allergy, and/or atopy; (b) comparing the level of IL-33 activity or expression in the sample with an IL-33 reference, and (c) performing one of the following actions:
(i) administering to the patient a composition comprising a TIGIT inhibitor and/or an Fgl2 inhibitor, when the level of IL-33 activity or expression is greater than the IL-33 reference;
(ii) administering an alternative, Th2-dampening therapy or immunotherapy without the TIGIT inhibitor or Fgl2 inhibitor, when the level of IL-33 activity or expression is the same as or less than the IL-33 reference; or
(iii) determining if the level of at least one other inhibitory immune regulator in the sample is greater than the level of the corresponding reference, or if the level of at least one activating immune regulator in the sample is less than the level of the corresponding reference, when the level of IL-33 activity or expression is the same as or less than the IL-33 reference.

Examples of inhibitory immune regulators include, but are not limited to Fgl2, TIGIT, ST2, CD155, CD112, PD-1, PD-L1, DD1α, TIM-3, galectin-9, CTLA-4, Lag-3, and any combination thereof. Examples of activating immune regulators include, but are not limited to CD28, ICOS, 4-1BB, OX40, CD27, and any combination thereof.
In some embodiments, the IL-33 reference can correspond to the level of expression or activity of IL-33 in a normal healthy subject. In some embodiments, the IL-33 reference can correspond to the level of expression or activity of IL-33 in normal blood or a normal tissue of the same type or lineage as a tissue biopsy obtained from a patient. The normal tissue of the same type or lineage can be obtained from the same or a different patient. In some embodiments, the IL-33 reference can correspond to the level of expression or activity of IL-33 in a patient's sample obtained at a different or prior time point. In some embodiments, the IL-33 reference can correspond to a threshold level of expression or activity of IL-33, above which the level of IL-33 expression or activity measured in a sample from a patient diagnosed with asthma, allergy, and/or atopy would indicate the likelihood of the patient to respond to a treatment. When the level of IL-33 activity or expression is greater than the IL-33 reference, e.g., by at least about 10% or more, including, e.g., at least about 20%, at least about 30%, at least about 40%, at least about 50%, at least about 60%, at least about 70%, at least about 80%, at least about 90%, at least about 100% or more, the patient diagnosed with asthma, allergy, and/or atopy is identified to be more likely to be responsive to a TIGIT inhibitor and/or Fgl2 inhibitor. In some embodiments, when the level of IL-33 activity or expression is greater than the IL-33 reference, e.g., by at least about 1.1-fold or more, including, e.g., at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold or more, the patient diagnosed with asthma, allergy, and/or atopy is identified to be more likely to be responsive to a TIGIT inhibitor and/or Fgl2 inhibitor. On the other hand, when the level of IL-33 activity or expression is substantially the same as or less than the IL-33 reference, e.g., by at least about 10% or more, including, e.g., at least about 20%, at least about 30%, at least about 40%, at least about 50%, at least about 60%, at least about 70%, at least about 80%, at least about 90%, or more, the patient diagnosed with asthma, allergy, and/or atopy is identified as likely to respond to an alternative, Th2-dampening therapy or immunotherapy. In some embodiments, the alternative, Th2-dampening therapy or immunotherapy can comprise, e.g., an activator of a pro-inflammatory T cell response pathway and/or a suppressor of an anti-inflammatory T cell response pathway, without the need to suppress the TIGIT axis.

In some embodiments where the level of IL-33 activity or expression is the same as or less than the IL-33 reference, the method can further comprise (a) measuring the level of Fgl2 activity or expression in a sample from the patient diagnosed with asthma, allergy, and/or atopy, (b) comparing the level of Fgl2 activity or expression in the sample with an Fgl2 reference, and (c) administering to the patient a composition comprising a TIGIT inhibitor and/or an Fgl2 inhibitor, when the level of Fgl2 activity or expression is greater than the Fgl2 reference (e.g., by at least about 30% or more, including, e.g., at least about 1.1-fold, at least about 1.5-fold, at least about 2-fold or higher); or administering an alternative, Th2-dampening therapy or immunotherapy without a TIGIT inhibitor or Fgl2 inhibitor, when the level of Fgl2 activity or expression is the same as or less than the reference (e.g., by at least about 30% or more).

In some embodiments where the level of IL-33 and/or Fgl2 activity or expression is the same as or less than the reference (e.g., by at least about 30% or more, including, e.g., at least about 40%, at least about 50%, at least about 60%, at least about 70%, at least about 80%, at least about 90%, at least about 95% or more), a Th2-dampening therapy or immunotherapy without a TIGIT inhibitor or Fgl2 inhibitor can also be administered. In some embodiments, the alternative, Th2-dampening therapy or immunotherapy can comprise, e.g., an activator of a pro-inflammatory T cell response pathway and/or a suppressor of an anti-inflammatory T cell response pathway as described earlier.

In some aspects of the present disclosure, TIGIT, Fgl2 and/or IL-33 can be used as a marker to determine or monitor the efficacy of an anti-TIGIT, anti-Fgl2 and/or anti-IL-33 therapy administered to a patient diagnosed with asthma, allergy, and/or atopy. In some embodiments, Fgl2 can be used as a predictive marker to determine or monitor the efficacy of an anti-TIGIT and/or anti-IL-33 therapy administered to a patient diagnosed with asthma, allergy, and/or atopy. In some embodiments, TIGIT can be used as a predictive marker to determine or monitor the efficacy of an anti-IL-33 therapy and/or anti-Fgl2 therapy administered to a patient diagnosed with asthma, allergy, and/or atopy. In some embodiments, IL-33 can be used as a predictive marker to determine or monitor the efficacy of an anti-TIGIT and/or anti-Fgl2 therapy administered to a patient diagnosed with asthma, allergy, and/or atopy.

As an example, methods of treating a patient diagnosed with asthma, allergy, and/or atopy that has an elevated level of Fgl2 are provided herein. The method comprises: (a) determining a first level of Fgl2 expression or activity in a sample from a patient diagnosed with asthma, allergy, and/or atopy that has an elevated level of Fgl2; (b) administering an agent that inhibits IL-33 activity and/or TIGIT activity; (c) determining a second level of Fgl2 expression or activity after the administering; and (d) comparing the first and second levels of Fgl2 expression or activity, wherein the agent administered in (b) is effective if the second level of Fgl2 expression or activity is lower than the first level, and wherein the agent administered in (b) is ineffective if the second level of Fgl2 expression is the same as or higher than the first level.

By monitoring the effects of the anti-IL-33 and/or anti-TIGIT therapy on the level of Fgl2 expression or activity, one can determine the efficacy of the treatment regimen and adjust the treatment regimen if necessary. Accordingly, in some embodiments, the method can further comprise, when the anti-IL-33 or anti-TIGIT therapy is effective, continuing to administer the agent that inhibits IL-33 activity and/or TIGIT activity. In some embodiments, the method can further comprise, when the anti-IL-33 therapy or the anti-TIGIT therapy is ineffective, administering the agent that inhibits IL-33 activity and/or TIGIT activity at a higher dose. In some embodiments, the method can further comprise, when the anti-IL-33 therapy or the anti-TIGIT therapy is ineffective, discontinuing the anti-IL-33 therapy or the anti-TIGIT therapy. In these embodiments, the method can further comprise administering a therapy comprising an activator of a pro-inflammatory T cell response pathway and/or a suppressor of an anti-inflammatory T cell response pathway.

Similarly, further aspects disclosed herein relate to methods of treating a patient diagnosed with asthma, allergy, and/or atopy that exhibits an elevated level of IL-33. The method comprises: (a) determining a first level of TIGIT and/or Fgl2 expression or activity in a sample from a patient diagnosed with asthma, allergy, and/or atopy that exhibits an elevated level of IL-33; (b) administering an agent that inhibits IL-33 activity; (c) determining a second level of TIGIT or Fgl2 expression or activity after the administering; and (d) comparing the first and second levels of TIGIT and/or Fgl2 expression or activity, wherein anti-IL-33 therapy is effective if the second level of TIGIT and/or Fgl2 expression or activity is lower that the first level, and wherein anti-IL-33 therapy is ineffective if the second level of TIGIT and/or Fgl2 expression is the same as or higher than the first level.

In some embodiments, the method can further comprise, when the anti-IL-33 therapy is effective, continuing to administer the agent that inhibits IL-33 activity. In some embodiments, the method can further comprise, when the anti-IL-33 therapy is ineffective, administering the agent that inhibits IL-33 activity at a higher dose. In other embodiments, the method can further comprise, when the anti-IL-33 therapy is ineffective, discontinuing the anti-IL-33 therapy. In these embodiments, the method can further comprise administering a therapeutic agent for treatment of asthma, allergy, and/or atopy. In some embodiments, the therapeutic agent can comprise, e.g., an activator of a pro-inflammatory T cell response pathway and/or a suppressor of an anti-inflammatory T cell response pathway.

In yet other aspects of the present disclosure are methods of treating a patient diagnosed with asthma, allergy, and/or atopy comprising administering to a patient diagnosed with asthma, allergy, and/or atopy one or more embodiments of the pharmaceutical compositions described herein that provide a Th2-dampening effect are also provided. The pharmaceutical composition can be taken alone or in combination with another agent for treatment of asthma, allergy, and/or atopy. In some embodiments, the method can further comprise administering the patient an immunotherapy for treatment of asthma, allergy, and/or atopy. For example, an immunotherapy for treatment of asthma, allergy, and/or atopy can comprise an agent that increases a pro-inflammatory T cell response and/or an agent that suppresses an anti-inflammatory T cell response.

In some embodiments of this aspect and other related aspects of the present disclosure, the patient diagnosed with asthma, allergy, and/or atopy can be previously or currently being treated for the disease or disorder. Thus, the anti-TIGIT, anti-Fgl2 and/or anti-IL-33 therapy can be used, alone or in combination with another anti-asthmatic agent, anti-allergy agent, and/or anti-atopic agent. Examples of anti-asthmatic agents include, but are not limited to beta adrenergic agonists, xanthine derivatives, corticosteroids, antileukotrienes, and any combinations thereof. Exemplary anti-allergy agents and anti-atopic agents include, but are not limited to antihistamines, corticosteroids, and combinations thereof. In some embodiments of this aspect and other related aspects of the present disclosure, the methods described herein can further comprise administering to the patient diagnosed with asthma, allergy, and/or atopy a selected therapy (e.g., anti-TIGIT or anti-IL-33 therapy) after they have been identified to be more likely to benefit from one immunotherapy over another.

In some embodiments of this aspect and other related aspects of the present disclosure, the TIGIT inhibitor, Fgl2 inhibitor and/or IL-33 inhibitor administered to a patient diagnosed with asthma, allergy, and/or atopy can be constructed to specifically target TIGIT+ regulatory T cells (Tregs). For example, the TIGIT inhibitor, Fgl2 inhibitor and/or IL-33 inhibitor can comprise a cell-targeting moiety.

As used herein, the term "asthma" is intended to cover all types of asthma. Asthma is a chronic lung disease or disorder that inflames and narrows the airways.

As used herein, the term "allergy" refers to a disorder (or improper reaction) of the immune system often also referred to as "atopy." Allergic reactions can occur when a subject's immune system reacts to environmental substances that are normally harmless to those without allergy. The substances that cause such allergic reactions are known as allergens. In some embodiments, allergy refers to type I (or immediate) hypersensitivity. Allergic reactions occur when there is excessive activation of certain white blood cells (e.g., mast cells and basophils) by immunoglobulin E (IgE). Common allergic reactions include eczema, hives, hay fever, asthma, food allergies, and reactions to the venom of stinging insects such as wasps and bees. Mild allergies like hay fever are highly prevalent in the human population and cause symptoms such as allergic conjunctivitis, itchiness, and runny nose. Allergies can play a role in conditions such as asthma.

### TIGIT and antagonists (inhibitors) or agonists thereof

TIGIT is an immune receptor known as T cell Ig and ITIM (immunoreceptor tyrosine-based inhibitor motif) domain protein. TIGIT is also known as WUCAM and VSTM3. Boles et al. European Journal of Immunology; 39: 695-703; and Levin et al. European Journal of Immunology; 41:902-915. Generally, TIGIT is expressed as a cell surface protein on a variety of immune cells, e.g., regulatory T cells (Tregs), memory T cells, natural killer cells, and follicular T helper cells. TIGIT can directly suppress T cell responses, e.g., but not limited to T cell proliferation and/or proinflammatory cytokine production. In some embodiments, TIGIT+ T cells can suppress proliferation of other, TIGIT negative T cells and other immune cells such as antigen presenting cells.

As used herein, the term "TIGIT" generally refers to a TIGIT polypeptide or a TIGIT polynucleotide that is similar or identical to the sequence of a wild-type TIGIT.

In some embodiments, the term "TIGIT" refers to a TIGIT polypeptide having an amino acid sequence that is at least 70% or more (including at least 75%, at least 80%, at least 85%, at least 90%, at least 95%, at least 97%, at least 99%, or 100%) identical to that of a wild-type TIGIT, and is capable of suppressing Th1 and/or Th17 responses. In some embodiments, the TIGIT polypeptide can also increase expression and/or activity of Fgl2 to mediate the suppression of Th1 and/or Th17 responses.

In some embodiments, the term "TIGIT" refers to a TIGIT polynucleotide having a nucleotide sequence that is at least 70% or more (including at least 75%, at least 80%, at least 85%, at least 90%, at least 95%, at least 97%, at least 99%, or 100%) identical to that of a wild-type TIGIT or a portion thereof, and encodes a TIGIT polypeptide as described herein.

The wild-type TIGIT sequences of various species are available on the world wide web from the NCBI, including human, mouse, rat, dog, and chimpanzee. For example, the nucleotide sequence encoding human TIGIT is available at NCBI under Accession No. NM_173799 and its corresponding amino acid sequence is under Accession No. NP_776160.

Where the term "TIGIT" refers to a TIGIT polypeptide, the term "TIGIT polypeptide" also encompasses a portion or fragment of such a TIGIT polypeptide that retains at least about 70% or more (including at least 75%, at least 80%, at least 85%, at least 90%, at least 95%, at least 97%, at least 99%, or 100%) of the immunosuppressive activity of the wild-type TIGIT polypeptide. The term "TIGIT polypeptide" as used herein also encompasses conservative substitution variants of a TIGIT polypeptide that retain at least about 70% or more (including at least 75%, at least 80%, at least 85%, at least 90%, at least 95%, at least 97%, at least 99%, or 100%) of the immunosuppressive activity of the wild-type TIGIT polypeptide. Accordingly, a TIGIT polypeptide refers to any immunosuppressive form of TIGIT, including functional variants of TIGIT. For example, in some embodiments, a TIGIT polypeptide can be a full-length TIGIT. In some embodiments, a TIGIT polypeptide refers to a functional domain or domains of TIGIT that induces immunosuppression and expression and/or activity of Fgl2.

The amino acid identity between two polypeptides can be determined, for example, by first aligning the two polypeptide sequences using an alignment algorithm, such as BLAST® or by other methods well-known in the art.

In various aspects of the present disclosure, methods for measuring TIGIT or a fragment thereof from a sample are known in the art, including, but not limited to mRNA expression using PCR or real-time PCR, protein analysis using western blot, immunoassay, and/or ELISA, and/or sequencing analysis. Thus, in some embodiments, nucleic acid molecules can be isolated from a patient's sample to measure TIGIT mRNA expression, or proteins can be isolated to measure TIGIT protein expression.

As used interchangeably herein, the term "antagonist" or "inhibitor" is used in the broadest sense, and includes any molecule that partially or fully blocks, inhibits, or neutralizes a biological activity of a native polypeptide disclosed herein. In a similar manner, the term "agonist" is used in the broadest sense and includes any molecule that mimics a biological activity of a native polypeptide disclosed herein. Suitable agonist or antagonist molecules specifically include agonist or antagonist antibodies or antibody fragments, fragments or amino acid sequence variants of native polypeptides, peptides, antisense oligonucleotides, small organic molecules, recombinant proteins or peptides, etc. Methods for identifying agonists or antagonists of a polypeptide can comprise contacting a polypeptide with a candidate agonist or antagonist molecule and measuring a detectable change in one or more biological activities normally associated with the polypeptide.

The term "TIGIT antagonist" is used interchangeably with the terms "TIGIT inhibitor" and "anti-TIGIT therapy" and refers to an agent that interferes with the normal functioning of TIGIT, either by decreasing transcription or translation of TIGIT-encoding nucleic acid, or by inhibiting or blocking TIGIT polypeptide activity, or both. Examples of TIGIT antagonists include, but are not limited to, antisense polynucleotides, interfering RNAs, catalytic RNAs, RNA-DNA chimeras, TIGIT-specific aptamers, anti-TIGIT antibodies, TIGIT-binding fragments of anti-TIGIT antibodies, TIGIT-binding small molecules, TIGIT-binding peptides, and other polypeptides that specifically bind TIGIT (including, but not limited to, TIGIT-binding fragments of one or more TIGIT ligands, optionally fused to one or more additional domains), such that the interaction between the TIGIT antagonist and TIGIT results in a reduction or cessation of TIGIT activity or expression. It will be understood by one of ordinary skill in the art that in some instances, a TIGIT antagonist can antagonize one TIGIT activity without affecting another TIGIT activity. For example, a desirable TIGIT antagonist for use in certain of the methods herein is a TIGIT antagonist that antagonizes TIGIT activity in response to one of ligand interaction, CD112 interaction, or CD155 interaction, e.g., without affecting or minimally affecting any of the other TIGIT interactions.

In some embodiments, a TIGIT inhibitor is an agent that directly or indirectly inhibits or reduces the TIGIT-mediated suppression of proinflammatory Th1 and/or Th17 responses. Accordingly, a TIGIT inhibitor can target the TIGIT receptor or its corresponding ligand, or any of TIGIT's upstream molecules. Examples of TIGIT inhibitors include, without limitations, TIGIT-/- immune cells (e.g., T cells), anti-TIGIT molecules, ST2 inhibitors, CD112 inhibitors, CD155 inhibitors, and a combination thereof. A TIGIT inhibitor can be a protein, a peptide, a peptidomimetic, an aptamer, a nucleic acid, an antibody, a small molecule, a vaccine, or any combinations thereof.

In some embodiments, a TIGIT inhibitor is an anti-TIGIT antibody (e.g., an anti-human TIGIT antibody). Anti-TIGIT antibodies are commercially available (e.g., from R&D Systems (Clone 741182; Cat. No. MAB7898), Affymetrix eBioscience (clone MBSA43; Cat. No. 12-9500-41 or 12-9500-42), and Abcam (Cat. No. ab107664)).

In some embodiments, a TIGIT inhibitor can be a fragment or variant of TIGIT itself, e.g., a fragment that binds a TIGIT ligand (e.g., CD112 and/or CD155) but does not transmit an immunosuppressive signal (e.g., via Fgl2 expression). A TIGIT inhibitor of this type can be a dominant negative inhibitor.

In some embodiments, a TIGIT inhibitor is a recombinant soluble TIGIT Fc fusion protein. An exemplary recombinant soluble TIGIT Fc fusion protein can be obtained from R&D Systems (Cat. No. 7267-TG-050).

As used herein, the term "TIGIT agonist" refers to an agent that enhances or stimulates the normal functioning of TIGIT, by increasing transcription or translation of TIGIT-encoding nucleic acid, and/or by inhibiting or blocking activity of a molecule that inhibits TIGIT expression or TIGIT activity, and/or by enhancing normal TIGIT activity (including, but not limited to, enhancing the stability of TIGIT or enhancing binding of TIGIT to one or more target ligands such as CD112 or CD155). For example, the TIGIT agonist can be selected from an antibody, an antigen-binding fragment, an aptamer, an interfering RNA, a small molecule, a peptide, an antisense molecule, and another binding polypeptide. In another example, the TIGIT agonist can be a polynucleotide selected from an aptamer, interfering RNA, or antisense molecule that interferes with the transcription and/or translation of a TIGIT-inhibitory molecule. It will be understood by one of ordinary skill in the art that in some instances, a TIGIT agonist can agonize one TIGIT activity without affecting another TIGIT activity. For example, a desirable TIGIT agonist for use in certain of the methods herein is a TIGIT agonist that agonizes TIGIT activity in response to one of ligand interaction, CD155 interaction, or CD112 interaction, e.g., without affecting or minimally affecting any of the other TIGIT interactions.

In some embodiments, a TIGIT agonist is an agent that directly or indirectly enhances or stimulates the TIGIT-mediated suppression of proinflammatory Th1 and/or Th17 responses. Accordingly, a TIGIT agonist can target the TIGIT receptor or its corresponding ligand, or any of TIGIT's upstream molecules. Examples of TIGIT agonists include, without limitations, TIGIT-overexpressing immune cells (e.g., T cells), ST2 agonists, CD112 agonists, CD155 agonists, and a combination thereof. The TIGIT agonists can be a protein, a peptide, peptidomimetic, an aptamer, a nucleic acid, an antibody, a small molecule, a vaccine, a fusion protein, or any combinations thereof.

In some embodiments, a TIGIT agonist is an agonistic TIGIT antibody (e.g., an agonistic antibody to human TIGIT). Agonistic TIGIT antibody can be provided by ZymoGenetics, Inc. Agonistic TIGIT antibodies against human TIGIT are described by Lozano et al. (Journal of Immunology, 2012; 188: 3869-3875)

TIGIT antagonists or agonists can be obtained from known sources or prepared using known techniques such as recombinant or synthetic technology. The nucleic acid and protein sequences of TIGIT and its ligands of different species (e.g., but not limited to, human, mouse, rat, dog, chimpanzee) are known in the art, e.g., accessible at world wide web from NCBI. Thus, one of skill in the art can generate TIGIT antagonists or agonists based on these sequences using art-recognized molecular technologies such as cloning and expression technologies. For example, a human TIGIT antagonist (e.g., an antibody) can be generated using protein based on the nucleic acid sequence of human TIGIT accessible at NCBI under Accession No. NM_173799 and/or the corresponding amino acid sequence under Accession No. NP_776160, or fragments thereof. In some embodiments, a human TIGIT agonist (e.g., a TIGIT ligand) can be generated based on the nucleic acid sequence of human CD155 accessible at NCBI under Accession No. NM_001135768, NM_001135769, NM_001135770, or NM_006505 and/or the corresponding amino acid sequence under Accession No. NP_001129240, NP001129241, NP001129242, or NP_006496, or fragments thereof. In some embodiments, a human TIGIT agonist (e.g., a TIGIT ligand) can be generated based on the nucleic acid sequence of human CD112 accessible at NCBI under Accession No. NM_001042724 or NM_002856 and/or the corresponding amino acid sequence under Accession No. NP_001036189 or NP_002847, or fragments thereof.

In some embodiments, antagonists or agonists of TIGIT disclosed in the International Patent Publication WO 2009/126688 can be used in various embodiments of the methods and compositions described herein.

### Fgl2 and antagonists (inhibitors) or agonists thereof

Fgl2, also known as fibroleukin or fibrinogen-like protein 2, is a member of the fibrinogen-related protein superfamily of proteins. Fgl2 was first cloned from human CTLs and is secreted by CD4+ and CD8+ T cells or Tregs. As used herein, the term "Fgl2" generally refers to an Fgl2 polypeptide or an Fgl2 polynucleotide that is similar or identical to the sequence of a wild-type Fgl2.

In some embodiments, the term "Fgl2" refers to an Fgl2 polypeptide having an amino acid sequence that is at least 70% or more (including at least 75%, at least 80%, at least 85%, at least 90%, at least 95%, at least 97%, at least 99%, or 100%) identical to that of a wild-type Fgl2, and is capable of mediating suppression of Th1 and/or Th17 responses.

In some embodiments, the term "Fgl2" refers to an Fgl2 polynucleotide having a nucleotide sequence that is at least 70% or more (including at least 75%, at least 80%, at least 85%, at least 90%, at least 95%, at least 97%, at least 99%, or 100%) identical to that of a wild-type Fgl2, and encodes an Fgl2 polypeptide as described herein.

The wild-type Fgl2 sequences of various species are available on the world wide web from the NCBI, including human, mouse, rat, dog, and chimpanzee. For example, the nucleotide sequence encoding human Fgl2 is available at NCBI under Accession No. NM_006682 and its corresponding amino acid sequence is under Accession No. NP_00673.

As used herein, the term "Fgl2 polypeptide" also encompasses a portion or fragment of such an Fgl2 polypeptide that retains at least about 70% or more (including at least 75%, at least 80%, at least 85%, at least 90%, at least 95%, at least 97%, at least 99%, or 100%) of the activity of the wild-type FGL2 polypeptide to mediate the suppression of Th1 and/or Th17 responses. The term "Fgl2 polypeptide" as used herein also encompasses conservative substitution variants of an Fgl2 polypeptide that retain at least about 70% or more (including at least 75%, at least 80%, at least 85%, at least 90%, at least 95%, at least 97%, at least 99%, or 100%) of the activity of the wild-type FGL2 polypeptide to mediate the suppression of Th1 and/or Th7 responses.

As used herein, the term "FGL2 antagonist" is used interchangeably with the terms "Fgl2 inhibitor" and "anti-Fgl2 therapy" and refers to an agent that interferes with the normal functioning of Fgl2, either by decreasing transcription or translation of Fgl2-encoding nucleic acid, or by inhibiting or blocking Fgl2 polypeptide activity, or both. Examples of Fgl2 antagonists include, but are not limited to, antisense polynucleotides, interfering RNAs, catalytic RNAs, RNA-DNA chimeras, Fgl2-specific aptamers, anti-Fgl2 antibodies, Fgl2-binding fragments of anti-Fgl2 antibodies, Fgl2-binding small molecules, Fgl2-binding peptides, and other polypeptides that specifically bind Fgl2 (including, but not limited to, Fgl2-binding fragments of one or more Fgl2 ligands, optionally fused to one or more additional domains), such that the interaction between the Fgl2 antagonist and Fgl2 results in a reduction or cessation of Fgl2 activity or expression. It will be understood by one of ordinary skill in the art that in some instances, an Fgl2 antagonist can antagonize one Fgl2 activity without affecting another Fgl2 activity. For example, a desirable Fgl2 antagonist for use in certain of the methods herein is an Fgl2 antagonist that antagonizes Fgl2 activity in response to one of binding partner interactions, e.g., without affecting or minimally affecting any of the other Fgl2 interactions.

In some embodiments, an Fgl2 inhibitor is an agent that directly or indirectly reduces the expression/secretion and/or activity of Fgl2. Accordingly, an Fgl2 inhibitor can target Fgl2 molecule or its corresponding receptors. Alternatively, an Fgl2 inhibitor can bind to TIGIT or a TIGIT ligand (e.g., CD112 and/or CD155) and inhibit TIGIT-mediated activation of Fgl2 expression or activity. Examples of Fgl2 inhibitors include, but are not limited to, Fgl2 neutralizing agents, TIGIT inhibitors, CEBPα inhibitors (i.e., agents that decreases expression and/or activity of CEBPα and inhibits binding of CEBPα to Fgl2 gene), and/or ST2 inhibitors. The Fgl2 inhibitors can be a protein, a peptide, a peptidomimetic, an aptamer, a nucleic acid, an antibody, a fusion construct, a small molecule, a vaccine, a fusion protein, or any combination thereof.

In some embodiments, an Fgl2 inhibitor is an anti-Fgl2 antibody (e.g., an anti-human Fgl2 antibody). Anti-Fgl2 antibodies are commercially available (e.g., from Abcam (Cat. No. ab103584)).

The term "FGL2 agonist" refers to an agent that enhances or stimulates the normal functioning of FGL2, by increasing transcription or translation of FGL2-encoding nucleic acid, and/or by inhibiting or blocking activity of a molecule that inhibits FGL2 expression or FGL2 activity, and/or by enhancing normal FGL2 activity (including, but not limited to, enhancing the stability of FGL2 or enhancing binding of FGL2 to one or more target binding partners). For example, the FGL2 agonist can be selected from an antibody, an antigen-binding fragment, an aptamer, an interfering RNA, a small molecule, a peptide, an antisense molecule, and another binding polypeptide. In another example, the FGL2 agonist can be a polynucleotide selected from an aptamer, interfering RNA, or antisense molecule that interferes with the transcription and/or translation of an Fgl2-inhibitory molecule. It will be understood by one of ordinary skill in the art that in some instances, an Fgl2 agonist can agonize one FGL2 activity without affecting another FGL2 activity. For example, a desirable FGL2 agonist for use in certain of the methods herein is an Fgl2 agonist that agonizes FGL2 activity in response to one of its binding partner interactions, e.g., without affecting or minimally affecting any of the other FGL2 interactions.

In some embodiments, an Fgl2 agonist is an agent that directly or indirectly increases the expression/secretion and/or activity of Fgl2. Accordingly, an Fgl2 agonist can target Fgl2 molecule or its corresponding receptors. Examples of Fgl2 agonists include, but are not limited to, Fgl2 soluble molecules, TIGIT agonists, CEBPα-inducing agents (i.e., agents that increases expression and/or activity of CEBPα and promotes binding of CEBPα to Fgl2 gene), and/or ST2 agonists. The Fgl2 agonists can be a protein, a peptide, a peptidomimetic, a fusion protein, an aptamer, a nucleic acid, an antibody, a small molecule, a vaccine, a fusion construct, or any combination thereof.

In some embodiments, an Fgl2 agonist is a recombinant Fgl2 protein (e.g., a recombinant human Fgl2 protein). Recombinant Fgl2 proteins are commercially available (e.g., from OriGene (Cat. No. TP307557)).

Fgl2 antagonists or agonists can be obtained from known sources or prepared using known techniques such as recombinant or synthetic technology. The nucleic acid and protein sequences of Fgl2 of different species (e.g., but not limited to, human, mouse, rat, dog, chimpanzee) are known in the art, e.g., accessible at NCBI. Thus, one of skill in the art can readily generate Fgl2 antagonists or agonists based on these sequences using art-recognized molecular technologies. For example, a human Fgl2 antagonist (e.g., an antibody) or agonist (e.g., a soluble protein) can be generated based on the nucleic acid sequence of human Fgl2 accessible at NCBI under Accession No. NM_006682 and/or the corresponding amino acid sequence under Accession No. NP_00673 or fragments thereof.

In some embodiments, antagonists or agonists of Fgl2 disclosed in the International Patent Publication WO 2003/074068 can be used in various embodiments of the methods and compositions described herein.

### IL-33 and antagonists (inhibitors) or agonists thereof

IL-33 is interleukin-33 cytokine and is a ligand for ST2 receptor and the co-receptor IL-1 receptor accessory protein (IL-1RAcP). As used herein, the term "IL-33" generally refers to an IL-33 polypeptide or an IL-33 polynucleotide that is similar or identical to the sequence of a wild-type IL-33.

In some embodiments, the term "IL-33" refers to an IL-33 polypeptide having an amino acid sequence that is at least 70% or more (including at least 75%, at least 80%, at least 85%, at least 90%, at least 95%, at least 97%, at least 99%, or 100%) identical to that of a wild-type IL-33, and is capable of inducing TIGIT expression and/or activity, and/or increasing or expanding a TIGIT+ Treg population.

In some embodiments, the term "IL-33" refers to an IL-33 polynucleotide having a nucleotide sequence that is at least 70% or more (including at least 75%, at least 80%, at least 85%, at least 90%, at least 95%, at least 97%, at least 99%, or 100%) identical to that of a wild-type IL-33, and encodes an IL-33 polypeptide as described herein.

The wild-type IL-33 sequences of various species and isoforms thereof are available on the world wide web from the NCBI, including human, mouse, rat, pig, and chimpanzee. For example, the nucleotide sequences encoding human IL-33 and isoforms thereof are available at NCBI under Accession Nos. NM_001186569, NM_001196640, and NM_001199641, and their corresponding amino acid sequence are under Accession Nos. NP_254274, NP_001186569, and NP_001186570, respectively.

As used herein, the term "IL-33 polypeptide" also encompasses a portion or fragment of such an IL-33 polypeptide that retains at least about 70% or more (including at least 75%, at least 80%, at least 85%, at least 90%, at least 95%, at least 97%, at least 99%, or 100%) of the activity of the wild-type IL-33 polypeptide to induce TIGIT expression and/or activity and/or increasing or expanding a TIGIT+ Treg population. The term "IL-33 polypeptide" as used herein also encompasses conservative substitution variants of an IL-33 polypeptide that retain at least about 70% or more (including at least 75%, at least 80%, at least 85%, at least 90%, at least 95%, at least 97%, at least 99%, or 100%) of the activity of the wild-type IL-33 polypeptide to induce TIGIT expression and/or activity and/or increasing or expanding a TIGIT+ Treg population.

In various aspects of the present disclosure, methods for measuring IL-33 or a fragment thereof from a sample are known in the art, including, but not limited to mRNA expression using PCR or real-time PCR, protein analysis using western blot, immunoassay, and/or ELISA, and/or sequencing analysis. Thus, in some embodiments, nucleic acid molecules can be isolated from a patient's sample to measure IL-33 mRNA expression, or proteins can be isolated to measure IL-33 protein expression.

The term "IL-33 antagonist" is used interchangeably with the terms "IL-33 inhibitor" and "anti-IL-33 therapy" and refers to an agent that interferes with the normal functioning of IL-33, either by decreasing transcription or translation of IL-33-encoding nucleic acid, or by inhibiting or blocking IL-33 polypeptide activity, or both. Examples of IL-33 antagonists include, but are not limited to, antisense polynucleotides, interfering RNAs, catalytic RNAs, RNA-DNA chimeras, IL-33-specific aptamers, anti-IL-33 antibodies, IL-33-binding fragments of anti-IL-33 antibodies, IL-33-binding small molecules, IL-33-binding peptides, and other polypeptides that specifically bind IL-33 (including, but not limited to, IL-33-binding fragments of one or more IL-33 ligands, optionally fused to one or more additional domains), such that the interaction between the IL-33 antagonist and IL-33 results in a reduction or cessation of IL-33 activity or expression. It will be understood by one of ordinary skill in the art that in some instances, an IL-33 antagonist can antagonize one IL-33 activity without affecting another IL-33 activity. For example, a desirable IL-33 antagonist for use in certain of the methods herein is an IL-33 antagonist that antagonizes IL-33 activity in response to one of binding partner interactions such as ST2, e.g., without affecting or minimally affecting any of the other IL-33 interactions.

In some embodiments, an IL-33 inhibitor is an agent that directly or indirectly reduces the expression/secretion and/or activity of IL-33. Accordingly, an IL-33 inhibitor can target IL-33 molecule or its corresponding receptors. Examples of IL-33 inhibitors include, but are not limited to, ST2 inhibitors or IL-33 neutralizing agents. The IL-33 inhibitors can be a protein, a peptide, a peptidomimetic, an aptamer, a nucleic acid, an antibody, a small molecule, a fusion protein, a vaccine, or any combination thereof.

In some embodiments, an IL-33 inhibitor is an anti-IL-33 antibody (e.g., an anti-human IL-33 antibody). Anti-IL-33 antibodies are commercially available (e.g., from AbD Serotec, a Bio-Rad Company (Cat. No. AHP1482), Biolegend (Clone BL35175; Cat. No. 517201 or 517202), and Abcam (Cat. No. ab72844)).

In some embodiments, an IL-33 inhibitor can be a soluble ST2 receptor or a soluble IL-1RAcP receptor (e.g., without a transmembrane domain) that binds to IL-33, thereby decreasing the concentration of IL-33 that is available for functionally interacting with ST2/IL-1RAcP receptors present on an immune cell (e.g., T cell) or a TIGIT+ Treg.

The term "IL-33 agonist" refers to an agent that enhances or stimulates the normal functioning of IL-33, by increasing transcription or translation of IL-33-encoding nucleic acid, and/or by inhibiting or blocking activity of a molecule that inhibits IL-33 expression or IL-33 activity, and/or by enhancing normal IL-33 activity (including, but not limited to, enhancing the stability of IL-33 or enhancing binding of IL-33 to one or more target binding partners such as ST2. For example, the IL-33 agonist can be selected from an antibody, an antigen-binding fragment, an aptamer, an interfering RNA, a small molecule, a peptide, an antisense molecule, and another binding polypeptide. In another example, the IL-33 agonist can be a polynucleotide selected from an aptamer, interfering RNA, or antisense molecule that interferes with the transcription and/or translation of an IL-33-inhibitory molecule. It will be understood by one of ordinary skill in the art that in some instances, an IL-33 agonist can agonize one IL-33 activity without affecting another IL-33 activity. For example, a desirable IL-33 agonist for use in certain of the methods herein is an IL-33 agonist that agonizes IL-33 activity in response to one of its binding partner interactions such as ST2, e.g., without affecting or minimally affecting any of the other IL-33 interactions.

In some embodiments, an IL-33 agonist is an agent that directly or indirectly increases the expression/secretion and/or activity of IL-33. Accordingly, an IL-33 agonist can target IL-33 molecule or its corresponding receptors. Examples of IL-33 agonists include, but are not limited to, ST2 agonists or IL-33 soluble molecules. The IL-33 agonists can be a protein, a peptide, a peptidomimetic, an aptamer, a nucleic acid, an antibody, a small molecule, a fusion protein, a vaccine, or any combination thereof.

In some embodiments, an IL-33 agonist is a recombinant IL-33 protein (e.g., a recombinant human IL-33 protein). Recombinant IL-33 proteins are commercially available (e.g., from Life Technologies (Cat. No. PHC9254); InVivoGen (Cat. No. rhil-33); and R&D Systems (Cat. No. 3625-IL-010)).

IL-33 antagonists or agonists can be obtained from known sources or prepared using known techniques such as recombinant or synthetic technology. The nucleic acid and protein sequences of IL-33 of different species (e.g., but not limited to, human, mouse, pig, chimpanzee) are known in the art, e.g., accessible at NCBI. Thus, one of skill in the art can readily generate IL-33 antagonists or agonists based on these sequences using art-recognized molecular technologies. For example, a human IL-33 antagonist (e.g., an antibody) or agonist (e.g., a soluble protein) can be generated based on the nucleic acid sequence of human IL-33, e.g., accessible at NCBI under Accession No. NM_001186569, NM_001196640, or NM_001199641 and/or the corresponding amino acid sequence under Accession No. NP_254274, NP_001186569, or NP_001186570, or fragments thereof.

In some embodiments, antagonists or agonists of IL-33 disclosed in the International Patent Publication WO 2005/079844 can be used in various embodiments of the methods and compositions described herein.

### ST2 and antagonists (inhibitors) or agonists thereof

ST2 is interleukin 1 receptor-like 1 protein that binds IL-33, and is also known as IL1RL1, IL-1 R4, ST2L, DER4, Fit-1, Ly84, and T1. The ST2 protein has two isoforms: a soluble form (soluble ST2 or sST2) and a membrane bound receptor form (ST2 receptor). As used herein, the term "ST2" generally refers to an ST2 polypeptide or an ST2 polynucleotide that is similar or identical to the sequence of a wild-type ST2.

In some embodiments, the term "ST2" refers to an ST2 polypeptide having an amino acid sequence that is at least 70% or more (including at least 75%, at least 80%, at least 85%, at least 90%, at least 95%, at least 97%, at least 99%, or 100%) identical to that of a wild-type ST2, and is capable of binding IL-33 to induce TIGIT expression and/or activity and/or increase or expand a TIGIT+ Treg population.

In some embodiments, the term "ST2" refers to an ST2 polynucleotide having a nucleotide sequence that is at least 70% or more (including at least 75%, at least 80%, at least 85%, at least 90%, at least 95%, at least 97%, at least 99%, or 100%) identical to that of a wild-type ST2 or a portion thereof, and encodes an ST2 polypeptide as described herein.

The wild-type ST2 sequences of various species and isoforms thereof are available on the world wide web from the NCBI, including human, mouse, rat, monkey and dog. For example, the nucleotide sequences encoding human ST2 and isoforms thereof are available at NCBI under Accession Nos. NM_001282408, NM_003853, and NM_016232 and their corresponding amino acid sequences are under Accession Nos. NP_001269337, NP_003847, and NP_057316, respectively.

Where the term "ST2" refers to an ST2 polypeptide, the term "ST2 polypeptide" also encompasses a portion or fragment of such an ST2 polypeptide that retains at least about 70% or more (including at least 75%, at least 80%, at least 85%, at least 90%, at least 95%, at least 97%, at least 99%, or 100%) of the activity of the wild-type ST2 polypeptide to bind IL-33, which in turn induces TIGIT expression and/or activity and/or increases or expands a TIGIT+ Treg population. The term "ST2 polypeptide" as used herein also encompasses conservative substitution variants of an ST2 polypeptide that retain at least about 70% or more (including at least 75%, at least 80%, at least 85%, at least 90%, at least 95%, at least 97%, at least 99%, or 100%) of the activity of the wild-type ST2 polypeptide to bind IL-33, which in turn induces TIGIT expression and/or activity and/or increases or expands a TIGIT+ Treg population. Accordingly, an ST2 polypeptide refers to any form of ST2 that binds IL-33 and induces immunosuppression (e.g., via TIGIT activity and/or expression), including functional variants of ST2. For example, in some embodiments, an ST2 polypeptide can be a full-length ST2. In some embodiments, an ST2 polypeptide refers to a functional domain or domains of ST2 that binds IL-33, which in turn induces TIGIT expression and/or activity and/or increases or expands a TIGIT+ Treg population.

In various aspects of the present disclosure, methods for measuring ST2 or a fragment thereof (including sST2 and ST2 receptor or fragments thereof) from a sample are known in the art, including, but not limited to mRNA expression using PCR or real-time PCR, protein analysis using western blot, immunoassay, and/or ELISA, and/or sequencing analysis. Thus, in some embodiments, nucleic acid molecules can be isolated from a patient's sample to measure ST2 mRNA expression, or proteins can be isolated to measure ST2 protein expression.

The term "ST2 antagonist" is used interchangeably with the term "ST2 inhibitor" and refers to an agent that interferes with the normal functioning of ST2, either by decreasing transcription or translation of ST2-encoding nucleic acid, or by inhibiting or blocking ST2 polypeptide activity, or both. Examples of ST2 antagonists include, but are not limited to, antisense polynucleotides, interfering RNAs, catalytic RNAs, RNA-DNA chimeras, ST2-specific aptamers, anti-ST2 antibodies, ST2-binding fragments of anti-ST2 antibodies, ST2-binding small molecules, ST2-binding peptides, and other polypeptides that specifically bind ST2 (including, but not limited to, ST2-binding fragments of one or more ST2 ligands, optionally fused to one or more additional domains), such that the interaction between the ST2 antagonist and ST2 results in a reduction or cessation of ST2 activity or expression. It will be understood by one of ordinary skill in the art that in some instances, an ST2 antagonist can antagonize one ST2 activity without affecting another ST2 activity. For example, a desirable ST2 antagonist for use in certain of the methods herein is an ST2 antagonist that antagonizes ST2 activity in response to one of ligand interaction, IL-33 interaction, or other binding partner interaction, e.g., without affecting or minimally affecting any of the other ST2 interactions.

In some embodiments, an ST2 inhibitor is an agent that directly or indirectly inhibits or reduces the ST2-mediated suppression of proinflammatory Th1 and/or Th17 responses, e.g., by inhibiting or reducing proliferation of TIGIT+ Tregs. Accordingly, an ST2 inhibitor can target the corresponding ligand of ST2, or any of ST2's upstream molecules. Examples of ST2 inhibitors include, without limitations, ST2-/- immune cells (e.g., T cells), anti-ST2 molecules, IL-33 inhibitors, and any combination thereof. The ST2 inhibitors can be a protein, a peptide, peptidomimetic, an aptamer, a nucleic acid, an antibody, a small molecule, a vaccine, a fusion protein, or any combinations thereof.

In some embodiments, an ST2 inhibitor is an anti-ST2 antibody (e.g., an anti-human ST2 antibody). Anti-ST2 antibodies are commercially available (e.g., from R&D Systems (Clone 97203; Cat. No. MAB523); Abcam (Cat. No. ab89741); and AdipoGen (Clone ST33868; Cat No. AG-20A-0044)).

In some embodiments, an ST2 inhibitor is a recombinant ST2 Fc fusion protein (e.g., a recombinant mouse ST2 Fc fusion protein). Recombinant ST2 proteins are commercially available (e.g., from R&D Systems (Cat. No. 1004-MP-050)).

In some embodiments, an ST2 inhibitor can be a fragment or variant of ST2 itself, e.g., a fragment that binds IL-33 but does not induce an immunosuppressive signal (e.g., via TIGIT activity and/or expression). An ST2 inhibitor of this type can be a dominant negative inhibitor.

The term "ST2 agonist" refers to an agent that enhances or stimulates the normal functioning of ST2, by increasing transcription or translation of ST2-encoding nucleic acid, and/or by inhibiting or blocking activity of a molecule that inhibits ST2 expression or ST2 activity, and/or by enhancing normal ST2 activity (including, but not limited to, enhancing the stability of ST2 or enhancing binding of ST2 to one or more target ligands such as IL-33). For example, the ST2 agonist can be selected from an antibody, an antigen-binding fragment, an aptamer, an interfering RNA, a small molecule, a peptide, an antisense molecule, and another binding polypeptide. In another example, the ST2 agonist can be a polynucleotide selected from an aptamer, interfering RNA, or antisense molecule that interferes with the transcription and/or translation of an ST2-inhibitory molecule. It will be understood by one of ordinary skill in the art that in some instances, an ST2 agonist can agonize one ST2 activity without affecting another ST2 activity. For example, a desirable ST2 agonist for use in certain of the methods herein is an ST2 agonist that agonizes ST2 activity in response to one of ligand interaction, IL-33 interaction, or other binding partner interaction, e.g., without affecting or minimally affecting any of the other ST2 interactions.

In some embodiments, an ST2 agonist is an agent that directly or indirectly enhances or stimulates the ST2-mediated suppression of proinflammatory Th1 and/or Th17 responses, e.g., by inducing or expanding the TIGIT+ cell population. Accordingly, an ST2 agonist can target its corresponding ligand such as IL-33, or any of ST2's upstream molecules. Examples of ST2 agonists include, without limitations, ST2-overexpressing immune cells (e.g., T cells), IL-33 agonists, and a combination thereof. The ST2 agonists can be a protein, a peptide, peptidomimetic, an aptamer, a nucleic acid, an antibody, a small molecule, a vaccine, a fusion protein, or any combinations thereof.

ST2 antagonists or agonists can be obtained from known sources or prepared using known techniques such as recombinant or synthetic technology. The nucleic acid and protein sequences of ST2 of different species (e.g., but not limited to, human, mouse, pig, chimpanzee) are known in the art, e.g., accessible at NCBI. Thus, one of skill in the art can readily generate ST2 antagonists or agonists based on these sequences using art-recognized molecular technologies. For example, a human ST2 antagonist (e.g., an antibody) or agonist (e.g., a soluble protein) can be generated based on the nucleic acid sequence of human ST2, e.g., accessible at NCBI under Accession No. NM_001282408, NM_003853, or NM_016232 and/or the corresponding amino acid sequence under Accession No. NP_001269337, NP_003847, or NP_057316, or fragments thereof.

### CD112 and antagonists (inhibitors) or agonists thereof

CD112 (cluster of differentiation 112) is a single-pass type 1 membrane glycoprotein with two Ig-like C2-type domains and an Ig-like V-type domain. CD112 is also known as PVRL2 (poliovirus receptor-related 2), herpesvirus entry mediator B or nectin 2. Yu et al. Nat. Immunol. 10:48-57 (2009). Generally, CD112 is expressed as a cell surface protein on a variety of cells, e.g., myelomonocytic cells, megakaryocytes, dendritic cells, mast cells, CD34-positive stem cells, endothelial cells, epithelial cells, and neuronal cells, macrophages, and other antigen-presenting cells, e.g., but not limited to cancer cells. CD112 is a ligand for TIGIT with a lower affinity than CD155 (as discussed below).

As used herein, the term "CD112" generally refers to a CD112 polypeptide or a CD112 polynucleotide that is similar or identical to the sequence of a wild-type CD112.

In some embodiments, the term "CD112" refers to a CD112 polypeptide having an amino acid sequence that is at least 70% or more (including at least 75%, at least 80%, at least 85%, at least 90%, at least 95%, at least 97%, at least 99%, or 100%) identical to that of a wild-type CD112, and is capable of binding TIGIT and suppressing Th1 and/or Th17 responses. In some embodiments, the CD112 polypeptide can also increase expression and/or activity of Fgl2 to mediate the suppression of Thl and/or Th17 responses.

In some embodiments, the term "CD112" refers to a CD112 polynucleotide having a nucleotide sequence that is at least 70% or more (including at least 75%, at least 80%, at least 85%, at least 90%, at least 95%, at least 97%, at least 99%, or 100%) identical to that of a wild-type CD112 polynucleotide or a portion thereof, and encodes a CD112 polypeptide as described herein.

The wild-type CD112 sequences of various species are available on the world wide web from the NCBI, including human, mouse, and monkey. For example, the nucleotide sequence encoding human CD112 is available at NCBI under Accession No. NM_001042724 or NM_002856 and its corresponding amino acid sequence is under Accession No. NP_001036189 or NP_002847.

Where the term "CD112" refers to a CD112 polypeptide, the term "CD112 polypeptide" also encompasses a portion or fragment of such a CD112 polypeptide that retains at least about 70% or more (including at least 75%, at least 80%, at least 85%, at least 90%, at least 95%, at least 97%, at least 99%, or 100%) of the TIGIT binding activity of the wild-type CD112 polypeptide. The term "CD112 polypeptide" as used herein also encompasses conservative substitution variants of a CD112 polypeptide that retain at least about 70% or more (including at least 75%, at least 80%, at least 85%, at least 90%, at least 95%, at least 97%, at least 99%, or 100%) of the TIGIT binding activity of the wild-type CD112 polypeptide. Accordingly, a CD112 polypeptide refers to any form of CD112 that can bind TIGIT, including functional variants of CD112. For example, in some embodiments, a CD112 polypeptide can be a full-length CD112. In some embodiments, a CD112 polypeptide refers to a functional domain or domains (e.g., an extracellular domain) of CD112 that binds TIGIT and induces immunosuppression and expression and/or activity of Fgl2.

The term "CD112 antagonist" is used interchangeably with the term "CD112 inhibitor" and refers to an agent that interferes with the normal functioning of CD112, either by decreasing transcription or translation of CD112-encoding nucleic acid, or by inhibiting or blocking CD112 polypeptide activity, or both. Examples of CD112 antagonists include, but are not limited to, antisense polynucleotides, interfering RNAs, catalytic RNAs, RNA-DNA chimeras, CD112-specific aptamers, anti-CD 112 antibodies, CD112-binding fragments of anti-CD 112 antibodies, CD112-binding small molecules, CD112-binding peptides, and other polypeptides that specifically bind CD112 (including, but not limited to, CD112-binding fragments of soluble TIGIT, optionally fused to one or more additional domains), such that the interaction between the CD112 antagonist and CD112 results in a reduction or cessation of CD112 activity or expression. It will be understood by one of ordinary skill in the art that in some instances, a CD112 antagonist can antagonize one CD112 activity without affecting another CD112 activity. For example, a desirable CD112 antagonist for use in certain of the methods herein is a CD112 antagonist that antagonizes CD112 binding to TIGIT, e.g., without affecting or minimally affecting any of the other CD112 interactions.

In some embodiments, a CD112 inhibitor is an agent that directly or indirectly inhibits or reduces CD112 binding to TIGIT, which in turn induces suppression of proinflammatory Th1 and/or Th17 responses. Accordingly, a CD112 inhibitor can target the CD112 ligand itself or its corresponding receptor, or any molecule that regulates expression of CD112. Examples of CD112 inhibitors include, without limitations, anti-CD112 molecules, soluble TIGIT molecules, and a combination thereof. A CD112 inhibitor can be a protein, a peptide, a peptidomimetic, an aptamer, a nucleic acid, an antibody, a small molecule, a vaccine, or any combinations thereof.

In some embodiments, a CD112 inhibitor is an anti-CD112 antibody (e.g., an anti-human CD112 antibody). Anti-CD112 antibodies are commercially available (e.g., from BioLegend (Clone TX31; Cat. No. 337402), EMD Millipore (Clone R2.525; Cat. No. MABT62), R&D Systems (Cat. No. AF2229), or Abcam (Clone EPR6717; Cat. No. ab135246)).

In some embodiments, a CD112 inhibitor can be a fragment or variant of CD112 itself, e.g., a fragment that binds TIGIT but does not transmit an immunosuppressive signal (e.g., via Fgl2 expression). A CD112 inhibitor of this type can be a dominant negative inhibitor. For example, a CD112 inhibitor is a recombinant soluble CD112 protein (e.g., with the transmembrane domain substantially removed).

As used herein, the term "CD112 agonist" refers to an agent that enhances or stimulates the normal functioning of CD112, by increasing transcription or translation of CD112-encoding nucleic acid, and/or by inhibiting or blocking activity of a molecule that inhibits CD112 expression or CD112 activity, and/or by enhancing normal CD112 activity (including, but not limited to, enhancing the stability of CD112 or enhancing binding of CD112 to one or more target receptors such as TIGIT). For example, the CD112 agonist can be selected from an antibody, an antigen-binding fragment, an aptamer, an interfering RNA, a small molecule, a peptide, an antisense molecule, and another binding polypeptide. In another example, the CD112 agonist can be a polynucleotide selected from an aptamer, interfering RNA, or antisense molecule that interferes with the transcription and/or translation of a CD112 molecule. It will be understood by one of ordinary skill in the art that in some instances, a CD112 agonist can agonize one CD112 activity without affecting another CD112 activity. For example, a desirable CD112 agonist for use in certain of the methods herein is a CD112 agonist that agonizes CD112 binding to TIGIT, e.g., without affecting or minimally affecting any of the other CD112 interactions.

In some embodiments, a CD112 agonist is an agent that directly or indirectly enhances or stimulates CD112 binding to TIGIT, which in turn induces the TIGIT-mediated suppression of proinflammatory Th1 and/or Th17 responses. Accordingly, a CD112 agonist can target the CD112 ligand itself or its corresponding receptor, or any molecule that modulates expression of CD112. CD112 agonists can be a protein, a peptide, peptidomimetic, an aptamer, a nucleic acid, an antibody, a small molecule, a vaccine, a fusion protein, or any combinations thereof. Exemplary CD112 agonists include recombinant CD112 proteins or peptides.

CD112 antagonists or agonists can be obtained from known sources or prepared using known techniques such as recombinant or synthetic technology. The nucleic acid and protein sequences of CD112 and its ligands of different species (e.g., but not limited to, human, mouse, and monkey) are known in the art, e.g., accessible at world wide web from NCBI. Thus, one of skill in the art can generate CD112 antagonists or agonists based on these sequences using art-recognized molecular technologies such as cloning and expression technologies. For example, a human CD112 antagonist (e.g., an antibody) or agonist (e.g., recombinant protein) can be generated using protein based on the nucleic acid sequence of human CD112 accessible at NCBI under Accession No. NM_001042724 or NM_002856 and its corresponding amino acid sequence is under Accession No. NP_001036189 or NP_002847.

### CD155 and antagonists (inhibitors) or agonists thereof

CD155 (cluster of differentiation 155) is a type 1 membrane glycoprotein with three extracellular immunoglobulin-like domains, D1-D3. In humans, the immunoglobulin-like domain D1 of the CD155 polypeptide binds TIGIT. CD155 is also known as PVR (poliovirus receptor) or nectin-like 5. Yu et al. Nat. Immunol. 10:48-57 (2009). Generally, CD155 is expressed as a cell surface protein on a variety of cells, e.g., endothelial cells, monocytes, epithelia, central nervous system, dendritic cells, macrophages, and other antigen-presenting cells, e.g., but not limited to cancer cells. CD155 is a ligand for TIGIT with a higher affinity than CD112 (as discussed above).

As used herein, the term "CD155" generally refers to a CD155 polypeptide or a CD155 polynucleotide that is similar or identical to the sequence of a wild-type CD155.

In some embodiments, the term "CD155" refers to a CD155 polypeptide having an amino acid sequence that is at least 70% or more (including at least 75%, at least 80%, at least 85%, at least 90%, at least 95%, at least 97%, at least 99%, or 100%) identical to that of a wild-type CD155, and is capable of binding TIGIT and suppressing Th1 and/or Th17 responses. In some embodiments, the CD155 polypeptide can also increase expression and/or activity of Fgl2 to mediate the suppression of Thl and/or Th17 responses.

In some embodiments, the term "CD155" refers to a CD155 polynucleotide having a nucleotide sequence that is at least 70% or more (including at least 75%, at least 80%, at least 85%, at least 90%, at least 95%, at least 97%, at least 99%, or 100%) identical to that of a wild-type CD155 polynucleotide or a portion thereof, and encodes a CD155 polypeptide as described herein.

The wild-type CD155 sequences of various species are available on the world wide web from the NCBI, including human, mouse, and chimpanzee. For example, the nucleotide sequence encoding human CD155 is available at NCBI under Accession No. NM_001135768, NM_001135769, NM_001135770, or NM_006505 and its corresponding amino acid sequence is under Accession No. NP_001129240, NP_ 001129241, NP_001129242 or NP_006496.

Where the term "CD155" refers to a *CD155* polypeptide, the term "CD155 polypeptide" also encompasses a portion or fragment of such a *CD155* polypeptide that retains at least about 70% or more (including at least 75%, at least 80%, at least 85%, at least 90%, at least 95%, at least 97%, at least 99%, or 100%) of the TIGIT binding activity of the wild-type CD155 polypeptide. The term "CD155 polypeptide" as used herein also encompasses conservative substitution variants of a CD155 polypeptide that retain at least about 70% or more (including at least 75%, at least 80%, at least 85%, at least 90%, at least 95%, at least 97%, at least 99%, or 100%) of the TIGIT binding activity of the wild-type CD155 polypeptide. Accordingly, a CD155 polypeptide refers to any form of CD155 that can bind TIGIT, including functional variants of CD155. For example, in some embodiments, a CD155 polypeptide can be a full-length CD155. In some embodiments, a CD155 polypeptide refers to a functional domain or domains (e.g., one or more extracellular domains) of CD155 that binds TIGIT and induces immunosuppression and expression and/or activity of Fgl2.

The term "CD155 antagonist" is used interchangeably with the term "CD155 inhibitor" and refers to an agent that interferes with the normal functioning of CD155, either by decreasing transcription or translation of CD155-encoding nucleic acid, or by inhibiting or blocking CD155 polypeptide activity, or both. Examples of CD155 antagonists include, but are not limited to, antisense polynucleotides, interfering RNAs, catalytic RNAs, RNA-DNA chimeras, CD155-specific aptamers, anti-CD155 antibodies, CD155-binding fragments of anti-CD155 antibodies, CD155-binding small molecules, CD155-binding peptides, and other polypeptides that specifically bind CD155 (including, but not limited to, CD155-binding fragments of soluble TIGIT, optionally fused to one or more additional domains), such that the interaction between the CD155 antagonist and CD155 results in a reduction or cessation of CD155 activity or expression. It will be understood by one of ordinary skill in the art that in some instances, a *CD155* antagonist can antagonize one *CD155* activity without affecting another CD155 activity. For example, a desirable CD155 antagonist for use in certain of the methods herein is a CD155 antagonist that antagonizes CD155 binding to TIGIT, e.g., without affecting or minimally affecting any of the other CD155 interactions.

In some embodiments, a CD155 inhibitor is an agent that directly or indirectly inhibits or reduces CD155 binding to TIGIT, which in turn induces suppression of proinflammatory Th1 and/or Th17 responses. Accordingly, a CD155 inhibitor can target the CD155 ligand itself or its corresponding receptor, or any molecule that regulates expression of CD155. Examples of *CD155* inhibitors include, without limitations, anti-CD155 molecules, soluble TIGIT molecules, and a combination thereof. A CD155 inhibitor can be a protein, a peptide, a peptidomimetic, an aptamer, a nucleic acid, an antibody, a small molecule, a vaccine, or any combinations thereof.

In some embodiments, a CD155 inhibitor is an anti-CD155 antibody (e.g., an anti-human CD155 antibody). Anti-CD155 antibodies are commercially available (e.g., from BioLegend (Clone SKII.4; Cat. No. 337609), Affymetrix eBioscience (Clone 2H7CD155; Cat. No. 12-1550-41), or R&D Systems (Clone 300907; Cat. No. MAB25301)).

In some embodiments, a CD155 inhibitor can be a fragment or variant of CD155 itself, e.g., a fragment that binds TIGIT but does not transmit an immunosuppressive signal (e.g., via Fgl2 expression). A *CD155* inhibitor of this type can be a dominant negative inhibitor. For example, a CD155 inhibitor is a recombinant soluble CD155 protein (e.g., with the transmembrane domain substantially removed).

As used herein, the term "CD155 agonist" refers to an agent that enhances or stimulates the normal functioning of CD155, by increasing transcription or translation of CD155-encoding nucleic acid, and/or by inhibiting or blocking activity of a molecule that inhibits *CD155* expression or CD155 activity, and/or by enhancing normal CD155 activity (including, but not limited to, enhancing the stability of CD155 or enhancing binding of CD155 to one or more target receptors such as TIGIT). For example, the *CD155* agonist can be selected from an antibody, an antigen-binding fragment, an aptamer, an interfering RNA, a small molecule, a peptide, an antisense molecule, and another binding polypeptide. In another example, the *CD155* agonist can be a polynucleotide selected from an aptamer, interfering RNA, or antisense molecule that interferes with the transcription and/or translation of a *CD155* molecule. It will be understood by one of ordinary skill in the art that in some instances, a CD155 agonist can agonize one CD155 activity without affecting another CD155 activity. For example, a desirable CD155 agonist for use in certain of the methods herein is a CD155 agonist that agonizes CD155 binding to TIGIT, e.g., without affecting or minimally affecting any of the other *CD155* interactions.

In some embodiments, a CD155 agonist is an agent that directly or indirectly enhances or stimulates CD155 binding to TIGIT, which in turn induces the TIGIT-mediated suppression of proinflammatory Th1 and/or Th17 responses. Accordingly, a CD155 agonist can target the CD155 ligand itself or its corresponding receptor, or any molecule that modulates expression of CD155. CD155 agonists can be a protein, a peptide, peptidomimetic, an aptamer, a nucleic acid, an antibody, a small molecule, a vaccine, a fusion protein, or any combinations thereof. Exemplary CD155 agonists include recombinant CD155 proteins or peptides.

*CD155* antagonists or agonists can be obtained from known sources or prepared using known techniques such as recombinant or synthetic technology. The nucleic acid and protein sequences of CD155 and its ligands of different species (e.g., but not limited to, human, mouse, and monkey) are known in the art, e.g., accessible at world wide web from NCBI. Thus, one of skill in the art can generate CD155 antagonists or agonists based on these sequences using art-recognized molecular technologies such as cloning and expression technologies. For example, a human CD155 antagonist (e.g., an antibody) or agonist (e.g., recombinant protein) can be generated using protein based on the nucleic acid sequence of human CD155 accessible at NCBI under Accession No. NM_001135768, NM_001135769, NM_001135770, or NM_006505 and its corresponding amino acid sequence is under Accession No. NP_001129240, NP_001129241, NP_001129242 or NP_006496. Additionally or alternatively, one of skill in the art can generate *CD155* antagonists or agonists based on the crystal structure of *CD155* known in the art. See, e.g., Zhang et al. Proc Natl Acad Sci U.S.A. (2008)105: 18284-18289.

### Pharmaceutical compositions for treatment of immune-related diseases or disorders

Pharmaceutical compositions for treatment of cancer and/or infections (including, e.g., but not limited to chronic viral infection, intracellular bacterial infection, extracellular bacterial infection, and/or fungal infection) are also provided herein. More specifically, the pharmaceutical composition comprises a pharmaceutically-acceptable excipient and at least one (including, e.g., at least two, at least three or more) of the following therapeutic agents: (a) a TIGIT inhibitor; (b) an IL-33 inhibitor; (c) an ST2 inhibitor; and (d) an Fgl2 inhibitor. For example, in some embodiments, the composition can comprise a TIGIT inhibitor and an IL-33 inhibitor, or a TIGIT inhibitor and an ST2 inhibitor. In some embodiments, the composition can comprise a TIGIT inhibitor and an Fgl2 inhibitor. In some embodiments, the composition can comprise an IL-33 inhibitor and an Fgl2 inhibitor, or an ST2 inhibitor and an Fgl2 inhibitor. In some embodiments, the composition can comprise a TIGIT inhibitor, an IL-33 inhibitor and/or an ST2 inhibitor, and an Fgl2 inhibitor.

In some embodiments, pharmaceutical compositions for treatment of cancer can further comprise an anti-cancer agent. Examples of an anti-cancer agent include, but are not limited to, vaccine, chemotherapy, targeted therapy (e.g., kinase inhibitors), radiation therapy, surgery, immunotherapy, and any combinations thereof.

In some embodiments, pharmaceutical compositions for treatment of extracellular and/or intracellular bacterial infection can further comprise an anti-bacterial agent.

In some embodiments, pharmaceutical compositions for treatment of fungal infection can further comprise an anti-fungal agent.

In some embodiments, pharmaceutical compositions for treatment of chronic viral infections can further comprise an anti-viral agent (e.g., small molecules and/or immunotherapy) as described herein. Examples of anti-viral agents include, but are not limited to, virus protein specific antibodies, reverse transcriptase inhibitors, protease inhibitors, immunomodulatory agents (e.g., cytokines, various nucleoside analogs, and/or Zn²⁺), plant extracts demonstrated to have an antiviral effect, and any combinations thereof.

In some embodiments, pharmaceutical compositions for treatment of asthma, allergy, and/or atopy can further comprise an anti-asthmatic agent, an anti-allergy agent, and/or an anti-atopic agent. Examples of anti-asthmatic agents include, but are not limited to beta adrenergic agonists, xanthine derivatives, corticosteroids, antileukotrienes, and any combinations thereof. Exemplary anti-allergy agents and anti-atopic agents include, but are not limited to antihistamines, corticosteroids, and combinations thereof.

Examples of an immunotherapy for treatment of cancer, infections, and/or asthma, allergy and/or atopy can comprise an agent that increases a proinflammatory T cell response and/or an agent that suppresses an anti-inflammatory T cell response.

Pharmaceutical compositions for treatment of inflammatory diseases or disorders are also provided herein. In some embodiments, the pharmaceutical composition for treatment of an inflammatory disease or disorder where an inhibition of Th1 and/or Th17 responses and/or a shift of balance toward Th2 responses is desirable (e.g., autoimmune diseases and/or parasitic infection) can comprise a pharmaceutically-acceptable excipient and at least one (including, e.g., at least two, at least three or more) of the following therapeutic agents: (a) a TIGIT agonist; (b) an IL-33 agonist; (c) an ST2 agonist; and (d) an Fgl2 agonist. For example, in some embodiments, the composition can comprise a TIGIT agonist and an IL-33 agonist, or a TIGIT agonist and an ST2 agonist. In some embodiments, the composition can comprise a TIGIT agonist and an Fgl2 agonist. In some embodiments, the composition can comprise an IL-33 agonist and an Fgl2 agonist, or an ST2 agonist and an Fgl2 agonist. In some embodiments, the composition can comprise a TIGIT agonist, an IL-33 agonist and/or an ST2 agonist, and an Fgl2 agonist.

In some embodiments, the pharmaceutical composition can further comprise an agent for treatment of an inflammatory disease or disorder where an inhibition of Thl and/or Th17 responses and/or a shift of balance toward Th2 responses is desirable. For example, the agent can comprise an agent that increases an anti-inflammatory T cell response and/or an agent that suppresses a proinflammatory T cell response.

The phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" as used herein means a pharmaceutically acceptable material, composition or vehicle, such as a liquid or solid filler, diluent, excipient, solvent, media, encapsulating material, manufacturing aid (e.g., lubricant, talc magnesium, calcium or zinc stearate, or steric acid), or solvent encapsulating material, involved in maintaining the stability, solubility, or activity of, an agent for modulating expression and/or activity of TIGIT, Fgl2 and/or IL-33. Each carrier must be "acceptable" in the sense of being compatible with the other ingredients of the formulation and not injurious to the patient. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, methylcellulose, ethyl cellulose, microcrystalline cellulose and cellulose acetate; (4) powdered tragacanth; (5) malt; (6) gelatin; (7) excipients, such as cocoa butter and suppository waxes; (8) oils, such as peanut oil, cottonseed oil, safflower oil, sesame oil, olive oil, corn oil and soybean oil; (9) glycols, such as propylene glycol; (10) polyols, such as glycerin, sorbitol, mannitol and polyethylene glycol (PEG); (11) esters, such as ethyl oleate and ethyl laurate; (12) agar; (13) buffering agents, such as magnesium hydroxide and aluminum hydroxide; (14) alginic acid; (15) pyrogen-free water; (16) isotonic saline; (17) Ringer's solution; (19) pH buffered solutions; (20) polyesters, polycarbonates and/or polyanhydrides; (21) bulking agents, such as polypeptides and amino acids (22) serum components, such as serum albumin, HDL and LDL; (23) C2-C12 alchols, such as ethanol; and (24) other non-toxic compatible substances employed in pharmaceutical formulations. Release agents, coating agents, preservatives, and antioxidants can also be present in the formulation. The terms such as "excipient", "carrier", "pharmaceutically acceptable carrier" or the like are used interchangeably herein.

The agents for modulating expression and/or activity of TIGIT, Fgl2 and/or IL-33 can be specially formulated for administration of the compound to a subject in solid, liquid or gel form, including those adapted for the following: (1) parenteral administration, for example, by subcutaneous, intramuscular, intravenous or epidural injection as, for example, a sterile solution or suspension, or sustained-release formulation; (2) topical application, for example, as a cream, ointment, or a controlled-release patch or spray applied to the skin; (3) intravaginally or intrarectally, for example, as a pessary, cream or foam; (4) ocularly; (5) transdermally; (6) transmucosally; or (79) nasally. Additionally, a bispecific or multispecific polypeptide agent can be implanted into a patient or injected using a drug delivery system. See, for example, Urquhart, et al., Ann. Rev. Pharmacol. Toxicol. 24: 199-236 (1984); Lewis, ed. "Controlled Release of Pesticides and Pharmaceuticals" (Plenum Press, New York, 1981); U.S. Pat. No. 3,773,919; and U.S. Pat. No. 35 3,270,960.

Further embodiments of the formulations and modes of administration of an agent for expression and/or activity of TIGIT, Fgl2 and/or IL-33 that can be used in the methods described herein are illustrated below.

*Parenteral Dosage Forms.* Parenteral dosage forms of an agent for modulating expression and/or activity of TIGIT, Fgl2 and/or IL-33 can also be administered to a subject by various routes, including, but not limited to, subcutaneous, intravenous (including bolus injection), intramuscular, and intraarterial. Since administration of parenteral dosage forms typically bypasses the patient's natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, controlled-release parenteral dosage forms, and emulsions.

Suitable vehicles that can be used to provide parenteral dosage forms of the disclosure are well known to those skilled in the art. Examples include, without limitation: sterile water; water for injection USP; saline solution; glucose solution; aqueous vehicles such as but not limited to, sodium chloride injection, Ringer's injection, dextrose Injection, dextrose and sodium chloride injection, and lactated Ringer's injection; water-miscible vehicles such as, but not limited to, ethyl alcohol, polyethylene glycol, and propylene glycol; and non-aqueous vehicles such as, but not limited to, corn oil, cottonseed oil, peanut oil, sesame oil, ethyl oleate, isopropyl myristate, and benzyl benzoate.

*Aerosol formulations.* An agent for modulating expression and/or activity of TIGIT, Fgl2 and/or IL-33 can be packaged in a pressurized aerosol container together with suitable propellants, for example, hydrocarbon propellants like propane, butane, or isobutane with conventional adjuvants. An agent for modulating expression and/or activity of TIGIT, Fgl2 and/or IL-33 can also be administered in a non-pressurized form such as in a nebulizer or atomizer. An agent for modulating expression and/or activity of TIGIT, Fgl2 and/or IL-33 can also be administered directly to the airways in the form of a dry powder, for example, by use of an inhaler.

Suitable powder compositions include, by way of illustration, powdered preparations of an agent for modulating expression and/or activity of TIGIT, Fgl2 and/or IL-33 thoroughly intermixed with lactose, or other inert powders acceptable for intrabronchial administration. The powder compositions can be administered via an aerosol dispenser or encased in a breakable capsule which can be inserted by the subject into a device that punctures the capsule and blows the powder out in a steady stream suitable for inhalation. The compositions can include propellants, surfactants, and co-solvents and can be filled into conventional aerosol containers that are closed by a suitable metering valve.

Aerosols for the delivery to the respiratory tract are known in the art. See for example, Adjei, A. and Garren, J. Pharm. Res., 1: 565-569 (1990); Zanen, P. and Lamm, J.-W. J. Int. J. Pharm., 114: 111-115 (1995); Gonda, I. "Aerosols for delivery of therapeutic and diagnostic agents to the respiratory tract," in Critical Reviews in Therapeutic Drug Carrier Systems, 6:273-313 (1990); Anderson et al., Am. Rev. Respir. Dis., 140: 1317-1324 (1989)) and have potential for the systemic delivery of peptides and proteins as well (Patton and Platz, Advanced Drug Delivery Reviews, 8:179-196 (1992)); Timsina et. al., Int. J. Pharm., 101: 1-13 (1995); and Tansey, I. P., Spray Technol. Market, 4:26-29 (1994); French, D. L., Edwards, D. A. and Niven, R. W., Aerosol Sci., 27: 769-783 (1996); Visser, J., Powder Technology 58: 1-10 (1989)); Rudt, S. and R. H. Muller, J. Controlled Release, 22: 263-272 (1992); Tabata, Y, and Y. Ikada, Biomed. Mater. Res., 22: 837-858 (1988); Wall, D. A., Drug Delivery, 2: 10 1-20 1995); Patton, J. and Platz, R., Adv. Drug Del. Rev., 8: 179-196 (1992); Bryon, P., Adv. Drug. Del. Rev., 5: 107-132 (1990); Patton, J. S., et al., Controlled Release, 28: 15 79-85 (1994); Damms, B. and Bains, W., Nature Biotechnology (1996); Niven, R. W., et al., Pharm. Res., 12(9); 1343-1349 (1995); and Kobayashi, S., et al., Pharm. Res., 13(1): 80-83 (1996).

The formulations of the agents for modulating expression and/or activity of TIGIT, Fgl2 and/or IL-33 further encompass anhydrous pharmaceutical compositions and dosage forms comprising the disclosed compounds as active ingredients, since water can facilitate the degradation of some compounds. For example, the addition of water *(e.g.,* 5%) is widely accepted in the pharmaceutical arts as a means of simulating long-term storage in order to determine characteristics such as shelf life or the stability of formulations over time. See, *e.g.,* Jens T. Carstensen, Drug Stability: Principles & Practice, 379-80 (2nd ed., Marcel Dekker, NY, N.Y.: 1995). Anhydrous pharmaceutical compositions and dosage forms of the disclosure can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected. Anhydrous compositions are preferably packaged using materials known to prevent exposure to water such that they can be included in suitable formulary kits. Examples of suitable packaging include, but are not limited to, hermetically sealed foils, plastics, unit dose containers (e.g., vials) with or without desiccants, blister packs, and strip packs.

*Controlled and Delayed Release Dosage Forms.* In some embodiments of the methods described herein, an agent for modulating expression and/or activity of TIGIT, Fgl2 and/or IL-33 can be administered to a subject by controlled- or delayed-release means. Ideally, the use of an optimally designed controlled-release preparation in medical treatment is characterized by a minimum of drug substance being employed to cure or control the condition in a minimum amount of time. Advantages of controlled-release formulations include: 1) extended activity of the drug; 2) reduced dosage frequency; 3) increased patient compliance; 4) usage of less total drug; 5) reduction in local or systemic side effects; 6) minimization of drug accumulation; 7) reduction in blood level fluctuations; 8) improvement in efficacy of treatment; 9) reduction of potentiation or loss of drug activity; and 10) improvement in speed of control of diseases or conditions. (Kim, Cherng-ju, Controlled Release Dosage Form Design, 2 (Technomic Publishing, Lancaster, Pa.: 2000)). Controlled-release formulations can be used to control a compound of formula (I)'s onset of action, duration of action, plasma levels within the therapeutic window, and peak blood levels. In particular, controlled- or extended-release dosage forms or formulations can be used to ensure that the maximum effectiveness of a compound of formula (I) is achieved while minimizing potential adverse effects and safety concerns, which can occur both from under-dosing a drug (*i.e*., going below the minimum therapeutic levels) as well as exceeding the toxicity level for the drug.

A variety of known controlled- or extended-release dosage forms, formulations, and devices can be adapted for use with the agents for modulating expression and/or activity of TIGIT, Fgl2 and/or IL-33. Examples include, but are not limited to, those described in U.S. Pat. Nos.: 3,845,770; 3,916,899; 3,536,809; 3,598,123; 4,008,719; 5674,533; 5,059,595; 5,591 ,767; 5,120,548; 5,073,543; 5,639,476; 5,354,556; 5,733,566; and 6,365,185 B1. These dosage forms can be used to provide slow or controlled-release of one or more active ingredients using, for example, hydroxypropylmethyl cellulose, other polymer matrices, gels, permeable membranes, osmotic systems (such as OROS® (Alza Corporation, Mountain View, Calif. USA)), multilayer coatings, microparticles, liposomes, or microspheres or a combination thereof to provide the desired release profile in varying proportions. Additionally, ion exchange materials can be used to prepare immobilized, adsorbed salt forms of the disclosed compounds and thus effect controlled delivery of the drug. Examples of specific anion exchangers include, but are not limited to, Duolite® A568 and Duolite® AP143 (Rohm&Haas, Spring House, Pa. USA).

In some embodiments, an agent for modulating expression and/or activity of TIGIT, Fgl2 and/or IL-33 for use in the methods described herein is administered to a subject by sustained release or in pulses. Pulse therapy is not a form of discontinuous administration of the same amount of a composition over time, but comprises administration of the same dose of the composition at a reduced frequency or administration of reduced doses. Sustained release or pulse administrations are particularly preferred when the disorder occurs continuously in the subject, for example where the subject has continuous or chronic symptoms of an infection. Each pulse dose can be reduced and the total amount of the agent for modulating expression and/or activity of TIGIT, Fgl2 and/or IL-33 administered over the course of treatment to the patient is minimized.

The interval between pulses, when necessary, can be determined by one of ordinary skill in the art. Often, the interval between pulses can be calculated by administering another dose of the composition when the composition or the active component of the composition is no longer detectable in the subject prior to delivery of the next pulse. Intervals can also be calculated from the *in vivo* half-life of the composition. Intervals can be calculated as greater than the *in vivo* half-life, or 2, 3, 4, 5 and even 10 times greater the composition half-life. Various methods and apparatus for pulsing compositions by infusion or other forms of delivery to the patient are disclosed in U.S. Pat. Nos. 4,747,825; 4,723,958; 4,948,592; 4,965,251 and 5,403,590.

### Methods for modulating Th17 response

In still another aspect of the present disclosure, methods for modulating Th17 response based on the level of TIGIT, Fgl2 and/or IL-33 activity or expression are also provided herein. For example, in some embodiments, methods for enhancing Th17 response comprise contacting Tregs with or administering to a subject with a deficiency in Th17 response a TIGIT inhibitor, a Flg2 inhibitor and/or an IL-33 inhibitor. In other embodiments, methods for reducing or suppressing Th17 response comprise contacting Tregs with or administering to a subject with an over-stimulation in Th17 response a TIGIT agonist, a Flg2 agonist and/or an IL-33 agonist.

As used herein, the term "Th17 response" refers to response of T helper 17 cells (Th17) producing interleukin 17 (IL-17). They are developmentally distinct from Th1 and Th2 cells.

### Sample

In accordance with various embodiments described herein, a sample, including any fluid or specimen (processed or unprocessed) or other biological sample, can be subjected to the methods of various aspects disclosed herein.

In some embodiments, the sample can include a biological fluid obtained from a subject. Exemplary biological fluids obtained from a subject can include, but are not limited to, blood (including whole blood, plasma, cord blood and serum), lactation products (e.g., milk), amniotic fluids (e.g., a sample collected during amniocentesis), sputum, saliva, urine, semen, cerebrospinal fluid, bronchial aspirate, perspiration, mucus, liquefied feces, synovial fluid, lymphatic fluid, tears, tracheal aspirate, and fractions thereof. In some embodiments, a biological fluid can include a homogenate of a tissue specimen (e.g., biopsy) from a subject. In one embodiment, a test sample can comprises a suspension obtained from homogenization of a solid sample obtained from a solid organ or a fragment thereof.

In some embodiments, a sample can be obtained from a subject who has or is suspected of having an immune-related disease or disorder, e.g., cancer and/or inflammatory disease or disorder. In some embodiments, the sample can be obtained from a subject who has or is suspected of having cancer, or who is suspected of having a risk of developing cancer. In some embodiments, the sample can be obtained from a subject who has or is suspected of having an inflammatory disease or disorder or who is suspected of having a risk of developing an inflammatory disease or disorder.

In some embodiments, a sample can be obtained from a subject who is being treated for the immune-related disease or disorder. In other embodiments, the sample can be obtained from a subject whose previously-treated disease or disorder is in remission. In other embodiments, the test sample can be obtained from a subject who has a recurrence of a previously-treated disease or disorder. For example, in the case of cancer such as breast cancer, a test sample can be obtained from a subject who is undergoing a cancer treatment, or whose cancer was treated and is in remission, or who has cancer recurrence.

As used herein, a "subject" can mean a human or an animal. Examples of subjects include primates (e.g., humans, and monkeys). Usually the animal is a vertebrate such as a primate, rodent, domestic animal or game animal. Primates include chimpanzees, cynomologous monkeys, spider monkeys, and macaques, e.g., Rhesus. Rodents include mice, rats, woodchucks, ferrets, rabbits and hamsters. Domestic and game animals include cattle, cows, horses, pigs, deer, bison, sheep, goats, buffalo, feline species, e.g., domestic cat, canine species, e.g., dog, fox, wolf, and avian species, e.g., chicken, ducks, geese, turkeys, emu, ostrich. A patient or a subject includes any subset of the foregoing, e.g., all of the above, or includes one or more groups or species such as humans, primates or rodents. In certain embodiments of the aspects of the present disclosure, the subject is a mammal, e.g., a primate, e.g., a human. The terms "patient" and "subject" are used interchangeably herein. A subject can be male or female. The term "patient" and "subject" does not denote a particular age. Thus, any mammalian subjects from adult (e.g., young adult, middle-aged adult or senior adult) to pediatric subjects (e.g, infant, child, adolescent) to newborn subjects, as well as fetuses, are intended to be covered. When the term is used in conjunction with administration of a compound or drug, then the subject or patient has been the object of treatment, observation, and/or administration of the compound or drug. The methods and/or pharmaceutical compositions described herein are also contemplated to be used to treat domesticated animals or pets such as cats and dogs.

In one embodiment, the subject or patient is a mammal. The mammal can be a human, non-human primate, mouse, rat, dog, cat, horse, or cow, but are not limited to these examples. In one embodiment, the subject is a human being. In another embodiment, the subject can be a domesticated animal and/or pet.

In some embodiments, the sample can be a blood sample or a sample of a tissue at a target site from a patient. For example, for treatment of cancer, the sample can be a blood sample or a tumor biopsy from a patient. For treatment of inflammatory diseases or disorders, the sample can be a blood sample or a tissue biopsy from an inflammatory site in a patient. Without wishing to be bound by theory, since Fg2 and IL-33 are soluble molecules while TIGIT is a cell surface molecule, Fgl2 and IL-33 can be more easily measured, e.g., from a blood sample, as compared to TIGIT measured, e.g., from a tissue sample.

It should be understood that this invention is not limited to the particular methodology, protocols, and reagents, etc., described herein and as such may vary. The terminology used herein is for the purpose of describing particular embodiments only, and is not intended to limit the scope of the present invention, which is defined solely by the claims.

### Some selected definitions

As used herein and in the claims, the singular forms include the plural reference and vice versa unless the context clearly indicates otherwise. The term "or" is inclusive unless modified, for example, by "either." Other than in the operating examples, or where otherwise indicated, all numbers expressing quantities of ingredients or reaction conditions used herein should be understood as modified in all instances by the term "about." The term "about" with respect to numerical values means within 5%

As used herein, the term "greater than" in the context of an increase in the activity level and/or expression of a target molecule (e.g., TIGIT, Fgl2, and/or IL-33) relative to its corresponding reference (e.g., a TIGIT reference, an Fgl2 reference and/or an IL-33 reference), the increase can be at least about 30% or more, including, e.g., at least about 40%, at least about 50%, at least about 60%, at least about 70%, at leasta bout 80%, at least about 90%, at least about 95%, at least about 100% or more. In some embodiments, the increase can be at least about 1.1-fold or more, including, e.g., at least about 2-fold, at least about 3-fold, at least about 4-fold, at least about 5-fold, at least about 6-fold, at least about 7-fold, at least about 8-fold, at least about 9-fold, at least about 10-fold, at least about 50-fold, at least about 100-fold, or more.

The term "antibody" as used herein, whether in reference to an anti-TIGIT, anti-Fgl2, or anti-IL-33 antibody, refers to a full length antibody or immunoglobulin, IgG, IgM, IgA, IgD or IgE molecules, or a protein portion thereof that comprises only a portion of an intact antibody, generally including an antigen binding site of the intact antibody and thus retaining the ability to bind a target, such as an epitope or antigen. Examples of portions of antibodies or epitope-binding proteins encompassed by the present definition include: (i) the Fab fragment, having VL, CL, VH and CH1 domains; (ii) the Fab' fragment, which is a Fab fragment having one or more cysteine residues at the C-terminus of the CH1 domain; (iii) the Fd fragment having VH and CH1 domains; (iv) the Fd' fragment having VH and CH1 domains and one or more cysteine residues at the C terminus of the CH1 domain; (v) the Fv fragment having the VL and VH domains of a single arm of an antibody; (vi) the dAb fragment (Ward et al., 341 Nature 544 (1989)) which consists of a VH domain or a VL domain that binds antigen; (vii) isolated CDR regions or isolated CDR regions presented in a functional framework; (viii) F(ab')2 fragments which are bivalent fragments including two Fab' fragments linked by a disulfide bridge at the hinge region; (ix) single chain antibody molecules (e.g., single chain Fv; scFv) (Bird et al., 242 Science 423 (1988); and Huston et al., 85 PNAS 5879 (1988)); (x) "diabodies" with two antigen binding sites, comprising a heavy chain variable domain (VH) connected to a light chain variable domain (VL) in the same polypeptide chain (see, e.g., EP 404,097; WO 93/11161; Hollinger et al., 90 PNAS 6444 (1993)); (xi) "linear antibodies" comprising a pair of tandem Fd segments (VH-CH1-VH-CH1) which, together with complementary light chain polypeptides, form a pair of antigen binding regions (Zapata et al., 8 Protein Eng. 1057 (1995); and U.S. Patent No. 5,641,870).

"Antibodies" include antigen-binding portions of antibodies such as epitope- or antigen-binding peptides, paratopes, functional CDRs; recombinant antibodies; chimeric antibodies; tribodies; midibodies; or antigen-binding derivatives, analogs, variants, portions, or fragments thereof.

The term "aptamer" refers to a nucleic acid molecule that is capable of binding to a target molecule, such as a polypeptide. For example, an aptamer of the invention can specifically bind to a TIGIT, Fgl2 and/or IL-33 polypeptide, or to a molecule in a signaling pathway that modulates the expression and/or activity of TIGIT, Fgl2 and/or IL-33. The generation and therapeutic use of aptamers are well established in the art. See, e.g., U.S. Pat. No. 5,475,096.

As used herein, the term "fusion protein" refers to a fusion polypeptide comprising a target polypeptide (e.g., TIGIT, Fgl2 or IL-33) and a second, heterologous fusion partner polypeptide. The fusion partner can, for example, increase the in vivo stability of the fusion polypeptide, modulate its biological activity or localization, or facilitate purification of the fusion polypeptide. Exemplary heterologous fusion partner polypeptides that can be used to generate such fusion polypeptides for use in the compositions and methods described herein include, but are not limited to, polyhistidine (His or 6His tag), Glu-Glu tag, glutathione S transferase (GST), thioredoxin, polypeptide A, polypeptide G, an immunoglobulin heavy chain constant region (Fc), and maltose binding polypeptide (MBP), which are particularly useful for isolation of the fusion polypeptides by affinity chromatography. For the purpose of affinity purification, relevant matrices for affinity chromatography, such as glutathione-, amylase-, and nickel- or cobalt- conjugated resins are used. Fusion polypeptides can also include "epitope tags," which are usually short peptide sequences for which a specific antibody is available. Well known epitope tags for which specific monoclonal antibodies are readily available include FLAG, influenza virus haemagglutinin (HA), and c myc tags. In some embodiments, the fusion polypeptides can have a protease cleavage site, such as for Factor Xa or Thrombin, which allows the relevant protease to partially digest the fusion polypeptides and thereby liberate the recombinant polypeptides therefrom. The liberated polypeptides can then be isolated from the fusion polypeptides by subsequent chromatographic separation.

Unless otherwise defined herein, scientific and technical terms used in connection with the present application shall have the meanings that are commonly understood by those of ordinary skill in the art to which this disclosure belongs. It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims. Definitions of common terms in immunology and molecular biology can be found in The Merck Manual of Diagnosis and Therapy, 19th Edition, published by Merck Sharp & Dohme Corp., 2011 (ISBN 978-0-911910-19-3); Robert S. Porter et al. (eds.), The Encyclopedia of Molecular Cell Biology and Molecular Medicine, published by Blackwell Science Ltd., 1999-2012 (ISBN 9783527600908); and Robert A. Meyers (ed.), Molecular Biology and Biotechnology: a Comprehensive Desk Reference, published by VCH Publishers, Inc., 1995 (ISBN 1-56081-569-8); Immunology by Werner Luttmann, published by Elsevier, 2006; Janeway's Immunobiology, Kenneth Murphy, Allan Mowat, Casey Weaver (eds.), Taylor & Francis Limited, 2014 (ISBN 0815345305, 9780815345305); Lewin's Genes XI, published by Jones & Bartlett Publishers, 2014 (ISBN-1449659055); Michael Richard Green and Joseph Sambrook, Molecular Cloning: A Laboratory Manual, 4th ed., Cold Spring Harbor Laboratory Press, Cold Spring Harbor, N.Y., USA (2012) (ISBN 1936113414); Davis et al., Basic Methods in Molecular Biology, Elsevier Science Publishing, Inc., New York, USA (2012) (ISBN 044460149X); Laboratory Methods in Enzymology: DNA, Jon Lorsch (ed.) Elsevier, 2013 (ISBN 0124199542); Current Protocols in Molecular Biology (CPMB), Frederick M. Ausubel (ed.), John Wiley and Sons, 2014 (ISBN 047150338X, 9780471503385), Current Protocols in Protein Science (CPPS), John E. Coligan (ed.), John Wiley and Sons, Inc., 2005; and Current Protocols in Immunology (CPI) (John E. Coligan, ADA M Kruisbeek, David H Margulies, Ethan M Shevach, Warren Strobe, (eds.) John Wiley and Sons, Inc., 2003 (ISBN 0471142735, 9780471142737).

### EXAMPLES

### Example 1. Expression of TIGIT in subsets of regulatory T cells (Treg) cells

Regulatory T cells (Tregs) are a subset of CD4+ T cells that is marked by Foxp3 expression and act as a central component in regulating immune responses to pathogens and in maintaining self-tolerance. Other regulatory populations also contribute to this balance, but Foxp3+ Tregs are critical for maintaining immune homeostasis as demonstrated by the devastating multi-organ autoimmune disease caused by genetic deficiencies in Foxp3 (Brunkow et *al.,* 2001; Wildin et *al.,* 2001). Foxp3+ Tregs are not all identical, but comprised of multiple, functionally diverse subtypes with distinct phenotypes and specialized functions. Foxp3+ Tregs have been previously reported to specialize to selectively regulate specific effector T cell responses and control inflammation at defined anatomical tissue sites (Chaudhry et *al.,* 2009; Cipolletta et *al.,* 2012; Koch et *al.,* 2009; Zheng et *al.,* 2009). Although the transcription factors that differentially induce specialized suppressor functions in Tregs have been identified, the molecules that mediate these selective effector functions remain largely unknown. Identification of cytokines and cell surface molecules that mediate specialization of Treg function would allow the development of therapeutic approaches that target Tregs and selectively regulate specific types of T cell responses.

In conventional T cells, cytokines and co-stimulatory molecules act in concert for their differentiation and acquisition of effector functions. For example, OX40 was shown to augment Th2 responses by increasing IL-4 secretion and to favor the induction of Th9 cells (Flynn et *al.,* 1998; Xiao et *al.,* 2012). Similarly, ICOS regulates TFH expansion and critically contributes to Th17 function by regulating IL-23R expression in an IL-21 and c-Maf-dependent manner (Bauquet et *al.,* 2009). In Tregs, co-inhibitory molecules, such as PD-1 and CTLA-4 promote suppressive function. PD-1 plays an important role in iTreg stability and suppressive function (Francisco et *al.,* 2009). CTLA-4 is essential for Treg function (Wing et *al.,* 2008) and can mediate suppression by enabling Tregs to compete with effector T cells for co-stimulatory signals on APCs and by inducing the production of indoleamine 2,3-dioxygenase (IDO) in APCs, thereby limiting T cell proliferation (Fallarino et *al.,* 2003). While co-stimulatory molecules have been shown to promote effector functions of defined T helper lineages, there are no reports that implicate co-inhibitory molecules in the specialized function of Treg subsets, despite their important role in promoting the suppressive function of Tregs in general.

Recently, the novel co-inhibitory molecule TIGIT has been reported as an inhibitor of autoimmune responses (Joller et *al.,* 2011; Levin et *al.,* 2011). TIGIT can inhibit T cell responses by binding the ligand *CD155* on dendritic cells (DCs) and thereby inhibiting IL-12 while inducing IIL-10 production (Yu et *al.,* 2009). In addition, TIGIT engagement also directly inhibits T cell activation and proliferation (Joller et *al.,* 2011; Levin et al., 2011; Lozano et *al.,* 2012). Like other co-inhibitory molecules, TIGIT is highly expressed on Tregs (Levin et *al.,* 2011; Yu et *al.,* 2009); however, whether it plays a functional role in these cells has not been explored.

It was sought in this Example to determine whether TIGIT expression defines a functionally distinct Treg subset. To this end, expression of TIGIT was determined in natural as well as in vitro differentiated induced Tregs (nTregs and iTregs, respectively) populations (Figure 1A). nTregs could be separated into distinct TIGIT+ and TIGIT- populations, while TIGIT was uniformly upregulated in iTregs. To assess whether TIGIT functionally contributes to Treg differentiation, the ability of TIGIT-deficient T cells to differentiate into Foxp3+ iTreg in vitro was evaluated. As iTregs express high levels of TIGIT, it was next sought to determine whether TIGIT+ Tregs present in vivo might also be generated peripherally. However, TIGIT+ Tregs were primarily Neuropilin-1+ and express high levels of Helios, indicating that the majority of TIGIT+ Tregs are nTregs (Figures 7A and 7B). TIGIT+ Tregs also do not appear to be a terminally differentiated lineage as both TIGIT+ and TIGIT- Tregs can convert into the other subset as evidenced by the loss of TIGIT from TIGIT+ Tregs upon adoptive transfer and conversely gain of TIGIT expression where TIGIT- Tregs were injected (Figure 7C).

As nTregs can be separated into distinct TIGIT+ and TIGIT- populations, the functional differences between these two populations were also characterized. To this end, TIGIT+ and TIGIT- nTregs were sorted based on Foxp3-GFP reporter expression and their ability to suppress CD4+Foxp3- effector T cells was compared in vitro. TIGIT+ nTreg showed an increased ability to suppress TCR-stimulated proliferation of conventional T cells (Figure 1B). TIGIT therefore marks a functionally distinct subset of nTregs with superior suppressive capacity.

Next, it was sought to determine whether TIGIT+ Tregs are also detected in humans and whether they might represent a similarly potent Treg subset as in mice. TIGIT expression in human CD4+ T cells was analyzed and it was found that a large proportion of human Tregs are TIGIT+ (Figure 1C). In vitro suppression assays were then performed to assess whether human TIGIT+ and TIGIT- Tregs also differ in their suppressive capacity. Indeed, increased suppression by TIGIT+ Tregs was compared to TIGIT- Tregs (Figures 1D-1F), indicating that TIGIT+ Tregs are highly suppressive and may also represent a functionally distinct Treg subset in humans. Accordingly, TIGIT expression defines a functionally distinct Treg subset.

### Example 2. Characterization of TIGIT+ Tregs display an activated phenotype

To determine the differences between TIGIT+ and TIGIT- Tregs, their gene expression patterns were analyzed by microarray profiling. Overall, a total of 472 and 184 genes were over- or under-expressed in TIGIT+ cells relative to their TIGIT- counterparts (with an arbitrary cut-off of at fold change > 2 and t-test p<0.05; Figure 7D, Table 1). These belonged to several functional families including chemokines/cytokines or their receptors, transcription factors, and costimulatory and other surface receptors, as well as molecules typical of activated Treg cells such as Klrg1 and Il10. Overall, TIGIT+ Tregs seemed to display a more activated phenotype than their TIGIT- counterparts (Figures 7E and 7F). Despite appearing anergic in vitro, Tregs proliferate extensively upon activation in vivo. Therefore, it was sought to determine whether the activated phenotype of TIGIT+ Tregs that was observed translates into higher proliferation in vivo. TIGIT+ Tregs indeed expressed 3-fold higher levels of Ki67, which serves as an indirect marker for proliferation (Figure 7G). In addition, TIGIT+ Tregs also incorporated four times higher amounts of BrdU than their TIGIT- counterparts when we labeled proliferating cells in vivo (Figure 2H). The activated phenotype of TIGIT+ Tregs characterized by transcriptional profiling therefore translates into a higher rate of proliferation in vivo.

### Example 3. Comparison of TIGIT+ Tregs with pro-inflammatory T cell lineages

Tregs are generally known to share a number of features with the effector population they suppress, including the expression of chemokine receptors as well as the transcription factors that induce the development of those effector T cells (Chaudhry et *al.,* 2009; Chung et *al.,* 2011; Koch et *al.,* 2009; Linterman et *al.,* 2011; Zheng et *al.,* 2009). Unexpectedly, the pattern of chemokine receptors expressed by TIGIT+ Tregs does not overlap with that of any particular Th effector subset (Figure 2A), but includes receptors that are expressed by several lineages, mainly by the pro-inflammatory Th1 and Th17 subsets (Ccr2, Ccr5, Ccr6, Cxcr3, and Cxcr6), but to a lesser degree also those expressed by Th2 (Ccr3) or T_{FH} cells (Cxcr5). Without wishing to be bound by theory, these findings can indicate that TIGIT+ Tregs are equipped to target a broad spectrum of effector cells and tissues, for example, in some embodiments, under pro-inflammatory conditions.

Similarly, the transcription factors that are more highly expressed in TIGIT+ Tregs do not specifically fall within the fingerprint of a particular effector lineage (Figure 2B). On the contrary, transcription factors that are expressed at higher levels in TIGIT+ Tregs include those that are specific for Th1 (Tbx21) and Th17 cells (Rora, Rorc, Irf4, Ahr), while only minor or no differences could be observed in the expression of the Th2 lineage factor Gata3 and the TFH lineage specific transcription factor Bcl6 (Figure 3C). Prdm1 was expressed at higher levels in TIGIT+, which is consistent with higher production of IL-10 by TIGIT+ Tregs (Figure 2A, 2C and 8A).

The expression profile of TIGIT+ Tregs was analyzed in relation to signatures of the Treg subsets as described above. TIGIT+ Tregs were enriched for a gene set that distinguishes CXCR3+ Tregs (Figure 2D). Cells that express T-bet and were previously reported to be specialized in suppression of Th1 responses (Koch et *al.,* 2012; Koch et *al.,* 2009). IRF4 expression in Tregs is important for control of Th2 responses as demonstrated by the dysregulated Th2 responses observed in mice that lack IRF4 in Foxp3+ Tregs (Zheng et *al.,* 2009). Many of the IRF4-dependent genes are upregulated in TIGIT+ Tregs (Figure 2E), which is in line with the increased expression of IRF4 by TIGIT+ Tregs (Figure 2C). Further, it was found that TIGIT+ Tregs share features with Tregs from mice in which Foxp3 is modified by an N-terminal fusion with GFP (Figure 2F). The modification of As previously reported, Foxp3 can lead to modified interaction with Foxp3 cofactors (Bettini et *al.,* 2012; Fontenot et *al.,* 2005). Thus, in these mice the GFP-fusion altered the molecular characteristics of Foxp3, reducing HIF-1α and increasing IRF4 interactions, modifying the Treg transcriptome and resulting in enhanced suppression of Th2/Th17 responses but weaker suppression of Th1 responses (Bettini et *al.,* 2012; Darce et *al.,* 2012). Overall these data indicate that, rather than representing a subset specialized to suppress a specific T effector lineage, TIGIT+ Tregs express features of multiple pro-inflammatory Th subsets.

### Example 4. Functional roles of TIGIT+ Tregs in mediating immune suppression

Effector as well as regulatory T cell function is shaped through the cytokine environment as well as engagement of co-stimulatory ligands. By analyzing the transcriptional profile for differential expression of membrane receptors, a distinct pattern of co-stimulatory molecules in TIGIT+ vs. TIGIT- Tregs was indeed detected. TIGIT+ Tregs express higher levels of the co-stimulatory molecule ICOS, but also showed increased expression of a number of co-inhibitory molecules, such as CTLA-4, PD-1 (Pdcd1), Lag3, and Tim3 (Havcr2) (Figures 3A-3C, and Figures 8A-8C). Co-inhibitory molecules such as CTLA-4 and PD-1 not only serve as markers for T cell activation but also contribute to Treg stability and function (Francisco et *al.,* 2009; Wing et *al.,* 2008). Therefore, increased expression of these molecules by TIGIT+ Tregs indicates that they might be better equipped for mediating suppression. Indeed, Treg signature genes and mediators of the suppressive function were found to also be differentially expressed in TIGIT+ vs. TIGIT- Tregs (Figures 3C-3G and 8A-8C). TIGIT+ Tregs expressed higher levels of CTLA-4, CD25, and GITR but showed no or only slight differences in the expression of Lag3. In mice, CD39 and CCR6 expression in TIGIT+ Tregs is comparable to TIGIT- Tregs while human TIGIT+ Tregs show an upregulation of these markers (Figures 3C and 8B). When comparing TIGIT+ to TIGIT- Tregs, the immunosuppressive cytokine IL-10 appears to be primarily produced by TIGIT+ Tregs (Figure 3C). Expression of the master transcription factor of Treg function Foxp3 was not significantly different between TIGIT+ and TIGIT- Tregs in the microarray analysis, but when analyzed by qPCR and flow cytometry Foxp3 was expressed at higher levels in TIGIT+ Tregs (Figures 3F and 3G). Since Foxp3 regulates transactivation of CD25 (Fontenot et *al.,* 2003), higher expression of CD25 was also observed in TIGIT+ Tregs (Figures 3C and 3F). In addition, Treg effector molecules such as Granzyme B, IL-10 and Fgl2 were also expressed at significantly higher levels in TIGIT+ Tregs (Figures 3C-3D and 3G and Figure 8A). TIGIT+ Tregs therefore represent an activated, highly suppressive Treg subset.

### Example 5. TIGIT ligation induces the Treg effector molecule Fgl2

As TIGIT marks a highly suppressive Treg population, it was next sought to determine whether TIGIT ligation could directly induce Treg effectors. Among these, IL-10 and Fgl2 stood out as particularly interesting molecules as both were highly expressed in TIGIT+ Tregs (Figure 3G) and these molecules were previously reported to be able to suppress pro-inflammatory responses (Chan et *al.,* 2003; Kuhn et *al.,* 1993). To assess whether Fgl2 and IL-10 could be induced through TIGIT, effector and regulatory T cells were isolated and stimulated in vitro in the presence of an agonistic anti-TIGIT antibody (Ab). As shown in Figure 4A, *Il10* mRNA in effector T cells was slightly reduced by TIGIT. Without wishing to be bound by theory, this is most likely due to the inhibition of activation of effector T cells when TIGIT is engaged by the agonistic antibody. In contrast, TIGIT ligation triggered a 2-fold increase in *Il10* gene expression by Tregs in vitro. TIGIT did not induce a profound induction of Fgl2 mRNA in effector T cells. However, Fgl2 expression levels in Tregs were dramatically increased in the presence of agonistic anti-TIGIT Ab indicating that TIGIT signaling induces Fgl2 in TIGIT+ Tregs (Figures 4A and 4B). Taken together, this finding indicates that TIGIT ligation induces Il10 and Fgl2 mRNA in Tregs.

It was next sought to determine whether TIGIT was also able to induce Fgl2 and IL-10 in vivo. As the functional effects of Abs can differ dramatically in vitro and in vivo, the anti-TIGIT Ab was evaluated to assess whether the Ab also acted agonistically in vivo. To this end, mice were immunized and treated with anti-TIGIT Ab and Ag-specific proliferation was determined 10 days later. The anti-TIGIT Ab was indeed able to reduce the Ag-specific T cell response and therefore also acts agonistically in vivo (Figure 9A). Next, the same immunization and Ab treatment regimen were used, cells from spleen and LN were restimulated10 days after immunization for 2 days in vitro, and Fgl2 and IL-10 in the culture supernatants were analyzed. IL-10 could not be detected in these cultures (data not shown). However, Fgl2 was significantly increased in cell supernatants from anti-TIGIT treated mice without affecting Treg frequencies or composition (Figure 4C, Figure 9B). TIGIT therefore induces Fgl2 in vitro and in vivo.

To further investigate the role of IL-10 and Fgl2 as Treg effector molecules, it was next sought to determine whether neutralizing them would abolish the differences in suppressive capacity of TIGIT+ and TIGIT- Tregs observed in vitro. Blocking or deletion of IL-10 had no effect on suppression by TIGIT+ or TIGIT- Tregs in vitro (Figure 4D). Similarly, neutralizing or deleting Fgl2 had no effect on the suppression by TIGIT- Tregs, which express only minimal amounts of Fgl2 (Figures 4A and 4E). In contrast, neutralization of Fgl2 reduced the level of suppression from TIGIT+ Tregs to that observed for TIGIT- Tregs (Figure 4E), indicating that Fgl2 is a major effector molecule driving the increased suppression by TIGIT+ Tregs observed in vitro. Similarly, Fgl2-deficient TIGIT+ Tregs showed a significant decrease in their ability to suppress in vitro, down to the level observed in TIGIT- Tregs (Figure 4E). Therefore, TIGIT ligation triggers secretion of Fgl2 by Tregs, which enables them to act as highly potent suppressors.

To understand how TIGIT could induce Fgl2 expression, the genomic region of Fgl2 was searched for binding sites of transcription factors that showed differential expression in our microarray analysis of TIGIT+ vs. TIGIT- Tregs (Figure 2B). The analysis indicated that the Fgl2 gene contains binding sites for the transcription factor CEBPα, which was differentially expressed in TIGIT+ Tregs. Quantitative PCR confirmed that CEBPα is highly expressed in TIGIT+ but not TIGIT- Tregs (Figure 4F). To assess whether TIGIT signaling is able to upregulate Cebpa expression, T cells were stimulated with agonistic anti-TIGIT Ab in vitro and Cebpa induction in Tregs was observed in response to TIGIT engagement (Figure 4G). Chromatin Immunoprecipitation (ChIP)-PCR using an anti-CEBPa Ab together with primer pairs specific for the Fgl2 genomic region indicated that CEBPα binds to the Fgl2 gene (Figure 4H). To further analyze whether CEBPα can promote transcription of Fgl2, CEBPα was overexpressed in nTregs. An increase in Fgl2 expression following CEBPα transfection in nTregs was observed (Figure 4I), indicating that CEBPα drives Fgl2 expression. TIGIT might therefore equip Tregs for superior suppression by inducing CEBPα, thereby promoting Fgl2 expression.

### Example 6. TIGIT+ Tregs inhibit Th1 and/or Th17 but not Th2 responses

Fgl2 not only suppresses effector T cell proliferation, it also shifts the cytokine profile towards a Th2 response as it inhibits Th1 responses while promoting Th2 polarization and induction of IL-10 and IL-4 (Chan et *al.,* 2003; Shalev et *al.,* 2008). Furthermore, Fgl2 is important for Treg function in vivo as Fgl2-deficient Tregs show impaired control of effector T cell expansion in lymphopenic hosts (Figure 10A). As TIGIT+ Tregs produce high levels of Fgl2, without wishing to be bound by theory, they might similarly affect the cytokine profile of a T cell response by having differential suppressive effects on different Th lineages. To test this hypothesis, naive effector T cells and TIGIT- and TIGIT+ Tregs were co-cultured under differentiation conditions for Th1, Th2, and Th17 cells and then expression of lineage-specific cytokines were assessed. TIGIT+ Tregs showed no difference in suppressing Th1 differentiation when compared to TIGIT- Tregs as indicated by reduced expression of IFNγ (Figures 5A and 10B). Similarly, both subsets potently suppressed expression of IL-17 by Th17 cells. However, in contrast to TIGIT- Tregs, TIGIT+ Tregs did not suppress differentiation of Th2 cells as indicated by IL-4 production comparable to unsuppressed controls (Figures 5A and 10B). This effect was dependent on the ability of TIGIT to induce Fgl2 as TIGIT+ Tregs from Fgl2-/- mice were able to suppress Th2 differentiation, indicating that Fgl2 produced by TIGIT+ Tregs interferes with suppression of Th2 responses (Figures 5A and 10B). In line with these results, analysis of human effector T cells observed in the presence of TIGIT+ or TIGIT- Tregs also showed a potent inhibition of Th1 and/or Th17 but not Th2 responses (Figure 5B). To determine whether these results also translated into selective suppression of Th1 and/or Th17 vs. Th2 responses in vivo, TIGIT+ and TIGIT- OVA-specific OT-II Tregs together with OT-II effector cells were transferred into WT recipients, which were immunized with OVA in CFA, the ability of the different Treg subsets to suppress the Th1 and/or Th17 responses was analyzed upon immunization. Surprisingly, TIGIT+ and TIGIT- were equally capable of suppressing effector T cell expansion as determined by the number of Vβ5⁺ OT-II cells. In addition, differentiation of Th1 and Th17 cells was suppressed equally well by TIGIT+ vs. TIGIT- Tregs in vivo as determined by IFNγ and IL-17 production (Figures 5C-5F). When mice that had received OT-II effector T cells together with TIGIT+ or TIGIT- Tregs were immunized for induction of allergic airway inflammation, TIGIT- Tregs were able to suppress the disease. In contrast, TIGIT+ Tregs failed to inhibit recruitment of antigen-specific Vβ5⁺ OT-II cells to the lung and production of Th2 cytokines (IL-4 and IL-13) was significantly higher than in mice that had received TIGIT- Tregs (Figures 5G, 5H). Consistent with an increase in Th2 cells in the presence of TIGIT+ Tregs, high numbers of eosinophils were detected in the bronchio-alveolar lavage of these mice (Figure 5I). Taken together these data indicate that TIGIT+ Tregs selectively suppress pro-inflammatory Th1 and Th17 cells, but not Th2 responses. TIGIT+ Tregs appear to mediate this effect by inhibiting Th1 and/or Th17 but promoting and/or sparing Th2 differentiation.

### Example 7. TIGIT+ Tregs suppress pro-inflammatory Th1 and/or Th17 but not Th2 responses in vivo

To assess the effects of TIGIT+ Tregs in a setting where multiple pro-inflammatory effector T cell lineages contribute to disease, their role was evaluated in experimental autoimmune encephalomyelitis (EAE), in which disease progression is promoted by Th1 as well as Th17 cells. To engage TIGIT in this model, mice immunized for EAE were treated with an anti-TIGIT antibody (Ab) that acts agonistically in vivo (Figures 9A-9B). While this treatment does not necessarily distinguish between TIGIT on effector or regulatory T cells, the vast majority of TIGIT expressing cells are Tregs. The observed effects are therefore most likely at least in part mediated through TIGIT+ Tregs. Animals that received the anti-TIGIT agonistic Ab indeed showed significantly reduced EAE (Figures 9C-9D), indicating that TIGIT also plays a role in suppressing mixed pro-inflammatory responses. To determine whether both Th1 and Th17 responses were affected, the cytokine secretion by T cells from spleen and draining LN at the induction phase of the disease (day 10) was analyzed. Both IFN- and IL-17 levels were significantly reduced in mice treated with the anti-TIGIT Ab (Figures 9E-9F), indicating that Th1 as well as Th17 responses are suppressed. TIGIT+ Tregs therefore not only suppress one specific effector subset but are capable of inhibiting multiple pro-inflammatory effector T cell responses including Th1 and Th17 responses.

Based on the in vitro and in vivo data (Figures 5A-5I) in Example 6, it was next sought to determine whether TIGIT+ Tregs might be able to mediate similar effects in inhibiting effector Th1 and/or Th17 responses by skewing the effector T cell response towards a Th2 phenotype if the system is not biased by immunization with adjuvant. To this end, the Rag-transfer model of colitis in which disease induction does not rely on immunization and therefore does not introduce a cytokine bias through the choice of adjuvant (Izcue et *al.,* 2008) was used. To induce disease, congenically marked CD45.1 effector T cells were transferred into Rag1-/- recipients; either alone or together with CD45.2+ TIGIT+Foxp3+ or TIGIT-Foxp3+ Tregs. Mice that received the effector T cells alone lost weight over time, while co-transfer of either TIGIT+ or TIGIT- Tregs was able to suppress the disease (Figure 6A). Surprisingly, both Treg populations were able to suppress the disease equally well. Co-transfer of either Treg population prevented tissue inflammation, as indicated by the histopathological colitis score (Figure 6B), and suppressed the expansion of effector T cells in vivo (Figure 6C). In addition, both groups that received Tregs showed comparable frequencies of Foxp3+CD4+ Tregs, indicating that both TIGIT+ and TIGIT- Tregs are able to expand and persist to the same degree in vivo (Figure 6D). They also showed comparable stability, as approximately 80% of the transferred Tregs still expressed Foxp3 10 weeks after transfer (Figure 6E). TIGIT+ as well as TIGIT- Tregs were able to suppress pro-inflammatory cytokines as T cells from the mesenteric LNs produced significantly lower levels of IFN-γ and TNF-α than those from mice that did not receive Tregs (Figures 6F and 11). In contrast, TIGIT+ Treg did not suppress, or may have even increased the expression of the Th2 cytokines IL-4 and IL-10 when compared to the control group (no Tregs) (Figure 6F). Intracellular cytokine staining indicated that, while IL-10 was produced by both effector T cells and Tregs, IL-4 was entirely produced by effector T cells (Figure 11). No significant IL-17 was observed under any conditions of in vivo transfer (data not shown). TIGIT+ Treg therefore potently suppress pro-inflammatory responses in vivo, while sparing or promoting Th2-like responses.

### Discussion for Examples 1-7

Examples 1-7 show that TIGIT+Foxp3+ T cells were identified as a distinct Treg subset that specifically suppresses pro-inflammatory Th1 and/or Th17 responses through the secretion of Fgl2, which contributes to the higher suppressive capacity of TIGIT+ Treg in vitro. The findings presented herein further indicate that engagement of TIGIT induces Fgl2. Through the secretion of Fgl2, TIGIT+ Tregs are able to selectively suppress pro-inflammatory effector Th1 and Th17 responses, shifting the balance towards Th2 responses. This is one of the first examples of how a co-inhibitory molecule can mediate selective inhibition of certain effector responses while leaving others intact.

Uncontrolled Th1 and/or Th17 responses can lead to chronic immune activation and inflammation that results in induction of autoimmune diseases such as psoriasis, rheumatoid arthritis, inflammatory bowel disease and multiple sclerosis. The findings presented herein indicate that TIGIT+ Tregs can play an important role in preventing these autoimmune disorders and maintaining self-tolerance. Fgl2-/- mice were previously discussed to show increased Th1 but diminished Th2 responses and spontaneously develop autoimmune glomerulonephritis as they age (Shalev et al., 2008). In addition, the findings presented herein can indicate that Fgl2-deficient mice would also show enhanced Th17 responses. That the TIGIT+ Treg-specific effector molecule Fgl2 results in inhibition of Th1 and/or Th17 responses while increasing Th2 responses indicate that TIGIT+ Tregs can act as a specialized subset that does not globally suppress all effector T cell responses but may specifically suppress inflammatory immune responses and tissue inflammation mediated by Th1 and/or Th17 cells, but does not affect Th2 responses.

TIGIT was first described as an inhibitory molecule that suppresses immune responses indirectly by regulating DC function. By interacting with its ligand *CD155* on DCs, TIGIT was shown to induce IL-10 and suppress IL-12 production in DCs and thereby inhibit Th1 responses (Yu et *al.,* 2009). TIGIT was previously reported to have T cell intrinsic inhibitory effects (Joller et *al.,* 2011). Since Tregs are the primary cell type that constitutively expresses TIGIT, without wishing to be bound by theory, many of the DC effects that have been observed might be mediated by TIGIT+ Tregs. In addition to TIGIT-induced IL-10 produced by the DCs themselves, it is contemplated that increased amounts of IL-10 and Fgl2 produced by TIGIT+ Tregs may also contribute to the generation of tolerogenic DCs and thereby inhibit the generation of effector Th1 responses. Although TIGIT-induced IL-10 was shown to suppress both Il-12p35 and IL-12p40 (Yu et *al.,* 2009), the effect of Fgl2 in suppressing these key differentiating cytokines has not been evaluated. We propose that IL-10 and Fgl2 secreted by TIGIT+ Tregs may act in concert to suppress both IL-12 and IL-23 production from activated DCs and thereby inhibit development of both Th1 and Th17 responses.

Tregs represent a heterogeneous population that encompasses many specialized subpopulations. While Foxp3 is necessary to equip T cells with basic Treg functions (Fontenot et *al.,* 2005), additional factors are required for efficient suppression of effector T cell responses in vivo and for maintaining immune tolerance. Several transcription factors have been identified that drive additional programs in Tregs to efficiently control certain classes of effector T cells and autoimmunity and inflammation in defined target tissues. For instance, tissue specific Treg subsets, such as "fat Tregs", have very distinct transcriptional signatures that are shaped by the expression of tissue-specific transcription factors that allow them to adapt their function to the specific tissue requirements (Cipolletta et *al.,* 2012). Similarly, Tregs that are specialized in controlling specific effector T cell lineages co-express lineage-specific transcription factors from T helper cells, such as T-bet, IRF4, Stat3, or Bcl6 to fulfill their subset-specific inhibitory functions (Chaudhry et *al.,* 2009; Chung et *al.,* 2011; Koch et *al.,* 2009; Linterman et *al.,* 2011; Zheng et *al.,* 2009). These Examples show that TIGIT+ Tregs share features with several different Treg subsets and express elevated levels of T-bet and IRF4 as well as Th17-specific transcription factors such as RORα and RORγ, when compared to TIGIT- Treg cells. The finding that TIGIT+ Tregs express elevated levels of IRF4 would indicate that they are well equipped for suppression of Th2 responses, because IRF4-deficieny in Foxp3+ T cells results in spontaneous Th2 pathology (Zheng et *al.,* 2009). However, the findings presented herein indicate that TIGIT+ Treg effectively inhibit pro-inflammatory Th1 and/or Th17 responses but not Th2 responses. It should be noted that IRF4 is not only expressed in Th2 cells but is also required for Th17 differentiation. While conditional deletion of IRF4 in Foxp3+ Tregs most prominently affects control of Th2 responses, these mice also have slightly elevated IL-17 levels (Zheng et *al.,* 2009) and in settings where the immune response is dominated by Th17 effector cells, such as arthritis, diminished function of IRF4 in Tregs results in impaired control of Th17 responses (Darce et *al.,* 2012). While TIGIT+ Tregs seem to share functional aspects with IRF4-deficient Tregs, their ability to potently suppress Th17 responses distinguishes them from IRF4-deficient Tregs.

The findings presented herein show that in addition to the lineage- and tissue-specific transcription factors, co-inhibitory molecules like TIGIT also contribute to the functional specialization of Tregs by inducing a distinct set of suppressive mediators that can selectively suppress certain classes of effector T cell responses. In the case of TIGIT+ Tregs, expression of Fgl2 allows them to selectively suppress pro-inflammatory responses, including Th1 and/or Th17 responses. In some embodiments, co-inhibitory receptors can therefore tailor the suppressive function of Foxp3+ Tregs to what is required in a specific inflammatory environment. The expression pattern of these receptors and/or engagement through their ligands in a particular tissue environment can thereby alter the molecular signature of Tregs and equip them with specialized suppressive mechanisms that are tailored for a specific tissue or type of inflammation.

Besides transcription factors, the present findings show that that cell surface molecules like TIGIT expressed on Foxp3+ Tregs can differentially suppress effector T cell responses, providing a target by which defined subsets of Tregs can be manipulated to regulate immune and autoimmune responses.

### Exemplary Experimental Procedures for Examples 1-7

***Animals.*** C57BL/6 (B6), B6.SJL-*Ptprc^{a}Pepc^{b}*/BoyJ (CD45.1), B6.129P2-Il10*^{tm1Cgn}*/J (IL-10^{-/-}) and B6.129S7-Rag1^{tm1Mom}/J (RAG1^{-/-}) mice were purchased from the Jackson Laboratories. Foxp3-GFP.KI reporter mice (Bettelli et *al.,* 2006), and Fgl2-/- mice (Shalev et *al.,* 2008) have been previously described. Animals were maintained in a conventional, pathogen-free facility and all experiments were carried out in accordance with guidelines prescribed by the Institutional Animal Care and Use Committee (IACUC).

***Human samples.*** Peripheral venous blood was obtained from healthy control volunteers in compliance with Institutional Review Board protocols. Total CD4+ T cells were isolated by negative selection (CD4+ T cell isolation kit II, Miltenyi Biotec, Auburn, CA) and then sorted by flow cytometry.

***Treg differentiation and suppression assays.*** Cells were cultured in DMEM supplemented with 10% (vol/vol) FCS, 50 mM mercaptoethanol, 1 mM sodium pyruvate, nonessential amino acids, L-glutamine, and 100 U/ml penicillin and 100 g/ml streptomycin. CD4+ T cells from splenocytes and lymph node cells were isolated using anti-CD4 beads (Miltenyi). For in vitro Treg differentiation, naïve CD4⁺CD62L⁺CD44⁻ cells were sorted by flow cytometry and stimulated with plate bound anti-CD3 (145-2C11, 0.3 µg/ml) and anti-CD28 (PV-1, 2 µg/ml) in the presence of 2.5ng/ml TGF-β (R&D). Foxp3 expression was assessed by flow cytometry 4 days later. For suppression assays, CD4+Foxp3- responder cells and CD4+Foxp3+ Tregs were flow sorted from Foxp3-GFP.KI reporter mice based on GFP expression. CD4+Foxp3- (2 × 10⁴/well) and CD4+Foxp3+ cells were cultured in triplicate in the presence of soluble anti-CD3 (1 µg/ml) and irradiated splenic APCs (1.2 × 10⁵/well). After 48 h cells were pulsed with 1 µCi [³H]thymidine for an additional 18h, harvested and [3H]thymidine incorporation was analyzed to assess proliferation. Percentage of suppression = 100 - C.P.M. of well with the indicated ratio of effector: Tregs / mean C.P.M. of wells with CD4⁺Foxp3- effectors alone. Where indicated, anti-Fgl2 Ab (clone 6D9, 30 µg/ml, Abnova), anti-IL-10 Ab (clone JES5-16E3, Biolegend) or an isotype control was added to the cultures. For human Treg suppression assays, CD25-depleted T cells were CFSE - labeled and co-cultured with FACS-sorted Tregs (TIGIT+ or TIGIT-) at indicated ratios. Cells were stimulated with Treg Inspector Beads (Miltenyi) at manufacturer's recommended concentration. At day 4, cells were stained with LIVE/DEAD Fixable Dead Cell Stain Kit (Molecular Probes) to allow gating on viable cells and proliferation was measured by CFSE dilution. Samples were analyzed by flow cytometry.

***Microarray.*** CD4+ T cells were pre-purified from splenocytes and lymph node cells of naïve Foxp3-GFP.KI reporter mice using Dynal beads (Invitrogen) and CD4+Foxp3+TIGIT+ and CD4+Foxp3+TIGIT- cells were sorted by flow cytometry. CD4+Foxp3+CXCR3+ and CD4+Foxp3+CXCR3+ were similarly sorted from spleens of Foxp3-GFP.KI mice. All cells were double-sorted for purity, the final sort being directly into TRIzol (Invitrogen). RNA was extracted and used to prepare probes for microarray analysis on the Affymetrix Mouse Gene1.0ST platform, using ImmGen protocols (Heng and Painter, 2008). Microarray data was analyzed using the GeneSpring 11 (Agilent, Santa Clara, CA; quantile normalization) or GenePattern (RMA normalization) software. Genes of interest (fold change >1.5) were manually selected and two-way hierarchical clustering using Euclidean distance metric was performed to generate heat-maps. Analysis of signature genes within the TIGIT+/TIGIT- comparison used previously determined genesets: a T cell activation/proliferation signature from in vivo activated T cells (Hill et *al.,* 2007); the canonical Treg/Tconv signature (Hill et *al.,* 2007); a geneset that distinguishes Treg cells which express the chimeric GFP-Foxp3 fusion protein (Darce et *al.,* 2012), and the IRF4-dependent signature in Treg cells (Zheng et *al.,* 2009). P values form a chi-squared test. The genes and Probe IDs included in these signatures are listed in Table 2.

***Flow cytometry.*** Surface staining was performed for 20 minutes at 4°C in PBS containing 0.1% sodium azide and 0.5% BSA. For intracellular cytokine stainings, cells were re-stimulated with phorbol 12-myristate 13-acetate (PMA, 50 ng/ml, Sigma), ionomycin (1 µg/ml, Sigma), and GolgiStop (1 µl/1ml, BD Bioscience) at 37°C in 10% CO₂ for 4 h before staining was performed using the Cytofix/Cytoperm kit (BD Biosciences). Intracellular staining for Foxp3 was performed using the Foxp3 Staining Buffer Set (eBioscience). Antibodies were from BioLegend except for anti-Foxp3 (eBioscience), and anti-Ki67 (BD Biosciences). 7AAD was purchased from BD Biosciences. Samples were acquired on a FACSCalibur or LSRII flow cytometer (BD Biosciences) and analyzed using the FlowJo software (Tree Star).

***Quantitative RT-PCR.*** RNA was extracted with RNAeasy mini Kits (Qiagen) and cDNA was prepared using the iScript cDNA synthesis kit (BioRad). Real-time PCR (RT-PCR) was performed using Taqman probes and the 7500 Fast Real-Time PCR system (Applied Biosystems). All samples were normalized to b-actin internal control.

***In vitro antibody treatment.*** CD4+Foxp3- effector T cells and CD4+Foxp3+ Tregs were sorted from Foxp3-GFP.KI reporter mice and stimulated at a density of 1 × 10⁶/ml with plate bound anti-CD3 (145-2C11, 1 µg/ml), anti-CD28 (PV-1, 2 µg/ml), and anti-TIGIT (4D4, 100 µg/ml) or isotype control antibody. RNA was isolated on day 3. Antibodies to human TIGIT were provided by ZymoGenetics, Inc. (a wholly-owned subsidiary of Bristol-Myers Squibb). Cells were stimulated with anti-CD3 (UCHT1, 1 µg/ml), anti-CD28 (28.2, 1 µg/ml) and IL-2 (10 U/ml) in the presence of agonistic anti-TIGIT at 20 µg/ml or IgG isotype control. Gene expression was assessed on day 4.

***In vivo antibody treatment.*** Mice were immunized s.c. with 200 µl of an emulsion containing 100 µg of MOG_{35 - 55} peptide (MEVGWYRSPFSRVVHLYRNGK) in adjuvant oil (CFA) on day 0 and treated i.p. with 100 µg of anti - TIGIT (clone 4D4) or isotype control Ab (armenian hamster IgG) on days 0, 2, 4, 10 and 17. For antigen-specific proliferation assays spleens and lymph nodes were collected on day 10 and 2.5 x 10⁶ cells/ ml were re-stimulated with 50 µg/ml MOG₃₅₋₅₅ peptide. After 48h Fgl2 concentrations in culture supernatants were determined by ELISA (Biolegend).

***CHIP-PCR and over-expression.*** ChIP assays were performed on P815 cells expressing TIGIT using the SimpleChIP Enzymatic Chromatin IP Kit (Cell Signaling) according to the manufacturer's instructions. Lysates were immunoprecipitated using anti-C/EBPα antibody (8 µg; Santa Cruz Biotech, sc-61) or rabbit IgG isotype control. Quantitative PCR reactions were performed with SYBR-Green on ChIP-bound and input DNA. % input = 2% x 2^{(CT 2% input sample - CT sample)}. For CEBPα over-expression CD4+Foxp3+ Tregs were flow sorted from Foxp3-GFP.KI reporter mice. 5x10⁵ Tregs / ml were stimulated with Mouse T-Activator CD3/CD28 Dynabeads (Invitrogen) and transfected with 10 µg/ml of Cebpa cDNA in pCMV6-Kan/Neo or the empty vector, which had been pre-incubated with FuGene 6 (Roche Diagnostics). RNA was extracted on day 4 with RNAeasy mini Kits (Qiagen), samples were treated with DNAse (RNAse-free DNAse set, Qiagen) and cDNA was prepared using the iScript cDNA synthesis kit (BioRad). Cebpa over-expression was verified by Taqman PCR.

***Suppression of Th differentiation.*** For in vitro experiments CD4+CD62L+ naive T cell from CD45.1 mice and CD4+Foxp3+TIGIT+ or TIGIT- Tregs from Foxp3-GFP.KI mice (CD45.2) were sorted and cultured at 10⁵ Teff and 10⁴ Treg /well. Cells were stimulated with Mouse T-Activator CD3/CD28 Dynabeads (Invitrogen, 0.6 µl/well) in the presence of polarizing cytokines (Th1: 4ng/ml IL-12; Th2: 4ng/ml IL-4; Th17: 10ng/ml IL-6, 2ng/ml TGF-β; all cytokines from R&D). RNA was extracted after 3 days and flow cytometric analysis was performed on day 5.

For in vivo experiments 1-2 x 10⁵ CD4+CD62L+CD25+ sorted naive effector T cell and 2.5-5 x 10⁴ CD4+CD25+TIGIT+ or TIGIT- Tregs (Teff: Treg 4:1) from OT-II mice were transferred i.v. into WT recipients one day before immunization. To elicit a mixed Th1/Th17 response, mice were immunized with 10 µg OVA (Sigma) emulsified in CFA and spleens and draining LN were analyzed 10 days later. Allergic airway inflammation was induced as described previously (Haworth et *al.,* 2008; Rogerio et *al.,* 2012). For example, mice were sensitized with 10 µg OVA in alum i.p. on days 0 and 7 and challenged with 6% (wt/vol) OVA aerosol for 25 min on days 14, 15, 16 and 17. Cells from lung and bronchioalveolar lavage were analyzed directly following challenge on day 17.

***Colitis and histopathology.*** CD4+CD45RBhigh naive T cell from CD45.1 mice and CD4+Foxp3+ Tregs from Foxp3-GFP.KI mice were purified by cell sorting after enrichment for CD4+ cells using anti-CD4 MACS beads. 8 × 10⁵ CD4⁺CD45RB^{high} cells were transferred i.v. into RAG1-/- mice, either alone or with Tregs (4:1 effector T cell:Treg ratio) and mice were weighed weekly. At the time of sacrifice small and large intestine samples were fixed in neutral buffered formalin.

Routinely processed, paraffin-embedded tissue samples were stained with hematoxylin and eosin (H&E). The presence and severity of colitis was evaluated in a blinded manner and graded semi-quantitatively from 0 to 3 for the three following criteria: epithelial hyperplasia; leukocyte infiltration; and the presence of crypt abscesses. Scores for each criterion were added to give an overall inflammation score for each sample of 0-9.

***In vivo Treg stability.*** TIGIT+ or TIGIT- Tregs from Foxp3-GFP.KI mice were purified by cell sorting after enrichment for CD4+ cells using anti-CD4 MACS beads. 10⁶ CD4+Foxp3-GFP+TIGIT+ or TIGIT- Tregs were transferred i.v. into CD45.1 mice and transferred cells were analyzed for TIGIT expression 20 days later by flow cytometry gating on CD45.2+ donor cells.

***BrdU labeling.*** For BrdU labeling, naïve Foxp3-GFP.KI mice were injected i.p. daily with 1mg of BrdU for 4 days and spleens and LNs were harvested on day5. BrdU was stained in sorted CD4+Foxp3+TIGIT+ and CD4+Foxp3+TIGIT- cells using the APC BrdU Flow Kit (BD Biosciences).

***In vivo antibody treatment.*** Mice were immunized s.c. with 200 µl of an emulsion containing 100 µg of MOG₃₅₋₅₅ peptide (MEVGWYRSPFSRVVHLYRNGK) in adjuvant oil (CFA) on day 0 and treated i.p. with 100 µg of anti-TIGIT (clone 4D4) or isotype control Ab (armenian hamster IgG) on days 0, 2, 4, 10 and 17. Spleens and lymph nodes were collected on day 10 and 2.5 x 10⁶ cells / ml were re-stimulated in the presence of a range of concentrations of MOG₃₅₋₅₅ peptide (0.07 µg/ml - 50 µg/ml). After 48 h, plates were pulsed with 1 µCi/well [³H]thymidine and incubated for an additional 18h before being harvested onto glass fiber filters. ³H-thymidine incorporation was analyzed using a β-counter (1450 Microbeta, Trilux, Perkin Elmer).

***In vitro Treg differentiation.*** CD4+ T cells were isolated from mice using anti-CD4 beads (Miltenyi). Naïve CD4+CD62L+CD44- cells were sorted by flow cytometry and stimulated with plate bound anti-CD3 (145-2C11, 0.3 µg/ml) and anti-CD28 (PV-1, 2 µg/ml) in the presence of 2.5ng/ml TGF-β (R&D) and where indicated 10µg/ml recombinant Fgl2. Foxp3 expression was assessed by flow cytometry 4 days later.

***In vivo suppression.*** CD4+ T cell from CD45.1 mice and CD4+CD25+ Tregs from WT or Fgl2-/- mice were purified by cell sorting after enrichment for CD4+ cells using anti-CD4 MACS beads. CD45.1 CD4+ T cell were labeled with CFSE and transferred i.v. into RAG1-/- mice, either alone or with Tregs (5:1 effector T cell/Treg ratio). On day 8 mice were sacrificed and CFSE dilution in CD45.1+ effector T cells was analyzed by flow cytometry.

***Statistical analysis.*** Statistical significance was assessed either by 2-tailed Student's T-test (two groups) or ANOVA for multiple groups with a post hoc Tukey's test; P values < 0.05 were considered statistically significant. Statistical significance values indicated as follows: p < 0.05 (*), p < 0.01 (**) and p < 0.005 (***).

**Table 1: Differentially regulated genes in TIGIT⁻ vs. TIGIT⁺ Tregs**

| **Comparison** TIGIT- vs TIGIT+ SpLN Tregs | | | |
|---|---|---|---|
| **Name** | **Description** | **Fold change in gene expression** (TIGIT+ Treg cells relative to their TIGIT-counterparts) | **T-test** |
| 10547590 | Klrg 1 | 16.949153 | 0.08277953 |
| 10349603 | Il10 | 12.5 | 0.00502744 |
| 10352178 | Sccpdh | 11.235955 | 0.02691577 |
| 10503098 | Lyn | 8.6206897 | 0.05098679 |
| 10421517 | Cysltr2 | 7.518797 | 0.01049428 |
| 10454015 | Ttc39c | 7.4626866 | 0.0022178 |
| 10368970 | Prdm1 | 7.0921986 | 0.00621068 |
| 10439527 | Tigit | 6.993007 | 0.00186793 |
| 10574524 | Ces2c | 6.7114094 | 0.0165658 |
| 10603551 | Cybb | 6.6666667 | 0.00373176 |
| 10375443 | Havcr2 | 6.5359477 | 0.03476303 |
| 10420308 | Gzmb | 6.2111801 | 0.00857544 |
| 10519983 | Fgl2 | 6.0606061 | 0.00621597 |
| 10568714 | Mki67 | 5.7803468 | 0.10430529 |
| 10408689 | Nrn1 | 5.7142857 | 0.00067242 |
| 10526832 | LOC100504914 | 5.6818182 | 0.0054879 |
| 10466521 | Gcnt1 | 5.6497175 | 0.01675728 |
| 10399148 | Rapgef5 | 5.5555556 | 0.02740164 |
| 10363082 | Lilrb4 | 5.4945055 | 0.03565153 |
| 10487480 | Bub1 | 5.4347826 | 0.17528406 |
| 10403229 | Itgb8 | 5.2910053 | 0.00645382 |
| 10586448 | 2810417H13Rik | 5.0251256 | 0.1783378 |
| 10511363 | Penk | 5 | 0.03340601 |
| 10521731 | Ncapg | 5 | 0.07555397 |
| 10365933 | Eea1 | 4.9019608 | 0.01919938 |
| 10384458 | Plek | 4.9019608 | 0.03442288 |
| 10601350 | Fgf16 | 4.9019608 | 0.04717276 |
| 10590242 | Ccr8 | 4.8543689 | 0.00390674 |
| 10518313 | Tnfrsf8 | 4.8543689 | 0.01295347 |
| 10523156 | Cxcl2 | 4.8076923 | 0.0196782 |
| 10540472 | Bhlhe40 | 4.7619048 | 0.03546062 |
| 10411611 | Naip5 | 4.7169811 | 0.00649136 |
| 10462796 | Kif11 | 4.6948357 | 0.13286043 |
| 10363415 | Spock2 | 4.6728972 | 0.00454537 |
| 10408935 | Gm10786 | 4.587156 | 0.00029438 |
| 10443980 | Myo1f | 4.587156 | 0.00064718 |
| 10411622 | Naip6 | 4.4642857 | 0.01417191 |
| 10553354 | Nav2 | 4.4052863 | 0.00044491 |
| 10482528 | Neb | 4.4052863 | 0.0100309 |
| 10594762 | Fam81a | 4.3859649 | 0.03835893 |
| 10487506 | Gm14005 | 4.3668122 | 0.00130514 |
| 10471555 | Angptl2 | 4.3668122 | 0.00161377 |
| 10588243 | Ryk | 4.3290043 | 0.0003589 |
| 10382200 | Ccdc46 | 4.3290043 | 0.00354873 |
| 10464905 | Npas4 | 4.2918455 | 0.00373534 |
| 10363070 | Gp49a | 4.2918455 | 0.02607473 |
| 10562657 | Gm5595 | 4.2194093 | 0.00090933 |
| 10494001 | Tdpoz4 | 4.2194093 | 0.14975638 |
| 10500720 | Slc22a15 | 4.2016807 | 0.0013601 |
| 10345752 | Il1r2 | 4.1841004 | 0.0327554 |
| 10582997 | Casp4 | 4.1666667 | 0.02254515 |
| 10582985 | Casp 1 | 4.1322314 | 0.01204709 |
| 10505674 | Cntln | 4.1322314 | 0.01308681 |
| 10356866 | Pdcd1 | 4.1322314 | 0.01705969 |
| 10574532 | Ces2d-ps | 4.1322314 | 0.01858489 |
| 10542896 | Bicd1 | 4.0816327 | 0.00159197 |
| 10439895 | Alcam | 4.0816327 | 0.00447 |
| 10511779 | Atp6v0d2 | 4.0160643 | 0.00162049 |
| 10606910 | Mcart6 | 4.0160643 | 0.00225771 |
| 10423599 | Matn2 | 4.0160643 | 0.01580438 |
| 10380289 | Mmd | 3.9840637 | 0.00058236 |
| 10399087 | Ncapg2 | 3.9525692 | 0.09359614 |
| 10571399 | Zdhhc2 | 3.9215686 | 0.00022571 |
| 10497831 | Ccna2 | 3.8910506 | 0.10693279 |
| 10607738 | Car5b | 3.875969 | 0.02088748 |
| 10499062 | Fhdc1 | 3.8461538 | 0.01202645 |
| 10435907 | Cd200r1 | 3.8167939 | 0.00586522 |
| 10548307 | Klrb1c | 3.8022814 | 0.00089588 |
| 10462866 | Cep55 | 3.8022814 | 0.11031612 |
| 10476443 | Plcb4 | 3.7735849 | 0.0136415 |
| 10389207 | Ccl5 | 3.7735849 | 0.11229956 |
| 10447383 | Epcam | 3.7593985 | 0.06264469 |
| 10590494 | Kif15 | 3.7453184 | 0.13765528 |
| 10412517 | Gm3002 | 3.7313433 | 0.00303162 |
| 10586781 | Myole | 3.7174721 | 0.00013348 |
| 10390707 | Top2a | 3.7174721 | 0.12225475 |
| 10356299 | Gpr55 | 3.7037037 | 0.04268382 |
| 10476740 | Slc24a3 | 3.6900369 | 0.01069883 |
| 10436945 | Slc5a3 | 3.6764706 | 0.00823772 |
| 10417235 | Gm2897 | 3.6630037 | 0.00038087 |
| 10440186 | Crybg3 | 3.6630037 | 0.01962173 |
| 10544660 | Osbpl3 | 3.6363636 | 0.00222121 |
| 10412495 | Gm3002 | 3.6363636 | 0.00583372 |
| 10417408 | D830030K20Rik | 3.6363636 | 0.02832373 |
| 10519857 | Hgf | 3.6231884 | 0.01510521 |
| 10417302 | Gm3002 | 3.5971223 | 0.00194254 |
| 10590628 | Ccr3 | 3.5714286 | 0.00826259 |
| 10409278 | Nfil3 | 3.5587189 | 0.05973224 |
| 10553598 | Cyfip1 | 3.5335689 | 0.00160251 |
| 10543120 | Ica1 | 3.5335689 | 0.00197274 |
| 10417258 | Gm3002 | 3.5335689 | 0.00416404 |
| 10462005 | Tmem2 | 3.5211268 | 0.00284602 |
| 10418341 | Il17rb | 3.5087719 | 0.00756839 |
| 10417458 | Gm5458 | 3.5087719 | 0.02039524 |
| 10476759 | Rin2 | 3.4965035 | 0.00151299 |
| 10417359 | Gm3002 | 3.4965035 | 0.00286363 |
| 10417226 | Gm3002 | 3.4965035 | 0.00559209 |
| 10492890 | Lrba | 3.4843206 | 0.00287007 |
| 10417239 | Gm1973 | 3.4843206 | 0.00715288 |
| 10601312 | Chic1 | 3.4843206 | 0.00715637 |
| 10394770 | Odc1 | 3.4843206 | 0.03679442 |
| 10417366 | ENSMUSG00000068790 | 3.4722222 | 0.0060115 |
| 10461844 | Gnaq | 3.4722222 | 0.00665844 |
| 10359375 | Gpr52 | 3.4722222 | 0.00861866 |
| 10361110 | Dtl | 3.4722222 | 0.12548646 |
| 10367919 | Stx11 | 3.4602076 | 0.00460918 |
| 10412537 | Gm3002 | 3.4482759 | 0.00149724 |
| 10542993 | Pon3 | 3.4364261 | 0.0075274 |
| 10552143 | Slc7a10 | 3.4364261 | 0.03125043 |
| 10531415 | Cxcl10 | 3.4364261 | 0.03126957 |
| 10592201 | Chek1 | 3.4246575 | 0.12067932 |
| 10454198 | Rnf125 | 3.4129693 | 0.0031176 |
| 10590635 | Ccr5 | 3.4129693 | 0.03010649 |
| 10417411 | Gm3002 | 3.4013605 | 0.00520539 |
| 10422013 | Klf12 | 3.4013605 | 0.00624066 |
| 10482687 | Arl5a | 3.4013605 | 0.00684182 |
| 10412549 | D830030K20Rik | 3.4013605 | 0.00799923 |
| 10515836 | Ccnb1 | 3.4013605 | 0.04611089 |
| 10357833 | Atp2b4 | 3.3898305 | 0.00197782 |
| 10519527 | Abcbla | 3.3898305 | 0.00466438 |
| 10599174 | Il13ra1 | 3.3898305 | 0.00736274 |
| 10417501 | Gm5458 | 3.3898305 | 0.00875546 |
| 10462973 | Hells | 3.3898305 | 0.15731102 |
| 10544829 | Jazf1 | 3.3783784 | 0.00342215 |
| 10417319 | D830030K20Rik | 3.3783784 | 0.00404997 |
| 10417245 | Gm1973 | 3.3670034 | 0.00440714 |
| 10491848 | Larp1b | 3.3557047 | 0.00761383 |
| 10369932 | Susd2 | 3.3557047 | 0.00835697 |
| 10358816 | Lamc1 | 3.3444816 | 7.93E-05 |
| 10392415 | Rgs9 | 3.3333333 | 0.00077218 |
| 10545154 | Il23r | 3.3333333 | 0.05666823 |
| 10543031 | Slc25a13 | 3.3222591 | 0.00062077 |
| 10346799 | Icos | 3.3112583 | 0.00480172 |
| 10417461 | Gm10406 | 3.30033 | 0.00164351 |
| 10411739 | Ccnb1 | 3.2894737 | 0.04922262 |
| 10605674 | Pola1 | 3.2894737 | 0.06416331 |
| 10478973 | Cass4 | 3.2786885 | 0.00787172 |
| 10417421 | Gm3696 | 3.2679739 | 0.00321407 |
| 10387257 | Alox8 | 3.257329 | 0.01148882 |
| 10571696 | Casp3 | 3.257329 | 0.04059645 |
| 10417253 | Gm1973 | 3.2467532 | 0.00036471 |
| 10581992 | Maf | 3.2467532 | 0.00346861 |
| 10435920 | Cd200r4 | 3.236246 | 0.02525016 |
| 10590631 | Ccr2 | 3.2154341 | 0.04830149 |
| 10385248 | Hmmr | 3.2154341 | 0.2101738 |
| 10466745 | Tjp2 | 3.1948882 | 0.03325137 |
| 10417264 | Gm3002 | 3.1847134 | 0.00059997 |
| 10591781 | Anln | 3.1847134 | 0.13299794 |
| 10499108 | Glt28d2 | 3.1545741 | 0.00418673 |
| 10503107 | 6330407A03Rik | 3.1446541 | 0.07580716 |
| 10350733 | Rgs16 | 3.125 | 0.02095813 |
| 10345791 | Il1rl1 | 3.125 | 0.10437169 |
| 10484888 | Ptprj | 3.1152648 | 0.02062208 |
| 10402325 | Asb2 | 3.1152648 | 0.02131779 |
| 10417373 | Gm10406 | 3.1055901 | 0.00041173 |
| 10412520 | Gm3002 | 3.1055901 | 0.0005167 |
| 10417286 | Gm3002 | 3.1055901 | 0.00073736 |
| 10486396 | Ehd4 | 3.0959752 | 0.00264405 |
| 10587350 | Ddx43 | 3.0959752 | 0.00302497 |
| 10586700 | Rora | 3.0959752 | 0.00426848 |
| 10417504 | Gm1973 | 3.0864198 | 0.00217757 |
| 10483178 | Cobll1 | 3.0864198 | 0.02366217 |
| 10562637 | Ccnb1 | 3.0864198 | 0.0489732 |
| 10368060 | Ect21 | 3.0864198 | 0.20420133 |
| 10602068 | Mid2 | 3.0769231 | 0.00599048 |
| 10420362 | Gjb2 | 3.0769231 | 0.1902505 |
| 10429754 | Nrbp2 | 3.0487805 | 0.03126596 |
| 10585194 | Il18 | 3.0487805 | 0.09548274 |
| 10594501 | Ptplad1 | 3.0395137 | 0.00279641 |
| 10355567 | Tmbim1 | 3.030303 | 0.04004553 |
| 10436106 | C330027C09Rik | 3.021148 | 0.09961112 |
| 10497122 | Depdcla | 3.021148 | 0.24710867 |
| 10491699 | Fgf2 | 3.0120482 | 0.00396892 |
| 10417326 | Gm3002 | 3.003003 | 0.00029084 |
| 10401320 | Adam4 | 3.003003 | 0.04067599 |
| 10406334 | Mctp1 | 2.994012 | 0.00710938 |
| 10477187 | Tpx2 | 2.9850746 | 0.1065914 |
| 10350838 | 2810417H13Rik | 2.9850746 | 0.15896679 |
| 10417773 | Gm5458 | 2.9673591 | 0.00378186 |
| 10375121 | C530030P08Rik | 2.9585799 | 0.01034509 |
| 10524878 | Vsig10 | 2.9585799 | 0.01488337 |
| 10404840 | Cd83 | 2.9411765 | 0.00229696 |
| 10561104 | Axl | 2.9411765 | 0.01659645 |
| 10411595 | Naip2 | 2.9325513 | 0.00146451 |
| 10576661 | Itgb1 | 2.9325513 | 0.00551884 |
| 10556266 | Wee1 | 2.9325513 | 0.00617181 |
| 10424543 | Wisp1 | 2.9325513 | 0.01202386 |
| 10498952 | Gucyla3 | 2.9325513 | 0.03400079 |
| 10523182 | Areg | 2.9239766 | 0.00497611 |
| 10415021 | Abhd4 | 2.9069767 | 0.00507733 |
| 10375123 | C530030P08Rik | 2.9069767 | 0.00911151 |
| 10486875 | Frmd5 | 2.9069767 | 0.01761078 |
| 10605431 | Rab39b | 2.8985507 | 0.00342792 |
| 10561702 | Kcnk6 | 2.8901734 | 0.01942935 |
| 10484201 | Ccdc141 | 2.8901734 | 0.02140296 |
| 10378286 | Itgae | 2.8818444 | 0.00852384 |
| 10493812 | S100a4 | 2.8818444 | 0.01944604 |
| 10417446 | 4930555G01Rik | 2.8735632 | 0.00558711 |
| 10564805 | Pex11a | 2.8735632 | 0.01736591 |
| 10474381 | Kif18a | 2.8735632 | 0.07955153 |
| 10394978 | Rrm2 | 2.8735632 | 0.09466237 |
| 10482772 | Nr4a2 | 2.8653295 | 0.08787412 |
| 10497149 | WIs | 2.8571429 | 0.00128082 |
| 10478364 | Tox2 | 2.8571429 | 0.01701987 |
| 10452980 | Eif2ak2 | 2.8490028 | 0.01607352 |
| 10592001 | St14 | 2.8490028 | 0.0580992 |
| 10484894 | Ptprj | 2.8409091 | 0.00314912 |
| 10417124 | B930095G15Rik | 2.8328612 | 0.0140005 |
| 10388591 | Cpd | 2.8248588 | 0.00701122 |
| 10524308 | Mir701 | 2.8248588 | 0.01073409 |
| 10350630 | Fam129a | 2.8089888 | 0.00213696 |
| 10593497 | Zc3h12c | 2.8011204 | 0.01017535 |
| 10402136 | Gpr68 | 2.8011204 | 0.03628006 |
| 10362005 | Ahi1 | 2.7932961 | 0.00128988 |
| 10359890 | Nuf2 | 2.7932961 | 0.23297012 |
| 10367945 | Phactr2 | 2.7855153 | 0.00266881 |
| 10356082 | Plscr1 | 2.7855153 | 0.00872677 |
| 10395328 | Snx13 | 2.7855153 | 0.02508373 |
| 10511617 | Fam92a | 2.7700831 | 0.00255819 |
| 10345807 | Il18r1 | 2.7700831 | 0.00449592 |
| 10583320 | BC017612 | 2.7624309 | 0.00646737 |
| 10521678 | Cd38 | 2.7624309 | 0.00774699 |
| 10511382 | Nsmaf | 2.7548209 | 0.00280194 |
| 10505064 | Tmem38b | 2.7548209 | 0.01044574 |
| 10493820 | S100a6 | 2.7548209 | 0.02699151 |
| 10606182 | Mir421 | 2.7548209 | 0.06443783 |
| 10496204 | Cenpe | 2.7548209 | 0.12279284 |
| 10368062 | Ect21 | 2.7472527 | 0.00151131 |
| 10484402 | Ctnnd1 | 2.739726 | 0.01174898 |
| 10428534 | Trps1 | 2.739726 | 0.01884088 |
| 10399024 | Adam6b | 2.739726 | 0.14639423 |
| 10459643 | 4930503L19Rik | 2.7322404 | 0.00484409 |
| 10491835 | Larp1b | 2.7322404 | 0.02052749 |
| 10474984 | Nusap1 | 2.7322404 | 0.14846887 |
| 10417769 | Gm2897 | 2.7247956 | 0.00034888 |
| 10361790 | Fuca2 | 2.7247956 | 0.00836353 |
| 10523595 | Ptpn13 | 2.7247956 | 0.0121956 |
| 10515090 | Cdkn2c | 2.7173913 | 0.00122007 |
| 10345824 | Il18rap | 2.7173913 | 0.01132779 |
| 10374500 | Vps54 | 2.7100271 | 0.0072985 |
| 10456296 | Malt1 | 2.7100271 | 0.03715951 |
| 10601011 | Kif4 | 2.7100271 | 0.15030573 |
| 10349383 | Slc35f5 | 2.6954178 | 0.00316724 |
| 10449452 | Fkbp5 | 2.6954178 | 0.00844298 |
| 10602020 | Tbcld8b | 2.6954178 | 0.04446942 |
| 10518300 | Tnfrsflb | 2.688172 | 0.00219415 |
| 10556583 | Nucb2 | 2.688172 | 0.01037182 |
| 10498309 | Pfn2 | 2.688172 | 0.01090422 |
| 10366586 | Ifng | 2.6809651 | 0.03645065 |
| 10606640 | Nox1 | 2.6666667 | 0.00492862 |
| 10352918 | Mir29c | 2.6595745 | 0.02079424 |
| 10410756 | Ankrd32 | 2.6595745 | 0.03124248 |
| 10519988 | Fam185a | 2.6595745 | 0.07678483 |
| 10563883 | Depdcla | 2.6595745 | 0.26481196 |
| 10432511 | Racgap 1 | 2.6525199 | 0.02623238 |
| 10548735 | Dusp16 | 2.6455026 | 0.0091817 |
| 10418927 | Bmprla | 2.6455026 | 0.01148904 |
| 10479010 | Spo11 | 2.6455026 | 0.08476739 |
| 10606058 | Cxcr3 | 2.6385224 | 0.00059492 |
| 10446771 | Lclat1 | 2.6385224 | 0.00557771 |
| 10511588 | Tmem67 | 2.6385224 | 0.00831089 |
| 10347036 | Mtap2 | 2.6385224 | 0.019728 |
| 10405785 | 0610007P08Rik | 2.6385224 | 0.03150043 |
| 10388065 | Nlrp1b | 2.6385224 | 0.03222866 |
| 10365286 | Eid3 | 2.6315789 | 0.00490943 |
| 10524515 | Myo1h | 2.6315789 | 0.03197403 |
| 10594774 | Ccnb2 | 2.6315789 | 0.04620604 |
| 10452516 | Ankrd12 | 2.6315789 | 0.06380163 |
| 10408937 | Atxn1 | 2.617801 | 0.00172482 |
| 10540897 | Pparg | 2.617801 | 0.20092261 |
| 10359339 | Rabgap11 | 2.6109661 | 0.00208466 |
| 10476648 | Dstn | 2.6109661 | 0.02540187 |
| 10345241 | Dst | 2.6041667 | 0.03799886 |
| 10400589 | C79407 | 2.6041667 | 0.24546253 |
| 10473281 | Itgav | 2.5974026 | 0.002269 |
| 10346790 | Ctla4 | 2.5974026 | 0.01623906 |
| 10601303 | Chic1 | 2.5974026 | 0.02815456 |
| 10369993 | Gstt3 | 2.5839793 | 0.01439467 |
| 10465244 | Malat1 | 2.5839793 | 0.03915652 |
| 10392010 | 1700081L11Rik | 2.5773196 | 0.00373539 |
| 10534456 | Hip1 | 2.5641026 | 0.00090612 |
| 10355050 | Raph1 | 2.5575448 | 0.00033097 |
| 10498935 | Gucylb3 | 2.5575448 | 0.0008303 |
| 10366073 | Cep290 | 2.5575448 | 0.12416189 |
| 10517364 | A330049M08Rik | 2.5510204 | 0.00376354 |
| 10535065 | Adap 1 | 2.5380711 | 0.00965112 |
| 10556820 | Tmem159 | 2.5380711 | 0.12464267 |
| 10408975 | Kif13a | 2.5188917 | 0.00011864 |
| 10539617 | Alms1 | 2.5188917 | 0.00780576 |
| 10414374 | Ktn1 | 2.5188917 | 0.03076519 |
| 10474875 | Casc5 | 2.5188917 | 0.19047732 |
| 10538791 | Tnip3 | 2.5125628 | 0.08090276 |
| 10554667 | Tmc3 | 2.5062657 | 0.00965655 |
| 10582295 | Odc1 | 2.5062657 | 0.02176383 |
| 10571384 | Efha2 | 2.5 | 0.06702998 |
| 10580457 | N4bp1 | 2.4875622 | 0.00314058 |
| 10542791 | Ppfibpl | 2.4875622 | 0.00355237 |
| 10572170 | D130040H23Rik | 2.4875622 | 0.00740691 |
| 10424188 | Mtbp | 2.4875622 | 0.05750915 |
| 10458589 | Prelid2 | 2.4813896 | 0.01795025 |
| 10458581 | Gm10008 | 2.4813896 | 0.02598396 |
| 10354286 | Kdelc1 | 2.4813896 | 0.02892792 |
| 10462632 | Kif20b | 2.4813896 | 0.12489398 |
| 10436662 | Mir155 | 2.4813896 | 0.17654312 |
| 10519951 | Pion | 2.4752475 | 0.00127643 |
| 10581813 | Mlkl | 2.4752475 | 0.05280226 |
| 10554445 | Prc1 | 2.4752475 | 0.0706501 |
| 10416155 | Kctd9 | 2.4691358 | 0.00116305 |
| 10411728 | Cenph | 2.4691358 | 0.23008238 |
| 10355312 | Ikzf2 | 2.4630542 | 0.00918153 |
| 10398039 | Serpina3f | 2.4630542 | 0.01679921 |
| 10357115 | Dsel | 2.4630542 | 0.05387983 |
| 10538394 | Plekha8 | 2.4570025 | 0.01308491 |
| 10488459 | Zfp442 | 2.4570025 | 0.08163826 |
| 10396645 | Zbtb 1 | 2.4509804 | 0.0039396 |
| 10587792 | Plscr1 | 2.4449878 | 0.00073185 |
| 10453082 | Hnrpll | 2.4449878 | 0.00493423 |
| 10355227 | 1110028C15Rik | 2.4449878 | 0.03236243 |
| 10349593 | Faim3 | 2.4449878 | 0.0380532 |
| 10503617 | F730047E07Rik | 2.4449878 | 0.04447421 |
| 10435712 | Cd80 | 2.4449878 | 0.07761971 |
| 10452508 | Twsg1 | 2.4390244 | 0.01931803 |
| 10594251 | Kif23 | 2.4390244 | 0.03323231 |
| 10421877 | Diap3 | 2.4390244 | 0.05932931 |
| 10370544 | 2610008E11Rik | 2.43309 | 0.02088547 |
| 10574023 | Mt2 | 2.43309 | 0.24469845 |
| 10601449 | Sh3bgrl | 2.4271845 | 0.01427406 |
| 10428536 | Trps1 | 2.4271845 | 0.0156411 |
| 10394783 | Hpcal 1 | 2.4271845 | 0.02267103 |
| 10423556 | Pgcp | 2.4271845 | 0.06123205 |
| 10487340 | Ncaph | 2.4271845 | 0.11463912 |
| 10501629 | Cdc14a | 2.4213075 | 0.00402386 |
| 10602385 | Pfkfb 1 | 2.4213075 | 0.00811626 |
| 10590479 | Zfp167 | 2.4213075 | 0.01973184 |
| 10578690 | Nei13 | 2.4213075 | 0.20210435 |
| 10469720 | Acbd5 | 2.4154589 | 0.03806352 |
| 10507112 | Stil | 2.4154589 | 0.26073984 |
| 10603151 | Gpm6b | 2.4096386 | 0.09804744 |
| 10571870 | Hmgb2 | 2.4096386 | 0.12672335 |
| 10587639 | Nt5e | 2.4038462 | 0.0008956 |
| 10442224 | BC049807 | 2.4038462 | 0.00576263 |
| 10554325 | 5730590G19Rik | 2.4038462 | 0.045224 |
| 10367076 | Prim1 | 2.4038462 | 0.09828601 |
| 10593492 | Zc3h12c | 2.3866348 | 0.00601872 |
| 10407211 | Ppap2a | 2.3866348 | 0.02630078 |
| 10543067 | Asns | 2.3809524 | 0.01008112 |
| 10590597 | Sacm11 | 2.3809524 | 0.01294225 |
| 10495186 | AI504432 | 2.3809524 | 0.02193077 |
| 10459905 | Setbp1 | 2.3809524 | 0.05812016 |
| 10497971 | Sclt1 | 2.3809524 | 0.08800643 |
| 10505213 | E130308A19Rik | 2.3696682 | 0.00173153 |
| 10595371 | Hmgn3 | 2.3696682 | 0.01334807 |
| 10449935 | Zfp870 | 2.3696682 | 0.01872383 |
| 10518350 | Hmgb2 | 2.3696682 | 0.13314054 |
| 10480432 | Mastl | 2.3696682 | 0.27433254 |
| 10440388 | Hspa13 | 2.3640662 | 0.00339499 |
| 10521136 | Whsc1 | 2.3640662 | 0.00446787 |
| 10455647 | Tnfaip8 | 2.3584906 | 0.00021297 |
| 10591614 | Dock6 | 2.3584906 | 0.01325737 |
| 10579049 | Gm10033 | 2.3584906 | 0.02444929 |
| 10535883 | Katnal1 | 2.3584906 | 0.04351106 |
| 10395273 | Gdap10 | 2.3584906 | 0.12686918 |
| 10383897 | Nf2 | 2.3529412 | 0.00466102 |
| 10510580 | Tnfrsf9 | 2.3529412 | 0.01860989 |
| 10369815 | Cdk1 | 2.3529412 | 0.07141248 |
| 10368050 | Ect2l | 2.3474178 | 0.00385925 |
| 10405804 | 0610007P08Rik | 2.3474178 | 0.00543767 |
| 10428310 | Azin1 | 2.3474178 | 0.01111183 |
| 10445977 | Ebi3 | 2.3474178 | 0.01516525 |
| 10365845 | Fgd6 | 2.3474178 | 0.02901281 |
| 10406757 | Col4a3bp | 2.3474178 | 0.03391371 |
| 10440288 | Zfp654 | 2.3419204 | 0.02826963 |
| 10491805 | Plk4 | 2.3419204 | 0.08724536 |
| 10415911 | Kif13b | 2.3364486 | 0.00252308 |
| 10531256 | AU017193 | 2.3364486 | 0.00833691 |
| 10462535 | Pten | 2.3364486 | 0.01977837 |
| 10579052 | Gm10033 | 2.3364486 | 0.05030866 |
| 10384373 | Fignl1 | 2.3364486 | 0.08904826 |
| 10382890 | Sec14l1 | 2.3310023 | 0.03723295 |
| 10374895 | 1700034F02Rik | 2.3255814 | 0.01281525 |
| 10412921 | Nid2 | 2.3201856 | 0.00918366 |
| 10601844 | Bhlhb9 | 2.3201856 | 0.01930196 |
| 10599416 | Gm10483 | 2.3201856 | 0.0193513 |
| 10607774 | Mospd2 | 2.3201856 | 0.02461639 |
| 10546855 | Srgap3 | 2.3201856 | 0.07156709 |
| 10384974 | Il9r | 2.3201856 | 0.07218936 |
| 10422028 | Tbcld4 | 2.3148148 | 0.00338815 |
| 10578300 | Mtmr7 | 2.3148148 | 0.02188629 |
| 10545958 | Anxa4 | 2.3148148 | 0.07808225 |
| 10389025 | Myold | 2.3094688 | 0.00695187 |
| 10569707 | Myadm | 2.3041475 | 0.04818941 |
| 10401317 | Gm4787 | 2.2988506 | 0.01407323 |
| 10503196 | Chd7 | 2.2988506 | 0.01590884 |
| 10583326 | Slc36a4 | 2.2988506 | 0.02031783 |
| 10491780 | Hspa41 | 2.2988506 | 0.03303836 |
| 10436169 | Ift57 | 2.2988506 | 0.04472591 |
| 10510172 | Hmgb2 | 2.2988506 | 0.132583 |
| 10420877 | Esco2 | 2.2988506 | 0.27628066 |
| 10379127 | Spag5 | 2.293578 | 0.07839113 |
| 10496262 | Nhedc2 | 2.2883295 | 0.01981333 |
| 10417579 | 4930452B06Rik | 2.2883295 | 0.0611149 |
| 10521927 | Tbcld19 | 2.283105 | 0.00666418 |
| 10541114 | Rasgefla | 2.283105 | 0.0075592 |
| 10441436 | Snx9 | 2.283105 | 0.02399696 |
| 10395612 | G2e3 | 2.2779043 | 0.01471304 |
| 10389134 | Slfn9 | 2.2779043 | 0.05108747 |
| 10394954 | Grhl1 | 2.2727273 | 0.00025111 |
| 10412267 | Itga2 | 2.2727273 | 0.00212204 |
| 10595718 | Chst2 | 2.2727273 | 0.01464964 |
| 10396421 | Hifla | 2.2624434 | 0.02758493 |
| 10448247 | Zfp40 | 2.2624434 | 0.03927672 |
| 10547469 | Wnk1 | 2.2624434 | 0.10076335 |
| 10466835 | Snora19 | 2.2624434 | 0.10512874 |
| 10380116 | Rnf43 | 2.2573363 | 0.00180114 |
| 10399691 | Id2 | 2.2573363 | 0.00624245 |
| 10590909 | Endod1 | 2.2573363 | 0.01808855 |
| 10603881 | Zfp182 | 2.2573363 | 0.03851246 |
| 10485963 | Arhgaplla | 2.2573363 | 0.0866043 |
| 10451761 | Tbcld5 | 2.2573363 | 0.23402181 |
| 10442219 | Zfp52 | 2.2522523 | 0.0034822 |
| 10407467 | Akr1e1 | 2.2522523 | 0.00960602 |
| 10357436 | Mcm6 | 2.2522523 | 0.01942241 |
| 10476945 | Cst7 | 2.2522523 | 0.02205786 |
| 10447084 | Galm | 2.2522523 | 0.02988624 |
| 10584615 | Pvrl1 | 2.2522523 | 0.07439894 |
| 10606714 | Gla | 2.247191 | 0.06098277 |
| 10409994 | Gm5665 | 2.247191 | 0.11000257 |
| 10468762 | 4930506M07Rik | 2.2421525 | 0.00761633 |
| 10592515 | Ubash3b | 2.2371365 | 0.00885917 |
| 10483679 | Gpr155 | 2.2371365 | 0.02116137 |
| 10399973 | Hdac9 | 2.2371365 | 0.06215052 |
| 10353004 | Cks2 | 2.2371365 | 0.07932277 |
| 10441195 | Dscam | 2.2321429 | 0.0056505 |
| 10379363 | Atad5 | 2.2321429 | 0.1076887 |
| 10356329 | Snora75 | 2.2321429 | 0.1775966 |
| 10593332 | Bco2 | 2.2321429 | 0.19013999 |
| 10394611 | Nbas | 2.2271715 | 0.00355172 |
| 10503218 | Chd7 | 2.2271715 | 0.02083899 |
| 10441633 | Ccr6 | 2.2271715 | 0.03100126 |
| 10562651 | C330019L16Rik | 2.2271715 | 0.0354997 |
| 10476297 | Mir103-2 | 2.2271715 | 0.0608872 |
| 10420670 | Dleu2 | 2.2271715 | 0.07459412 |
| 10405185 | Cks2 | 2.2271715 | 0.107097 |
| 10594221 | Lrrc49 | 2.2222222 | 0.03825033 |
| 10529741 | Rab28 | 2.2172949 | 0.01176421 |
| 10518352 | Hmgb2 | 2.2172949 | 0.14011238 |
| 10497399 | Pde7a | 2.2123894 | 0.00753532 |
| 10381588 | Grn | 2.2123894 | 0.01370998 |
| 10504692 | Tmod1 | 2.2123894 | 0.0257923 |
| 10592106 | Tirap | 2.2123894 | 0.10197148 |
| 10406270 | Glrx | 2.2075055 | 0.01001241 |
| 10467110 | Lipo 1 | 2.2075055 | 0.04823091 |
| 10531737 | Hpse | 2.2075055 | 0.0484914 |
| 10533844 | Rilpl2 | 2.2026432 | 0.00438382 |
| 10406905 | Ccdc125 | 2.2026432 | 0.01098253 |
| 10513141 | Ptpn3 | 2.1978022 | 0.01652439 |
| 10349510 | Mir128-1 | 2.1978022 | 0.04433153 |
| 10357363 | Nckap5 | 2.1929825 | 0.03936668 |
| 10379153 | Aldoc | 2.1929825 | 0.06289367 |
| 10390519 | Plxdc1 | 2.1929825 | 0.06869935 |
| 10597279 | Ccrl2 | 2.1881838 | 0.02338559 |
| 10602009 | Rnf128 | 2.1881838 | 0.03506544 |
| 10594301 | Coro2b | 2.1881838 | 0.12097252 |
| 10385323 | Mir146 | 2.1834061 | 0.02385294 |
| 10406364 | 2210408I21Rik | 2.1786492 | 0.01275564 |
| 10519324 | Cdk6 | 2.1786492 | 0.01549031 |
| 10608138 | Ddx3y | 2.1786492 | 0.03732003 |
| 10389395 | Brip 1 | 2.1786492 | 0.08775499 |
| 10485405 | Cd44 | 2.173913 | 0.01271296 |
| 10350594 | Ivnslabp | 2.173913 | 0.04840403 |
| 10421555 | Mir687 | 2.173913 | 0.05565063 |
| 10467230 | Ide | 2.1691974 | 0.00386768 |
| 10407792 | Gpr137b-ps | 2.1691974 | 0.00858809 |
| 10587683 | Bcl2ala | 2.1691974 | 0.03382114 |
| 10369102 | Gm9766 | 2.1645022 | 0.00916278 |
| 10595633 | Bcl2ald | 2.1645022 | 0.02794013 |
| 10425207 | H1f0 | 2.1645022 | 0.03520816 |
| 10587107 | Myo5a | 2.1598272 | 0.02041305 |
| 10602827 | A830080D01Rik | 2.1598272 | 0.04816672 |
| 10603598 | Rpgr | 2.1551724 | 0.01262466 |
| 10523012 | Dck | 2.1551724 | 0.02170034 |
| 10474769 | Bub1b | 2.1551724 | 0.04249554 |
| 10352756 | Lpgat1 | 2.1505376 | 0.00778529 |
| 10480329 | Dnajc1 | 2.1505376 | 0.00964467 |
| 10587690 | Bcl2alb | 2.1505376 | 0.01734898 |
| 10594110 | Neo1 | 2.1505376 | 0.02541233 |
| 10405733 | 6720457D02Rik | 2.1505376 | 0.04428622 |
| 10514865 | Acot1 1 | 2.1505376 | 0.04791578 |
| 10587733 | Ctsh | 2.1505376 | 0.17161953 |
| 10547906 | Lag3 | 2.1459227 | 0.00569861 |
| 10421737 | Tnfsf11 | 2.1459227 | 0.01318614 |
| 10394625 | Nbas | 2.1459227 | 0.01676718 |
| 10430006 | Slc39a4 | 2.1459227 | 0.03234083 |
| 10568150 | Kif22 | 2.1459227 | 0.08123392 |
| 10497077 | Mir186 | 2.1459227 | 0.09669853 |
| 10412543 | Gm1973 | 2.1413276 | 0.00102788 |
| 10603567 | Dynlt3 | 2.1413276 | 0.00663607 |
| 10354647 | Pgap 1 | 2.1413276 | 0.03769364 |
| 10513166 | Ptpn3 | 2.1367521 | 0.00559333 |
| 10536390 | Glcci1 | 2.1367521 | 0.0086892 |
| 10346330 | Plcl1 | 2.1367521 | 0.01303019 |
| 10442240 | Zfp760 | 2.1321962 | 0.02403225 |
| 10539080 | St3gal5 | 2.1321962 | 0.10672212 |
| 10393559 | Timp2 | 2.1276596 | 0.0131688 |
| 10414537 | Rnase4 | 2.1276596 | 0.01691999 |
| 10408519 | Hus1b | 2.1276596 | 0.0274425 |
| 10481857 | Pbx3 | 2.1231423 | 0.02611634 |
| 10410560 | Trip13 | 2.1231423 | 0.07295799 |
| 10490872 | Lrrcc 1 | 2.1231423 | 0.08667311 |
| 10435789 | Zbtb20 | 2.1231423 | 0.08964086 |
| 10400006 | Ahr | 2.1141649 | 0.01354896 |
| 10535747 | Gm10858 | 2.1141649 | 0.01496187 |
| 10461856 | Gna14 | 2.1141649 | 0.01721532 |
| 10599369 | Xiap | 2.1141649 | 0.03468285 |
| 10501164 | Csf1 | 2.1141649 | 0.03582429 |
| 10565292 | Arnt2 | 2.1141649 | 0.04983575 |
| 10346365 | Sgol2 | 2.1141649 | 0.18050565 |
| 10589884 | Bcl2alc | 2.1097046 | 0.0682851 |
| 10592471 | Gramdlb | 2.1052632 | 0.00074598 |
| 10597420 | Ccr4 | 2.1052632 | 0.00466806 |
| 10371220 | Gna15 | 2.1052632 | 0.05997275 |
| 10503264 | Ccne2 | 2.1052632 | 0.14016493 |
| 10436402 | Cldnd1 | 2.1008403 | 0.00093241 |
| 10394749 | Nol10 | 2.1008403 | 0.02963964 |
| 10358459 | BC003331 | 2.1008403 | 0.0567876 |
| 10585338 | Kdelc2 | 2.0964361 | 0.00070799 |
| 10371356 | Appl2 | 2.0964361 | 0.00749817 |
| 10563780 | E2f8 | 2.0964361 | 0.18388031 |
| 10350392 | Aspm | 2.0964361 | 0.20422759 |
| 10435704 | Cd80 | 2.0920502 | 0.00273045 |
| 10601834 | Gprasp2 | 2.0920502 | 0.00742276 |
| 10503194 | Chd7 | 2.0920502 | 0.01333232 |
| 10434291 | B3gnt5 | 2.0920502 | 0.02474341 |
| 10468949 | Dclrelc | 2.0920502 | 0.0323231 |
| 10468527 | 5830416P10Rik | 2.0876827 | 0.01308794 |
| 10572724 | Zfp709 | 2.0876827 | 0.01743369 |
| 10355998 | Fam124b | 2.0876827 | 0.02278189 |
| 10424404 | Pvt1 | 2.0833333 | 0.00758977 |
| 10417095 | Farp1 | 2.0833333 | 0.00915935 |
| 10558773 | B4galnt4 | 2.0833333 | 0.03058125 |
| 10453867 | Rbbp8 | 2.0833333 | 0.05294243 |
| 10606263 | Atrx | 2.0833333 | 0.06987161 |
| 10428763 | Atad2 | 2.0833333 | 0.07035945 |
| 10371591 | 4930547N16Rik | 2.0833333 | 0.16138153 |
| 10603814 | Slc9a7 | 2.0790021 | 0.01344989 |
| 10474902 | Rad51 | 2.0790021 | 0.05271723 |
| 10416956 | Mir19b-1 | 2.0790021 | 0.19045001 |
| 10599411 | Sh2dla | 2.0746888 | 0.00059302 |
| 10424221 | Wdr67 | 2.0746888 | 0.01206853 |
| 10499138 | Dclk2 | 2.0746888 | 0.02070704 |
| 10346695 | Nbeal1 | 2.0746888 | 0.0296774 |
| 10389606 | Prr11 | 2.0746888 | 0.10753061 |
| 10545707 | Actg2 | 2.0703934 | 0.03709829 |
| 10478928 | Tshz2 | 2.0661157 | 0.00087833 |
| 10524866 | Vsig10 | 2.0661157 | 0.00174265 |
| 10406086 | Tert | 2.0661157 | 0.01749025 |
| 10388042 | 6330403K07Rik | 2.0661157 | 0.01833252 |
| 10467578 | Pik3ap1 | 2.0661157 | 0.03302219 |
| 10482030 | Stom | 2.0618557 | 0.00600923 |
| 10357345 | Nckap5 | 2.0618557 | 0.07845241 |
| 10359982 | Atf6 | 2.0576132 | 0.00108407 |
| 10417526 | Dnase113 | 2.0576132 | 0.00962298 |
| 10345423 | Plekhb2 | 2.0576132 | 0.01085091 |
| 10457429 | Rock1 | 2.0576132 | 0.10276042 |
| 10407126 | Plk2 | 2.0576132 | 0.1387202 |
| 10496638 | Odf21 | 2.0576132 | 0.19410796 |
| 10363265 | Lims1 | 2.0533881 | 0.00661461 |
| 10590623 | Cxcr6 | 2.0533881 | 0.00962734 |
| 10435514 | Ildr1 | 2.0533881 | 0.01643374 |
| 10413517 | Chdh | 2.0533881 | 0.01902125 |
| 10409990 | 6720489N17Rik | 2.0533881 | 0.03708042 |
| 10400304 | Egln3 | 2.0533881 | 0.05740125 |
| 10409978 | 6720457D02Rik | 2.0533881 | 0.05875589 |
| 10543779 | Mir29a | 2.0533881 | 0.17557649 |
| 10587854 | Slc9a9 | 2.0491803 | 0.00438505 |
| 10351015 | Serpinc1 | 2.0491803 | 0.00578176 |
| 10361834 | Txlnb | 2.0491803 | 0.01143309 |
| 10455873 | Slc12a2 | 2.0491803 | 0.01797121 |
| 10356880 | St8sia4 | 2.0491803 | 0.02363305 |
| 10606876 | Morf4l2 | 2.0491803 | 0.0384018 |
| 10374406 | Cnrip1 | 2.0491803 | 0.06007526 |
| 10464128 | Casp7 | 2.0449898 | 0.00166227 |
| 10588091 | Cep70 | 2.0449898 | 0.02267903 |
| 10517287 | Man1c1 | 2.0449898 | 0.02552988 |
| 10354529 | 1700019D03Rik | 2.0408163 | 0.01566418 |
| 10405783 | Mir24-1 | 2.0408163 | 0.02445097 |
| 10531610 | Rasgeflb | 2.0408163 | 0.05800715 |
| 10369171 | 9530009G21Rik | 2.0408163 | 0.06855793 |
| 10515431 | Kif2c | 2.0408163 | 0.08892337 |
| 10431424 | Plxnb2 | 2.0366599 | 0.0123898 |
| 10365899 | Ccdc41 | 2.0366599 | 0.05169979 |
| 10600765 | Pcytlb | 2.0325203 | 0.00560068 |
| 10552264 | 9430025M13Rik | 2.0325203 | 0.00895905 |
| 10424779 | Cks2 | 2.0325203 | 0.07938786 |
| 10480275 | Nebl | 2.0283976 | 0.00163009 |
| 10507286 | Ipp | 2.0283976 | 0.01100194 |
| 10461369 | Ahnak | 2.0283976 | 0.01144069 |
| 10503251 | 2610301B20Rik | 2.0283976 | 0.01893445 |
| 10592725 | Gm10688 | 2.0283976 | 0.02243805 |
| 10430113 | Arhgap39 | 2.0283976 | 0.02249738 |
| 10490826 | Zbtb10 | 2.0283976 | 0.16655707 |
| 10368144 | Tnfaip3 | 2.0283976 | 0.18745235 |
| 10551852 | Clip3 | 2.0242915 | 0.01122009 |
| 10352916 | Mir29b-2 | 2.0242915 | 0.0194867 |
| 10399588 | Zfp125 | 2.0242915 | 0.05567409 |
| 10462398 | Pdcd1lg2 | 2.020202 | 0.01877215 |
| 10428698 | Sntb1 | 2.016129 | 0.00092118 |
| 10476314 | Prnp | 2.016129 | 0.03114167 |
| 10563659 | Spty2d1 | 2.016129 | 0.1008961 |
| 10456005 | Cd74 | 2.0120724 | 0.01858586 |
| 10371092 | Atcay | 2.0120724 | 0.0197594 |
| 10456357 | Pmaip 1 | 2.0120724 | 0.02046887 |
| 10346960 | Ccnyl1 | 2.0120724 | 0.02305044 |
| 10582941 | Cwf1912 | 2.0120724 | 0.10122588 |
| 10601903 | Zcchc18 | 2.0080321 | 0.00283264 |
| 10372028 | Plxnc1 | 2.0080321 | 0.04271335 |
| 10556280 | Swap70 | 2.0080321 | 0.04782103 |
| 10562132 | Cd22 | 2.0080321 | 0.12021187 |
| 10538617 | Lancl2 | 2.004008 | 0.0086513 |
| 10445373 | B230354K17Rik | 2.004008 | 0.0248183 |
| 10565840 | Neu3 | 2.004008 | 0.0316306 |
| 10513818 | Stmn1 | 2.004008 | 0.10169755 |
| 10530492 | Nfx11 | 2 | 0.00829441 |
| 10494978 | Ptpn22 | 2 | 0.01136496 |
| 10456346 | Sec11c | 1.996008 | 0.08923052 |
| 10474596 | Aven | 0.5 | 0.11649388 |
| 10472587 | Rpl13 | 0.4997501 | 0.00400507 |
| 10605113 | Llcam | 0.4995005 | 0.00445563 |
| 10544596 | Tmem176b | 0.4995005 | 0.17169004 |
| 10537909 | Rny3 | 0.4992511 | 0.18015923 |
| 10405693 | Dapk1 | 0.4982561 | 0.00579729 |
| 10536010 | C87414 | 0.4977601 | 0.00795618 |
| 10510391 | Srm | 0.4977601 | 0.02840111 |
| 10545479 | Tmsb10 | 0.4962779 | 0.00121938 |
| 10513420 | Mup7 | 0.4962779 | 0.0026631 |
| 10482814 | Acvr1c | 0.4962779 | 0.02069101 |
| 10512827 | Gm568 | 0.4962779 | 0.04440213 |
| 10404053 | Histlh2bc | 0.4960317 | 0.04462586 |
| 10445877 | Gm16489 | 0.4952947 | 0.28933281 |
| 10456001 | Rps14 | 0.4940711 | 0.09360483 |
| 10419162 | 4930503E14Rik | 0.4926108 | 0.07017411 |
| 10356333 | Snord82 | 0.4918839 | 0.0004474 |
| 10445774 | B430306N03Rik | 0.4918839 | 0.00836391 |
| 10570513 | Kbtbd 11 | 0.4918839 | 0.0260371 |
| 10508651 | Sdc3 | 0.490918 | 0.07270972 |
| 10560911 | Rabac1 | 0.4901961 | 0.00336931 |
| 10598218 | Gm2799 | 0.4894763 | 0.06129231 |
| 10551009 | Tmsb10 | 0.4882813 | 0.00020919 |
| 10513504 | Mup2 | 0.4882813 | 0.01211184 |
| 10608263 | Sly | 0.487567 | 0.26411037 |
| 10452257 | Slc25a23 | 0.4870921 | 0.06174372 |
| 10494411 | Rnulbl | 0.486618 | 0.04126581 |
| 10560780 | Vmn1r101 | 0.4863813 | 0.02147802 |
| 10545210 | Gm1524 | 0.4859086 | 0.04889538 |
| 10385872 | Slc22a5 | 0.4859086 | 0.04909365 |
| 10608410 | Sly | 0.4859086 | 0.18330955 |
| 10608625 | LOC100040235 | 0.4854369 | 0.09419057 |
| 10503695 | Bach2 | 0.4852014 | 0.01594984 |
| 10522467 | Rasl11b | 0.4844961 | 0.03888329 |
| 10553092 | Dbp | 0.4826255 | 0.10018767 |
| 10385533 | Tgtp1 | 0.4823927 | 0.0513553 |
| 10479463 | Slc17a9 | 0.4814636 | 0.00014776 |
| 10393449 | Socs3 | 0.481232 | 0.01955942 |
| 10380571 | Gngt2 | 0.4805382 | 0.00445514 |
| 10503833 | Rplp1 | 0.4800768 | 0.00144009 |
| 10608237 | Sly | 0.4796163 | 0.23349111 |
| 10399677 | Cox7a2l | 0.4791567 | 0.03681363 |
| 10419125 | Gm8005 | 0.4786979 | 0.00290623 |
| 10532085 | Tgfbr3 | 0.4784689 | 0.00153001 |
| 10491058 | Rprl2 | 0.4768717 | 0.00447126 |
| 10608628 | LOC100041704 | 0.4768717 | 0.1973275 |
| 10461162 | Snord22 | 0.4764173 | 0.18146542 |
| 10375058 | Hba-a2 | 0.4759638 | 0.01757608 |
| 10608460 | LOC665698 | 0.4739336 | 0.06223302 |
| 10444041 | Ndufa7 | 0.4737091 | 0.03355838 |
| 10544891 | Nod1 | 0.4732608 | 0.00018331 |
| 10408613 | Tubb2b | 0.4728132 | 0.17980019 |
| 10608260 | Srsy | 0.4725898 | 0.02186593 |
| 10419122 | Gm8165 | 0.4725898 | 0.08673251 |
| 10471503 | Tafld | 0.4725898 | 0.09294577 |
| 10499748 | Rps27 | 0.4721435 | 0.01239656 |
| 10608385 | Sly | 0.4716981 | 0.25443181 |
| 10597490 | Rps27 | 0.4708098 | 0.01298652 |
| 10571325 | Mfhas 1 | 0.4699248 | 0.00524913 |
| 10598225 | Gm2799 | 0.4677268 | 0.06050661 |
| 10576795 | Cd209a | 0.4675082 | 0.00435424 |
| 10461012 | Trmt112 | 0.4670715 | 0.02437115 |
| 10608308 | Srsy | 0.4664179 | 0.01262089 |
| 10414767 | Rps19 | 0.4659832 | 0.03882346 |
| 10570516 | Kbtbd 11 | 0.4649 | 0.01845397 |
| 10398599 | Rps19 | 0.464684 | 0.03972966 |
| 10472757 | Cybrd1 | 0.4631774 | 0.06063082 |
| 10479362 | Rps21 | 0.462963 | 0.02994749 |
| 10460968 | Rasgrp2 | 0.4627487 | 0.00650458 |
| 10375051 | Hba-a1 | 0.4627487 | 0.01119691 |
| 10360832 | 1700056E22Rik | 0.4625347 | 0.02445717 |
| 10487021 | Slc30a4 | 0.4621072 | 0.01842405 |
| 10351515 | Rnulbl | 0.4614675 | 0.03562672 |
| 10552964 | Ftl1 | 0.4612546 | 0.04358579 |
| 10608488 | LOC665128 | 0.4612546 | 0.06741966 |
| 10598183 | Gm2799 | 0.461042 | 0.15727075 |
| 10502510 | Lmo4 | 0.4599816 | 0.01712604 |
| 10608407 | Srsy | 0.4599816 | 0.04384627 |
| 10351206 | Selp | 0.4593477 | 0.09488342 |
| 10608615 | LOC380994 | 0.4585053 | 0.21947653 |
| 10603232 | Gm2799 | 0.4580852 | 0.11902275 |
| 10545192 | Rprl1 | 0.4562044 | 0.01280426 |
| 10550778 | Vmn1r132 | 0.4557885 | 0.01813138 |
| 10550765 | Vmn1r148 | 0.4545455 | 0.03033933 |
| 10489065 | Ndrg3 | 0.4537205 | 0.00260561 |
| 10437668 | Socs1 | 0.4526935 | 0.04555823 |
| 10424607 | Ptp4a3 | 0.452284 | 0.02292982 |
| 10608531 | LOC100504530 | 0.4518753 | 0.06981132 |
| 10530560 | Slain2 | 0.4514673 | 0.09070758 |
| 10462618 | Ifit3 | 0.4512635 | 0.00449285 |
| 10401244 | Actn1 | 0.4504505 | 0.00247861 |
| 10400635 | Rps29 | 0.4502476 | 0.00063662 |
| 10607870 | Tlr7 | 0.450045 | 0.00467426 |
| 10608506 | LOC100039753 | 0.4498426 | 0.12685831 |
| 10516908 | Snora73a | 0.4492363 | 0.00527233 |
| 10608277 | Ssty2 | 0.4492363 | 0.11866777 |
| 10559635 | Hspbp1 | 0.4490346 | 0.05004011 |
| 10608342 | LOC100041704 | 0.4490346 | 0.08283511 |
| 10608212 | Sly | 0.448833 | 0.17849941 |
| 10560795 | Vmn1r158 | 0.4476276 | 0.02368124 |
| 10608302 | Ssty1 | 0.4472272 | 0.12510276 |
| 10608420 | Ssty1 | 0.4472272 | 0.25077694 |
| 10380059 | Rnu3b1 | 0.4462294 | 0.01437788 |
| 10608551 | Srsy | 0.4446421 | 0.01751658 |
| 10349102 | Bcl2 | 0.4440497 | 0.06237449 |
| 10591739 | Acp5 | 0.4432624 | 0.02297332 |
| 10494413 | Rnulbl | 0.4415011 | 0.0411497 |
| 10364102 | Chchd10 | 0.4411116 | 0.0316024 |
| 10447429 | Gm4832 | 0.4403347 | 0.07690707 |
| 10608247 | LOC100042196 | 0.4393673 | 0.11704582 |
| 10560754 | Vmn1r132 | 0.4382121 | 0.02421074 |
| 10577226 | 2610019F03Rik | 0.4376368 | 0.04884058 |
| 10608371 | LOC665406 | 0.4370629 | 0.07867391 |
| 10608361 | LOC100042359 | 0.4357298 | 0.07551582 |
| 10549495 | Rps29 | 0.4355401 | 0.00268464 |
| 10541307 | Usp18 | 0.4353505 | 0.03279837 |
| 10490126 | Rps29 | 0.4347826 | 0.00044543 |
| 10608549 | LOC100039753 | 0.4347826 | 0.17861321 |
| 10548875 | Art4 | 0.4338395 | 0.02500971 |
| 10608567 | Srsy | 0.4330879 | 0.00864567 |
| 10550189 | Gm10679 | 0.4315926 | 0.00625779 |
| 10521134 | Rps29 | 0.4306632 | 0.00291539 |
| 10590365 | Vipr1 | 0.4304778 | 0.00648496 |
| 10590620 | Ccr9 | 0.4304778 | 0.07149516 |
| 10392910 | C630004H02Rik | 0.4295533 | 0.03632227 |
| 10608630 | Ssty2 | 0.4293688 | 0.12434134 |
| 10550786 | Vmn1r132 | 0.4269855 | 0.02916742 |
| 10422493 | Gpr18 | 0.4257131 | 0.01053923 |
| 10608573 | LOC100504530 | 0.4253509 | 0.02315258 |
| 10550782 | Vmn1r148 | 0.4246285 | 0.02386071 |
| 10608613 | LOC100042196 | 0.4246285 | 0.10963456 |
| 10573427 | Nfix | 0.4224757 | 0.00279782 |
| 10608424 | Ssty1 | 0.4215852 | 0.15634008 |
| 10508719 | Snora16a | 0.4208754 | 0.11172156 |
| 10560728 | Vmnlr158 | 0.4203447 | 0.01905504 |
| 10608394 | Srsy | 0.4191115 | 0.0159561 |
| 10590267 | Snora62 | 0.41841 | 0.01913618 |
| 10550193 | Gm3994 | 0.4177109 | 0.00831483 |
| 10608273 | LOC100040223 | 0.4177109 | 0.07251798 |
| 10608222 | LOC100504530 | 0.4175365 | 0.03370856 |
| 10608327 | LOC100040031 | 0.4175365 | 0.12815183 |
| 10353034 | Snord87 | 0.4171882 | 0.20536112 |
| 10362674 | Rnu3a | 0.4168404 | 0.02916955 |
| 10499130 | Rnu73b | 0.4166667 | 0.06375527 |
| 10608293 | Srsy | 0.41511 | 0.00790248 |
| 10551881 | Sdhaf1 | 0.41511 | 0.07553797 |
| 10454807 | Snora74a | 0.4147657 | 0.05923466 |
| 10379633 | Slfn1 | 0.4140787 | 0.00041818 |
| 10608209 | Srsy | 0.4116921 | 0.00732101 |
| 10608480 | LOC100039147 | 0.4116921 | 0.03408665 |
| 10608282 | LOC100039753 | 0.4098361 | 0.13112934 |
| 10550208 | Gm3994 | 0.4093328 | 0.00665008 |
| 10560732 | Vmnlr-ps79 | 0.4091653 | 0.01638949 |
| 10499378 | Sema4a | 0.4088307 | 0.067101 |
| 10608484 | Ssty2 | 0.4088307 | 0.12129823 |
| 10402512 | Scarna13 | 0.4088307 | 0.15531227 |
| 10608377 | LOC100039753 | 0.4086637 | 0.122138 |
| 10608348 | Ssty2 | 0.4078303 | 0.15572877 |
| 10377265 | Pik3r5 | 0.4071661 | 0.01043105 |
| 10432190 | Adcy6 | 0.4058442 | 0.00856264 |
| 10489235 | 9430008C03Rik | 0.4056795 | 0.03194117 |
| 10560719 | 2210010C17Rik | 0.4056795 | 0.03920352 |
| 10560742 | Vmnlr103 | 0.4053506 | 0.01423602 |
| 10550768 | Vmnlr122 | 0.4051864 | 0.01337363 |
| 10516906 | Snora73b | 0.4051864 | 0.02263855 |
| 10362896 | Cd24a | 0.4045307 | 0.1707852 |
| 10608482 | LOC665746 | 0.4042037 | 0.06695088 |
| 10608350 | LOC100039552 | 0.4040404 | 0.07989928 |
| 10501591 | A930005H10Rik | 0.4035513 | 0.11059473 |
| 10560744 | Vmnlr117 | 0.4019293 | 0.01896927 |
| 10608368 | LOC100041256 | 0.4014452 | 0.14889401 |
| 10540542 | LOC100503669 | 0.4003203 | 0.00097644 |
| 10560789 | Vmnlr151 | 0.4001601 | 0.01495732 |
| 10550770 | Vmn1r114 | 0.4001601 | 0.02510794 |
| 10608295 | LOC100039753 | 0.3992016 | 0.12280064 |
| 10399943 | Cdhr3 | 0.3980892 | 0.00193339 |
| 10608608 | Ssty2 | 0.3976143 | 0.11231776 |
| 10376885 | Snord49b | 0.3972984 | 0.01124483 |
| 10608365 | Ssty2 | 0.396668 | 0.12169133 |
| 10376887 | Snord49a | 0.3963535 | 0.03005858 |
| 10439237 | Rps21 | 0.3957262 | 0.00080957 |
| 10560797 | Vmnlr-ps79 | 0.3954132 | 0.01686886 |
| 10461156 | Snhg1 | 0.3947888 | 0.00690243 |
| 10560785 | Vmnlr-ps79 | 0.3938558 | 0.02374871 |
| 10608477 | Ssty2 | 0.3904725 | 0.1419859 |
| 10459766 | Scarna17 | 0.3892565 | 0.00479583 |
| 10365098 | Tbxa2r | 0.3888025 | 0.00404659 |
| 10608339 | Ssty2 | 0.3881988 | 0.12323147 |
| 10560740 | Gm10670 | 0.3866976 | 0.02285695 |
| 10560730 | Vmnlr93 | 0.3853565 | 0.02435977 |
| 10608373 | Ssty2 | 0.385208 | 0.11609829 |
| 10608457 | Ssty2 | 0.3847634 | 0.12523244 |
| 10608606 | LOC100039753 | 0.3844675 | 0.17145298 |
| 10358713 | 1700025G04Rik | 0.382995 | 0.05347887 |
| 10606654 | Xkrx | 0.3827019 | 0.01247528 |
| 10608521 | Ssty1 | 0.381971 | 0.14062238 |
| 10608454 | Ssty2 | 0.3797949 | 0.12602111 |
| 10376269 | Galnt10 | 0.3786445 | 0.00615746 |
| 10480238 | St8sia6 | 0.3785011 | 0.01070054 |
| 10414953 | Gml6591 | 0.3736921 | 0.0070053 |
| 10382104 | Snord104 | 0.3735525 | 0.00270416 |
| 10500204 | Ecm1 | 0.3727171 | 0.00054722 |
| 10550760 | Vmnlr100 | 0.3698225 | 0.02430528 |
| 10432176 | Snora34 | 0.3681885 | 0.03375434 |
| 10360145 | B930036N10Rik | 0.3676471 | 0.15867117 |
| 10524621 | Oasl2 | 0.367242 | 0.02150953 |
| 10503856 | Gabrr2 | 0.3644315 | 0.11913457 |
| 10585803 | Stra6 | 0.3619254 | 0.03613085 |
| 10346876 | Snora41 | 0.3601008 | 0.01110707 |
| 10547073 | Snora7a | 0.3597122 | 0.02766423 |
| 10560752 | Vmnlr125 | 0.3589375 | 0.01428597 |
| 10604076 | Snora69 | 0.3584229 | 0.07649178 |
| 10425799 | Rnu12 | 0.3541076 | 0.27760186 |
| 10585286 | Arhgap20 | 0.3533569 | 0.01122687 |
| 10350159 | Lad1 | 0.3529827 | 0.00211499 |
| 10430851 | Cyp2d22 | 0.3513703 | 0.01398453 |
| 10414781 | Gm13926 | 0.3508772 | 0.00322421 |
| 10598178 | Disp 1 | 0.3497726 | 0.01907486 |
| 10380719 | Sp6 | 0.3376097 | 0.00824907 |
| 10526943 | Gpr146 | 0.3314551 | 0.00504248 |
| 10465059 | Ctsw | 0.3307972 | 0.01628608 |
| 10368277 | Rps12 | 0.3292723 | 0.00083038 |
| 10450920 | AY036118 | 0.3286231 | 0.11252538 |
| 10563114 | Snord32a | 0.3278689 | 0.00455642 |
| 10467420 | Pdlim1 | 0.3274394 | 0.00277614 |
| 10570432 | Snora3 | 0.3270111 | 0.04721699 |
| 10520950 | Pdlim1 | 0.3244646 | 0.00070513 |
| 10556206 | Snora3 | 0.3244646 | 0.04610858 |
| 10580752 | 9330175E14Rik | 0.3234153 | 0.06396521 |
| 10344750 | Sgk3 | 0.3233107 | 0.04867129 |
| 10565813 | Snord15a | 0.3219575 | 0.19731484 |
| 10556528 | Pde3b | 0.3214401 | 0.03639524 |
| 10583286 | Gpr83 | 0.3212335 | 0.00034837 |
| 10564183 | Snord116 | 0.3212335 | 0.01784823 |
| 10390763 | Ccr7 | 0.32 | 0.00608044 |
| 10564177 | Snord116 | 0.317965 | 0.01101963 |
| 10549162 | St8sia1 | 0.3164557 | 0.03366039 |
| 10351043 | Snord47 | 0.3145643 | 0.01937336 |
| 10563108 | Snord35a | 0.312989 | 0.00192573 |
| 10598087 | ND6 | 0.3098853 | 0.25208048 |
| 10554658 | A530021J07Rik | 0.3085467 | 0.14545011 |
| 10572800 | Klf2 | 0.3051572 | 0.03619069 |
| 10583310 | Tafld | 0.303859 | 0.02566778 |
| 10431935 | Amigo2 | 0.3021148 | 0.02486963 |
| 10564161 | Snord116 | 0.3005711 | 0.00675119 |
| 10564163 | Snord116 | 0.2965599 | 0.0081746 |
| 10407435 | Akrlc18 | 0.295858 | 0.20866961 |
| 10455015 | Vaultrc5 | 0.2953337 | 0.03404918 |
| 10544523 | Rny1 | 0.2899391 | 0.04496609 |
| 10563937 | Snord115 | 0.2855511 | 0.00214888 |
| 10508723 | Snora61 | 0.2853881 | 0.05992672 |
| 10358717 | 1700025G04Rik | 0.2840909 | 0.00339189 |
| 10564011 | Snord115 | 0.2832861 | 0.00106156 |
| 10377429 | Snord118 | 0.280112 | 0.06398083 |
| 10563112 | Snord33 | 0.2790179 | 0.07864334 |
| 10564013 | Snord115 | 0.27894 | 0.00333173 |
| 10450363 | Snord52 | 0.2764722 | 0.00194155 |
| 10508721 | Snora44 | 0.2751032 | 0.16392721 |
| 10563110 | Snord34 | 0.2724053 | 0.01176541 |
| 10563099 | Snord35b | 0.2659574 | 0.01981001 |
| 10451763 | Satb1 | 0.2585984 | 0.00372421 |
| 10529515 | Sorcs2 | 0.2478929 | 0.00285724 |
| 10576216 | Snord68 | 0.2351281 | 0.00038855 |
| 10394054 | Cd7 | 0.2329916 | 0.02198172 |
| 10598083 | LOC100503984 | 0.2275831 | 0.06239005 |
| 10445767 | Treml2 | 0.2255809 | 0.00723648 |
| 10565811 | Snord15b | 0.2142245 | 0.06820383 |
| 10603417 | Gata1 | 0.2137666 | 0.00288082 |
| 10569017 | Ifitm3 | 0.1954652 | 0.00836967 |
| 10495659 | Cnn3 | 0.1945525 | 0.05981146 |
| 10461594 | Ms4a4c | 0.1907669 | 0.00190196 |
| 10406852 | Cnn3 | 0.1666944 | 0.10549489 |
| 10403825 | Tcrg-C | 0.1312336 | 0.04662083 |
| 10429573 | Ly6c2 | 0.1183292 | 0.00484551 |
| 10429568 | Ly6c1 | 0.0956572 | 0.00227583 |
| 10381096 | Igfbp4 | 0.095338 | 0.00020963 |
| 10472235 | Dapl1 | 0.0660415 | 0.0335448 |
| 10403821 | Tcrg-V3 | 0.0614213 | 0.03222249 |
| 10407940 | Tcrg-V2 | 0.0598372 | 0.02340547 |

**Table 2: Genes and Probe IDs included in Treg signatures**

| | | | | | | | | | | | | | |
|---|---|---|---|---|---|---|---|---|---|---|---|---|---|
| **Upregulated** | | | **Upregulated** | | | **Upregulated** | | | **Upregulated** | | | **Upregulated** | |
| **Probe** | **Gene** | | **Probe** | **Gene** | | **Probe** | **Gene** | | **Probe** | **Gene** | | **Probe** | **Gene** |
| 10344624 | Lypla1 | | 10346330 | Plcl1 | | 10349603 | Il10 | | 10345791 | Ilrl1 | | 10346799 | Icos |
| 10344713 | Ahcy | | 10346790 | Ctla4 | | 10357833 | Atp2b4 | | 10349603 | Il10 | | 10349593 | Faim3 |
| 10346168 | Stat4 | | 10346799 | Icos | | 10363070 | Gp49a | | 10355567 | Tmbim1 | | 10349603 | Il10 |
| 10346365 | Sgol2 | | 10349603 | 1110 | | 10363082 | Lilrb4 | | 10363070 | Gp49a | | 10349648 | Ctse |
| 10346523 | Bzw1 | | 10350159 | Lad1 | | 10366586 | Ing | | 10363082 | Lilrb4 | | 10357488 | Cd55 |
| 10346764 | Abi2 | | 10350630 | Fam129a | | 10368970 | Prdm1 | | 10366586 | Ifng | | 10357808 | Snrpe |
| 10346790 | Ctla4 | | 10353450 | Gm4956 | | 10389207 | Ccl5 | | 10367919 | Stx11 | | 10357986 | Ptprv |
| 10346799 | Icos | | 10354563 | Dnahc7b | | 10390328 | Tbx21 | | 10368970 | Prdm1 | | 10359890 | Nuf2 |
| 10346943 | Creb1 | | 10355312 | Ikzf2 | | 10398039 | Serpina3f | | 10369525 | 2010107G23Rik | | 10360370 | BC094916 |
| 10347106 | Rpe | | 10356082 | Plscr1 | | 10399691 | Id2 | | 10369932 | Susd2 | | 10368970 | Prdm1 |
| 10348775 | Ppp1 r7 | | 10356866 | Pdcd1 | | 10402325 | Asb2 | | 10375443 | Havcr2 | | 10378286 | Itgae |
| 10349637 | Fam72a | | 10357833 | Atp2b4 | | 10406270 | Glrx | | 10378286 | Itgae | | 10389207 | Ccl5 |
| 10349733 | Nucks1 | | 10358408 | Rgs1 | | 10414708 | Gm7124 | | 10399555 | Kcnf1 | | 10398039 | Serpina3f |
| 10349744 | Slc45a3 | | 10358816 | Lamc1 | | 10414802 | Gm10893 | | 10402136 | Gpr68 | | 10399148 | Rapgef5 |
| 10350090 | Ube2t | | 10359339 | Rabgap1l | | 10414909 | Gm8721 | | 10402325 | Asb2 | | 10400006 | Ahr |
| 10350392 | Aspm | | 10359375 | Gpr52 | | 10414981 | Gm13893 | | 10404840 | Cd83 | | 10408081 | Hist1h1b |
| 10350489 | Uchl5 | | 10360173 | Slamf7 | | 10420308 | Gzmb | | 10408689 | Nrn1 | | 10408689 | Nrn1 |
| 10350630 | Fam129a | | 10361771 | Plagl1 | | 10443980 | Myo1f | | 10420308 | Gzmb | | 10409278 | Nfil3 |
| 10350838 | 2810417H13Rik | | 10363415 | Spock2 | | 10445977 | Ebi3 | | 10427744 | Rai14 | | 10411739 | Ccnb1 |
| 10351047 | Cenpl | | 10365933 | Eea1 | | 10454015 | Ttc39c | | 10440206 | Arl6 | | 10421517 | Cysltr2 |
| 10351277 | Nme7 | | 10367919 | Stx11 | | 10466127 | AW112010 | | 10441633 | Ccr6 | | 10439527 | Tigit |
| 10351404 | Tmco1 | | 10372069 | Socs2 | | 10476759 | Rin2 | | 10454015 | Ttc39c | | 10441233 | Mx1 |
| 10351636 | Refbp2 | | 10373502 | Ikzf4 | | 10478633 | Mmp9 | | 10464905 | Npas4 | | 10444814 | H2-gs10 |
| 10351640 | Refbp2 | | 10375402 | Adam19 | | 10493812 | S100a4 | | 10466521 | Gcnt1 | | 10450723 | H2-T10 |
| 10351658 | Cd48 | | 10378286 | Itgae | | 10493820 | S100a6 | | 10466745 | Tjp2 | | 10450733 | H2-t9 |
| 10352048 | Exo1 | | 10379176 | Unc119 | | 10498576 | Lxn | | 10482824 | Acvr1 | | 10455961 | ligp1 |
| 10352709 | Nsl1 | | 10380719 | Sp6 | | 10519527 | Abcb1a | | 10487508 | Gm14005 | | 10462618 | Ifit3 |
| 10352756 | Lpgat1 | | 10381187 | Atp6v0a1 | | 10519983 | Fgl2 | | 10493812 | S100a4 | | 10463263 | Lztfl1 |
| 10352767 | Nek2 | | 10388591 | Cpd | | 10526832 | LOC 100504914 | | 10507137 | Pdzk1 ip1 | | 10473367 | Slc43a1 |
| 10352954 | Hmgb3 | | 10394674 | Socs2 | | 10531415 | Cxcl10 | | 10511363 | Penk | | 10474875 | Casc5 |
| 10353004 | Cks2 | | 10398039 | Serpina3f | | 10547590 | Klrg1 | | 10521626 | Cc2d2a | | 10477187 | Tpx2 |
| 10353050 | Cops5 | | 10399087 | Ncapg2 | | 10552406 | Nkg7 | | 10523182 | Areg | | 10482528 | Neb |
| 10353181 | Lactb2 | | 10401935 | BC005685 | | 10571399 | Zdhhc2 | | 10523231 | Art3 | | 10482687 | Arl5a |
| 10353250 | Gapdh | | 10402325 | Asb2 | | 10584870 | Tmprss13 | | 10539135 | Capg | | 10487480 | Bub1 |
| 10353733 | Prim2 | | 10403229 | Itgb8 | | 10590628 | Ccr3 | | 10542993 | Pon3 | | 10487506 | Gm14005 |
| 10354275 | 1700029F09Rik | | 10403821 | Tcrg-V3 | | 10590631 | Ccr2 | | 10544660 | Osbpl3 | | 10511779 | Atp6v0d2 |
| 10354307 | Txn1 | | 10403941 | Hist1h3h | | 10590635 | Ccr5 | | 10545707 | Actg2 | | 10515836 | Ccnb1 |
| 10355037 | Wdr12 | | 10403948 | Hist1h2bn | | 10594774 | Ccnb2 | | 10547590 | Klrg1 | | 10519983 | Fgl2 |
| 10355050 | Raph1 | | 10403978 | Hist1h2bk | | 10595718 | Chst2 | | 10552143 | Slc7a10 | | 10521731 | Ncapg |
| 10355115 | Prelid1 | | 10403980 | Hist1h2bj | | 10603151 | Gpm6b | | 10565292 | Arnt2 | | 10554863 | Sytl2 |
| 10355931 | Farsb | | 10404028 | Hist1h3g | | 10603551 | Cybb | | 10571788 | Vegfc | | 10562637 | Ccnb1 |
| 10356082 | Plscr1 | | 10404049 | Hist1h3d | | 10606058 | Cxcr3 | | 10574524 | Ces2c | | 10568714 | Mki67 |
| 10356859 | Dtymk | | 10404061 | Hist1h2bb | | | | | 10574532 | Ces2d-ps | | 10582545 | Mela |
| | | | | | | | | | | | | | |
| 10357242 | Dbi | | 10404063 | Hist1h2ab | | | | | 10576661 | Itgb1 | | 10582549 | Mela |
| 10357436 | Mcm6 | | 10404065 | Hist1h3b | | | | | 10578904 | Cpe | | 10586933 | Nedd4 |
| 10358259 | Nek7 | | 10404389 | Irf4 | | 10344750 | Sgk3 | | 10587683 | Bcl2a1 a | | 10586967 | Gm7265 |
| 10358713 | 1700025G04Rik | | 10404840 | Cd83 | | 10349102 | Bcl2 | | 10587690 | Bcl2a1b | | 10590620 | Ccr9 |
| 10358717 | 1700025G04Rik | | 10406270 | Glrx | | 10350159 | Lad1 | | 10590623 | Cxcr6 | | 10590631 | Ccr2 |
| 10359849 | Uck2 | | 10406334 | Mctp1 | | 10351197 | Sell | | 10590631 | Ccr2 | | 10593332 | Bco2 |
| 10359851 | Uck2 | | 10406982 | Adamts6 | | 10355141 | Klf7 | | 10595633 | Bcl2a1d | | 10603328 | Ccdc22 |
| 10359890 | Nuf2 | | 10407940 | Tcrg-V2 | | 10357043 | Bcl2 | | 10600122 | Xlr3b | | 10607738 | Car5b |
| 10360147 | Refbp2 | | 10408070 | Hist1h2bl | | 10358717 | 1700025G04Rik | | 10604996 | Xlr3a | | | |
| 10360806 | Capn2 | | 10408077 | Hist1h2ak | | 10359689 | Atp1 b1 | | 10605007 | Xlr3c | | | |
| 10360985 | Cenpf | | 10408081 | Hist1h1b | | 10375019 | Nsg2 | | | | | | |
| 10361110 | Dtl | | 10408083 | Hist1h3i | | 10378855 | Ssh2 | | | | | | |
| 10361375 | Fbxo5 | | 10408200 | Hist1h4f | | 10381096 | Igfbp4 | | | | | | |
| 10361995 | Fam54a | | 10408202 | Hist1h3e | | 10403821 | Tcrg-V3 | | | | | | |
| 10362581 | Tube1 | | 10408210 | Hist1h2bf | | 10403825 | Tcrg-C | | | | | | |
| 10362941 | Prep | | 10408239 | Hist1h3c | | 10406852 | Cnn3 | | | | | | |
| 10363498 | Ppa1 | | 10408246 | Hist1h3a | | 10407940 | Tcrg-V2 | | | | | | |
| 10363575 | Dna2 | | 10408689 | Nrn1 | | 10429568 | Ly6c1 | | | | | | |
| 10365227 | Ap3m1 | | 10408693 | F13a1 | | 10429573 | Ly6c2 | | | | | | |
| 10365260 | Txnrd1 | | 10412517 | Gm3002 | | 10445767 | Treml2 | | | | | | |
| 10365420 | Al97468 | | 10412537 | Gm3002 | | 10451763 | Satb1 | | | | | | |
| 10365578 | Nup37 | | 10417258 | Gm3002 | | 10453026 | Prkd3 | | | | | | |
| 10365637 | Arl1 | | 10417264 | Gm3002 | | 10461594 | Ms4a4c | | | | | | |
| 10365933 | Eea1 | | 10417302 | Gm3002 | | 10472235 | Dapl1 | | | | | | |
| 10366277 | E2f7 | | 10417359 | Gm3002 | | 10472501 | Lass6 | | | | | | |
| 10366337 | Nap111 | | 10417411 | Gm3002 | | 10480238 | St8sia6 | | | | | | |
| 10366814 | Cdk4 | | 10417421 | Gm3696 | | 10485607 | Qser1 | | | | | | |
| 10367076 | Prim1 | | 10417461 | Gm10406 | | 10485622 | Qser1 | | | | | | |
| 10368612 | Gapdh | | 10420308 | Gzmb | | 10487021 | Slc30a4 | | | | | | |
| 10369815 | Cdk1 | | 10421517 | Cysltr2 | | 10498599 | Ift80 | | | | | | |
| 10370552 | Ppap2c | | 10424370 | Trib1 | | 10501494 | Amy2b | | | | | | |
| 10371591 | 4930547N16Rik | | 10425049 | Apol9b | | 10501544 | Amy2a5 | | | | | | |
| 10371770 | Gas2l3 | | 10427235 | Prr13 | | 10503695 | Bach2 | | | | | | |
| 10371846 | Apaf1 | | 10430344 | I12rb | | 10503709 | D130062J21 Rik | | | | | | |
| 10371888 | Tmpo | | 10432511 | Racgap1 | | 10514732 | Slc35d1 | | | | | | |
| 10371987 | Metap2 | | 10438626 | Etv5 | | 10529515 | Sorcs2 | | | | | | |
| 10372082 | Nudt4 | | 10439527 | Tigit | | 10530516 | Txk | | | | | | |
| 10372965 | Usp15 | | 10439895 | Alcam | | 10549162 | St8sia1 | | | | | | |
| 10374426 | Pno1 | | 10440393 | Samsn1 | | 10583286 | Gpr83 | | | | | | |
| 10374442 | C1d | | 10441436 | Snx9 | | 10585286 | Arhgap20 | | | | | | |
| 10374466 | Rab1 | | 10443009 | Ergic1 | | 10585976 | Myo9a | | | | | | |
| 10375880 | Nhp2 | | 10443980 | Myo1f | | 10585982 | Myo9a | | | | | | |
| 10375941 | Vdac1 | | 10444824 | H2-Q6 | | 10585986 | Myo9a | | | | | | |
| 10377405 | Aurkb | | 10446771 | Lclat1 | | 10587315 | Gsta4 | | | | | | |
| 10378802 | Blmh | | 10447383 | Epcam | | 10603417 | Gata1 | | | | | | |
| 10378848 | Hsp90aa1 | | 10450374 | D17H6S56E-5 | | 10606178 | Xist | | | | | | |
| 10379127 | Spag5 | | 10452047 | Ptprs | | 10606369 | Itm2a | | | | | | |
| 10379363 | Atad5 | | 10452508 | Twsg1 | | 10607870 | Tlr7 | | | | | | |
| 10379445 | Zfp207 | | 10454015 | Ttc39c | | 10608247 | LOC100042196 | | | | | | |
| 10379968 | Tubd1 | | 10456005 | Cd74 | | 10608273 | LOC 100040223 | | | | | | |
| 10379989 | Fam33a | | 10457225 | Map3k8 | | 10608282 | LOC 100039753 | | | | | | |
| 10379998 | Trim37 | | 10461369 | Ahnak | | 10608302 | Ssty1 | | | | | | |
| 10380403 | Lrrc59 | | 10462398 | Pdcd1lg2 | | 10608342 | LOC100041704 | | | | | | |
| 10380411 | Mrpl27 | | 10463070 | Entpd1 | | 10608348 | Ssty2 | | | | | | |
| 10380815 | Psmb3 | | 10466779 | Pip5k1b | | 10608350 | LOC 100039552 | | | | | | |
| 10381072 | Cdc6 | | 10469151 | Itih5 | | 10608365 | Ssty2 | | | | | | |
| 10381526 | Ppih | | 10469278 | Il2ra | | 10608371 | LOC665406 | | | | | | |
| 10381664 | Kif18b | | 10473356 | Ube2l6 | | 10608373 | Ssty2 | | | | | | |
| 10381798 | Myl4 | | 10473367 | Slc43a1 | | 10608420 | Ssty1 | | | | | | |
| 10382998 | Birc5 | | 10474769 | Bub1b | | 10608424 | Ssty1 | | | | | | |
| 10384373 | Fignl1 | | 10476314 | Prnp | | 10608454 | Ssty2 | | | | | | |
| 10384474 | Pno1 | | 10476945 | Cst7 | | 10608482 | LOC665746 | | | | | | |
| 10384493 | Gapdh | | 10481210 | Vav2 | | 10608484 | Ssty2 | | | | | | |
| 10384579 | Ugp2 | | 10482528 | Neb | | 10608488 | LOC665128 | | | | | | |
| 10385248 | Hmmr | | 10482687 | Arl5a | | 10608521 | Ssty1 | | | | | | |
| 10385325 | Pttg1 | | 10484888 | Ptprj | | 10608549 | LOC 100039753 | | | | | | |
| 10385686 | Hnrnpab | | 10484894 | Ptprj | | 10608606 | LOC 100039753 | | | | | | |
| 10385966 | Anxa6 | | 10485405 | Cd44 | | 10608613 | LOC100042196 | | | | | | |
| 10386005 | Atp5f1 | | 10493820 | S100a6 | | 10608625 | LOC 100040235 | | | | | | |
| 10386947 | Gm10291 | | 10494402 | Hist2h3c1 | | 10608628 | LOC 100041704 | | | | | | |
| 10388234 | Gsg2 | | 10494405 | Hist2h3b | | 10608630 | Ssty2 | | | | | | |
| 10388745 | Lsm6 | | 10496379 | H2afz | | | | | | | | | |
| 10388971 | Utp6 | | 10496539 | Gbp5 | | | | | | | | | |
| 10389606 | Prr11 | | 10496580 | Gbp3 | | | | | | | | | |
| 10390707 | Top2a | | 10497149 | Wls | | | | | | | | | |
| 10391461 | Brca1 | | 10497831 | Ccna2 | | | | | | | | | |
| 10391811 | Kif18b | | 10499095 | Fam160a1 | | | | | | | | | |
| 10392284 | Kpna2 | | 10499216 | Pear1 | | | | | | | | | |
| 10392388 | Prkca | | 10500204 | Ecm1 | | | | | | | | | |
| 10393431 | Tk1 | | 10500656 | Cd101 | | | | | | | | | |
| 10393844 | Thoc4 | | 10501164 | Csf1 | | | | | | | | | |
| 10394770 | Odc1 | | 10502156 | Ccdc109b | | | | | | | | | |
| 10394978 | Rrm2 | | 10504753 | LOC641050 | | | | | | | | | |
| 10395259 | Nampt | | 10504757 | BC005685 | | | | | | | | | |
| 10396068 | Ppil5 | | 10504761 | LOC641050 | | | | | | | | | |
| 10396712 | Fut8 | | 10510580 | Tnfrsf9 | | | | | | | | | |
| 10397741 | Psmc1 | | 10511282 | Tnfrsf4 | | | | | | | | | |
| 10398173 | Vrk1 | | 10511290 | Tnfrsf18 | | | | | | | | | |
| 10398874 | Siva1 | | 10511363 | Penk | | | | | | | | | |
| 10399011 | 4930427A07Rik | | 10511617 | Fam92a | | | | | | | | | |
| 10399087 | Ncapg2 | | 10512774 | Coro2a | | | | | | | | | |
| 10399825 | Dld | | 10514466 | Jun | | | | | | | | | |
| 10400304 | Egln3 | | 10514732 | Slc35d1 | | | | | | | | | |
| 10400589 | C79407 | | 10518300 | Tnfrsf1 b | | | | | | | | | |
| 10401278 | Erh | | 10519527 | Abcb1a | | | | | | | | | |
| 10402615 | Hsp90aa1 | | 10519983 | Fgl2 | | | | | | | | | |

| **Upregulated** | | | **Upregulated** | | | **Downregulated** | | | **Upregulated** | | | **Upregulated** | |
|---|---|---|---|---|---|---|---|---|---|---|---|---|---|
| **Probe** | **Gene** | | **Probe** | **Gene** | | **Probe** | **Gene** | | **Probe** | **Gene** | | **Probe** | **Gene** |
| 10402648 | Brp44l | | 10521678 | Cd38 | | | | | | | | | |
| 10402650 | Cinp | | 10523595 | Ptpn13 | | | | | | | | | |
| 10403258 | Gdi2 | | 10525419 | P2rx7 | | | | | | | | | |
| 10403413 | Idi1 | | 10528238 | Phtf2 | | | | | | | | | |
| 10404053 | Hist1h2bc | | 10530819 | Hopx | | | | | | | | | |
| 10404422 | Serpinb6b | | 10535065 | Adap1 | | | | | | | | | |
| 10404429 | Serpinb9 | | 10535389 | Rnf216 | | | | | | | | | |
| 10405185 | Cks2 | | 10538890 | LOC641050 | | | | | | | | | |
| 10405427 | Prelid1 | | 10538892 | LOC641050 | | | | | | | | | |
| 10406482 | Ccnh | | 10538901 | BC005685 | | | | | | | | | |
| 10406581 | Dhfr | | 10539135 | Capg | | | | | | | | | |
| 10406898 | Taf9 | | 10540999 | H2afz | | | | | | | | | |
| 10406968 | Cenpk | | 10542880 | 4833442J19Rik | | | | | | | | | |
| 10407081 | Depdc1 b | | 10544660 | Osbpl3 | | | | | | | | | |
| 10407481 | Pfkp | | 10547590 | Klrg1 | | | | | | | | | |
| 10407993 | Srsf10 | | 10547906 | Lag3 | | | | | | | | | |
| 10408210 | Hist1h2bf | | 10548585 | Csda | | | | | | | | | |
| 10408223 | Hist1h2bc | | 10550509 | Pglyrp1 | | | | | | | | | |
| 10408321 | Gmnn | | 10552406 | Nkg7 | | | | | | | | | |
| 10408329 | Gmnn | | 10553598 | Cyfip1 | | | | | | | | | |
| 10408531 | Gmds | | 10555174 | Lrrc32 | | | | | | | | | |
| 10409190 | Cenpp | | 10555197 | Mtap6 | | | | | | | | | |
| 10409200 | Gapdh | | 10557156 | Plk1 | | | | | | | | | |
| 10409424 | Mxd3 | | 10559261 | Cd81 | | | | | | | | | |
| 10409866 | Ctla2b | | 10560945 | Grik5 | | | | | | | | | |
| 10409876 | Ctla2a | | 10560964 | Pou2f2 | | | | | | | | | |
| 10410092 | Zfp367 | | 10564507 | Arrdc4 | | | | | | | | | |
| 10410560 | Trip13 | | 10565315 | Fah | | | | | | | | | |
| 10411332 | Hmgcr | | 10565735 | A630091 E08Rik | | | | | | | | | |
| 10411359 | Plp2 | | 10565990 | Art2a-ps | | | | | | | | | |
| 10411373 | Hexb | | 10571312 | Dusp4 | | | | | | | | | |
| 10411452 | Gapdh | | 10571399 | Zdhhc2 | | | | | | | | | |
| 10411728 | Cenph | | 10571696 | Casp3 | | | | | | | | | |
| 10411739 | Ccnb1 | | 10572497 | Il12rb1 | | | | | | | | | |
| 10412466 | Hmgcs1 | | 10576639 | Nrp1 | | | | | | | | | |
| 10412559 | Slbp | | 10576661 | Itgb1 | | | | | | | | | |
| 10412909 | Fdft1 | | 10580077 | Rln3 | | | | | | | | | |
| 10413059 | Vcl | | 10581992 | Maf | | | | | | | | | |
| 10413542 | Tkt | | 10583286 | Gpr83 | | | | | | | | | |
| 10414315 | Cdkn3 | | 10585286 | Arhgap20 | | | | | | | | | |
| 10415791 | Rnaseh2b | | 10586744 | Anxa2 | | | | | | | | | |
| 10415844 | Ctsb | | 10586781 | Myo1e | | | | | | | | | |
| 10416037 | Pbk | | 10586933 | Nedd4 | | | | | | | | | |
| 10416736 | 6720463M24Rik | | 10587315 | Gsta4 | | | | | | | | | |
| 10416940 | Tpm3 | | 10587503 | Sh3bgrl2 | | | | | | | | | |
| 10417070 | Ipo5 | | 10587639 | Nt5e | | | | | | | | | |
| 10417359 | Gm3002 | | 10588577 | Cish | | | | | | | | | |
| 10417421 | Gm3696 | | 10590242 | Ccr8 | | | | | | | | | |
| 10417617 | Gapdh | | 10590909 | Endod1 | | | | | | | | | |
| 10417689 | Psmd6 | | 10590974 | Folr4 | | | | | | | | | |
| 10417787 | Gng2 | | 10592655 | Arhgef12 | | | | | | | | | |
| 10418004 | Ap3m1 | | 10592888 | Cxcr5 | | | | | | | | | |
| 10419136 | Cdv3 | | 10593497 | Zc3h12c | | | | | | | | | |
| 10419198 | Ero11 | | 10594774 | Ccnb2 | | | | | | | | | |
| 10419267 | Cnih | | 10597420 | Ccr4 | | | | | | | | | |
| 10419296 | Wdhd1 | | 10598289 | 4930524L23Rik | | | | | | | | | |
| 10419323 | Dlgap5 | | 10598292 | Foxp3 | | | | | | | | | |
| 10420155 | Dhrs1 | | 10603551 | Cybb | | | | | | | | | |
| 10420198 | Ripk3 | | 10603814 | Slc9a7 | | | | | | | | | |
| 10420308 | Gzmb | | 10606058 | Cxcr3 | | | | | | | | | |
| 10420426 | F630043A04Rik | | 10607738 | Car5b | | | | | | | | | |
| 10420637 | Kpna3 | | | | | | | | | | | | |
| | | | | | | | | | | | | | |
| | | | | | | | | | | | | | |
| | | | | | | | | | | | | | |
| 10420730 | Fdft1 | | | | | | | | | | | | |
| 10420988 | Dpysl2 | | | | | | | | | | | | |
| 10421029 | Cdca2 | | 10344750 | Sgk3 | | | | | | | | | |
| 10422161 | Gm10293 | | 10345777 | Ikllrl2 | | | | | | | | | |
| 10422655 | Gapdh | | 10351880 | E430029J22Rik | | | | | | | | | |
| 10423180 | Gapdh | | 10354506 | Mfsd6 | | | | | | | | | |
| 10424221 | Wdz67 | | 10356475 | Arl4c | | | | | | | | | |
| 10424349 | Sqle | | 10359689 | Atp1 b1 | | | | | | | | | |
| 10424379 | Srsf3 | | 10362073 | Sgk1 | | | | | | | | | |
| 10424779 | Cks2 | | 10362350 | Themis | | | | | | | | | |
| 10425161 | Lgals1 | | 10362861 | Scml4 | | | | | | | | | |
| 10425207 | H1f0 | | 10364072 | Ggt5 | | | | | | | | | |
| 10425226 | Eif3l | | 10367734 | Ust | | | | | | | | | |
| 10425903 | Gm2451 | | 10368647 | Dse | | | | | | | | | |
| 10426827 | Larp4 | | 10369911 | 1110038D17Rik | | | | | | | | | |
| 10427166 | Espl1 | | 10371356 | Appl2 | | | | | | | | | |
| 10427606 | Skp2 | | 10378549 | Rtn4rl1 | | | | | | | | | |
| 10428018 | Ube2v2 | | 10381096 | Igfbp4 | | | | | | | | | |
| 10428310 | Azin1 | | 10381809 | Itgb3 | | | | | | | | | |
| 10428672 | Dscc1 | | 10382532 | Slc16a5 | | | | | | | | | |
| 10430344 | I12rb | | 10385428 | Itk | | | | | | | | | |
| 10430778 | Phf5a | | 10385776 | Tcf7 | | | | | | | | | |
| 10432511 | Racgap1 | | 10388488 | Fam101b | | | | | | | | | |
| 10433088 | Cbx5 | | 10390763 | Ccr7 | | | | | | | | | |
| 10434643 | Psmb3 | | 10392910 | C630004H02Rik | | | | | | | | | |
| 10434869 | Ccdc50 | | 10399696 | Rnf144a | | | | | | | | | |
| 10434998 | Ncbp2 | | 10401244 | Actn1 | | | | | | | | | |
| 10435821 | Naa50 | | 10402096 | Ttc7b | | | | | | | | | |
| 10436048 | Prdx1 | | 10403604 | Lyst | | | | | | | | | |
| 10436106 | C330027C09Rik | | 10404359 | Mboat1 | | | | | | | | | |
| 10436182 | Cd47 | | 10406111 | Slc12a7 | | | | | | | | | |

| **Upregulated** | | | **Downregulated** | | | **Upregulated** | | | **Upregulated** | | | **Upregulated** | |
|---|---|---|---|---|---|---|---|---|---|---|---|---|---|
| **Probe** | **Gene** | | **Probe** | **Gene** | | **Probe** | **Gene** | | **Probe** | **Gene** | | **Probe** | **Gene** |
| 10437432 | Nmral1 | | 10406852 | Cnn3 | | | | | | | | | |
| 10437590 | Carhsp1 | | 10407072 | Elovl7 | | | | | | | | | |
| 10437748 | Gspt1 | | 10407124 | Al452195 | | | | | | | | | |
| 10437942 | Ube2v2 | | 10407327 | Emb | | | | | | | | | |
| 10437945 | Mcm4 | | 10414807 | Trav14-3 | | | | | | | | | |
| 10438091 | 2610318N02Rik | | 10423293 | Myo10 | | | | | | | | | |
| 10438308 | Ranbp1 | | 10425040 | Apol7e | | | | | | | | | |
| 10438378 | Cdc45 | | 10430179 | Apol7b | | | | | | | | | |
| 10438690 | Rfc4 | | 10439790 | Trat1 | | | | | | | | | |
| 10439762 | Ahcy | | 10440099 | St3gal6 | | | | | | | | | |
| 10439878 | Psmc1 | | 10446777 | Ehd3 | | | | | | | | | |
| 10440314 | Cadm2 | | 10451763 | Satb1 | | | | | | | | | |
| 10441642 | Brp44l | | 10455784 | Gramd3 | | | | | | | | | |
| 10442454 | Pgp | | 10460968 | Rasgrp2 | | | | | | | | | |
| 10443459 | Srsf3 | | 10467420 | Pdlim1 | | | | | | | | | |
| 10443527 | Pim1 | | 10472022 | Lypd6b | | | | | | | | | |
| 10444927 | Nrm | | 10472162 | Gpd2 | | | | | | | | | |
| 10445894 | Erh | | 10472235 | Dapl1 | | | | | | | | | |
| 10446074 | Uhrf1 | | 10472846 | Pdk1 | | | | | | | | | |
| 10447395 | Msh2 | | 10472860 | Rapgef4 | | | | | | | | | |
| 10447417 | Msh6 | | 10475990 | Slc20a1 | | | | | | | | | |
| 10447702 | Ppih | | 10481574 | Fam78a | | | | | | | | | |
| 10447880 | Mrpl18 | | 10483809 | Nfe2l2 | | | | | | | | | |
| 10448506 | Ccnf | | 10487208 | Atp8b4 | | | | | | | | | |
| 10448803 | Hn1l | | 10495685 | Arhgap29 | | | | | | | | | |
| 10449575 | Ppil1 | | 10496091 | Lef1 | | | | | | | | | |
| 10449581 | Mtch1 | | 10496438 | Adh1 | | | | | | | | | |
| 10450374 | D17H6S56E-5 | | 10497237 | Pag1 | | | | | | | | | |
| 10450519 | Tcf19 | | 10498345 | Gpr171 | | | | | | | | | |
| 10450605 | Tubb5 | | 10499378 | Sema4a | | | | | | | | | |
| 10451805 | Sgol1 | | 10500434 | Bcl9 | | | | | | | | | |
| 10452415 | Gapdh | | 10503161 | Chd7 | | | | | | | | | |
| 10452709 | Ndc80 | | 10503222 | Chd7 | | | | | | | | | |
| 10453512 | Kpna2 | | 10505187 | Ugcg | | | | | | | | | |
| 10453867 | Rbbp8 | | 10513729 | Tnfsf8 | | | | | | | | | |
| 10454093 | Mrpl27 | | 10514956 | Scp2 | | | | | | | | | |
| 10454198 | Rnf125 | | 10516823 | Epb4.1 | | | | | | | | | |
| 10454709 | Kif20a | | 10532085 | Tgfbr3 | | | | | | | | | |
| 10455595 | Eno1 | | 10533198 | Oas2 | | | | | | | | | |
| 10455647 | Tnfaip8 | | 10533659 | Clip1 | | | | | | | | | |
| 10455738 | Snx2 | | 10533729 | Vps37b | | | | | | | | | |
| 10455780 | Gapdh | | 10534570 | Orai2 | | | | | | | | | |
| 10455813 | Lmnb1 | | 10542981 | Gmfg | | | | | | | | | |
| 10455967 | 2610318N02Rik | | 10547795 | Atn1 | | | | | | | | | |
| 10456383 | Impa2 | | 10549162 | St8sia1 | | | | | | | | | |
| 10457409 | Usp14 | | 10554658 | A530021 J07Rik | | | | | | | | | |
| 10458033 | Stard4 | | 10556528 | Pde3b | | | | | | | | | |
| 10458195 | Cdc25c | | 10557069 | Mettl9 | | | | | | | | | |
| 10458213 | Etf1 | | 10562260 | Gramd1a | | | | | | | | | |
| 10458589 | Prelid2 | | 10563099 | Snord35b | | | | | | | | | |
| 10459375 | Txnl1 | | 10564539 | Mctp2 | | | | | | | | | |
| 10459755 | Ska1 | | 10571344 | D8Ertd82e | | | | | | | | | |
| 10459844 | Haus1 | | 10577226 | 2610019F03Rik | | | | | | | | | |
| 10460738 | Cdca5 | | 10583203 | Phxr4 | | | | | | | | | |
| 10461391 | Pcna | | 10583207 | Maml2 | | | | | | | | | |
| 10461439 | Fads1 | | 10589654 | Als2cl | | | | | | | | | |
| 10461452 | Fen1 | | 10590365 | Vipr1 | | | | | | | | | |
| 10461723 | Fam111a | | 10590381 | Vipr1 | | | | | | | | | |
| 10462632 | Kif20b | | 10595840 | Acpl2 | | | | | | | | | |
| 10462670 | Rpp30 | | 10597518 | Tgfbr2 | | | | | | | | | |
| 10462796 | Kif11 | | 10598101 | Maml2 | | | | | | | | | |
| 10462866 | Cep55 | | 10599802 | Cd40lg | | | | | | | | | |
| 10462973 | Hells | | 10606654 | Xkrx | | | | | | | | | |
| 10463064 | Gm4609 | | | | | | | | | | | | |
| 10464045 | Acsl5 | | | | | | | | | | | | |
| 10465005 | Banf1 | | | | | | | | | | | | |
| 10465553 | Fkbp2 | | | | | | | | | | | | |
| 10465686 | Rtn3 | | | | | | | | | | | | |
| 10465844 | Asrgl1 | | | | | | | | | | | | |
| 10465861 | Incenp | | | | | | | | | | | | |
| 10465912 | Fen1 | | | | | | | | | | | | |
| 10466410 | Psat1 | | | | | | | | | | | | |
| 10466606 | Anxa1 | | | | | | | | | | | | |
| 10466843 | Gapdh | | | | | | | | | | | | |
| 10466925 | Ak3 | | | | | | | | | | | | |
| 10467637 | Arhgap19 | | | | | | | | | | | | |
| 10469035 | Sephs1 | | | | | | | | | | | | |
| 10469070 | Nudt5 | | | | | | | | | | | | |
| 10469322 | Vim | | | | | | | | | | | | |
| 10469712 | Pdss1 | | | | | | | | | | | | |
| 10469732 | Yme1l1 | | | | | | | | | | | | |
| 10472782 | Hat1 | | | | | | | | | | | | |
| 10472916 | Cdca7 | | | | | | | | | | | | |
| 10472933 | Scrn3 | | | | | | | | | | | | |
| 10473022 | Plp2 | | | | | | | | | | | | |
| 10473240 | Eno1 | | | | | | | | | | | | |
| 10473250 | Mrpl18 | | | | | | | | | | | | |
| 10473384 | Slc43a3 | | | | | | | | | | | | |
| 10473919 | Ckap5 | | | | | | | | | | | | |
| 10474239 | Gapdh | | | | | | | | | | | | |
| 10474381 | Kif18a | | | | | | | | | | | | |
| 10474769 | Bub1b | | | | | | | | | | | | |
| 10474825 | D2Ertd750e | | | | | | | | | | | | |
| 10474875 | Casc5 | | | | | | | | | | | | |
| 10474902 | Rad51 | | | | | | | | | | | | |
| 10474984 | Nusap1 | | | | | | | | | | | | |
| 10475335 | Pdia3 | | | | | | | | | | | | |
| 10475610 | Dut | | | | | | | | | | | | |
| 10476252 | Cdc25b | | | | | | | | | | | | |
| 10476648 | Dstn | | | | | | | | | | | | |
| 10476834 | Xrn2 | | | | | | | | | | | | |
| 10476989 | Gins1 | | | | | | | | | | | | |
| 10477187 | Tpx2 | | | | | | | | | | | | |
| 10477942 | Rbl1 | | | | | | | | | | | | |
| 10478407 | Serinc3 | | | | | | | | | | | | |
| 10478572 | Ube2c | | | | | | | | | | | | |
| 10478943 | Pfdn4 | | | | | | | | | | | | |
| 10479379 | Slco4a1 | | | | | | | | | | | | |
| 10479736 | Polr3k | | | | | | | | | | | | |
| 10479811 | Mcm10 | | | | | | | | | | | | |
| 10480381 | Arhgap21 | | | | | | | | | | | | |
| 10480432 | Mastl | | | | | | | | | | | | |
| 10480628 | Tubb2c | | | | | | | | | | | | |
| 10481344 | Gapdh | | | | | | | | | | | | |
| 10481585 | 2900010J23Rik | | | | | | | | | | | | |
| 10482229 | Psmb7 | | | | | | | | | | | | |
| 10482687 | Arl5a | | | | | | | | | | | | |
| 10482762 | Idi1 | | | | | | | | | | | | |
| 10483046 | Dpp4 | | | | | | | | | | | | |
| 10483178 | CobIl1 | | | | | | | | | | | | |
| 10483381 | Stk39 | | | | | | | | | | | | |
| 10483401 | Spc25 | | | | | | | | | | | | |
| 10484425 | 2700094K13Rik | | | | | | | | | | | | |
| 10485294 | Hsd17b12 | | | | | | | | | | | | |
| 10485963 | Arhgap11a | | | | | | | | | | | | |
| 10486396 | Ehd4 | | | | | | | | | | | | |
| 10487033 | Myef2 | | | | | | | | | | | | |
| 10487175 | Cops2 | | | | | | | | | | | | |
| 10487340 | Ncaph | | | | | | | | | | | | |
| 10487480 | Bub1 | | | | | | | | | | | | |
| 10487577 | Ckap2l | | | | | | | | | | | | |
| 10487930 | Pcna | | | | | | | | | | | | |
| 10488785 | E2f1 | | | | | | | | | | | | |
| 10488816 | Ahcy | | | | | | | | | | | | |
| 10489127 | Rbl1 | | | | | | | | | | | | |
| 10489377 | Serinc3 | | | | | | | | | | | | |
| 10490104 | Aurka | | | | | | | | | | | | |
| 10490225 | Slmo2 | | | | | | | | | | | | |
| 10490838 | Fabp5 | | | | | | | | | | | | |
| 10490843 | Myef2 | | | | | | | | | | | | |
| 10490946 | Hsp90aa1 | | | | | | | | | | | | |
| 10491182 | Eif5a2 | | | | | | | | | | | | |
| 10491385 | Actl6a | | | | | | | | | | | | |
| 10491805 | Plk4 | | | | | | | | | | | | |
| 10491835 | Larp1b | | | | | | | | | | | | |
| 10491848 | Larp1b | | | | | | | | | | | | |
| 10492220 | 2810407C02Rik | | | | | | | | | | | | |
| 10492381 | Gmps | | | | | | | | | | | | |
| 10492679 | 4930579G24Rik | | | | | | | | | | | | |
| 10493137 | Iqgap3 | | | | | | | | | | | | |
| 10493548 | Pmvk | | | | | | | | | | | | |
| 10493633 | Tpm3 | | | | | | | | | | | | |
| 10493820 | S100a6 | | | | | | | | | | | | |
| 10493995 | S100a10 | | | | | | | | | | | | |
| 10494322 | Anp32e | | | | | | | | | | | | |
| 10494583 | Sec22b | | | | | | | | | | | | |
| 10494662 | Ywhah | | | | | | | | | | | | |
| 10495405 | Slc25a24 | | | | | | | | | | | | |
| 10496204 | Cenpe | | | | | | | | | | | | |
| 10496324 | Slc39a8 | | | | | | | | | | | | |
| 10496485 | Eif4e | | | | | | | | | | | | |
| 10496490 | Mir1956 | | | | | | | | | | | | |
| 10497105 | Lrrc40 | | | | | | | | | | | | |
| 10497503 | Kpna2 | | | | | | | | | | | | |
| 10497520 | Ect2 | | | | | | | | | | | | |
| 10497752 | Carhsp1 | | | | | | | | | | | | |
| 10497831 | Ccna2 | | | | | | | | | | | | |
| 10499639 | Cks1 b | | | | | | | | | | | | |
| 10500630 | Ttf2 | | | | | | | | | | | | |
| 10500990 | Atp5f1 | | | | | | | | | | | | |
| 10501402 | Gpsm2 | | | | | | | | | | | | |
| 10501661 | Srsf3 | | | | | | | | | | | | |
| 10503264 | Ccne2 | | | | | | | | | | | | |
| 10503315 | Rad54b | | | | | | | | | | | | |
| 10503617 | F730047E07Rik | | | | | | | | | | | | |
| 10503911 | Polr1d | | | | | | | | | | | | |
| 10504450 | Glipr2 | | | | | | | | | | | | |
| 10504470 | Melk | | | | | | | | | | | | |
| 10504957 | Smc2 | | | | | | | | | | | | |
| 10506118 | Usp1 | | | | | | | | | | | | |
| 10506680 | Tmem48 | | | | | | | | | | | | |
| 10506714 | Lrp8 | | | | | | | | | | | | |
| 10506822 | Orc1 | | | | | | | | | | | | |
| 10507112 | Stil | | | | | | | | | | | | |
| 10507286 | Ipp | | | | | | | | | | | | |
| 10507328 | Prdx1 | | | | | | | | | | | | |
| 10507885 | Mycbp | | | | | | | | | | | | |
| 10508151 | Clspn | | | | | | | | | | | | |
| 10508182 | Psmb2 | | | | | | | | | | | | |
| 10508217 | Sfpq | | | | | | | | | | | | |
| 10508444 | Zbtb8os | | | | | | | | | | | | |
| 10508986 | Stmn1 | | | | | | | | | | | | |
| 10509113 | Srsf10 | | | | | | | | | | | | |
| 10509168 | E2f2 | | | | | | | | | | | | |
| 10510165 | Gm13238 | | | | | | | | | | | | |
| 10510167 | Gm13051 | | | | | | | | | | | | |
| 10510172 | Hmgb2 | | | | | | | | | | | | |
| 10510219 | Gm13238 | | | | | | | | | | | | |
| 10510546 | Eno1 | | | | | | | | | | | | |
| 10510687 | Acot7 | | | | | | | | | | | | |
| 10511617 | Fam92a | | | | | | | | | | | | |
| 10511661 | Otud6b | | | | | | | | | | | | |
| 10511694 | Osgin2 | | | | | | | | | | | | |
| 10512061 | Taf9 | | | | | | | | | | | | |
| 10513181 | Gapdh | | | | | | | | | | | | |
| 10513195 | Txn1 | | | | | | | | | | | | |
| 10513320 | Ptgr1 | | | | | | | | | | | | |
| 10513608 | Alad | | | | | | | | | | | | |
| 10513818 | Stmn1 | | | | | | | | | | | | |
| 10513822 | Stmn1 | | | | | | | | | | | | |
| 10514201 | Haus6 | | | | | | | | | | | | |
| 10515090 | Cdkn2c | | | | | | | | | | | | |
| 10515257 | Rad54l | | | | | | | | | | | | |
| 10515337 | Nasp | | | | | | | | | | | | |
| 10515431 | Kif2c | | | | | | | | | | | | |
| 10515744 | Cdc20 | | | | | | | | | | | | |
| 10515836 | Ccnb1 | | | | | | | | | | | | |
| 10515884 | Ppih | | | | | | | | | | | | |
| 10516246 | Cdca8 | | | | | | | | | | | | |
| 10516943 | Atpif1 | | | | | | | | | | | | |
| 10517336 | Clic4 | | | | | | | | | | | | |
| 10517559 | Cdc42 | | | | | | | | | | | | |
| 10518344 | Gm13238 | | | | | | | | | | | | |
| 10518350 | Hmgb2 | | | | | | | | | | | | |
| 10518352 | Hmgb2 | | | | | | | | | | | | |
| 10519324 | Cdk6 | | | | | | | | | | | | |
| 10519488 | Tubb2c | | | | | | | | | | | | |
| 10520390 | Gapdh | | | | | | | | | | | | |
| 10520483 | Ept1 | | | | | | | | | | | | |
| 10520521 | Cenpa | | | | | | | | | | | | |
| 10521031 | Ywhah | | | | | | | | | | | | |
| 10521090 | Tacc3 | | | | | | | | | | | | |
| 10521136 | Whsc1 | | | | | | | | | | | | |
| 10521690 | Ppih | | | | | | | | | | | | |
| 10521731 | Ncapg | | | | | | | | | | | | |
| 10521863 | Anapc4 | | | | | | | | | | | | |
| 10523012 | Dck | | | | | | | | | | | | |
| 10523281 | 11-Sep | | | | | | | | | | | | |
| 10523365 | Mrpl1 | | | | | | | | | | | | |
| 10524169 | Pole | | | | | | | | | | | | |
| 10524266 | Chek2 | | | | | | | | | | | | |
| 10524790 | Cit | | | | | | | | | | | | |
| 10525591 | Kntc1 | | | | | | | | | | | | |
| 10525733 | Setd8 | | | | | | | | | | | | |
| 10525983 | Ran | | | | | | | | | | | | |
| 10526972 | Nudt1 | | | | | | | | | | | | |
| 10527559 | Polr1d | | | | | | | | | | | | |
| 10527801 | Brca2 | | | | | | | | | | | | |
| 10527888 | Gatad1 | | | | | | | | | | | | |
| 10527920 | Cyp51 | | | | | | | | | | | | |
| 10528077 | Dbf4 | | | | | | | | | | | | |
| 10528167 | Gapdh | | | | | | | | | | | | |
| 10528915 | Tyms | | | | | | | | | | | | |
| 10529299 | Slbp | | | | | | | | | | | | |
| 10530806 | Ppat | | | | | | | | | | | | |
| 10531707 | Lin54 | | | | | | | | | | | | |
| 10531724 | Plac8 | | | | | | | | | | | | |
| 10533090 | Rfc5 | | | | | | | | | | | | |
| 10533929 | Scarb1 | | | | | | | | | | | | |
| 10534842 | Gnb2 | | | | | | | | | | | | |
| 10534974 | Mcm7 | | | | | | | | | | | | |
| 10535979 | Rfc3 | | | | | | | | | | | | |
| 10536472 | Mdfic | | | | | | | | | | | | |
| 10536595 | Naa38 | | | | | | | | | | | | |
| 10538617 | Lancl2 | | | | | | | | | | | | |
| 10538832 | Mad2l1 | | | | | | | | | | | | |
| 10540273 | Ube2v2 | | | | | | | | | | | | |
| 10540738 | Fancd2 | | | | | | | | | | | | |
| 10541484 | M6pr | | | | | | | | | | | | |
| 10541729 | Cdca3 | | | | | | | | | | | | |
| 10542200 | Gabarapl1 | | | | | | | | | | | | |
| 10542355 | Emp1 | | | | | | | | | | | | |
| 10542445 | Strap | | | | | | | | | | | | |
| 10542460 | Dera | | | | | | | | | | | | |
| 10542750 | Med21 | | | | | | | | | | | | |
| 10543944 | Mtpn | | | | | | | | | | | | |
| 10544501 | Ezh2 | | | | | | | | | | | | |
| 10544660 | Osbpl3 | | | | | | | | | | | | |
| 10545534 | Rnf26 | | | | | | | | | | | | |
| 10545588 | Hk2 | | | | | | | | | | | | |
| 10545672 | Mthfd2 | | | | | | | | | | | | |
| 10545835 | 1700040103Rik | | | | | | | | | | | | |
| 10545958 | Anxa4 | | | | | | | | | | | | |
| 10546163 | Mcm2 | | | | | | | | | | | | |
| 10547830 | Tpi1 | | | | | | | | | | | | |
| 10547936 | Gapdh | | | | | | | | | | | | |
| 10547943 | Ncapd2 | | | | | | | | | | | | |
| 10548086 | Rad51ap1 | | | | | | | | | | | | |
| 10548143 | Gapdh | | | | | | | | | | | | |
| 10548585 | Csda | | | | | | | | | | | | |
| 10550098 | Wdr12 | | | | | | | | | | | | |
| 10550102 | Lig1 | | | | | | | | | | | | |
| 10552740 | Nup62 | | | | | | | | | | | | |
| 10553788 | Atp10a | | | | | | | | | | | | |
| 10554013 | Chsy1 | | | | | | | | | | | | |
| 10554445 | Prc1 | | | | | | | | | | | | |
| 10554574 | Tm6sf1 | | | | | | | | | | | | |
| 10554817 | Gm10291 | | | | | | | | | | | | |
| 10555055 | Ndufc2 | | | | | | | | | | | | |
| 10555695 | Rrm1 | | | | | | | | | | | | |
| 10556266 | Wee1 | | | | | | | | | | | | |
| 10556640 | 6330503K22Rik | | | | | | | | | | | | |
| 10557156 | Plk1 | | | | | | | | | | | | |
| 10557843 | Fus | | | | | | | | | | | | |
| 10558248 | Bub3 | | | | | | | | | | | | |
| 10558723 | Psmd13 | | | | | | | | | | | | |
| 10560000 | Tpm3 | | | | | | | | | | | | |
| 10560260 | Sae1 | | | | | | | | | | | | |
| 10561388 | Timm50 | | | | | | | | | | | | |
| 10562563 | Ccne1 | | | | | | | | | | | | |
| 10562637 | Ccnb1 | | | | | | | | | | | | |
| 10562639 | Gapdh | | | | | | | | | | | | |
| 10563780 | E2f8 | | | | | | | | | | | | |
| 10563838 | Nipa2 | | | | | | | | | | | | |
| 10564978 | Blm | | | | | | | | | | | | |
| 10565479 | I7Rn6 | | | | | | | | | | | | |
| 10565570 | 4632434111 Rik | | | | | | | | | | | | |
| 10565921 | Gapdh | | | | | | | | | | | | |
| 10567072 | Psma1 | | | | | | | | | | | | |
| 10567303 | Coq7 | | | | | | | | | | | | |
| 10568150 | Kif22 | | | | | | | | | | | | |
| 10568714 | Mki67 | | | | | | | | | | | | |
| 10569017 | Ifitm3 | | | | | | | | | | | | |
| 10569071 | Hras1 | | | | | | | | | | | | |
| 10570373 | Tfdp1 | | | | | | | | | | | | |
| 10571274 | Gsr | | | | | | | | | | | | |
| 10571288 | Gtf2e2 | | | | | | | | | | | | |
| 10571399 | Zdhhc2 | | | | | | | | | | | | |
| 10571696 | Casp3 | | | | | | | | | | | | |
| 10571870 | Hmgb2 | | | | | | | | | | | | |
| 10571876 | Gapdh | | | | | | | | | | | | |
| 10571911 | 2700029M09Rik | | | | | | | | | | | | |
| 10571978 | Cbr4 | | | | | | | | | | | | |
| 10572906 | Mcm5 | | | | | | | | | | | | |
| 10573217 | Ddx39 | | | | | | | | | | | | |
| 10573261 | Asf1 b | | | | | | | | | | | | |
| 10573451 | Syce2 | | | | | | | | | | | | |
| 10573615 | Orc6 | | | | | | | | | | | | |
| 10574033 | Nup93 | | | | | | | | | | | | |
| 10575153 | Cyb5b | | | | | | | | | | | | |
| 10575733 | Cenpn | | | | | | | | | | | | |
| 10576034 | Irf8 | | | | | | | | | | | | |
| 10576639 | Nrp1 | | | | | | | | | | | | |
| 10576661 | Itgb1 | | | | | | | | | | | | |
| 10576883 | Shcbp1 | | | | | | | | | | | | |
| 10577508 | Ckap2 | | | | | | | | | | | | |
| 10577598 | Lsm6 | | | | | | | | | | | | |
| 10578145 | Erh | | | | | | | | | | | | |
| 10578193 | Eri1 | | | | | | | | | | | | |
| 10578539 | Slc25a4 | | | | | | | | | | | | |
| 10578545 | Gm12070 | | | | | | | | | | | | |
| 10578690 | Neil3 | | | | | | | | | | | | |
| 10578916 | Sc4mol | | | | | | | | | | | | |
| 10579347 | Ifi30 | | | | | | | | | | | | |
| 10579769 | Gapdh | | | | | | | | | | | | |
| 10579833 | Lsm6 | | | | | | | | | | | | |
| 10580590 | Gapdh | | | | | | | | | | | | |
| 10582008 | 2310061 C15Rik | | | | | | | | | | | | |
| 10582190 | Gins2 | | | | | | | | | | | | |
| 10582295 | Odc1 | | | | | | | | | | | | |
| 10582809 | Tk1 | | | | | | | | | | | | |
| 10582843 | Itgb1 | | | | | | | | | | | | |
| 10582981 | Tfdp1 | | | | | | | | | | | | |
| 10583254 | Cwc15 | | | | | | | | | | | | |
| 10584710 | H2afx | | | | | | | | | | | | |
| 10585395 | Siva1 | | | | | | | | | | | | |
| 10585417 | Idh3a | | | | | | | | | | | | |
| 10585474 | Psma4 | | | | | | | | | | | | |
| 10585699 | Fabp5 | | | | | | | | | | | | |
| 10585932 | Pkm2 | | | | | | | | | | | | |
| 10586184 | Tipin | | | | | | | | | | | | |
| 10586284 | Dpp8 | | | | | | | | | | | | |
| 10586416 | Pif1 | | | | | | | | | | | | |
| 10586448 | 2810417H13Rik | | | | | | | | | | | | |
| 10586484 | Fam96a | | | | | | | | | | | | |
| 10586604 | Rps271 | | | | | | | | | | | | |
| 10586744 | Anxa2 | | | | | | | | | | | | |
| 10587104 | Arpp19 | | | | | | | | | | | | |
| 10587107 | Myo5a | | | | | | | | | | | | |
| 10587508 | Ttk | | | | | | | | | | | | |
| 10587792 | Plscr1 | | | | | | | | | | | | |
| 10588049 | Copb2 | | | | | | | | | | | | |
| 10588294 | Topbp1 | | | | | | | | | | | | |
| 10590325 | Ctnnb1 | | | | | | | | | | | | |
| 10590623 | Cxcr6 | | | | | | | | | | | | |
| 10590648 | Top2a | | | | | | | | | | | | |
| 10591556 | Spc24 | | | | | | | | | | | | |
| 10591781 | Anln | | | | | | | | | | | | |
| 10591816 | Dpy19l1 | | | | | | | | | | | | |
| 10592201 | Chek1 | | | | | | | | | | | | |
| 10592585 | Sc5d | | | | | | | | | | | | |
| 10592727 | Rnf26 | | | | | | | | | | | | |
| 10593356 | Sdhd | | | | | | | | | | | | |
| 10593789 | Etfa | | | | | | | | | | | | |
| 10594251 | Kif23 | | | | | | | | | | | | |
| 10594426 | Zwilch | | | | | | | | | | | | |
| 10594774 | Ccnb2 | | | | | | | | | | | | |
| 10595000 | Tmod3 | | | | | | | | | | | | |
| 10595604 | Syncrip | | | | | | | | | | | | |
| 10595702 | 1190002N15Rik | | | | | | | | | | | | |
| 10590325 | Ctnnb1 | | | | | | | | | | | | |
| 10596185 | Cdv3 | | | | | | | | | | | | |
| 10596575 | Manf | | | | | | | | | | | | |
| 10597095 | 3000002C10Rik | | | | | | | | | | | | |
| 10598638 | Mid1ip1 | | | | | | | | | | | | |
| 10599554 | Rbmx2 | | | | | | | | | | | | |
| 10599855 | Eif4e | | | | | | | | | | | | |
| 10600017 | Hmgb3 | | | | | | | | | | | | |
| 10600031 | 2610030H06Rik | | | | | | | | | | | | |
| 10601011 | Kif4 | | | | | | | | | | | | |
| 10601335 | 2610029G23Rik | | | | | | | | | | | | |
| 10601449 | Sh3bgrl | | | | | | | | | | | | |
| 10601567 | Gm12070 | | | | | | | | | | | | |
| 10601705 | Cenpi | | | | | | | | | | | | |
| 10603252 | Larp4 | | | | | | | | | | | | |
| 10603254 | Larp4 | | | | | | | | | | | | |
| 10603346 | Plp2 | | | | | | | | | | | | |
| 10603431 | Suv39h1 | | | | | | | | | | | | |
| 10596185 | Cdv3 | | | | | | | | | | | | |
| 10604187 | Lamp2 | | | | | | | | | | | | |
| 10604528 | Mbnl3 | | | | | | | | | | | | |
| 10605674 | Pola1 | | | | | | | | | | | | |
| 10605711 | Pdk3 | | | | | | | | | | | | |
| 10606071 | Ercc6l | | | | | | | | | | | | |
| 10606436 | Hmgn5 | | | | | | | | | | | | |
| 10607475 | Prdx4 | | | | | | | | | | | | |
| 10607952 | Vamp7 | | | | | | | | | | | | |
| | | | | | | | | | | | | | |
| | | | | | | | | | | | | | |
| | | | | | | | | | | | | | |
| 10350977 | 4930523C07Rik | | | | | | | | | | | | |
| 10352234 | Itpkb | | | | | | | | | | | | |
| 10352815 | Irf6 | | | | | | | | | | | | |
| 10353064 | Arfgef1 | | | | | | | | | | | | |
| 10353991 | Rpl12 | | | | | | | | | | | | |
| 10357604 | Ikbke | | | | | | | | | | | | |
| 10358389 | Rgs2 | | | | | | | | | | | | |
| 10359201 | Ralgps2 | | | | | | | | | | | | |
| 10359422 | Prdx6 | | | | | | | | | | | | |
| 10359689 | Atp1 b1 | | | | | | | | | | | | |
| 10360684 | Ephx1 | | | | | | | | | | | | |
| 10361323 | Cnksr3 | | | | | | | | | | | | |
| 10362861 | Scml4 | | | | | | | | | | | | |
| 10363641 | Herc4 | | | | | | | | | | | | |
| 10365971 | Btg1 | | | | | | | | | | | | |
| 10366346 | Phlda1 | | | | | | | | | | | | |
| 10366667 | Gns | | | | | | | | | | | | |
| 10368504 | Rpl12 | | | | | | | | | | | | |
| 10369210 | Serinc1 | | | | | | | | | | | | |
| 10369735 | Herc4 | | | | | | | | | | | | |
| 10370072 | Prmt2 | | | | | | | | | | | | |
| 10370544 | 2610008E11 Rik | | | | | | | | | | | | |
| 10371356 | Appl2 | | | | | | | | | | | | |
| 10373519 | Rpl12 | | | | | | | | | | | | |

| **Downregulated** | | | **Downregulated** | | | **Upregulated** | | | **Upregulated** | | | **Upregulated** | |
|---|---|---|---|---|---|---|---|---|---|---|---|---|---|
| **Probe** | **Gene** | | **Probe** | **Gene** | | **Probe** | **Gene** | | **Probe** | **Gene** | | **Probe** | **Gene** |
| 10373740 | Pik3ip1 | | | | | | | | | | | | |
| 10376839 | Ttc19 | | | | | | | | | | | | |
| 10377537 | Chd3 | | | | | | | | | | | | |
| 10377547 | Kdm6b | | | | | | | | | | | | |
| 10377593 | Zbtb4 | | | | | | | | | | | | |
| 10385747 | Phf15 | | | | | | | | | | | | |
| 10386850 | Ncor1 | | | | | | | | | | | | |
| 10387316 | Chd3 | | | | | | | | | | | | |
| 10387372 | Kdm6b | | | | | | | | | | | | |
| 10387699 | Acap1 | | | | | | | | | | | | |
| 10388488 | Fam101b | | | | | | | | | | | | |
| 10389143 | Slfn8 | | | | | | | | | | | | |
| 10389162 | Rpl12 | | | | | | | | | | | | |
| 10391301 | Stat3 | | | | | | | | | | | | |
| 10392063 | Limd2 | | | | | | | | | | | | |
| 10392259 | Smurf2 | | | | | | | | | | | | |
| 10392261 | Smurf2 | | | | | | | | | | | | |
| 10392300 | Bptf | | | | | | | | | | | | |
| 10392318 | Bptf | | | | | | | | | | | | |
| 10396956 | Pcnx | | | | | | | | | | | | |
| 10398267 | Evl | | | | | | | | | | | | |
| 10400405 | Nfkbia | | | | | | | | | | | | |
| 10401238 | Zfp36l1 | | | | | | | | | | | | |
| 10401473 | Aldh6a1 | | | | | | | | | | | | |
| 10402020 | Eml5 | | | | | | | | | | | | |
| 10402061 | Eml5 | | | | | | | | | | | | |
| 10402730 | Ppp1r13b | | | | | | | | | | | | |
| 10403273 | Asb13 | | | | | | | | | | | | |
| 10403765 | Vps41 | | | | | | | | | | | | |
| 10404848 | Jarid2 | | | | | | | | | | | | |
| 10404988 | C030044B11 Rik | | | | | | | | | | | | |
| 10406111 | SIc12a7 | | | | | | | | | | | | |
| 10406817 | Enc1 | | | | | | | | | | | | |
| 10407173 | Il6st | | | | | | | | | | | | |
| 10408032 | Zfp187 | | | | | | | | | | | | |
| 10408049 | Zfp192 | | | | | | | | | | | | |
| 10409170 | Fgd3 | | | | | | | | | | | | |
| 10410465 | BC018507 | | | | | | | | | | | | |
| 10410475 | BC018507 | | | | | | | | | | | | |
| 10411853 | Erbb2ip | | | | | | | | | | | | |
| 10413174 | Rps24 | | | | | | | | | | | | |
| 10414807 | Trav14-3 | | | | | | | | | | | | |
| 10414817 | A130082M07Rik | | | | | | | | | | | | |
| 10416251 | Egr3 | | | | | | | | | | | | |
| 10416522 | Tsc22d1 | | | | | | | | | | | | |
| 10417004 | Dzip1 | | | | | | | | | | | | |
| 10419343 | Atg14 | | | | | | | | | | | | |
| 10421810 | 1190002H23Rik | | | | | | | | | | | | |
| 10422075 | Mycbp2 | | | | | | | | | | | | |
| 10422321 | Dzip1 | | | | | | | | | | | | |
| 10425410 | Grap2 | | | | | | | | | | | | |
| 10427035 | Nr4a1 | | | | | | | | | | | | |
| 10427454 | Card6 | | | | | | | | | | | | |
| 10427459 | Card6 | | | | | | | | | | | | |
| 10427628 | Il7r | | | | | | | | | | | | |
| 10428912 | Fam84b | | | | | | | | | | | | |
| 10428918 | 9930014A18Rik | | | | | | | | | | | | |
| 10430179 | Apol7b | | | | | | | | | | | | |
| 10430201 | Myh9 | | | | | | | | | | | | |
| 10432294 | MII2 | | | | | | | | | | | | |
| 10435769 | Zbtb20 | | | | | | | | | | | | |
| 10435789 | Zbtb20 | | | | | | | | | | | | |
| 10435980 | Rps24 | | | | | | | | | | | | |
| 10437080 | Ttc3 | | | | | | | | | | | | |
| 10438583 | Rpl12 | | | | | | | | | | | | |
| 10440491 | App | | | | | | | | | | | | |
| 10441115 | Brwd1 | | | | | | | | | | | | |
| 10441601 | Tagap | | | | | | | | | | | | |
| 10441787 | Airn | | | | | | | | | | | | |
| 10441791 | Airn | | | | | | | | | | | | |
| 10442495 | Pkd1 | | | | | | | | | | | | |
| 10443852 | A530088E08Rik | | | | | | | | | | | | |
| 10444394 | Pbx2 | | | | | | | | | | | | |
| 10446334 | Glcci1 | | | | | | | | | | | | |
| 10446615 | Rps24 | | | | | | | | | | | | |
| 10449893 | Rasal3 | | | | | | | | | | | | |
| 10451763 | Satb1 | | | | | | | | | | | | |
| 10456745 | Smad7 | | | | | | | | | | | | |
| 10460202 | Suv420h1 | | | | | | | | | | | | |
| 10462035 | Ldhb | | | | | | | | | | | | |
| 10465244 | Malat1 | | | | | | | | | | | | |
| 10468309 | Sh3pxd2a | | | | | | | | | | | | |
| 10469867 | Pnpla7 | | | | | | | | | | | | |
| 10471550 | Rpl12 | | | | | | | | | | | | |
| 10472022 | Lypd6b | | | | | | | | | | | | |
| 10472277 | 7-Mar | | | | | | | | | | | | |
| 10472860 | Rapgef4 | | | | | | | | | | | | |
| 10474006 | Phf21 a | | | | | | | | | | | | |
| 10480238 | St8sia6 | | | | | | | | | | | | |
| 10482880 | Baz2b | | | | | | | | | | | | |
| 10484371 | Calcrl | | | | | | | | | | | | |
| 10489266 | Chd6 | | | | | | | | | | | | |
| 10491136 | Tnik | | | | | | | | | | | | |
| 10491300 | Skil | | | | | | | | | | | | |
| 10494306 | Mcl1 | | | | | | | | | | | | |
| 10496032 | Rpl12 | | | | | | | | | | | | |
| 10496438 | Adh1 | | | | | | | | | | | | |
| 10499160 | Cd1d1 | | | | | | | | | | | | |
| 10499748 | Rps27 | | | | | | | | | | | | |
| 10501879 | Usp53 | | | | | | | | | | | | |
| 10503709 | D130062J21 Rik | | | | | | | | | | | | |
| 10503723 | Mdn1 | | | | | | | | | | | | |
| 10503856 | Gabrr2 | | | | | | | | | | | | |
| 10504491 | Zcchc7 | | | | | | | | | | | | |
| 10504499 | Zcchc7 | | | | | | | | | | | | |
| 10506058 | Inadl | | | | | | | | | | | | |
| 10506335 | Pde4b | | | | | | | | | | | | |
| 10512949 | Abca1 | | | | | | | | | | | | |
| 10513551 | Fkbp15 | | | | | | | | | | | | |
| 10514985 | Zyg11b | | | | | | | | | | | | |
| 10516620 | Lck | | | | | | | | | | | | |
| 10518585 | Kif1b | | | | | | | | | | | | |
| 10518735 | Spsb1 | | | | | | | | | | | | |
| 10519105 | Ski | | | | | | | | | | | | |
| 10520371 | Rbm33 | | | | | | | | | | | | |
| 10520379 | Rbm33 | | | | | | | | | | | | |
| 10520388 | Rbm33 | | | | | | | | | | | | |
| 10524284 | Ttc28 | | | | | | | | | | | | |
| 10524310 | Ttc28 | | | | | | | | | | | | |
| 10524312 | Ttc28 | | | | | | | | | | | | |
| 10524398 | Wscd2 | | | | | | | | | | | | |
| 10527233 | Cyth3 | | | | | | | | | | | | |
| 10529239 | Pisd | | | | | | | | | | | | |
| 10530319 | Atp8a1 | | | | | | | | | | | | |
| 10536390 | Glcci1 | | | | | | | | | | | | |
| 10542557 | Aebp2 | | | | | | | | | | | | |
| 10543118 | Glcci1 | | | | | | | | | | | | |
| 10543319 | Fam3c | | | | | | | | | | | | |
| 10545608 | Sema4f | | | | | | | | | | | | |
| 10546510 | Lrig1 | | | | | | | | | | | | |
| 10546661 | Foxp1 | | | | | | | | | | | | |
| 10547789 | Grcc10 | | | | | | | | | | | | |
| 10548333 | Cd69 | | | | | | | | | | | | |
| 10549097 | Ldhb | | | | | | | | | | | | |
| 10551891 | Nfkbid | | | | | | | | | | | | |
| 10551989 | Tmem149 | | | | | | | | | | | | |
| 10552037 | Sbsn | | | | | | | | | | | | |
| 10552796 | Tsks | | | | | | | | | | | | |
| 10553336 | Zdhhc13 | | | | | | | | | | | | |
| 10555118 | Pak1 | | | | | | | | | | | | |
| 10558001 | Inpp5f | | | | | | | | | | | | |
| 10561920 | Hcst | | | | | | | | | | | | |
| 10562260 | Gramd1a | | | | | | | | | | | | |
| 10564573 | Chd2 | | | | | | | | | | | | |
| 10567702 | Arhgap17 | | | | | | | | | | | | |
| 10568553 | Chst15 | | | | | | | | | | | | |
| 10568780 | Mapk1 ip1 | | | | | | | | | | | | |
| 10569927 | Map2k7 | | | | | | | | | | | | |
| 10571344 | D8Ertd82e | | | | | | | | | | | | |
| 10575598 | Znrf1 | | | | | | | | | | | | |
| 10577226 | 2610019F03Rik | | | | | | | | | | | | |
| 10580452 | Siah1a | | | | | | | | | | | | |
| 10587419 | Senp6 | | | | | | | | | | | | |
| 10589654 | Als2cl | | | | | | | | | | | | |
| 10590365 | Vipr1 | | | | | | | | | | | | |
| 10590381 | Vipr1 | | | | | | | | | | | | |
| 10596492 | Parp3 | | | | | | | | | | | | |
| 10597258 | Tmie | | | | | | | | | | | | |
| 10597266 | Als2cl | | | | | | | | | | | | |
| 10597490 | Rps27 | | | | | | | | | | | | |
| 10597978 | Fyco1 | | | | | | | | | | | | |
| 10601819 | Gprasp1 | | | | | | | | | | | | |
| 10603328 | Ccdc22 | | | | | | | | | | | | |
| 10606261 | Rpl12 | | | | | | | | | | | | |
| 10606654 | Xkrx | | | | | | | | | | | | |
| 10606735 | Armcx2 | | | | | | | | | | | | |
| 10606989 | Tsc22d3 | | | | | | | | | | | | |

### References

Bauquet, A.T., Jin, H., Paterson, A.M., Mitsdoerffer, M., Ho, I.C., Sharpe, A.H., and Kuchroo, V.K. (2009). The costimulatory molecule ICOS regulates the expression of c-Maf and IL-21 in the development of follicular T helper cells and TH-17 cells. Nat Immunol 10, 167-175.
Bettelli, E., Carrier, Y., Gao, W., Korn, T., Strom, T.B., Oukka, M., Weiner, H.L., and Kuchroo, V.K. (2006). Reciprocal developmental pathways for the generation of pathogenic effector TH17 and regulatory T cells. Nature 441, 235-238.
Bettini, M.L., Pan, F., Bettini, M., Finkelstein, D., Rehg, J.E., Floess, S., Bell, B.D., Ziegler, S.F., Huehn, J., Pardoll, D.M., and Vignali, D.A. (2012). Loss of epigenetic modification driven by the Foxp3 transcription factor leads to regulatory T cell insufficiency. Immunity 36, 717-730.
Brunkow, M.E., Jeffery, E.W., Hjerrild, K.A., Paeper, B., Clark, L.B., Yasayko, S.A., Wilkinson, J.E., Galas, D., Ziegler, S.F., and Ramsdell, F. (2001). Disruption of a new forkhead/winged-helix protein, scurfin, results in the fatal lymphoproliferative disorder of the scurfy mouse. Nat Genet 27, 68-73.
Chan, C.W., Kay, L.S., Khadaroo, R.G., Chan, M.W., Lakatoo, S., Young, K.J., Zhang, L., Gorczynski, R.M., Cattral, M., Rotstein, O., and Levy, G.A. (2003). Soluble fibrinogen-like protein 2/fibroleukin exhibits immunosuppressive properties: suppressing T cell proliferation and inhibiting maturation of bone marrow-derived dendritic cells. J Immunol 170, 4036-4044.
Chaudhry, A., Rudra, D., Treuting, P., Samstein, R.M., Liang, Y., Kas, A., and Rudensky, A.Y. (2009). CD4+ regulatory T cells control TH17 responses in a Stat3-dependent manner. Science 326, 986-991.
Chung, Y., Tanaka, S., Chu, F., Nurieva, R.I., Martinez, G.J., Rawal, S., Wang, Y.H., Lim, H., Reynolds, J.M., Zhou, X.H., et al. (2011). Follicular regulatory T cells expressing Foxp3 and Bcl-6 suppress germinal center reactions. Nat Med 17, 983-988.
Cipolletta, D., Feuerer, M., Li, A., Kamei, N., Lee, J., Shoelson, S.E., Benoist, C., and Mathis, D. (2012). PPAR-gamma is a major driver of the accumulation and phenotype of adipose tissue Treg cells. Nature 486, 549-553.
Darce, J., Rudra, D., Li, L., Nishio, J., Cipolletta, D., Rudensky, A.Y., Mathis, D., and Benoist, C. (2012). An N-terminal mutation of the Foxp3 transcription factor alleviates arthritis but exacerbates diabetes. Immunity 36, 731-741.
Fallarino, F., Grohmann, U., Hwang, K.W., Orabona, C., Vacca, C., Bianchi, R., Belladonna, M.L., Fioretti, M.C., Alegre, M.L., and Puccetti, P. (2003). Modulation of tryptophan catabolism by regulatory T cells. Nat Immunol 4, 1206-1212.
Flynn, S., Toellner, K.M., Raykundalia, C., Goodall, M., and Lane, P. (1998). CD4 T cell cytokine differentiation: the B cell activation molecule, OX40 ligand, instructs CD4 T cells to express interleukin 4 and upregulates expression of the chemokine receptor, Blr-1. J Exp Med 188, 297-304.
Fontenot, J.D., Gavin, M.A., and Rudensky, A.Y. (2003). Foxp3 programs the development and function of CD4+CD25+ regulatory T cells. Nat Immunol 4, 330-336.
Fontenot, J.D., Rasmussen, J.P., Williams, L.M., Dooley, J.L., Farr, A.G., and Rudensky, A.Y. (2005). Regulatory T cell lineage specification by the forkhead transcription factor foxp3. Immunity 22, 329-341.
Francisco, L.M., Salinas, V.H., Brown, K.E., Vanguri, V.K., Freeman, G.J., Kuchroo, V.K., and Sharpe, A.H. (2009). PD-L1 regulates the development, maintenance, and function of induced regulatory T cells. J Exp Med 206, 3015-3029.
Haworth, O., Cernadas, M., Yang, R., Serhan, C.N., and Levy, B.D. (2008). Resolvin E1 regulates interleukin 23, interferon-gamma and lipoxin A4 to promote the resolution of allergic airway inflammation. Nat Immunol 9, 873-879.
Heng, T.S., and Painter, M.W. (2008). The Immunological Genome Project: networks of gene expression in immune cells. Nat Immunol 9, 1091-1094.
Hill, J.A., Feuerer, M., Tash, K., Haxhinasto, S., Perez, J., Melamed, R., Mathis, D., and Benoist, C. (2007). Foxp3 transcription-factor-dependent and -independent regulation of the regulatory T cell transcriptional signature. Immunity 27, 786-800.
Izcue, A., Hue, S., Buonocore, S., Arancibia-Carcamo, C.V., Ahern, P.P., Iwakura, Y., Maloy, K.J., and Powrie, F. (2008). Interleukin-23 restrains regulatory T cell activity to drive T cell-dependent colitis. Immunity 28, 559-570.
Joller, N., Hafler, J.P., Brynedal, B., Kassam, N., Spoerl, S., Levin, S.D., Sharpe, A.H., and Kuchroo, V.K. (2011). Cutting edge: TIGIT has T cell-intrinsic inhibitory functions. J Immunol 186, 1338-1342.
Klein, L., Khazaie, K., and von Boehmer, H. (2003). In vivo dynamics of antigen-specific regulatory T cells not predicted from behavior in vitro. Proc Natl Acad Sci U S A 100, 8886-8891.
Koch, M.A., Thomas, K.R., Perdue, N.R., Smigiel, K.S., Srivastava, S., and Campbell, D.J. (2012). T-bet(+) Treg cells undergo abortive Th1 cell differentiation due to impaired expression of IL-12 receptor beta2. Immunity 37, 501-510.
Koch, M.A., Tucker-Heard, G., Perdue, N.R., Killebrew, J.R., Urdahl, K.B., and Campbell, D.J. (2009). The transcription factor T-bet controls regulatory T cell homeostasis and function during type 1 inflammation. Nat Immunol 10, 595-602.
Kuhn, R., Lohler, J., Rennick, D., Rajewsky, K., and Muller, W. (1993). Interleukin-10-deficient mice develop chronic enterocolitis. Cell 75, 263-274.
Levin, S.D., Taft, D.W., Brandt, C.S., Bucher, C., Howard, E.D., Chadwick, E.M., Johnston, J., Hammond, A., Bontadelli, K., Ardourel, D., et al. (2011). Vstm3 is a member of the CD28 family and an important modulator of T-cell function. Eur J Immunol 41, 902-915.
Linterman, M.A., Pierson, W., Lee, S.K., Kallies, A., Kawamoto, S., Rayner, T.F., Srivastava, M., Divekar, D.P., Beaton, L., Hogan, J.J., et al. (2011). Foxp3(+) follicular regulatory T cells control the germinal center response. Nat Med 17, 975-982.
Lozano, E., Dominguez-Villar, M., Kuchroo, V., and Hafler, D.A. (2012). The TIGIT/CD226 Axis Regulates Human T Cell Function. J Immunol.
Rogerio, A.P., Haworth, O., Croze, R., Oh, S.F., Uddin, M., Carlo, T., Pfeffer, M.A., Priluck, R., Serhan, C.N., and Levy, B.D. (2012). Resolvin D1 and aspirin-triggered resolvin D1 promote resolution of allergic airways responses. J Immunol 189, 1983-1991.
Shalev, I., Liu, H., Koscik, C., Bartczak, A., Javadi, M., Wong, K.M., Maknojia, A., He, W., Liu, M.F., Diao, J., et al. (2008). Targeted deletion of fgl2 leads to impaired regulatory T cell activity and development of autoimmune glomerulonephritis. J Immunol 180, 249-260.
Thornton, A.M., and Shevach, E.M. (1998). CD4+CD25+ immunoregulatory T cells suppress polyclonal T cell activation in vitro by inhibiting interleukin 2 production. J Exp Med 188, 287-296.
Wildin, R.S., Ramsdell, F., Peake, J., Faravelli, F., Casanova, J.L., Buist, N., Levy-Lahad, E., Mazzella, M., Goulet, O., Perroni, L., et al. (2001). X-linked neonatal diabetes mellitus, enteropathy and endocrinopathy syndrome is the human equivalent of mouse scurfy. Nat Genet 27, 18-20.
Wing, K, Onishi, Y., Prieto-Martin, P., Yamaguchi, T., Miyara, M., Fehervari, Z., Nomura, T., and Sakaguchi, S. (2008). CTLA-4 control over Foxp3+ regulatory T cell function. Science 322, 271-275.
Xiao, X., Balasubramanian, S., Liu, W., Chu, X., Wang, H., Taparowsky, E.J., Fu, Y.X., Choi, Y., Walsh, M.C., and Li, X.C. (2012). OX40 signaling favors the induction of T(H)9 cells and airway inflammation. Nat Immunol 13, 981-990.
Yu, X., Harden, K., Gonzalez, L.C., Francesco, M., Chiang, E., Irving, B., Tom, I., Ivelja, S., Refino, C.J., Clark, H., et al. (2009). The surface protein TIGIT suppresses T cell activation by promoting the generation of mature immunoregulatory dendritic cells. Nat Immunol 10, 48-57.
Zheng, Y., Chaudhry, A., Kas, A., deRoos, P., Kim, J.M., Chu, T.T., Corcoran, L., Treuting, P., Klein, U., and Rudensky, A.Y. (2009). Regulatory T-cell suppressor program co-opts transcription factor IRF4 to control T(H)2 responses. Nature 458, 351-356.

## Claims

1. An agent that inhibits TIGIT activity for use in treating a patient diagnosed with cancer and/or infection, which patient has an elevated level of Fgl2, wherein the agent that inhibits TIGIT activity comprises an antibody or an antigen-binding fragment thereof that specifically binds TIGIT.

2. The agent for the use of claim 1 wherein the agent is intended to be administered with a therapy comprising an activator of a proinflammatory T cell response pathway and/or a suppressor of an anti-inflammatory T cell response pathway.

3. The agent for the use of claim 2 where the agent that inhibits TIGIT activity and the activator of a proinflammatory T cell response pathway and/or suppressor of an anti-inflammatory T cell response pathway are intended to be administered separately, sequentially or concurrently.

4. An *in vitro* method of determining the efficacy of an agent that inhibits TIGIT activity in the treatment of a patient diagnosed with cancer and/or infection, which patient has an elevated level of Fgl2, the method comprising:
a) determining a first level of Fgl2 expression or activity in a sample provided by the patient diagnosed with cancer and/or infection that has an elevated level of Fgl2 prior to the administration of the agent that inhibits TIGIT activity;
b) determining a second level of Fgl2 expression or activity in a sample provided by the patient after administration of the agent that inhibits TIGIT activity ; and
c) comparing said first and second levels of Fgl2 expression or activity, wherein the agent is considered effective if said second level of Fgl2 expression or activity is lower than said first level, and wherein the agent administered in (b) is ineffective if said second level of Fgl2 expression is the same as or higher than said first level; and
d) if said anti-TIGIT therapy is considered ineffective, administration of said agent that inhibits TIGIT activity at a higher dose or administration of a therapy comprising an activator of a proinflammatory T cell response pathway and/or a suppressor of an anti-inflammatory T cell response pathway is advised,
wherein the agent that inhibits TIGIT activity comprises an antibody or an antigen-binding fragment thereof that specifically binds TIGIT.

5. The agent for the use of claim 1, 2, or 3 or the method of claim 4, wherein the activator of the proinflammatory T cell response pathway comprises a TIM-3 inhibitor, an anti-galectin-9 molecule, a PD-1 antagonist, a PD-L1 antagonist, a CTLA-4 antagonist, a Lag-3 antagonist, an agonist of an immune checkpoint activating molecule, an antagonist of an immune checkpoint inhibitory molecule, or any combination thereof.

6. The method of claim 4, wherein the activator of the proinflammatory T cell response pathway comprises an antibody or an antigen-binding fragment thereof.

7. A composition for use in treating a patient diagnosed with cancer that has an elevated level of Fgl2, wherein the composition comprises a TIGIT inhibitor comprising an anti-TIGIT antibody or an antigen-binding fragment thereof and a therapeutic agent selected from the group consisting of a TIM-3 inhibitor, an anti-galectin-9 molecule, a PD-1 antagonist, a PD-L1 antagonist, a CTLA-4 antagonist, a Lag-3 antagonist, a CD155 inhibitor or any combination thereof.

8. The composition for the use of claim 7, wherein the therapeutic agent comprises an antibody or an antigen-binding fragment thereof that specifically binds to TIM-3, galectin-9, PD-1, PD-L1, CTLA-4, Lag-3 or CD155.

9. A composition for use in treating a patient diagnosed with cancer that has an elevated level of Fgl2 and who has received immunotherapy for the cancer, the composition comprising a TIGIT antagonist, wherein the TIGIT antagonist comprises an antibody or an antigen-binding fragment thereof that specifically binds TIGIT.

## Patentansprüche

1. Mittel, das die TIGIT-Aktivität hemmt, zur Verwendung bei der Behandlung eines Patienten, bei dem Krebs und/oder Infektion diagnostiziert worden ist, wobei der Patient einen erhöhten Fgl2-Spiegel aufweist, wobei das Mittel, das die TIGIT-Aktivität hemmt, einen Antikörper oder ein Antigen-bindendes Fragment davon umfasst, der/das spezifisch TIGIT bindet.

2. Mittel zur Verwendung nach Anspruch 1, wobei das Mittel mit einer Therapie verabreicht werden soll, die einen Aktivator eines proinflammatorischen T-Zell-Reaktionswegs und/oder einen Suppressor eines antiinflammatorischen T-Zell-Reaktionswegs umfasst.

3. Mittel zur Verwendung nach Anspruch 2, wobei das Mittel, das TIGIT-Aktivität hemmt, und der Aktivator eines proinflammatorischen T-Zell-Reaktionswegs und/oder der Suppressor eines antiinflammatorischen T-Zell-Reaktionswegs vorgesehen sind, separat, aufeinanderfolgend oder gleichzeitig verabreicht zu werden.

4. In-vitro-Verfahren zum Bestimmen der Wirksamkeit eines Mittels, das die TIGIT-Aktivität hemmt, bei der Behandlung eines Patienten, bei dem Krebs und/oder Infektion diagnostiziert worden ist, wobei der Patient einen erhöhten Fgl2-Spiegel aufweist, wobei das Verfahren Folgendes umfasst:
a) Bestimmen eines ersten Pegels der Fgl2-Expression oder -Aktivität in einer Probe, die von dem Patienten, bei dem Krebs und/oder Infektion diagnostiziert worden ist, der einen erhöhten Fgl2-Spiegel aufweist, vor dem Verabreichen des Wirkstoffs, der die TIGIT-Aktivität hemmt, bereitgestellt wird;
b) Bestimmen eines zweiten Pegels der Fgl2-Expression oder -Aktivität in einer Probe, die von dem Patienten nach dem Verabreichen des Wirkstoffs, der die TIGIT-Aktivität hemmt, bereitgestellt wird; und
c) Vergleichen des ersten und des zweiten Pegels der Fgl2-Expression oder -Aktivität, wobei das Mittel als wirksam angesehen wird, wenn der zweite Pegel der Fgl2-Expression oder -Aktivität niedriger als der erste Pegel ist, und wobei das in (b) verabreichte Mittel unwirksam ist, wenn der zweite Pegel der Fgl2-Expression ebenso hoch wie oder höher als der erste Pegel ist; und
d) falls die anti-TIGIT-Therapie als unwirksam angesehen wird, das Verabreichen des Mittels, das TIGIT-Aktivität hemmt, mit einer höheren Dosis oder das Verabreichen einer Therapie, die einen Aktivator eines proinflammatorischen T-Zell-Reaktionswegs und/oder einen Suppressor eines antiinflammatorischen T-Zell-Reaktionswegs umfasst, angeraten wird,
wobei das Mittel, das die TIGIT-Aktivität hemmt, einen Antikörper oder ein Antigen-bindendes Fragment davon umfasst, der/das spezifisch TIGIT bindet.

5. Mittel zur Verwendung nach Anspruch 1, 2 oder 3 oder Verfahren nach Anspruch 4, wobei der Aktivator des proinflammatorischen T-Zell-Reaktionswegs einen TIM-3-Inhibitor, ein anti-Galectin-9 Molekül, einen PD-1-Antagonisten, einen PD-L1-Antagonisten, einen CTLA-4-Antagonisten, einen Lag-3-Antagonisten, einen Agonisten eines Immun-Checkpoint-aktivierenden Moleküls, einen Antagonisten eines Immun-Checkpoint-inhibitorischen Moleküls oder eine beliebige Kombination daraus umfasst.

6. Verfahren nach Anspruch 4, wobei der Aktivator des proinflammatorischen T-Zell-Reaktionswegs einen Antikörper oder ein Antigen-bindendes Fragment davon umfasst.

7. Zusammensetzung zur Verwendung bei einem Patienten, bei dem Krebs diagnostiziert worden ist, der einen erhöhten Fgl2-Spiegel aufweist, wobei die Zusammensetzung einen TIGIT-Inhibitor umfasst, umfassend einen anti-TIGIT-Antikörper oder ein Antigen-bindendes Fragment davon und einen Wirkstoff, ausgewählt aus der Gruppe bestehend aus einem TIM-3-Inhibitor, einem Anti-Galectin-9-Molekül, einem PD-1-Antagonisten, einem PD-L1-Antagonisten, einem CTLA-4-Antagonisten, einem Lag-3-Antagonisten, einem CD155-Inhibitor oder einer beliebigen Kombination daraus.

8. Zusammensetzung zur Verwendung nach Anspruch 7, wobei der Wirkstoff einen Antikörper oder ein Antigen-bindendes Fragment davon umfasst, der/das spezifisch an TIM-3, Galectin-9, PD-1, PD-L1, CTLA-4, Lag-3 oder CD155 bindet.

9. Zusammensetzung zur Verwendung beim Behandeln eines Patienten, bei dem Krebs diagnostiziert worden ist, der einen erhöhten Fgl2-Spiegel aufweist, und der für den Krebs Immuntherapie erhalten hat, wobei die Zusammensetzung einen TIGIT-Antagonisten umfasst, wobei der TIGIT-Antagonist einen Antikörper oder ein Antigen-bindendes Fragment davon umfasst, der/das spezifisch TIGIT bindet.

## Revendications

1. Agent qui inhibe l'activité de TIGIT pour une utilisation dans le traitement d'un patient diagnostiqué avec un cancer et/ou une infection, ce patient présentant un niveau élevé de Fgl2, l'agent qui inhibe l'activité de TIGIT comprenant un anticorps ou un fragment de liaison à l'antigène de celui-ci, qui se lie spécifiquement à TIGIT.

2. Agent pour l'utilisation selon la revendication 1, l'agent étant destiné à être administré avec un traitement comprenant un activateur d'une voie de réponse des lymphocytes T pro-inflammatoires et/ou un suppresseur d'une voie de réponse des lymphocytes T anti-inflammatoires.

3. Agent pour l'utilisation selon la revendication 2, l'agent qui inhibe l'activité de TIGIT et l'activateur d'une voie de réponse des cellules T pro-inflammatoires et/ou le suppresseur d'une voie de réponse des lymphocytes T anti-inflammatoires sont destinés à être administrés séparément, successivement ou simultanément.

4. Procédé *in vitro* de détermination de l'efficacité d'un agent qui inhibe l'activité de TIGIT dans le traitement d'un patient diagnostiqué avec un cancer et/ou une infection, ce patient présentant un niveau élevé de Fgl2, le procédé comprenant :
a) la détermination d'un premier niveau d'expression ou d'activité de Fgl2 dans un échantillon fourni par le patient diagnostiqué avec un cancer et/ou une infection, qui présente un niveau élevé de Fgl2 avant l'administration de l'agent qui inhibe l'activité de TIGIT ;
b) la détermination d'un second niveau d'expression ou d'activité de Fgl2 dans un échantillon fourni par le patient après administration de l'agent qui inhibe l'activité de TIGIT ; et
c) la comparaison dudit premier et dudit second niveaux d'expression ou d'activité de Fgl2, l'agent étant considéré comme efficace si ledit second niveau d'expression ou d'activité de Fgl2 est inférieur audit premier niveau, et l'agent administré en (b) étant inefficace si ledit second niveau d'expression de Fgl2 est supérieur ou égal audit premier niveau ; et
d) si ledit traitement anti-TIGIT est considéré comme inefficace, l'administration dudit agent qui inhibe l'activité de TIGIT à une dose plus élevée, ou l'administration d'un traitement comprenant un activateur d'une voie de réponse des lymphocytes T pro-inflammatoires et/ou un suppresseur d'une voie de réponse des lymphocytes T anti-inflammatoires étant recommandée,
l'agent qui inhibe l'activité de TIGIT comprenant un anticorps ou un fragment de liaison à l'antigène qui se lie spécifiquement à TIGIT.

5. Agent pour l'utilisation selon la revendication 1, 2 ou 3, ou procédé selon la revendication 4, dans lequel l'activateur de la voie de réponse des lymphocytes T pro-inflammatoires est un inhibiteur de TIM-3, une molécule d'anti-galactine-9, un antagoniste de PD-1, un antagoniste de PD-L1, un antagoniste de CTLA-4, un antagoniste de Lag-3, un agoniste d'une molécule d'activation du point de contrôle immun, un antagoniste d'une molécule inhibitrice du point de contrôle immun, ou toute combinaison de ceux-ci.

6. Procédé selon la revendication 4, dans lequel l'activateur de la voie de réponse des lymphocytes T pro-inflammatoires comprend un anticorps ou un fragment de liaison à l'antigène de celui-ci.

7. Composition pour une utilisation dans le traitement d'un patient diagnostiqué avec un cancer, qui présente un niveau élevé de Fgl2, la composition comprenant un inhibiteur de TIGIT comprenant un anticorps anti-TIGIT ou un fragment de liaison à l'antigène de celui-ci, et un agent thérapeutique choisi dans le groupe consistant en un inhibiteur de TIM-3, une molécule d'anti-galectine-9, un antagoniste de PD-1, un antagoniste de PD-L1, un antagoniste de CTLA-4, un antagoniste de Lag-3, un inhibiteur de CD155 ou toute combinaison de ceux-ci.

8. Composition pour l'utilisation selon la revendication 7, dans laquelle l'agent thérapeutique comprend un anticorps ou un fragment de liaison à l'antigène de celui-ci, qui se lie spécifiquement à TIM-3, galectine-9, PD-1, PD-L1, CTLA-4, Lag-3 ou CD155.

9. Composition pour une utilisation dans le traitement d'un patient diagnostiqué avec un cancer, qui présente un niveau élevé de Fgl2 et qui a reçu une immunothérapie pour le cancer, la composition comprenant un antagoniste de TIGIT, l'antagoniste de TIGIT comprenant un anticorps ou un fragment de liaison à l'antigène de celui-ci, qui se lie spécifiquement à TIGIT.
